# EUROPEAN PATENT APPLICATION

(11) **EP 4 029 510 A1**
(43) Date of publication of application: **20.07.2022**
(21) Application number: 21215256.5
(22) Date of filing: 15.04.2011
(51) Int. Cl.: A61K 31/74, C08F 230/02, A61K 47/58, A61K 47/68

(54) **HIGH MOLECULAR WEIGHT ZWITTERION-CONTAINING POLYMERS**

(30) Priority: 15.04.2010 US 32441310 P
(62) Divisional of application: 19175761.6
(71) Applicant: Kodiak Sciences Inc., Palo Alto, CA 94304 (US)
(72) Inventor: CHARLES, Stephen A, San Jose, CA 95138 (US); PERLROTH, Victor D, Palo Alto, CA 94306 (US); BENOIT, Didier G, San Jose, CA 95123 (US); CLIZBE, Lane A, Redwood City, CA 94061 (US); TO, Wayne, Fremont, CA 94555 (US); ZADIK, Linda J, Palo Alto, CA 94303 (US); PRATT, Jeanne M, Palo Alto, CA 94304 (US)
(74) Representative: J A Kemp LLP

(57) **Abstract**

The present invention provides multi-armed high MW polymers containing hydrophilic groups and one or more functional agents, and methods of preparing such polymers.

## Description

### CROSS-REFERENCES TO RELATED APPLICATIONS

This application claims priority to U.S. Provisional Application No. 61/324,413, filed April 15, 2010, which is incorporated in its entirety herein for all purposes.

### REFERENCE TO A "SEQUENCE LISTING," A TABLE, OR A COMPUTER PROGRAM LISTING APPENDIX SUBMITTED ON A COMPACT DISK

### NOT APPLICABLE

### BACKGROUND OF THE INVENTION

An arms race of sorts is happening right now amongst the big pharma companies who are all trying to deliver 'medically differentiated products'. Biopharmaceuticals are seen as a key vehicle. The belief is that differentiation will come not necessarily through target novelty but through novel drug formats. These formats will be flexible such that resulting drugs can be *biology* centric rather than *format* centric. This next wave of biopharmaceuticals will be modular, multifunctional, and targeted. These drugs will be designed with a view towards understanding the broader disease biology being targeted and applying that knowledge in a multifaceted drug. Antibodies are fantastic drugs, but despite a significant amount of antibody protein engineering they are and will continue to be a rigid and inflexible format.

The pharma protein engineers are looking to smaller protein formats. There was a wave of progress in the 2006 timeframe with the likes of adnectins (developed by Adnexus and acquired by BMS), avimers (developed by Avidia and acquired by Amgen), diabodies (developed by Domantis and acquired by GSK), Haptogen (acquired by Wyeth), BiTES (developed by Micromet), camelids (developed by Ablynx), peptides (developed by the likes of Gryphon Therapeutics and Compugen and many others). But the conversion of these platform technologies into multiple products in the pharma pipeline has been slow to materialize. Over the past two decades, the problems besetting these non-whole antibody formats related to suboptimal affinity, poor stability, low manufacturing yield, as well as tools development. To a large degree, these problems have been or are being solved. But the Achilles heel of these formats remains their inadequate *in vivo* residence time, an issue which is holding back a wave of important product opportunities.

Whole antibodies have an elimination half life *in vivo* upwards of 250 hours, corresponding to more than one month of physical residency in the body. This makes them an excellent product format from a dosing point of view. Often they can achieve monthly or less frequent injection. The trajectory is also towards subcutaneous injection in smaller volumes (1mL, 0.8mL, 0.4mL), more stable liquid formulations (versus lyophilized formulations requiring physician reconstitution), storage at higher concentrations (50mg/mL, 100mg/mL, 200mg/mL) and at higher temperatures (-80 degrees, -20 degrees, 2 - 8 degrees, room temperature).

Antibodies are a tough act to follow, especially with all of the activity in the broad antibody discovery and development ecosystem. But antibodies do leave much to be desired. They are ungainly, inflexible, large, single-target limited, manufactured in mammalian systems, overall poorly characterized and are central to many different *in vivo* biologies of which target binding, epithelial FcRn receptor recycling, antibody-dependent cell-mediated cytotoxicity (ADCC), complement dependent cytoxicity (CDC), avidity, higher order architectures, to name just a few.

The smaller, modular formats can make a major contribution towards the development of safer, targeted, multifunctional, higher efficacy, well-characterized and cheaper therapeutics. In addition, there is a similar need to improve the serum residence time and associated physical properties of other types of drug agents such as recombinant proteins and peptides (either native or mutein) and oligonucleotides. The challenge is to devise a technical solution that dramatically increases *in vivo* residence time for these soluble biopharmaceuticals (the performance issue), does so without forcing compromises in other key parameters such as drug solubility, stability, viscosity, characterizability (the related physical properties issues), and employs an approach that allows predictability across target classes and across the drug development path from early animal studies through to manufacturing scale-up and late-stage human clinical trials (the portfolio planning issue).

The first attempted class of solutions is biology-based and depends on fusing the protein agents to transferrin, albumin, immunoglobulin gamma (IgG), IgG constant region (IgG-Fc) and/or other serum proteins. But fusing a biology-based serum extension moiety to a functional biologic moiety increases the number and complexity of concurrent biological interactions. These non-target-mediated interactions rarely promote the desired therapeutic action of the drug, but rather more often detract from the desired therapeutic action of the drug in complex and poorly understood ways. The net impact is to undermine predictability, performance, and safety.

The second attempted class of solutions is based broadly on a set of approaches that make use of polymers of different types which are attached to the drug. These polymers function largely on the basis of their ability to bind and structure water. The bound water decreases clearance by the myriad *in vivo* clearance mechanisms, both passive and active, while also improving physical properties of the polymer-drug conjugate such as solubility, stability, viscosity. This second class of solutions is subcategorized further in two ways: (1) by the water binding entity within the polymer, and (2) how the polymer is attached to the drug agent. Relating to (1), there are a number of different polymeric water binding moieties in use, such as sugars (carbohydrates), amino acids (hydrophilic protein domains), polyethylene oxide, polyoxazoline, polyvinyl alcohol, polyvinyl pyrrolidone, etc. Relating to (2), the distinction is largely whether the polymer is added to the drug agent by the cellular machinery or whether it is added in a semi-synthetic conjugation step.

Relating to polymers added to the drug agent by cellular machinery *(i.e.* not through a semi-synthetic step), one example is the addition of hydrophilic carbohydrate polymers to the surface of a translated protein through a cell-mediated glycosylation process by adding or modifying a glycosylation site at the level of the coding nucleotide sequence (e.g. Aranesp). Another example is the addition of a string of hydrophilic amino acids during protein translation by adding a series of repeating nucleotide units at the level of the open reading frame codons (i.e. Amunix's XTEN platform).

Relating to the semi-synthetics: The most experience exists with PEGylation in which polymers of polyethylene oxide are functionalized and then conjugated to the drug agent. Also, Fresenius employs a HESylation approach in which long-chain maize starches are functionalized and then conjugated to the drug agent. Also, Serina Therapeutics' employs a hydrophilic polyoxazoline backbone (as opposed to the polyethylene backbone of PEG). Another method termed polyPEG as described by Haddleton et al employs a polymer backbone capable of radical polymerization and a water binding entity that is either a short string of PEG or a sugar.

How well do these different technology approaches work in practice? In general, despite significant time and money spent by biopharma and pharma, the general conclusion is that these technologies are not delivering the level of performance benefit needed (especially *in vivo* residence time) and furthermore are at the flat of the curve in terms of their ability to deliver further progress through additional engineering. The level of improvement required depends on the drug and its biology and the required product profile , but in many cases is as high as three to fourfold. Many companies are working to achieve this level of improvement but in practice the technologies employed are falling short and delivering incremental improvements that are overall niche in their applicability.

For example:
PEGylation of an antibody fragment scFv (approximately 22 kDa in size) inhibitor of GM-CSF (Micromet data) with a 40 kDa branched PEG resulted in a murine elimination half life after intravenous injection of 59 hours which is inadequate. To be useful, the murine half-life should be over 150 hours (a 3x improvement) and preferably over 250 hours (a 4x improvement).

PEGylation of a recombinant interferon alfa of approximately 19.5 kDa with a 40 kDa branched PEG (Pegasys data) results in a murine elimination half life after subcutaneous injection of approximately 50 hours and a human half life in the range of 80 hours. Pegasys is dosed weekly in humans.

PEGylation of a Fab' antibody fragment of approximately 50 kDa against IL-8 (Genentech data, Leong et al, 2001) with a series of PEG polymers of increasing size and architecture. Half lives in rabbits after intravenous injection ranged from 44 hours with a PEG 20 kDa linear to 105 hours with a PEG 40 kDa branched. This can be correlated against the half-life of the approved product Cimzia which has a Fab' against TNFa conjugated with a 40 kDa branched polymer. Human half life after subcutaneous injection is 311 hours and is sufficient (as approved by the FDA for rheumatoid arthritis) for monthly subcutaneous dosing. But the properties driven by the PEG moiety (solubility, stability, viscosity) are not sufficient to enable the full dose amount (400mg) to be formulated in a single vial for subcutaneous injection (limit 1mL, preferably 0.8mL or less). Rather, Cimzia is formulated preferably as a solid and in two vials for two separate injections each delivering 200mg of product. Furthermore, the PEG reagent is very expensive and constitutes up to twenty percent of the average wholesale price of the drug. Therefore, the Cimzia product is not very competitive in the marketplace versus Humira (anti-TNFa antibody, in a liquid formulation, in a single use syringe, administered by single subcutaneous injection, twice monthly) and even less so versus Simponi (anti-TNFa antibody, in a liquid formulation, in a single use syringe, administered by single subcutaneous injection, once monthly).

PEGylation of a peptide mimetic (approximately 4kDa) of erythropoietin receptor (Hematide data) with a 40 kDa branched PEG polymer after subcutaneous injection showed between 23 and 31 hour half-life in rats (dose dependent). In monkeys the half-life ranged between 15 hours and 60 hours (Fan et al Experimental Hematology, 34, 2006). The projected dose frequency for the molecule is monthly. In this case, the ability to dose monthly with this molecule is enabled by a pharmacodynamic effect whose duration far exceeds the physical half-life and residence time of the drug itself. This property holds for certain potent agonistic drugs but generally does not hold for inhibitors that need to maintain a minimal inhibitory concentration nor does it hold for enzymes nor for high dose agonistic proteins.

Interferon beta (approximately 20 kDa) was PEGylated with a 40 kDa linear PEG polymer. Avonex, an unPEGylated form, demonstrates a mean terminal half life in monkeys after intravenous injection of 5.5 hours and a half-life of 10 hours after intramuscular injection. Conjugation of a 40 kDa linear PEG polymer can demonstrate a half life of approximately fifteen hours after intravenous administration and thirty hours after subcutaneous administration. Conjugation of a 40 kDa branched PEG polymer can demonstrate a half life of thirty hours after intravenous administration and sixty hours after subcutaneous administration. The projected dose frequency is twice monthly, so the ability to dose twice monthly with this molecule is enabled by a biological or pharmacodynamic effect whose duration exceeds the physical half-life and residence time of the drug itself. For an attractive target product profile to challenge the existing interferon beta products, a once a month dose frequency is required. Alternatively, a polymer conjugate that was dosed twice monthly but with very flat, potentially zero order, kinetics could be ideal. This is obtainable with a highly biocompatible conjugate and dosed at a lower overall dose. Furthermore, interferon beta is an unstable and overall 'difficult' protein to work with and further improvement in solubility and stability is desired.

PEGylation of recombinant human Factor VIII (upwards of 300 kDa) with a 60 kDa branched PEG polymer has been performed. UnPEGylated FVIII demonstrates a twelve to fourteen hour circulating half-life in humans. It is used acutely in response to a bleeding crisis. It is also being used for prophylaxis via three times weekly intravenous infusions. The murine mean terminal half-life is six hours in the unPEGylated form and eleven hours with a site-directed PEGylated form. In rabbits, with a full-length FVIII protein, an unPEGylated form showed a mean terminal half life of 6.7 hours. With a form PEGylated with a 60kDa branched PEG, the half life increased to twelve hours. The magnitude of increase in half-life of PEG-FVIII correlates to the increase in PEG mass. A key goal, however, is to enable prophylaxis with a once weekly intravenous infusion. The benefit delivered even by the very large (and expensive 60kDa PEG reagent) is not thought to, nor is it likely to, enable the once weekly dose frequency. It needs an additional >2x preferably a 4x versus PEG to be a game changer. Another in vivo performance metric to improve would be to substantially decrease the incidence of neutralizing antibodies generated against the administered FVIII drug. This goal is inadequately met via FVIII-PEG conjugates. Another *in vitro* performance metric to improve would be to achieve a stable, high concentration formulation sufficient to enable subcutaneous dosing rather than intravenous dosing - this would also require improvement of the *in vivo* immunogenicity properties as the subcutaneous areas are high in immune-stimulating antigen presenting cells. Recently, a Biogen-generated fusion of FVIII to immunoglobulin Fc fragment was tested and demonstrated to have similar level of *in vivo* half-life as the PEGylated FVIII but interestingly very poor bioavailability presumably due to FcRn-mediated endothelial cell clearance of the drug. These data have led FVIII drug developers to conclude the existing technologies have "hit a wall".

The Amunix XTEN technology fuses approximately 850 hydrophilic amino acids (approximately 80kDa in size) to the GLP-1 peptide. This boosts the half-life to sixty hours in a cynomolgus monkey which is slightly inferior to a GLP-1 equivalent conjugated to a 40kDa branched PEG polymer. So a polymer of 2x increased size delivers essentially the same performance benefit. A similar level of benefit was seen with XTEN attached to human growth hormone. In terms of trying to extend further the level of half life benefit, there are a number of challenges. First and foremost, the hydrophilic amino acids used to bind and structure the water are non-optimal in terms of their water binding characteristics. Second, the requisite use of the ribosomal translation machinery to add the polymer limits the architecture to single arm, linear structures which have been shown in many PEGylation examples to be inferior to branched architectures when holding molecular weight constant and increasing the level of branching. Third, a peptide bond used as a polymer backbone is sufficiently unstable such that it will demonstrate a polydispersity, which heterogeneity becomes limiting in practical terms such that the length of the hydrophilic polymer cannot be easily increased to achieve half lives superior to the 40kDa branched PEG (this on top of other complexity related to the use of multiple long repeating units in the encoding plasmid vector which itself becomes limiting). This technology then becomes niche in its application, for example, to allow a peptide formerly made synthetically via chemical synthesis to be made in a cell-based system which has some perceived advantages (as well as new disadvantages) but overall with similar *in vivo* performance as possible with other technologies, especially *in vivo* elimination half life.

rhEPO is a 30.4 kDa protein with 165 amino acids and 3 N-linked plus 1 O-linked glycosylation site. 40% of the mass is carbohydrate. The carbohydrates are not necessary for activity *in vitro,* but absolutely necessary for activity *in vivo.* Aranesp is a form of human erythropoietin modified at the genetic level to contain 5 N-linked oligosaccharide chains versus the native form which contains 3 chains. The additional carbohydrates increase the approximate molecular weight of the glycoprotein from 30kDa to 37kDa. In humans, the change increases mean terminal half life after intravenous injection from 7 hours to 21 hours and after subcutaneous injection from 16 hours to 46 hours, which is an approximate threefold improvement in both cases. Mircera which is a PEGylated form of recombinant human erythropietin demonstrated *in vivo* half life after subcutaneous injection of approximately 140 hours but in chronic renal disease patients, where patients because of renal filtration of the drug show a more than 2x increase in half life as well as a decreased receptor affinity which decreases mechanistic clearance, meaning the actual physical half life is less than 70 hours and in line with Affymax's Hematide peptidomimetic (PEGylated with a 40kDa branched PEG).

The HESylation technology employs a semi-synthetic conjugation of a maize derived starch polymer to a drug. Data shows that a 100kDa HESylation polymer is equivalent to a 30kDa linear PEG polymer on erythropoietin in mice (Mircera product equivalent). It is possible to use a bigger polymer, but the approach is fundamentally limited by the nature of the starch water binding. Also, equivalence of a 100kDa polymer to a 30kDa linear PEG (which is itself inferior to a 40kDa branched PEG) shows that there is a long way to go in terms of performance before this can equal a 40kDa branched PEG much less provide a requisite 4x benefit.

These examples are illustrative of several of the approaches being tried and the overall performance they achieve. In short, these approaches and technologies fall short. For non-antibody scaffolds, they converge and hit the wall at elimination half lives of around 60 to 80 hours in monkey. Although the line varies, it is generally desired to achieve at least 100 hour mean terminal half life in monkeys in order to enable once weekly dosing in humans. And when dose frequency is longer than the half life, this places additional demands on the formulation's solubility, stability, and viscosity. For other types of proteins, such as Factor VIII, the absolute value of the starting half life and thus the requisite target value is lower, but the performance multiple required to get to an attractive target product profile is similar and on the order of 3x to 4x. The question, then, is how to get here?

First, some more background. Efforts to formulate biologically active agents for delivery must deal with a variety of variables including the route of administration, the biological stability of the active agent and the solubility of the active agents in physiologically compatible media. Choices made in formulating biologically active agents and the selected routes of administration can affect the bioavailability of the active agents. For example, the choice of parenteral administration into the systemic circulation for biologically active proteins and polypeptides avoids the proteolytic environment found in the gastrointestinal tract. However, even where direct administration, such as by injection, of biologically active agents is possible, formulations may be unsatisfactory for a variety of reasons including the generation of an immune response to the administered agent and responses to any excipients including burning and stinging. Even if the active agent is not immunogenic and satisfactory excipients can be employed, biologically active agents can have a limited solubility and short biological half life that can require repeated administration or continuous infusion, which can be painful and/or inconvenient.

For some biologically active agents, a degree of success has been achieved in developing suitable formulations of functional agents by conjugating the agents to water soluble polymers. The conjugation of biologically active agents to water soluble polymers is generally viewed as providing a variety of benefits for the delivery of biologically active agents, and in particular, proteins and peptides. Among the water soluble polymers employed, polyethylene glycol (PEG) has been most widely conjugated to a variety of biologically active agents including biologically active peptides. A reduction in immunogenicity or antigenicity, increased half-life, increased solubility, decreased clearance by the kidney and decreased enzymatic degradation have been attributed to conjugates of a variety of water soluble polymers and functional agents, including PEG conjugates. As a result of these attributes, the polymer conjugates of biologically active agents require less frequent dosing and may permit the use of less of the active agent to achieve a therapeutic endpoint. Less frequent dosing reduces the overall number of injections, which can be painful and which require inconvenient visits to healthcare professionals.

Although some success has been achieved with PEG conjugation, "PEGylation" of biologically active agents remains a challenge. As drug developers progress beyond very potent agonistic proteins such as erythropoietin and the various interferons, the benefits of the PEG hydrophilic polymer are insufficient to drive (i) *in vitro* the increases in solubility, stability and the decreases in viscosity, and (ii) *in vivo* the increases in bioavailability, serum and/or tissue half-life and the decreases in immunogenicity that are necessary for a commercially successful product.

Branched forms of PEG for use in conjugate preparation have been introduced to alleviate some of the difficulties and limitations encountered with the use of long straight PEG polymer chains. Experience to date demonstrates that branched forms of PEG deliver a "curve-shift" in performance benefit versus linear straight PEG polymers chains of same total molecular weight. While branched polymers may overcome some of the limitations associated with conjugates formed with long linear PEG polymers, neither branched nor linear PEG polymer conjugates adequately resolve the issues associated with the use of conjugated functional agents, in particular, inhibitory agents. PEGylation does, though, represent the state of the art in conjugation of hydrophilic polymers to target agents. PEGylated compound products, among them peginterferon alfa-2a (PEGASYS), pegfilgrastim (Neulasta), pegaptanib (Macugen), and certolizumab pegol (Cimzia), had over $6 billion in annual sales in 2009. Functionalized PEG (suitable for conjugation) is manufactured through a laborious process that involves polymerization of short linear polymers which are then multiply functionalized then attached as two conjugation reactions to a lysine residue which becomes a two-arm PEG reagent. Due to the number of synthetic steps and the need for high quality, multiple chromatography steps are required. Low polydispersity (<1.2) linear PEG polymers have a size restriction of approximately 20kDa, 30kDa or 40kDa with 20kDa being the economically feasible limit. When formed into a branched reagent, then, the final reagent size is 40 kDa (2 x 20 kDa), 60 kDa (2 x 30 kDa), 80 kDa (2 x 40 kDa). The larger the size, the more expensive to manufacture with low polydispersity. Also, the larger the size, the less optimal the solubility, stability, and viscosity of the polymer and the associated polymer-drug conjugate.

In summary, PEG polymers work well with low-dose, high-potency agonistic molecules such as erythropoietin and interferon. However, despite its commercial success, PEGylated products have inadequate stability and solubility, the PEG reagent is expensive to manufacture and, most important, PEGylated products have limited further upside in terms of improving *in vivo* and *in vitro* performance.

In view of the recognized advantages of conjugating functional agents to water soluble polymers, and the limitations of water soluble polymers such as PEG in forming conjugates suitable for therapeutic purposes, additional water soluble polymers for forming conjugates with functional agents are desirable. Water soluble polymers, particularly those which have many of the advantages of PEG for use in conjugate formation, and which do not suffer from the disadvantages observed with PEG as a conjugating agent would be desirable for use in forming therapeutic and diagnostic agents.

PEGylation does nonetheless point the way to a solution to the entire biocompatibility issue. PEG works because of the polymer's hydrophilic characteristics which shield the conjugated biological agent from the myriad non-specific *in vivo* clearance mechanisms in the body. The importance of water is generally recognized, but the special insight in this technology is to dig deeper to appreciate that it is how the water is bound and the associated water structure that is critical to the performance enhancement. PEG works because of its hydrophilic nature, but the water is not tightly bound to the polymer and thus the conjugated agent. Water molecules are in free exchange between the PEGylated compound and the surrounding bulk water, enabling clearance systems to recognize the protein. The answer is to find a way to "glue" water so tightly to the polymer and thus conjugated moiety such as to tightly mask the complex entirely from non-specific interactions. To accomplish, it is necessary for the polymer to maintain both positive and negative charges, thus being net neutral, an essential zwitterion. Certain zwitterionic polymers hold and will not release water molecules bound to their structures.

To make further progress, then, it is necessary to take a closer look at: (i) other examples of hydrophilic moieties that bind water to a greater extent and with more favorable physical properties and therefore with improved fundamental biocompatibility *in vivo* and *in vitro,* and (ii) examples of much bigger, extended form polymers (size and architecture) which is the related key driver of the *in vivo* and *in vitro* performance.

What is important for these polymers is the extent to which they bind water molecules and the physical properties of those water binding interactions. This combination of properties drives the fundamental biocompatibility of the polymer and the extent to which such a polymer can impart biocompatibility to a functional agent to which it is conjugated. The ideal technology would use a water binding moiety which very tightly if not irreversibly binds a large amount of water, would format these water binding moieties into a polymer backbone of sufficient length and flexibility to shield a range of desired drugs and formats, may have an extended form *(i.e.* multi-armed) architecture, would be functionalized for high efficiency conjugation to the drug moiety, would be manufactured inexpensively with a minimal number of production steps, and would demonstrate very high quality as judged analytically and very high performance judged in functional *in vivo* (terminal half-life, immunogenicity, bioactivity) and *in vitro* (solubility, stability, viscosity, bioactivity) systems. A technology that allowed for the maximization of these elements would take the field to new levels *of in vivo* and *in vitro* performance.

One such technology uses as the water binding moiety the phosphorylcholine derived 2-methacryloyloxyethyl phosphorylcholine (HEMA-PC) or a related zwitterion, on a polymer of total size greater than 50 kDa peak molecular weight (Mp) as measured by multi-angle light scattering, with the possibility for highly branched architectures or pseudo architectures, functionalized for site-specific conjugation to a biopharmaceutical(s) of interest, manufactured with techniques enabling a well characterized therapeutic with high quality and low polydispersity, and when conjugated to a biopharmaceutical imparts a dramatic increase in mean terminal half-life versus an equivalent biopharmaceutical as modified with another half-life extension technology (for example, as conjugated with a PEG polymer) and which imparts solubility, stability, viscosity, and characterizability parameters to the conjugate that are a multiple of that seen with PEG or other technologies.

Of critical importance is the size of the polymer. When used for therapeutic purposes in the context of soluble polymer-drug conjugates, the prior art teaches that there is a well-defined and described trade-off between the size of the polymer and its quality. The polydispersity index (a key proxy for quality) is particularly important as it speaks to the heterogeneity of the underlying statistical polymer which when conjugated to a pharmaceutical of interest imparts such heterogeneity to the drug itself which significantly complicates the reliable synthesis of the therapeutic protein required for consistent effectiveness.and which is undesirable from a manufacturing, regulatory, clinical, and patient point of view.

The present invention describes very large polymers with very high quality and very low polydispersity index which are functionalized for chemical conjugation for example to a soluble drug. Importantly, the polymers are not inert, nor are they destined for attachment to a surface or gelled as hydrogel. This is wholly new, surprising, very useful and has not been described previously. For their therapeutic intent, a well-defined drug substance is essential. This manifests itself at the level of the polymer, the pharmaceutical, and the conjugate. Notably, there is a body of work on polymers having been made using a variety of approaches and components with unfunctionalized polymers. That body of work is not directly relevant here where a required step is a specific conjugation.

The current state of the art as it relates to functionalized polymers, constructed from hydrophilic monomers by conventional, pseudo or controlled radical polymerization, is that only low molecular weight polymers (typically <50 kDa) have been described. In addition, as this molecular weight is approached, control of molecular weight, as evidenced by the polydispersity index (PDI), is lost.

For instance, Ishihara et al (2004, Biomaterials 25, 71-76) utilized controlled radical polymerization to construct linear polymers of 2-methacryloyloxyethyl phosphorylcholine (HEMA-PC) up to a molecular weight of 37 kDa. The PDI was 1.35, which is too high to be pharmaceutically relevant. In addition, these authors clearly stated, "In this method, it is hard to control the molecular weight distribution and increase the molecular weight." Lewis et al (US Patent 2004/0063881) also describe homopolymerization of this monomer using controlled radical polymerization, and reported molecular weights up to 11 kDa with a PDI of 1.45. In a later publication, Lewis et al (2008, Bioconjugate Chem. 19, 2144-2155) again synthesized functionalized homopolymers of HEMA-PC this time to molecular weights up to 37 kDa. The PDI was 2.01. They stated that they achieved good control only at very limited (insufficient) molecular weights, with polydispersity increasing dramatically. They report loss of control at their high end molecular weight range (37 kDa) which they attribute to fast conversion at higher monomer concentrations which leads to the conclusion that it is not possible to create high molecular weight polymers of this type with tight control of polydispersity.

For instance, Haddleton et al (2004, JACS 126, 13220-13221) utilized controlled radical polymerization to construct small linear polymers of poly(methoxyPEG)methacrylates for use in conjugation with proteins and in a size range of 11,000 to 34,000 Daltons. In an attempt to build the larger of these polymers, the authors increased the reaction temperature and sought out catalysts that could drive a faster polymerization. In a later publication, Haddleton et al (2005, JACS 127, 2966-2973) again synthesized functionalized homopolymers of poly(methoxyPEG) methacrylates via controlled radical polymerization for protein conjugation in the size range of 4.1 to 35.4 kDa with PDI's ranging upwards of 1.25 even at this small and insufficient molecular weight distribution. In a subsequent publication, Haddleton et al (2007, JACS 129, 15156-15163) again synthesized functionalized polymers via controlled radical polymerization for protein conjugation in the low size range of 8 to 30 kDA with PDI range of 1.20 - 1.28. Haddleton et al's mindset and approach teach away from the methods that need to be used to make high molecular weight, low polydispersity polymers relevant to this invention. Further, the focus on low molecular weight polymers for protein conjugation reflects a lack of understanding as to the size, architecture, and quality of polymers needed to carry the biopharmaceutical field to the next level.

The present invention describes high molecular weight zwitterion-containing polymers (>50 kDa peak molecular weight measured using multi-angle light scattering) with concomitantly low PDIs. This is surprising in light of the foregoing summary of the current state of the art.

### BRIEF SUMMARY OF THE INVENTION

In some embodiments, the present invention provides a polymer having at least two polymer arms each having a plurality of monomers each independently selected from acrylate, methacrylate, acrylamide, methacrylamide, styrene, vinyl-pyridine, vinyl-pyrrolidone or vinyl-ester, wherein each monomer includes a hydrophilic group. The polymer also includes an initiator fragment linked to a proximal end of the polymer arm, wherein the initator moiety is suitable for radical polymerization. The polymer also includes an end group linked to a distal end of the polymer arm. At least one of the initiator fragment and the end group of the polymer includes a functional agent or a linking group.

In other embodiments, the present invention provides a conjugate including at least one polymer having at least two polymer arms each having a plurality of monomers each independently selected from the group consisting of acrylate, methacrylate, acrylamide, methacrylamide, styrene, vinyl-pyridine, vinyl-pyrrolidone or vinyl-ester, wherein each monomer includes a hydrophilic group, an initiator fragment linked to a proximal end of the polymer arm, wherein the initator moiety is suitable for radical polymerization, and an end group linked to a distal end of the polymer arm. The conjugates of the present invention also include at least one functional agent having a bioactive agent or a diagnostic agent, linked to the initiator fragment or the end group.

In some other embodiments, the present invention provides a polymer of the formula: 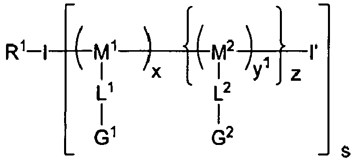 wherein R¹ can be H, L³-A¹, LG¹ or L³-LG¹. Each M¹ and M² can be independently selected from acrylate, methacrylate, acrylamide, methacrylamide, styrene, vinyl-pyridine, vinyl-pyrrolidone or vinyl-ester. Each of G¹ and G² is each independently a hydrophilic group. Each group I is an initiator fragment and I' a radical scavenger such that the combination of I-I' is an initiator, I¹, for the polymerization of the polymer via radical polymerization. Alternatively, each I' can be independently selected from H, halogen or C₁₋₆ alkyl. Each L¹, L² and L³ can be a linker. Each A¹ can be a functional agent. Each LG¹ can be a linking group. Subscripts x and y¹ can each independently be an integer of from 1 to 1000. Each subscript z can be independently an integer of from 1 to 10. Subscript s can be an integer of from 1 to 100.

### BRIEF DESCRIPTION OF THE DRAWINGS

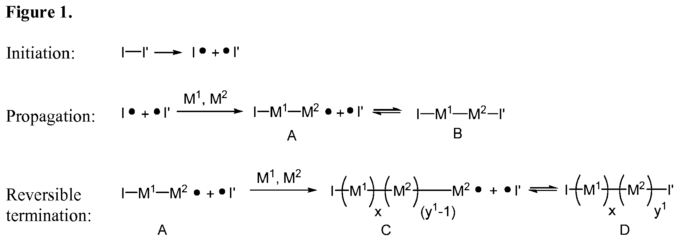

**Figure 1** shows a scheme for the preparation of the random copolymers of the present invention. The initiator I-I' is cleaved into initiator fragment I and radical scavenger I'. The initiator fragment I then reacts with comonomers M¹ and M² to initiate the polymerization process and generate species A. The radical scavenger I' can then reversibly react with species A to form species B. Alternatively, species A can react with additional monomers to continue propagation of the polymer (species C). Concomitantly, the growing polymer chain of species C reversibly reacts with radical scavenger I' to form the random copolymer, species D.

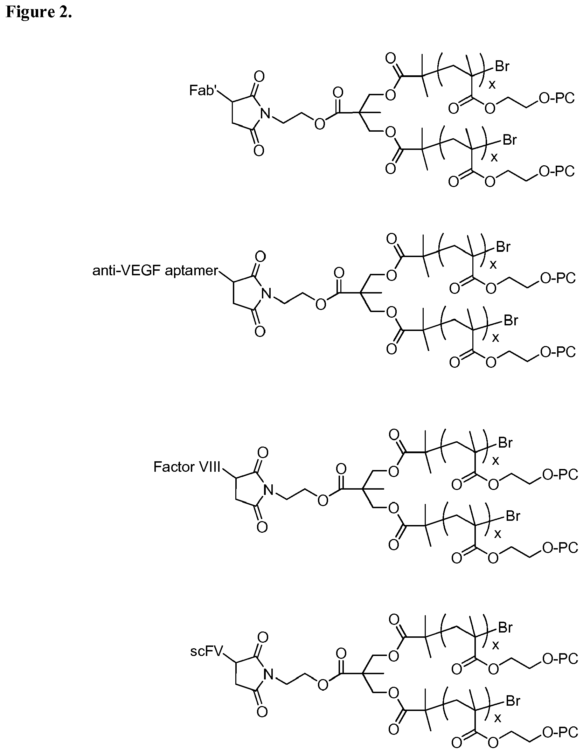

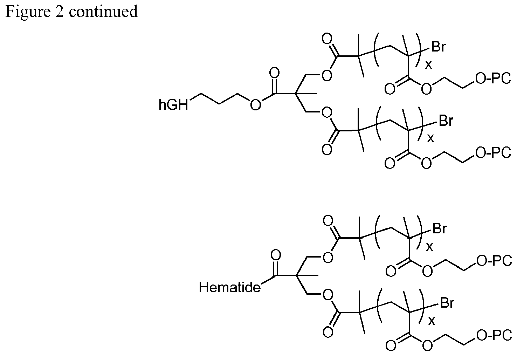

Figure 2 shows conjugates of the present invention.

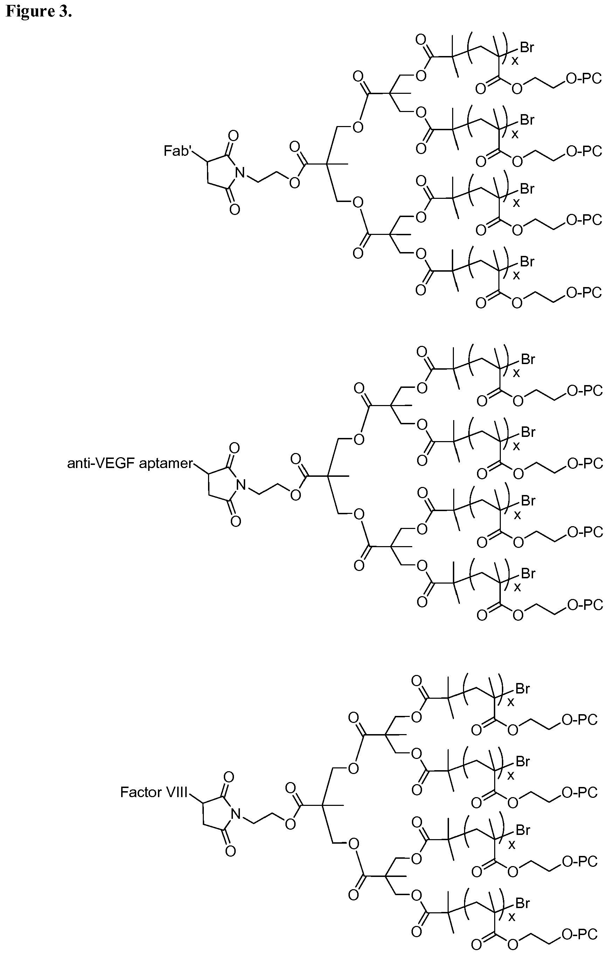

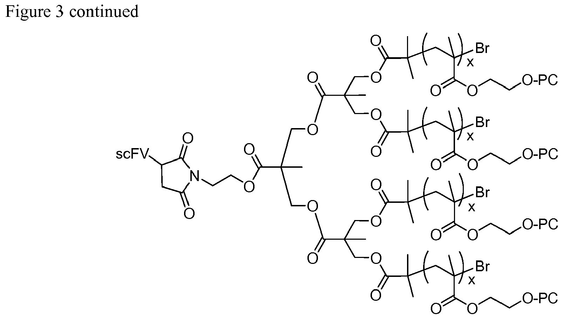

Figure 3 shows conjugates of the present invention.

### DETAILED DESCRIPTION OF THE INVENTION

### I. General

The present invention provides high MW polymers having hydrophilic groups or zwitterions, such as phosphorylcholine, and at least one functional agent (as defined herein). Phosphorylcholine as a highly biocompatible molecule drives fundamental biocompatibility. It also has chaperone type functions, in terms of protecting proteins under temperature or other stress. It also can allow other functions such as reversible cellular uptake. The functional agent can be a bioactive agent such as a drug, therapeutic protein or targeting agent, as well as a detection agent, imaging agent, labeling agent or diagnostic agent. The high MW polymers are useful for the treatment of a variety of conditions and disease states by selecting one or more appropriate functional agents. More than one bioactive agent can be linked to the high MW polymer, thus enabling treatment of not just a single disease symptom or mechanism, but rather the whole disease. In addition, the high MW polymers are useful for diagnostic and imaging purposes by attachment of suitable targeting agents and imaging agents. The high MW polymers can include both therapeutic and diagnostic agents in a single polymer, providing theranostic agents that treat the disease as well as detect and diagnose. The polymers can be linked to the bioactive agent(s) via stable or unstable linkages.

The polymers can be prepared via a conventional free-radical polymerization or controlled/living radical polymerization, such as atom transfer radical polymerization (ATRP), using monomers that contain zwitterions, such as phosphorylcholine. The initiators used for preparation of the high MW polymers can have multiple initiating sites such that multi-arm polymers, such as stars, can be prepared. The initiator can also contain either the bioactive agent, or linking groups that are able to link to the bioactive agent.

The invention also describes new ways to achieve branched polymer architectures on a bioactive surface. The concept is one of "branching points" or "proximal attachment points" on the target molecule such as to recreate an effective ≥2 arm polymer with ≥1 arm polymers attached to a localized site(s) on a target molecule. In the prior art, indiscriminate PEGylation of a protein with a non site-specific reagent (for example an NHS functionalized PEG reagent) would result in multiple PEG polymers conjugated to multiple amine groups scattered through the protein. Here, what is described is preferably a one step approach in which the target agent is modified to locate two unique conjugation sites (for example, cysteine amino acids) such that once the tertiary structure of the protein or peptide or agent is formed, the two sites will be in proximity one to the other. Then, this modified target agent is used in a conjugation reaction with a polymer containing the corresponding conjugation chemistry (for example, thiol reactive). The result is a single target agent which is conjugated with two polymers in close proximity to one another, thereby creating a branching point or "pseudo" branch. In another embodiment, the target agent would contain a single unique site, for example a free cysteine, and a tri(hetero)functional linking agent would be employed to attach ≥2 linear polymers to this single site, again creating a "pseudo" branch.

The invention also describes new ways to achieve very high efficiency and site specific conjugation to peptides and proteins by way of inteins.

### II. Definitions

"Polymer" refers to a series of monomer groups linked together. The high MW polymers are prepared from monomers that include, but are not limited to, acrylates, methacrylates, acrylamides, methacrylamides, styrenes, vinyl-pyridine, vinyl-pyrrolidone and vinyl esters such as vinyl acetate. Additional monomers are useful in the high MW polymers of the present invention. When two different monomers are used, the two monomers are called "comonomers," meaning that the different monomers are copolymerized to form a single polymer. The polymer can be linear or branched. When the polymer is branched, each polymer chain is referred to as a "polymer arm." The end of the polymer arm linked to the initiator moiety is the proximal end, and the growing-chain end of the polymer arm is the distal end. On the growing chain-end of the polymer arm, the polymer arm end group can be the radical scavenger, or another group.

"Hydrophilic group" refers to a compound or polymer that attracts water, and is typically water soluble. Examples of hydrophilic groups include hydrophilic polymers and zwitterionic moieties. Other hydrophilic groups include, but are not limited to, hydroxy, amine, carboxylic acid, amide, sulfonate and phosphonate. Hydrophilic polymers include, but are not limited to, polyethylene oxide, polyoxazoline, cellulose, starch and other polysaccharides. Zwitterionic moiety refers to a compound having both a positive and a negative charge. Zwitterionic moieties useful in the high MW polymers can include a quaternary nitrogen and a negatively charged phosphate, such as phosphorylcholine: RO-P(=O)(O⁻)-O-CH₂CH₂-N⁺(Me)₃. Other zwitterionic moieties are useful in the high MW polymers of the present invention, and Patents WO 1994/016748 and WO 1994/016749 are incorporated in their entirety herein.

"Initiator" refers to a compound capable of initiating a polymerization using the comonomers of the present invention. The polymerization can be a conventional free radical polymerization or a controlled/living radical polymerization, such as Atom Transfer Radical Polymerization (ATRP), Reversible Addition-Fragmentation-Termination (RAFT) polymerization or nitroxide mediated polymerization (NMP). The polymerization can be a "pseudo" controlled polymerization, such as degenerative transfer. When the initiator is suitable for ATRP, it contains a labile bond which can homolytically cleave to form an initiator fragment, I, being a radical capable of initiating a radical polymerization, and a radical scavenger, I', which reacts with the radical of the growing polymer chain to reversibly terminate the polymerization. The radical scavenger I' is typically a halogen, but can also be an organic moiety, such as a nitrile.

"Linker" refers to a chemical moiety that links two groups together. The linker can be cleavable or non-cleavable. Cleavable linkers can be hydrolyzable, enzymatically cleavable, pH sensitive, photolabile, or disulfide linkers, among others. Other linkers include homobifunctional and heterobifunctional linkers. A "linking group" is a functional group capable of forming a covalent linkage consisting of one or more bonds to a bioactive agent. Nonlimiting examples include those illustrated in Table 1.

"Hydrolyzable linker" refers to a chemical linkage or bond, such as a covalent bond, that undergoes hydrolysis under physiological conditions. The tendency of a bond to hydrolyze may depend not only on the general type of linkage connecting two central atoms between which the bond is severed, but also on the substituents attached to these central atoms. Non-limiting examples of hydrolytically susceptible linkages include esters of carboxylic acids, phosphate esters, acetals, ketals, acyloxyalkyl ether, imines, orthoesters, and some amide linkages.

"Enzymatically cleavable linker" refers to a linkage that is subject to degradation by one or more enzymes. Some hydrolytically susceptible linkages may also be enzymatically degradable. For example esterases may act on esters of carboxylic acid or phosphate esters, and proteases may act on peptide bonds and some amide linkages.

"pH sensitive linker" refers to a linkage that is stable at one pH and subject to degradation at another pH. For example, the pH sensitive linker can be stable at neutral or basic conditions, but labile at mildly acidic conditions.

"Photolabile linker" refers to a linkage, such as a covalent bond, that cleaves upon exposure to light. The photolabile linker includes an aromatic moiety in order to absorb the incoming light, which then triggers a rearrangement of the bonds in order to cleave the two groups linked by the photolabile linker.

"Self-immolative or double prodrug linker" refers to a linkage in which the main function of the linker is to release a functional agent only after selective trigger activation (for example, a drop in pH or the presence of a tissue-specific enzyme) followed by spontaneous chemical breakdown to release the functional agent.

"Functional agent" is defined to include a bioactive agent or a diagnostic agent. A "bioactive agent" is defined to include any agent, drug, compound, or mixture thereof that targets a specific biological location (targeting agent) and/or provides some local or systemic physiological or pharmacologic effect that can be demonstrated *in vivo* or *in vitro.* Non-limiting examples include drugs, vaccines, antibodies, antibody fragments, scFvs, diabodies, avimers, vitamins and cofactors, polysaccharides, carbohydrates, steroids, lipids, fats, proteins, peptides, polypeptides, nucleotides, oligonucleotides, polynucleotides, and nucleic acids *(e.g.,* mRNA, tRNA, snRNA, RNAi, DNA, cDNA, antisense constructs, ribozymes, etc). A "diagnostic agent" is defined to include any agent that enables the detection or imaging of a tissue or disease. Examples of diagnostic agents include, but are not limited to, radiolabels, fluorophores and dyes.

"Therapeutic protein" refers to peptides or proteins that include an amino acid sequence which in whole or in part makes up a drug and can be used in human or animal pharmaceutical applications. Numerous therapeutic proteins are known to practitioners of skill in the art including, without limitation, those disclosed herein.

"Phosphorylcholine," also denoted as "PC," refers to the following: 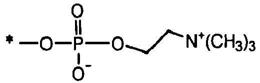 where ^{∗} denotes the point of attachment. The phosphorylcholine is a zwitterionic group and includes salts (such as inner salts), and protonated and deprotonated forms thereof.

"Phosphorylcholine containing polymer" is a polymer that contains phosphorylcholine. It is specifically contemplated that in each instance where a phosphorylcholine containing polymer is specified in this application for a particular use, a single phosphorylcholine can also be employed in such use. "Zwitterion containing polymer" refers to a polymer that contains a zwitterion.

"Poly(acryloyloxyethyl phosphorylcholine) containing polymer" refers to a polymer of acrylic acid containing at least one acryloyloxyethyl phosphorylcholine monomer such as 2-methacryloyloxyethyl phosphorylcholine (*i.e*., 2-methacryloyl-2'-trimethylammonium ethyl phosphate).

"Contacting" refers to the process of bringing into contact at least two distinct species such that they can react. It should be appreciated, however, that the resulting reaction product can be produced directly from a reaction between the added reagents or from an intermediate from one or more of the added reagents which can be produced in the reaction mixture.

"Water-soluble polymer" refers to a polymer that is soluble in water. A solution of a water-soluble polymer may transmit at least about 75%, more preferably at least about 95% of light, transmitted by the same solution after filtering. On a weight basis, a water-soluble polymer or segment thereof may be at least about 35%, at least about 50%, about 70%, about 85%, about 95% or 100% (by weight of dry polymer) soluble in water.

"Molecular weight" in the context of the polymer can be expressed as either a number average molecular weight, or a weight average molecular weight or a peak molecular weight. Unless otherwise indicated, all references to molecular weight herein refer to the peak molecular weight. These molecular weight determinations, number average, weight average and peak, can be measured using gel permeation chromatography or other liquid chromatography techniques. Other methods for measuring molecular weight values can also be used, such as the use of end-group analysis or the measurement of colligative properties (e.g., freezing-point depression, boiling-point elevation, or osmotic pressure) to determine number average molecular weight, or the use of light scattering techniques, ultracentrifugation or viscometry to determine weight average molecular weight. The polymeric reagents of the invention are typically polydisperse (*i.e*., number average molecular weight and weight average molecular weight of the polymers are not equal), possessing low polydispersity values of preferably less than about 1.5, as judged by gel permeation chromatography. In other embodiments the polydispersities may be in the range of about 1.4 to about 1.2, more preferably less than about 1.15, still more preferably less than about 1.10, yet still more preferably less than about 1.05, and most preferably less than about 1.03.

The phrase "a" or "an" entity as used herein refers to one or more of that entity; for example, a compound refers to one or more compounds or at least one compound. As such, the terms "a" (or "an"), "one or more", and "at least one" can be used interchangeably herein.

"About" as used herein means variation one might see in measurements taken among different instruments, samples, and sample preparations.

"Protected,", "protected form", "protecting group" and "protective group" refer to the presence of a group (*i.e.*, the protecting group) that prevents or blocks reaction of a particular chemically reactive functional group in a molecule under certain reaction conditions. Protecting group will vary depending upon the type of chemically reactive group being protected as well as the reaction conditions to be employed and the presence of additional reactive or protecting groups in the molecule, if any. The skilled artisan will recognize protecting groups known in the art, such as those found in the treatise by Greene et al., "Protective Groups In Organic Synthesis," 3rd Edition, John Wiley and Sons, Inc., New York, 1999.

"Spacer," and "spacer group" are used interchangeably herein to refer to an atom or a collection of atoms optionally used to link interconnecting moieties such as a terminus of a water-soluble polymer and a reactive group of a functional agent and a reactive group. A spacer may be hydrolytically stable or may include a hydrolytically susceptible or enzymatically degradable linkage.

"Alkyl" refers to a straight or branched, saturated, aliphatic radical having the number of carbon atoms indicated. For example, C₁-C₆ alkyl includes, but is not limited to, methyl, ethyl, propyl, isopropyl, butyl, isobutyl, sec-butyl, tert-butyl, pentyl, isopentyl, hexyl, etc. Other alkyl groups include, but are not limited to heptyl, octyl, nonyl, decyl, etc. Alkyl can include any number of carbons, such as 1-2, 1-3, 1-4, 1-5, 1-6, 1-7, 1-8, 1-9, 1-10, 2-3, 2-4, 2-5, 2-6, 3-4, 3-5, 3-6, 4-5, 4-6 and 5-6. The alkyl group is typically monovalent, but can be divalent, such as when the alkyl group links two moieties together.

The term "lower" referred to above and hereinafter in connection with organic radicals or compounds respectively defines a compound or radical which can be branched or unbranched with up to and including 7, preferably up to and including 4 and (as unbranched) one or two carbon atoms.

"Alkylene" refers to an alkyl group, as defined above, linking at least two other groups, *i.e.*, a divalent hydrocarbon radical. The two moieties linked to the alkylene can be linked to the same atom or different atoms of the alkylene. For instance, a straight chain alkylene can be the bivalent radical of -(CH₂)_{n,} where n is 1, 2, 3, 4, 5 or 6. Alkylene groups include, but are not limited to, methylene, ethylene, propylene, isopropylene, butylene, isobutylene, sec-butylene, pentylene and hexylene.

Substituents for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) can be a variety of groups selected from: -OR', =O, =NR', =N-OR', -NR'R", -SR', -halogen, -SiR'R"R"', -OC(O)R', -C(O)R', -CO₂R', -CONR'R", -OC(O)NR'R", -NR"C(O)R', -NR'-C(O)NR"R‴, -NR"C(O)₂R', -NH-C(NH₂)=NH, -NR'C(NH₂)=NH, -NH-C(NH₂)=NR', -S(O)R', -S(O)₂R', -S(O)₂NR'R", -CN and -NO₂ in a number ranging from zero to (2m'+1), where m' is the total number of carbon atoms in such radical. R', R" and R'" each independently refer to hydrogen, unsubstituted (C₁-C₈)alkyl and heteroalkyl, unsubstituted aryl, aryl substituted with 1-3 halogens, unsubstituted alkyl, alkoxy or thioalkoxy groups, or aryl-(C₁-C₄)alkyl groups. When R' and R" are attached to the same nitrogen atom, they can be combined with the nitrogen atom to form a 5-, 6-, or 7-membered ring. For example, -NR'R" is meant to include 1-pyrrolidinyl and 4-morpholinyl. From the above discussion of substituents, one of skill in the art will understand that the term "alkyl" is meant to include groups such as haloalkyl *(e.g.,* -CF₃ and -CH₂CF₃) and acyl *(e.g.,* -C(O)CH₃, -C(O)CF₃, -C(O)CH₂OCH₃, and the like). Preferably, the substituted alkyl and heteroalkyl groups have from 1 to 4 substituents, more preferably 1, 2 or 3 substituents. Exceptions are those perhalo alkyl groups (e.g., pentafluoroethyl and the like) which are also preferred and contemplated by the present invention.

Substituents for the alkyl and heteroalkyl radicals (including those groups often referred to as alkylene, alkenyl, heteroalkylene, heteroalkenyl, alkynyl, cycloalkyl, heterocycloalkyl, cycloalkenyl, and heterocycloalkenyl) can be one or more of a variety of groups selected from, but not limited to: -OR', =O, =NR', =N-OR', -NR'R", -SR', -halogen, -SiR'R"R‴, -OC(O)R', -C(O)R', -CO₂R', -CONR'R", -OC(O)NR'R", -NR"C(O)R', -NR'-C(O)NR"R‴, -NR"C(O)₂R',-NR-C(NR'R"R‴)=NRʺʺ, -NR-C(NR'R")=NR‴, -S(O)R', -S(O)₂R', -S(O)₂NR'R", -NRSO₂R', -CN and -NO₂ in a number ranging from zero to (2m'+1), where m' is the total number of carbon atoms in such radical. R', R", R‴ and Rʺʺ each preferably independently refer to hydrogen, substituted or unsubstituted heteroalkyl, substituted or unsubstituted aryl, *e.g.,* aryl substituted with 1-3 halogens, substituted or unsubstituted alkyl, alkoxy or thioalkoxy groups, or arylalkyl groups. When a compound of the invention includes more than one R group, for example, each of the R groups is independently selected as are each R', R", R'" and R"" groups when more than one of these groups is present. When R' and R" are attached to the same nitrogen atom, they can be combined with the nitrogen atom to form a 5-, 6-, or 7-membered ring. For example, -NR'R" is meant to include, but not be limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl *(e.g.,* -CF₃ and -CH₂CF₃) and acyl *(e.g.,* -C(O)CH₃, -C(O)CF₃, -C(O)CH₂OCH₃, and the like).

"Alkoxy" refers to alkyl group having an oxygen atom that either connects the alkoxy group to the point of attachment or is linked to two carbons of the alkoxy group. Alkoxy groups include, for example, methoxy, ethoxy, propoxy, iso-propoxy, butoxy, 2-butoxy, iso-butoxy, sec-butoxy, tert-butoxy, pentoxy, hexoxy, etc. The alkoxy groups can be further substituted with a variety of substituents described within. For example, the alkoxy groups can be substituted with halogens to form a "halo-alkoxy" group.

"Carboxyalkyl" means an alkyl group (as defined herein) substituted with a carboxy group. The term "carboxycycloalkyl" means an cycloalkyl group (as defined herein) substituted with a carboxy group. The term alkoxyalkyl means an alkyl group (as defined herein) substituted with an alkoxy group. The term "carboxy" employed herein refers to carboxylic acids and their esters.

"Haloalkyl" refers to alkyl as defined above where some or all of the hydrogen atoms are substituted with halogen atoms. Halogen (halo) preferably represents chloro or fluoro, but may also be bromo or iodo. For example, haloalkyl includes trifluoromethyl, fluoromethyl, 1,2,3,4,5-pentafluoro-phenyl, etc. The term "perfluoro" defines a compound or radical which has all available hydrogens that are replaced with fluorine. For example, perfluorophenyl refers to 1,2,3,4,5-pentafluorophenyl, perfluoromethyl refers to 1,1,1-trifluoromethyl, and perfluoromethoxy refers to 1,1,1-trifluoromethoxy.

"Fluoro-substituted alkyl" refers to an alkyl group where one, some, or all hydrogen atoms have been replaced by fluorine.

"Cytokine" in the context of this invention is a member of a group of protein signaling molecules that may participate in cell-cell communication in immune and inflammatory responses. Cytokines are typically small, water-soluble glycoproteins that have a mass of about 8-35 kDa.

"Cycloalkyl" refers to a cyclic hydrocarbon group that contains from about 3 to 12, from 3 to 10, or from 3 to 7 endocyclic carbon atoms. Cycloalkyl groups include fused, bridged and spiro ring structures.

"Endocyclic" refers to an atom or group of atoms which comprise part of a cyclic ring structure.

"Exocyclic" refers to an atom or group of atoms which are attached but do not define the cyclic ring structure.

"Cyclic alkyl ether" refers to a 4 or 5 member cyclic alkyl group having 3 or 4 endocyclic carbon atoms and 1 endocyclic oxygen or sulfur atom (e.g., oxetane, thietane, tetrahydrofuran, tetrahydrothiophene); or a 6 to 7 member cyclic alkyl group having 1 or 2 endocyclic oxygen or sulfur atoms (e.g., tetrahydropyran, 1,3-dioxane, 1,4-dioxane, tetrahydrothiopyran, 1,3-dithiane, 1,4-dithiane, 1,4-oxathiane).

"Alkenyl" refers to either a straight chain or branched hydrocarbon of 2 to 6 carbon atoms, having at least one double bond. Examples of alkenyl groups include, but are not limited to, vinyl, propenyl, isopropenyl, 1-butenyl, 2-butenyl, isobutenyl, butadienyl, 1-pentenyl, 2-pentenyl, isopentenyl, 1,3-pentadienyl, 1,4-pentadienyl, 1-hexenyl, 2-hexenyl, 3-hexenyl, 1,3-hexadienyl, 1,4-hexadienyl, 1,5-hexadienyl, 2,4-hexadienyl, or 1,3,5-hexatrienyl. Alkenyl groups can also have from 2 to 3, 2 to 4, 2 to 5, 3 to 4, 3 to 5, 3 to 6, 4 to 5, 4 to 6 and 5 to 6 carbons. The alkenyl group is typically monovalent, but can be divalent, such as when the alkenyl group links two moieties together.

"Alkenylene" refers to an alkenyl group, as defined above, linking at least two other groups, *i.e.,* a divalent hydrocarbon radical. The two moieties linked to the alkenylene can be linked to the same atom or different atoms of the alkenylene. Alkenylene groups include, but are not limited to, ethenylene, propenylene, isopropenylene, butenylene, isobutenylene, sec-butenylene, pentenylene and hexenylene.

"Alkynyl" refers to either a straight chain or branched hydrocarbon of 2 to 6 carbon atoms, having at least one triple bond. Examples of alkynyl groups include, but are not limited to, acetylenyl, propynyl, 1-butynyl, 2-butynyl, isobutynyl, sec-butynyl, butadiynyl, 1-pentynyl, 2-pentynyl, isopentynyl, 1,3-pentadiynyl, 1,4-pentadiynyl, 1-hexynyl, 2-hexynyl, 3-hexynyl, 1,3-hexadiynyl, 1,4-hexadiynyl, 1,5-hexadiynyl, 2,4-hexadiynyl, or 1,3,5-hexatriynyl. Alkynyl groups can also have from 2 to 3, 2 to 4, 2 to 5, 3 to 4, 3 to 5, 3 to 6, 4 to 5, 4 to 6 and 5 to 6 carbons. The alkynyl group is typically monovalent, but can be divalent, such as when the alkynyl group links two moieties together.

"Alkynylene" refers to an alkynyl group, as defined above, linking at least two other groups, *i.e.,* a divalent hydrocarbon radical. The two moieties linked to the alkynylene can be linked to the same atom or different atoms of the alkynylene. Alkynylene groups include, but are not limited to, ethynylene, propynylene, butynylene, sec-butynylene, pentynylene and hexynylene.

"Cycloalkyl" refers to a saturated or partially unsaturated, monocyclic, fused bicyclic or bridged polycyclic ring assembly containing from 3 to 12 ring atoms, or the number of atoms indicated. Monocyclic rings include, for example, cyclopropyl, cyclobutyl, cyclopentyl, cyclohexyl, and cyclooctyl. Bicyclic and polycyclic rings include, for example, norbornane, decahydronaphthalene and adamantane. For example, C₃₋₈cycloalkyl includes cyclopropyl, cyclobutyl, cyclopentyl, cyclohexyl, cyclooctyl, and norbornane.

"Cycloalkylene" refers to a cycloalkyl group, as defined above, linking at least two other groups, *i.e.,* a divalent hydrocarbon radical. The two moieties linked to the cycloalkylene can be linked to the same atom or different atoms of the cycloalkylene. Cycloalkylene groups include, but are not limited to, cyclopropylene, cyclobutylene, cyclopentylene, cyclohexylene, and cyclooctylene.

"Heterocycloalkyl" refers to a ring system having from 3 ring members to about 20 ring members and from 1 to about 5 heteroatoms such as N, O and S. Additional heteroatoms can also be useful, including, but not limited to, B, Al, Si and P. The heteroatoms can also be oxidized, such as, but not limited to, -S(O)- and -S(O)₂-. For example, heterocycle includes, but is not limited to, tetrahydrofuranyl, tetrahydrothiophenyl, morpholino, pyrrolidinyl, pyrrolinyl, imidazolidinyl, imidazolinyl, pyrazolidinyl, pyrazolinyl, piperazinyl, piperidinyl, indolinyl, quinuclidinyl and 1,4-dioxa-8-aza-spiro[4.5]dec-8-yl.

"Heterocycloalkylene" refers to a heterocyclalkyl group, as defined above, linking at least two other groups. The two moieties linked to the heterocycloalkylene can be linked to the same atom or different atoms of the heterocycloalkylene.

"Aryl" refers to a monocyclic or fused bicyclic, tricyclic or greater, aromatic ring assembly containing 6 to 16 ring carbon atoms. For example, aryl may be phenyl, benzyl or naphthyl, preferably phenyl. "Arylene" means a divalent radical derived from an aryl group. Aryl groups can be mono-, di- or tri-substituted by one, two or three radicals selected from alkyl, alkoxy, aryl, hydroxy, halogen, cyano, amino, amino-alkyl, trifluoromethyl, alkylenedioxy and oxy-C₂-C₃-alkylene; all of which are optionally further substituted, for instance as hereinbefore defined; or 1- or 2-naphthyl; or 1- or 2-phenanthrenyl. Alkylenedioxy is a divalent substitute attached to two adjacent carbon atoms of phenyl, e.g. methylenedioxy or ethylenedioxy. Oxy-C₂-C₃-alkylene is also a divalent substituent attached to two adjacent carbon atoms of phenyl, e.g. oxyethylene or oxypropylene. An example for oxy- C₂-C₃-alkylene-phenyl is 2,3-dihydrobenzofuran-5-yl.

Preferred as aryl is naphthyl, phenyl or phenyl mono- or disubstituted by alkoxy, phenyl, halogen, alkyl or trifluoromethyl, especially phenyl or phenyl-mono- or disubstituted by alkoxy, halogen or trifluoromethyl, and in particular phenyl.

Examples of substituted phenyl groups as R are, e.g. 4-chlorophen-1-yl, 3,4-dichlorophen-1-yl, 4-methoxyphen-1-yl, 4-methylphen-1-yl, 4-aminomethylphen-1-yl, 4-methoxyethylaminomethylphen-1-yl, 4-hydroxyethylaminomethylphen-1-yl, 4-hydroxyethyl-(methyl)-aminomethylphen-1-yl, 3-aminomethylphen-1-yl, 4-N-acetylaminomethylphen-1-yl, 4-aminophen-1-yl, 3-aminophen-1-yl, 2-aminophen-1-yl, 4-phenyl-phen-1-yl, 4-(imidazol-1-yl)-phen-yl, 4-(imidazol-1-ylmethyl)-phen-1-yl, 4-(morpholin-1-yl)-phen-1-yl, 4-(morpholin-1-ylmethyl)-phen-1-yl, 4-(2-methoxyethylaminomethyl)-phen-1-yl and 4-(pyrrolidin-1-ylmethyl)-phen-1-yl, 4-(thiophenyl)-phen-1-yl, 4-(3-thiophenyl)-phen-1-yl, 4-(4-methylpiperazin-1-yl)-phen-1-yl, and 4-(piperidinyl)-phenyl and 4-(pyridinyl)-phenyl optionally substituted in the heterocyclic ring.

"Arylene" refers to an aryl group, as defined above, linking at least two other groups. The two moieties linked to the arylene are linked to different atoms of the arylene. Arylene groups include, but are not limited to, phenylene.

"Arylene-oxy" refers to an arylene group, as defined above, where one of the moieties linked to the arylene is linked through an oxygen atom. Arylene-oxy groups include, but are not limited to, phenylene-oxy.

Similarly, substituents for the aryl and heteroaryl groups are varied and are selected from: -halogen, -OR', -OC(O)R', -NR'R", -SR', -R', -CN, -NO₂, -CO₂R', -CONR'R", -C(O)R', -OC(O)NR'R", -NR"C(O)R', -NR"C(O)₂R',,-NR'-C(O)NR"R‴, -NH-C(NH₂)=NH, -NR'C(NH₂)=NH, -NH-C(NH₂)=NR', -S(O)R', -S(O)₂R', -S(O)₂NR'R", -N₃, -CH(Ph)₂, perfluoro(C₁-C₄)alkoxy, and perfluoro(C₁-C₄)alkyl, in a number ranging from zero to the total number of open valences on the aromatic ring system; and where R', R" and R'" are independently selected from hydrogen, (C₁-C₈)alkyl and heteroalkyl, unsubstituted aryl and heteroaryl, (unsubstituted aryl)-(C₁-C₄)alkyl, and (unsubstituted aryl)oxy-(C₁-C₄)alkyl.

Two of the substituents on adjacent atoms of the aryl or heteroaryl ring may optionally be replaced with a substituent of the formula -T-C(O)-(CH₂)_{q}-U-, wherein T and U are independently -NH-, -O-, -CH₂- or a single bond, and q is an integer of from 0 to 2. Alternatively, two of the substituents on adjacent atoms of the aryl or heteroaryl ring may optionally be replaced with a substituent of the formula -A-(CH₂)ᵣ-B-, wherein A and B are independently -CH₂-, -O-, -NH-, -S-, -S(O)-, -S(O)₂-, -S(O)₂NR'- or a single bond, and r is an integer of from 1 to 3. One of the single bonds of the new ring so formed may optionally be replaced with a double bond. Alternatively, two of the substituents on adjacent atoms of the aryl or heteroaryl ring may optionally be replaced with a substituent of the formula -(CH₂)ₛ-X-(CH₂)ₜ-, where s and t are independently integers of from 0 to 3, and X is -O-, -NR'-, -S-, -S(O)-, -S(O)₂-, or -S(O)₂NR'-. The substituent R' in -NR'- and -S(O)₂NR'- is selected from hydrogen or unsubstituted (C₁-C₆)alkyl.

"Heteroaryl" refers to a monocyclic or fused bicyclic or tricyclic aromatic ring assembly containing 5 to 16 ring atoms, where from 1 to 4 of the ring atoms are a heteroatom each N, O or S. For example, heteroaryl includes pyridyl, indolyl, indazolyl, quinoxalinyl, quinolinyl, isoquinolinyl, benzothienyl, benzofuranyl, furanyl, pyrrolyl, thiazolyl, benzothiazolyl, oxazolyl, isoxazolyl, triazolyl, tetrazolyl, pyrazolyl, imidazolyl, thienyl, or any other radicals substituted, especially mono- or di-substituted, by e.g. alkyl, nitro or halogen. Pyridyl represents 2-, 3- or 4-pyridyl, advantageously 2- or 3-pyridyl. Thienyl represents 2- or 3-thienyl. Quinolinyl represents preferably 2-, 3- or 4-quinolinyl. Isoquinolinyl represents preferably 1-, 3- or 4-isoquinolinyl. Benzopyranyl, benzothiopyranyl represents preferably 3-benzopyranyl or 3-benzothiopyranyl, respectively. Thiazolyl represents preferably 2- or 4-thiazolyl, and most preferred, 4-thiazolyl. Triazolyl is preferably 1-, 2- or 5-(1,2,4-triazolyl). Tetrazolyl is preferably 5-tetrazolyl.

Preferably, heteroaryl is pyridyl, indolyl, quinolinyl, pyrrolyl, thiazolyl, isoxazolyl, triazolyl, tetrazolyl, pyrazolyl, imidazolyl, thienyl, furanyl, benzothiazolyl, benzofuranyl, isoquinolinyl, benzothienyl, oxazolyl, indazolyl, or any of the radicals substituted, especially mono- or di-substituted.

As used herein, the term "heteroalkyl" refers to an alkyl group having from 1 to 3 heteroatoms such as N, O and S. Additional heteroatoms can also be useful, including, but not limited to, B, Al, Si and P. The heteroatoms can also be oxidized, such as, but not limited to, -S(O)- and -S(O)₂-. For example, heteroalkyl can include ethers, thioethers, alkyl-amines and alkyl-thiols.

As used herein, the term "heteroalkylene" refers to a heteroalkyl group, as defined above, linking at least two other groups. The two moieties linked to the heteroalkylene can be linked to the same atom or different atoms of the heteroalkylene.

"Electrophile" refers to an ion or atom or collection of atoms, which may be ionic, having an electrophilic center, *i.e.,* a center that is electron seeking, capable of reacting with a nucleophile. An electrophile (or electrophilic reagent) is a reagent that forms a bond to its reaction partner (the nucleophile) by accepting both bonding electrons from that reaction partner.

"Nucleophile" refers to an ion or atom or collection of atoms, which may be ionic, having a nucleophilic center, *i.e.*, a center that is seeking an electrophilic center or capable of reacting with an electrophile. A nucleophile (or nucleophilic reagent) is a reagent that forms a bond to its reaction partner (the electrophile) by donating both bonding electrons. A "nucleophilic group" refers to a nucleophile after it has reacted with a reactive group. Non limiting examples include amino, hydroxyl, alkoxy, haloalkoxy and the like.

"Maleimido" refers to a pyrrole-2,5-dione-1-yl group having the structure: 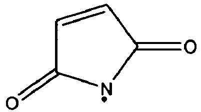 which upon reaction with a sulfhydryl (e.g., a thio alkyl) forms an -S-maleimido group having the structure 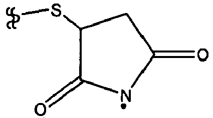 where "•" indicates the point of attachment for the maleimido group and " 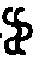 "indicates the point of attachment of the sulfur atom the thiol to the remainder of the original sulfhydryl bearing group.

For the purpose of this disclosure, "naturally occurring amino acids" found in proteins and polypeptides are L-alanine, L-arginine, L-asparagine, L-aspartic acid, L-cysteine, L-glutamine, L-glutamic acid, L-glycine, L-histidine, L-isoleucine, L-leucine, L-lysine, L-methionine, L-phenylalanine, L-proline, L-serine, L-threonine, L-tryptophan, L-tyrosine, and or L-valine. "Non-naturally occurring amino acids" found in proteins are any amino acid other than those recited as naturally occurring amino acids. Non-naturally occurring amino acids include, without limitation, the D isomers of the naturally occurring amino acids, and mixtures of D and L isomers of the naturally occurring amino acids. Other amino acids, such as 4-hydroxyproline, desmosine, isodesmosine, 5-hydroxylysine, epsilon-N-methyllysine, 3-methylhistidine, although found in naturally occurring proteins, are considered to be non-naturally occurring amino acids found in proteins for the purpose of this disclosure as they are generally introduced by means other than ribosomal translation of mRNA.

"Linear" in reference to the geometry, architecture or overall structure of a polymer, refers to polymer having a single polymer arm.

"Branched," in reference to the geometry, architecture or overall structure of a polymer, refers to polymer having 2 or more polymer "arms" extending from a core structure, such as an L group, that may be derived from an initiator employed in an atom transfer radical polymerization reaction. A branched polymer may possess 2 polymer arms, 3 polymer arms, 4 polymer arms, 5 polymer arms, 6 polymer arms, 7 polymer arms, 8 polymer arms, 9 polymer arms or more. For the purpose of this disclosure, compounds having three or more polymer arms extending from a single linear group are denoted as having a "comb" structure or "comb" architecture. Branched can also be achieved through "statistical" structures to create broader dendrimer-like architectures. The group linking the polymer arms can be a small molecule having multiple attachment points, such as glycerol, or more complex structures having 4 or more polymer attachment points, such as dendrimers and hyperbranched structures. The group can also be a nanoparticle appropriately functionalized to allow attachment of multiple polymer arms.

"Pharmaceutically acceptable" composition or "pharmaceutical composition" refers to a composition comprising a compound of the invention and a pharmaceutically acceptable excipient or pharmaceutically acceptable excipients.

"Pharmaceutically acceptable excipient" and "pharmaceutically acceptable carrier" refer to an excipient that can be included in the compositions of the invention and that causes no significant adverse toxicological effect on the patient. Non-limiting examples of pharmaceutically acceptable excipients include water, NaCl, normal saline solutions, lactated Ringer's, normal sucrose, normal glucose and the like.

"Patient" or "subject in need thereof' refers to a living organism suffering from or prone to a condition that can be prevented or treated by administration of a pharmaceutical composition as provided herein. Non-limiting examples include humans, other mammals and other non-mammalian animals.

"Therapeutically effective amount" refers to an amount of a conjugated functional agent or of a pharmaceutical composition useful for treating, ameliorating, or preventing an identified disease or condition, or for exhibiting a detectable therapeutic or inhibitory effect. The effect can be detected by any assay method known in the art.

The "biological half-life" of a substance is a pharmacokinetic parameter which specifies the time required for one half of the substance to be removed from an organism following introduction of the substance into the organism.

### III. High Molecular Weight Polymers

The present invention provides a high molecular weight polymer having hydrophilic groups and a functional group or linking group. In some embodiments, the present invention provides a polymer having at least two polymer arms each having a plurality of monomers each independently selected from acrylate, methacrylate, acrylamide, methacrylamide, styrene, vinyl-pyridine, vinyl-pyrrolidone or a vinyl ester such as vinyl acetate, wherein each monomer includes a hydrophilic group. The polymer also includes an initiator fragment linked to a proximal end of the polymer arm, wherein the initiator moiety is suitable for radical polymerization. The polymer also includes an end group linked to a distal end of the polymer arm. At least one of the initiator fragment and the end group of the polymer includes a functional agent or a linking group.

In other embodiments, the present invention provides a polymer having a polymer arm having a plurality of monomers each independently selected from acrylate, methacrylate, acrylamide, methacrylamide, styrene, vinyl-pyridine, vinyl-pyrrolidone or a vinyl ester such as vinyl acetate, wherein each monomer includes a hydrophilic group. The polymer also includes an initiator fragment linked to a proximal end of the polymer arm, wherein the initiator moiety is suitable for radical polymerization. The polymer also includes an end group linked to a distal end of the polymer arm. At least one of the initiator fragment and the end group of the polymer includes a functional agent or a linking group. In addition, the polymer has a peak molecular weight (Mp) of from about 50 kDa to about 1,500 kDa, as measured by multi-angle light scattering.

The polymers of the present invention can have any suitable molecular weight. Exemplary molecular weights for the high MW polymers of the present invention can be from about 50 to about 1,500 kilo-Daltons (kDa). In some embodiments, the high MW polymers of the present invention can have a molecular weight of about 50 kDa, about 100 kDa, about 200 kDa, about 250 kDa, about 300 kDa, about 350 kDa, about 400 kDa, about 450 kDa, about 500 kDa, about 750 kDa, about 1,000 kDa or about 1,500 kDa.

In some other embodiments, the present invention provides a polymer of the formula: 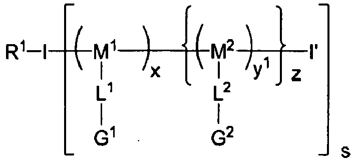 wherein R¹ can be H, L³-A¹, LG¹ or L³-LG¹. Each M¹ and M² can be independently selected from acrylate, methacrylate, acrylamide, methacrylamide, styrene, vinyl-pyridine, vinyl-pyrrolidone or vinyl-ester. Each of G¹ and G² is each independently a hydrophilic group. Each group I is an initiator fragment and I' a radical scavenger such that the combination of I-I' is an initiator, I¹, for the polymerization of the polymer via radical polymerization. Alternatively, each I' can be independently selected from H, halogen or C₁₋₆ alkyl. Each L¹, L² and L³ can be a linker. Each A¹ can be a functional agent. Each LG¹ can be a linking group. Subscripts x and y¹ can each independently be an integer of from 1 to 1000. Each subscript z can be independently an integer of from 1 to 10. Subscript s can be an integer of from 1 to 100.

In other embodiments, the present invention provides a polymer of Formula I: 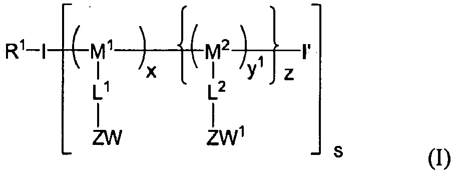 wherein R¹ of formula I can be H, L³-A¹, LG¹ or L³-LG¹. Each M¹ and M² of formula I can be independently selected from acrylate, methacrylate, acrylamide, methacrylamide, styrene, vinyl-pyridine, vinyl-pyrrolidone or vinyl-ester. Each of ZW and ZW¹ of formula I can be independently a zwitterionic moiety. Each I is an initiator fragment and I' a radical scavenger such that the combination of I-I' is an initiator, I¹, for the polymerization of the polymer of formula I via radical polymerization. Alternatively, each I' can be independently selected from H, halogen or C₁₋₆ alkyl. Each L¹, L² and L³ of formula I can be a linker. Each A¹ of formula I can be a functional agent. Each LG¹ of formula I can be a linking group. Subscripts x and y¹ of formula I can each independently be an integer of from 1 to 1000. Each subscript z of formula I can be independently an integer of from 1 to 10. Subscript s of formula I can be an integer of from 1 to 100. The sum of s, x, y¹ and z can be such that the polymer of formula I has a peak molecular weight of from about 50kDa to about 1,500kDa, as measured by multi-angle light scattering.

In other embodiments, the polymer can have the formula:

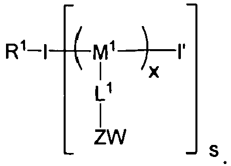

In some other embodiments, the polymer can have the formula: 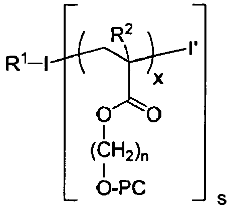 wherein R² can be selected from H or C₁₋₆ alkyl, and PC can be phosphorylcholine.

The high MW polymers of the present invention can also have any suitable number of comonomers, M². For example, the number of comonomers, subscript z, can be from 1 to 10, such as 1, 2, 3, 4, 5, 6, 7, 8, 9 or 10. The number of comonomers, subscript z, can also be from 1 to 5, 1 to 4, 1 to 3, or 1 to 2. In some embodiments, the high MW polymer of the present invention can have two different monomers where subscript z is 1, such as in formula Ia:

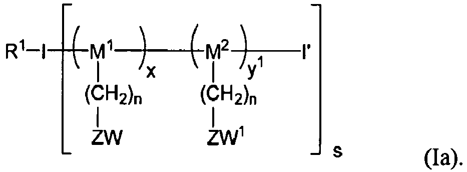

Additional comonomers M² can be present in the high MW polymers of the present invention, such as M^{2a}, M^{2b}, M^{2c}, M^{2d}, M^{2e}, M^{2f}, M^{2g}, M^{2h}, etc., and are defined as above for M², where each comonomer is present in a same or different y¹ value, and each comonomer having a corresponding ZW¹ group attached.

The different monomers of the high MW polymers can also be present in any suitable ratio. For example, the M² monomers, collectively or individually, can be present relative to the M¹ monomer in a ratio of 100:1, 50:1, 40:1, 30:1, 20:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:20, 1:30, 1:40, 1:50 and 1:100. In addition, each M² monomer can be present in any suitable ratio relative to the M¹ or any other M² monomer, such as 100:1, 50:1, 40:1, 30:1, 20:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:20, 1:30, 1:40, 1:50 and 1:100.

The high MW polymers of the present invention can have any suitable architecture. For example, the high MW polymers can be linear or branched. When the high MW polymers are branched, they can have any suitable number of polymer arms, as defined by subscript s of formula I, such as 2, 3, 4, 5, 6, 7, 8, 9, 10, 20, 30, 40, 50, 60, 70, 80, 90 and up to 100 arms. In some embodiments, subscript s can be from 1 to 32, 1 to 16, 1 to 10, 1 to 9, 1 to 8, 1 to 7, 1 to 6, 1 to 5, 1 to 4, 1 to 3 or 1 to 2. The high MW polymers of the present invention can adopt any suitable architecture. For example, the high MW polymers can be linear, branched, stars, dendrimers, combs, etc.

A functional agent of the high MW polymers can be linked to the initiator fragment I, or the radical scavenger I', or both. When multiple functional agents are present, L¹ can be a branching linker such that two or more functional agents can be linked to the initiator fragment I. In some embodiments, the high MW polymer has formula Ib:

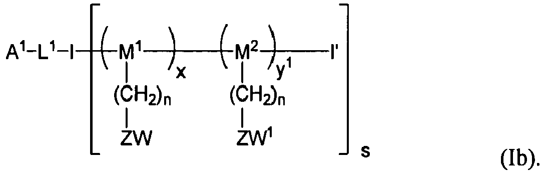

In formula Ib, functional agent A¹ can be a drug, therapeutic protein or a targeting agent. Linker L¹ can be a cleavable linker, such as when attached to a drug or therapeutic protein to facilitate release of the drug or therapeutic protein. Alternatively, linker L¹ can be a non-cleavable linker.

When multiple comonomers M² are present, each comonomer M² can have a different zwitterionic group attached. For example, the high MW polymer can have formula Ic: 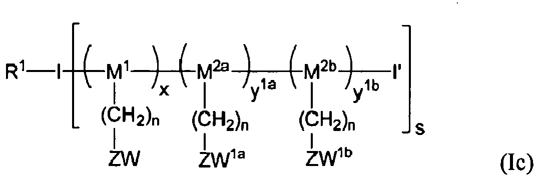 wherein each of ZW^{1a} and ZW^{1b} are as defined above for ZW, and each of y^{1a} and y^{1b} are as defined above for y¹.

In some embodiments, the high MW polymers have linking groups LG linked to the initiator fragment I, such as shown in the structures below:

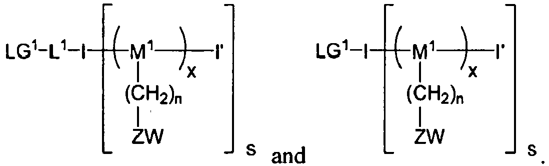

In some embodiments, the high MW polymers of the present invention can be modified via a subsequent polymerization with one or more additional monomers. For example, in formula Ic above, monomers M¹ and M^{2a} can be copolymerized in a first polymerization, and monomer M^{2b} can be polymerized in a second polymerization. A block copolymer would be formed having two blocks, the first block being a high MW polymer of M¹ and M^{2a}, and the second block a homopolymer of M^{2b}. Alternatively, following polymerization of monomers M¹ and M^{2a}, monomer M^{2b} can be copolymerized with monomer M^{2c}, thus forming a block copolymer where the first block is a high MW polymer of M¹ and M^{2a}, and the second block is a high MW polymer of M^{2b} and M^{2c}. Additional polymer structures can be prepared by copolymerizing monomers M¹, M^{2a} and M^{2b} in a first polymerization, followed by copolymerization of monomers M^{2c}, M^{2d}, and others, in a second copolymerization. Additional blocks can be prepared by yet a third polymerization using additional monomers. Such polymers provide blocks of copolymers that can have different properties, drugs and functional agents.

In some embodiments, the polymer can be 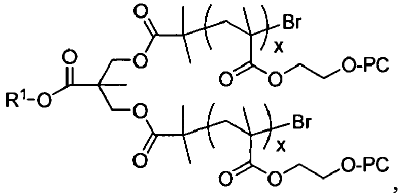 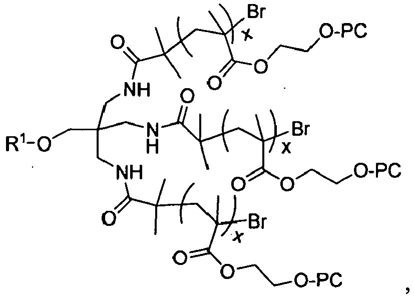 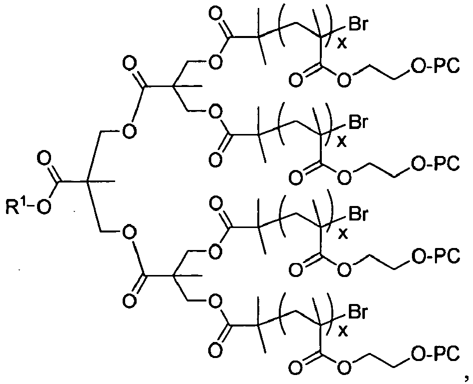 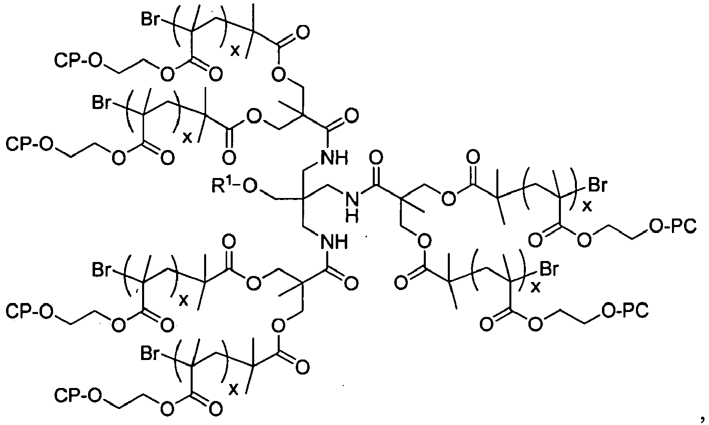 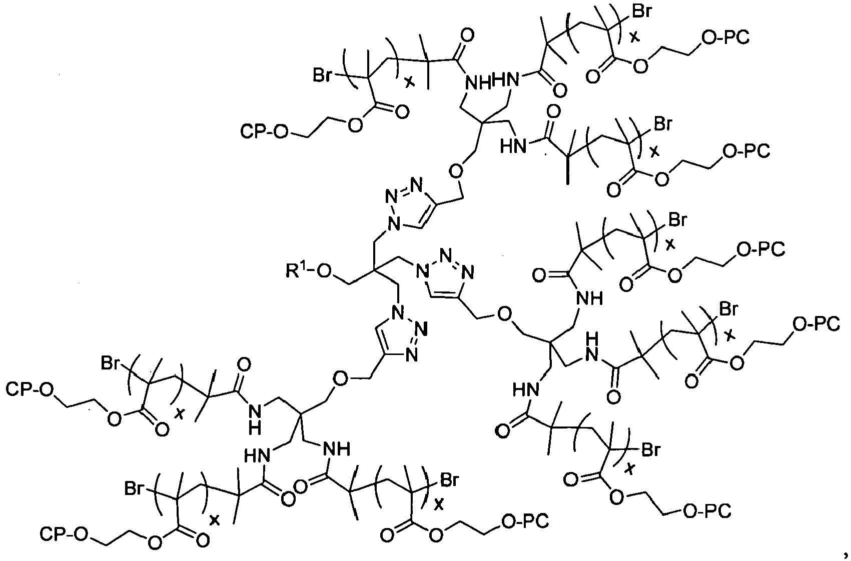 or 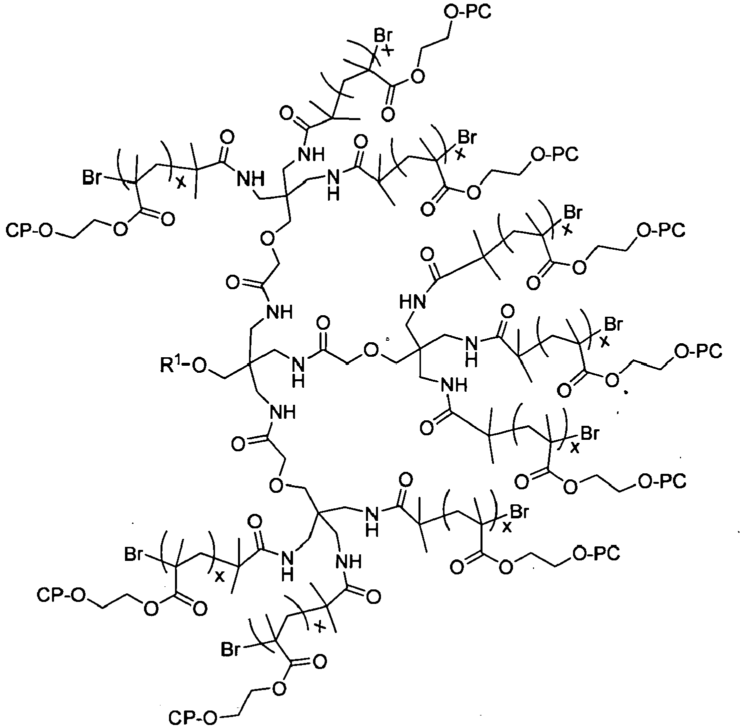 wherein PC is phosphorylcholine.

In some other embodiments, the polymer can be 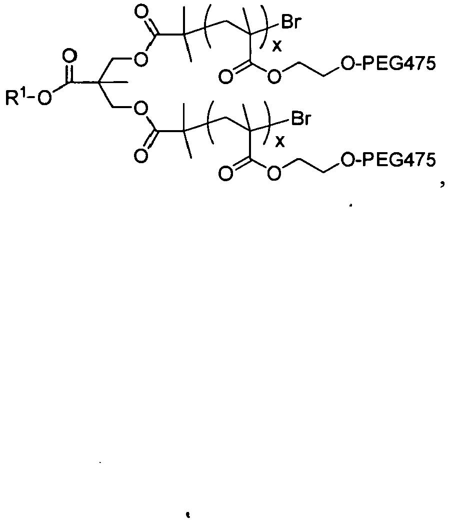 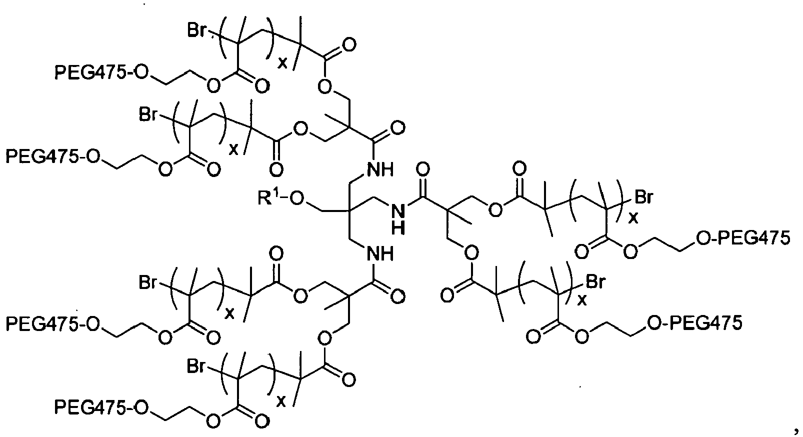 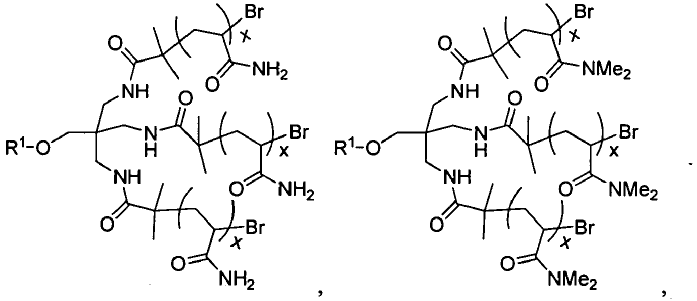 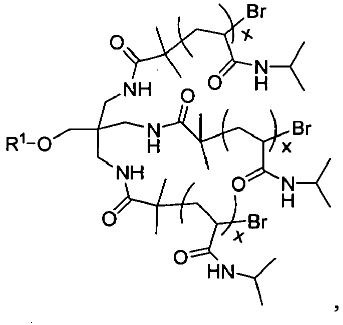 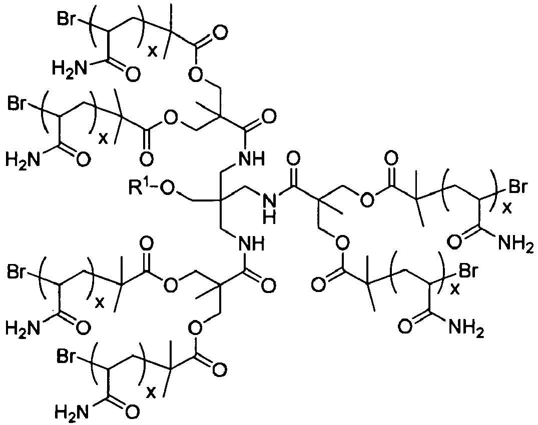 and

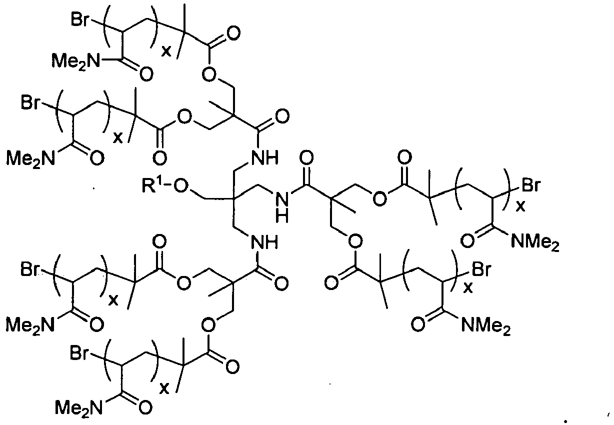

In some embodiments, R¹ is L³-A¹, LG¹ or L³-LG¹; A¹ is a drug, an antibody, an antibody fragment, a single domain antibody, an avimer, an adnectin, diabodies, a vitamin, a cofactor, a polysaccharide, a carbohydrate, a steroid, a lipid, a fat, a protein, a peptide, a polypeptide, a nucleotide, an oligonucleotide, a polynucleotide, a nucleic acid. a radiolabel, a contrast agent, a fluorophore or a dye; L³ is -(CH₂CH₂O)₁₋₁₀-; and LG¹ is maleimide, acetal, vinyl, allyl, aldehyde, -C(O)O-C₁₋₆ alkyl, hydroxy, diol, ketal, azide, alkyne, carboxylic acid, or succinimide. In other embodiments, each LG¹ can be hydroxy, carboxy, vinyl, vinyloxy, allyl, allyloxy, aldehyde, azide, ethyne, propyne, propargyl, -C(O)O-C₁₋₆ alkyl,

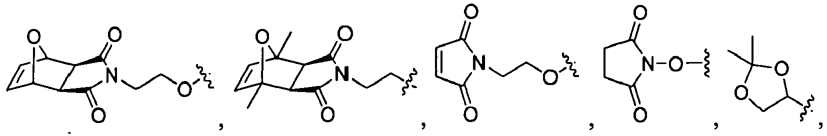

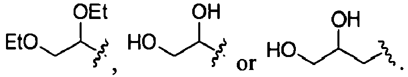

### A. Initiators

The high MW polymers of the present invention are polymerized using any suitable initiator. Initiators useful in the present invention can be described by the formula: I-(I')ₘ, where subscript m is an integer from 1 to 100. The initiator fragment I can be any group that initiates the polymerization. The radical scavenger I' can be any group that will reversibly terminate the growing polymer chain. The radical scavenger I' can be a halogen such as bromine, allowing the end of the polymer to be functionalized after polymerization. In some embodiments, the radical scavenger I' is referred to as an end group. In addition, the initiator fragment I can optionally be functionalized with an R¹ group that can include a variety of functional groups to tune the functionality of the high MW polymer.

Initiators useful in the present invention can have a single radical scavenger I', or any suitable number of branches such that there are multiple radical scavengers I' each capable of reversibly terminating a growing polymer chain. When the initiator fragment I is branched and is capable of initiating multiple polymer chains, subscript m is greater than one such that there are as many radical scavengers I' as there are growing polymer chains.

The polymer of the present invention can have a plurality of polymer arms. For example, the polymer can have from 1 to about 100 polymer arms, or from about 1 to about 50 polymer arms, or from about 1 to about 20 polymer arms, or from 1 to about 10 polymer arms, or from 2 to about 10 polymer arms, or from about 1 to about 8 polymer arms, or from about 2 to about 8 polymer arms, or from 1 to about 4 polymer arms, or from about 2 to about 4 polymer arms. The polymer can also have any sutiable polydispersity index (PDI), as measured by the weight average molecular weight (M_{w}) divided by the number average molecular weight (Mₙ), where a PDI of 1.0 indicates a perfectly monodisperse polymer. For example, the PDI can be less than about 2.0, or less than about 1.9, 1.8, 1.7, 1.6, 1.5, 1.4, 1.3, 1.2 or 1.1.

In some embodiments, the initiator fragment is linked to 1 polymer arm, and the polymer has a polydispersity index of less than about 1.5. In other embodiments, the initiator fragment is linked to the proximal end of from 2 to about 100 polymer arms. In some other embodiments, the polymer has a polydispersity index of less than about 2.0. In still other embodiments, the initiator fragment is linked to the proximal end of 2 polymer arms. In yet other embodiments, the initiator fragment is linked to the proximal end of 4 polymer arms. In other embodiments, the initiator fragment can be linked to the proximal end of 2, 3, 4, 5, 6, 8, 9 or 12 polymer arms.

Pseudo-branched polymers can also be obtained by linking multiple linear, unbranched, polymers of the present invention to a single functional agent such that the polymers are in close proximity. The proximity can be obtained by linking the polymers to nearby points on the functional agent, cysteines on a protein, for example. Alternatively, the proximity can be afforded by the structure of the functional agent, a protein for example, such that polymers attached to disparate regions of the protein are brought into close proximity due to the folding and secondary and tertiary structure of the protein. The close proximity of the two polymers of the present invention on a single functional agent, regardless of how the proximity is achieved, can impart properties similar to that of a polymer of the present invention having a plurality of polymer arms.

The bond between initiator fragment I and radical scavenger I' is labile, such that during the polymerization process monomers M¹ and comonomers M² are inserted between initiator fragment I and radical scavenger I'. For example, during a free radical polymerization, such as ATRP, initiator fragment I and radical scavenger I' dissociate, as shown in Figure 1, to form radicals of I and I'. The radical of initiator fragment I then reacts with the monomers in solution to grow the polymer and forms a propagating polymer radical (species A and species C of Figure 1). During the polymerization process, the radical of the radical scavenger I' will reversibly react with the propagating polymer radical to temporarily stop polymer growth. The bond between the monomer and the radical savenger I' is also labile, such that the bond can cleave and allow the propagating polymer radical to react with additional monomer to grow the polymer. The end result of the polymerization process is that initiator fragment I is at one end of the polymer chain and radical scavenger I' is at the opposite end of the polymer chain.

The radical of initiator fragment I is typically on a secondary or tertiary carbon, and can be stabilized by an adjacent carbonyl carbon. The radical scavenger I' is typically a halogen, such as bromine, chlorine or iodine. Together, initiator fragment I and radical scavenger I' form the initiator I¹ useful in the preparation of the high MW polymers of the present invention.

A broad variety of initiators can be used to prepare the high MW polymers of the invention, including a number of initiators set forth in US 6,852,816 (incorporated herein by reference). In some embodiments, the initiators employed for ATRP reactions to prepare high MW polymers of the invention are selected from alkanes, cycloalkanes, alkyl carboxylic acids or esters thereof, cycloalkylcarboxylic acids or esters thereof, ethers and cyclic alkyl ethers, alkyl aryl groups, alkyl amides, alkyl-aryl carboxylic acids and esters thereof, and also bearing one radical scavenger I' where unbranched high MW polymers are prepared, and more than one radical scavenger I' where branched molecules are prepared.

Radical scavengers I' useful in the present invention include, but are not limited to, halogens, such as Br, Cl and I, thiocyanate (-SCN) and isothiocyanate (-N=C=S). Other groups are useful for the radical scavenger I' of the present invention. In some embodiments, the radical scavenger I' is bromine.

Initiators employed for ATRP reactions can be hydroxylated. In some embodiments, the initiators employed for ATRP reactions to prepare high MW polymers of the invention are selected from alkanes, cycloalkanes, alkyl carboxylic acids or esters thereof, cycloalkylcarboxylic acids or esters thereof, ethers, cyclic alkyl ethers, alkyl aryl groups, alkyl amides, alkyl-aryl carboxylic acids and esters thereof, bearing a hydroxyl group, and also bearing one radical scavenger I' where unbranched high MW polymers are to be prepared, or alternatively, more than one radical scavenger I' where branched molecules are to be prepared.

Initiators employed for ATRP reactions can bear one or more amine groups. In some embodiments, the initiators employed for ATRP reactions to prepare high MW polymers of the invention are alkanes, cycloalkanes, alkyl carboxylic acids or esters thereof, cycloalkylcarboxylic acids or esters thereof, ethers, cyclic alkyl ethers alkyl aryl groups, alkyl amides, alkyl-aryl carboxylic acids and esters thereof, bearing an amine group and also bearing one radical scavenger I' where unbranched high MW polymers are to be prepared, or alternatively, more than one radical scavenger I' where branched molecules are to be prepared.

Alkylcarboxylic acids, including alkyl dicarboxylic acids, having at least one radical scavenger I', and substituted with amino or hydroxy groups can also be employed as initiators. In some embodiments of the invention where ATRP is employed to prepare high MW polymers of the present invention, the initiators can be alkylcarboxylic acids bearing one or more halogens selected from chlorine and bromine.

Alkanes substituted with two or more groups selected from -COOH, -OH and -NH₂, and at least one radical scavenger I', can also be employed as initiators for the preparation of high MW polymers where ATRP is employed to prepare high MW polymers of the present invention.

Initiators can also contain one or more groups including, but not limited to, -OH, amino, monoalkylamino, dialkylamino, -O-alkyl, -COOH, -COO-alkyl, or phosphate groups (or protected forms thereof).

A broad variety of initiators are commercially available, for example bromoacetic acid N-hydroxysuccinimide ester available from Sigma-Aldrich (St. Louis, MO). Suitably protected forms of those initiators can be prepared using standard methods in the art as necessary.

Other initiators include thermal, redox or photo initiators, including, for example, alkyl peroxide, substituted alkyl peroxides, aryl peroxides, substituted aryl peroxides, acyl peroxides, alkyl hydroperoxides, substituted aryl hydroperoxides, aryl hydroperoxides, substituted aryl hydroperoxides, heteroalkyl peroxides, substituted heteroalkyl peroxides, heteroalkyl hydroperoxides, substituted heteroalkyl hydroperoxides, heteroaryl peroxides, substituted heteroaryl peroxides, heteroaryl hydroperoxides, substituted heteroaryl hydroperoxides, alkyl peresters, substituted alkyl peresters, aryl peresters, substituted aryl peresters, azo compounds and halide compounds. Specific initiators include cumene hydroperoxide (CHP), tert-butyl hydroperoxide (TBHP), tert-butyl perbenzoate, (TBPB), sodium carbonateperoxide, benzoyl peroxide (BPO), lauroyl peroxide (LPO), methylethyl ketone 45%, potassium persulfate, ammonium persulfate, 2,2-azobis(2,4-dimethyl-valeronitrile), 1,1-azobis(cyclo-hexanecarbonitrile), 2,2-azobis(N,N-dimethyleneisobutyramidine) dihydrochloride, and 2,2-azobis (2-amido-propane) dihydrochloride. Redox pairs such as persulfate/sulfite and Fe (2+) peroxide or ammonium persulfate and N,N,N'N'-tetramethylethylenediamine (TEMED).

Still other initiators useful for preparing the high MW polymers of the present invention, are branched. Suitable initiators having a single branch point include the following: 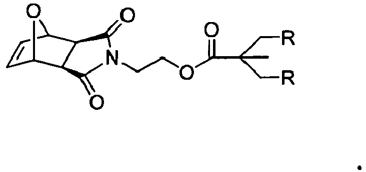 where radical R can be any of the following:

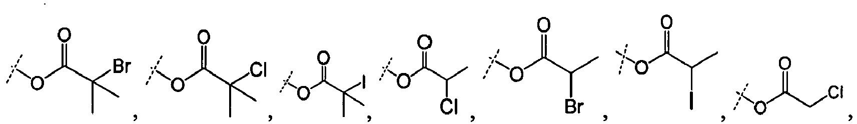

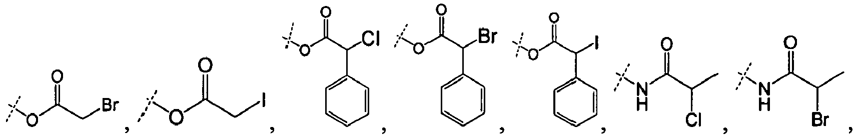

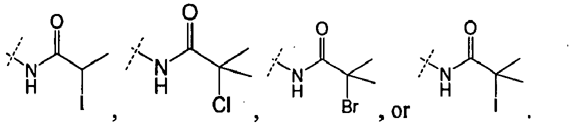

In some embodiments, the initiator can be: 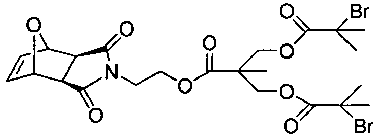 which is a protected maleimide that can be deprotected after polymerization to form the maleimide for reaction with additional functional groups.

Additional branched initiators include, but are not limited to, the following, where radical R is as defined above:

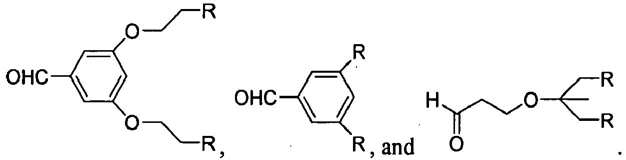

In some embodiments, the branched initiators include, but are not limited to, the following:

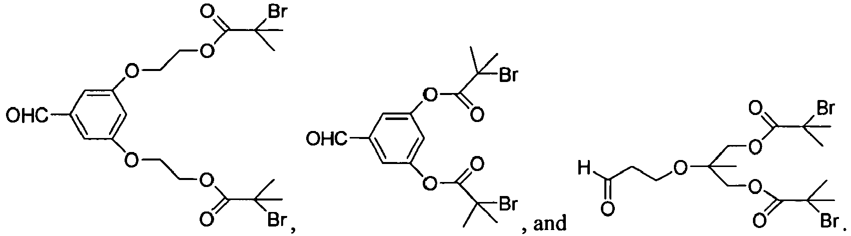

Other branched initiators useful for preparing the high MW polymers of the present invention include the following: 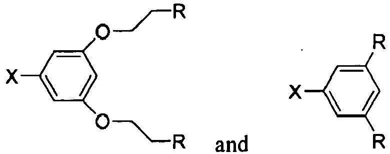 where radical R is as defined above, and radical X can be CHO, SO₂Cl, SO₂CH=CH₂, NHCOCH₂I, N=C=O and N=C=S, among others. Additional X groups can include the following:

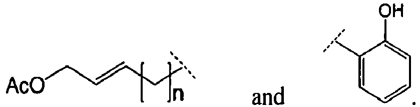

Still other initiators include, but are not limited to, the following:

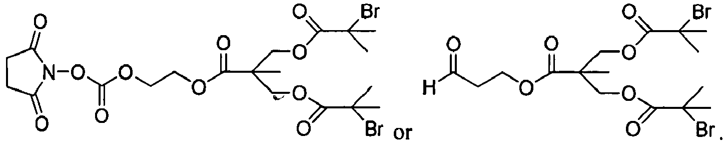

In other embodiments, the initiator can have several branch points to afford a plurality of polymer arms, such as: 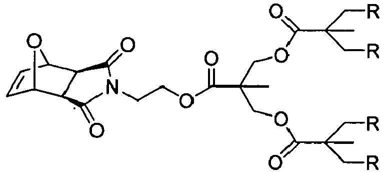 where radical R is as defined above. In some other embodiments, the initiator can have the following structure:

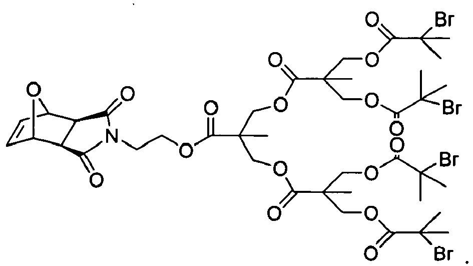

In some other embodiments, the initiator can have the following structures: 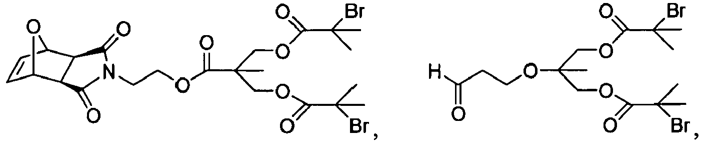 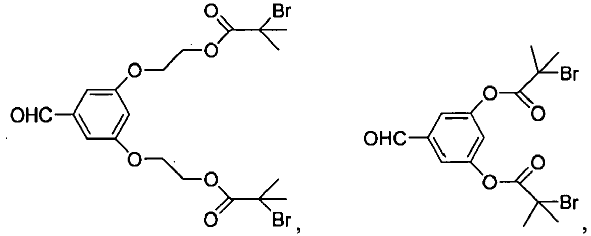 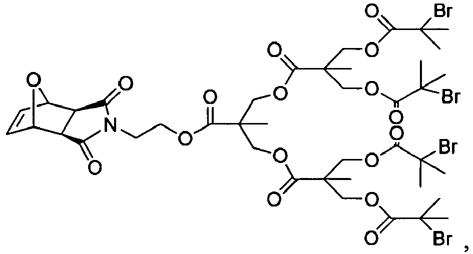 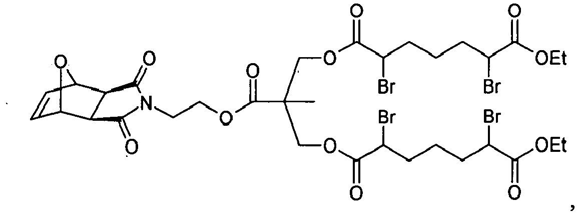 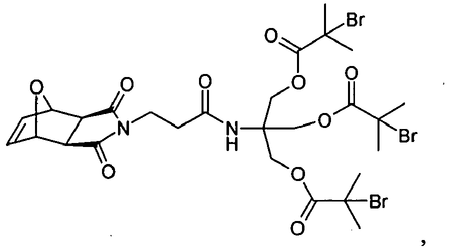 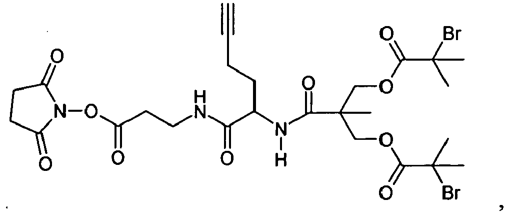 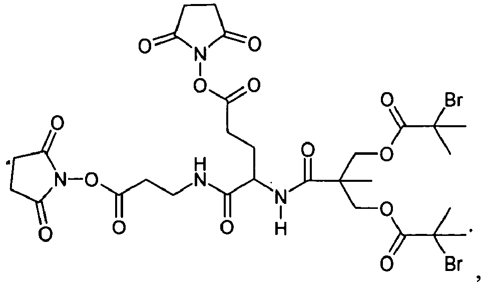 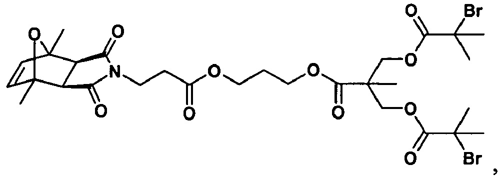 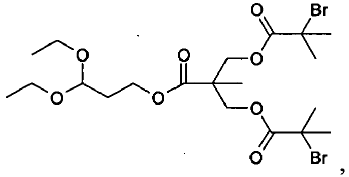 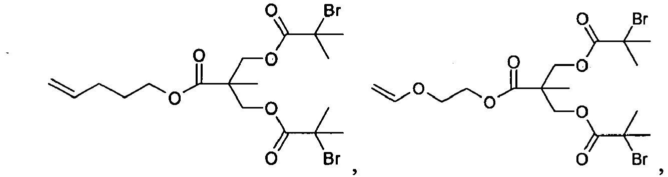 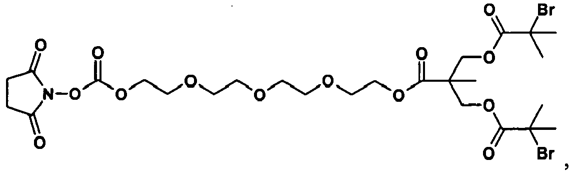 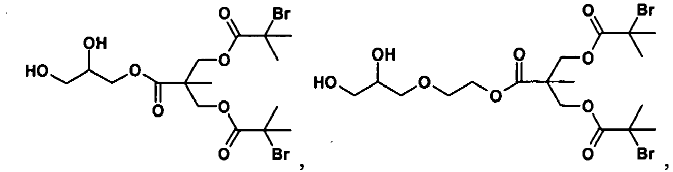 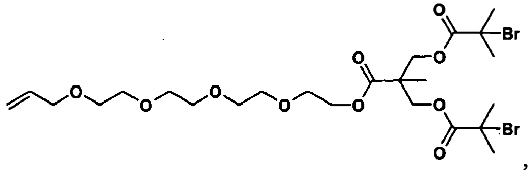 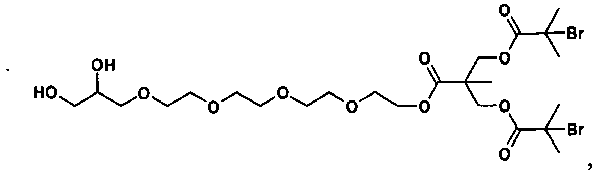 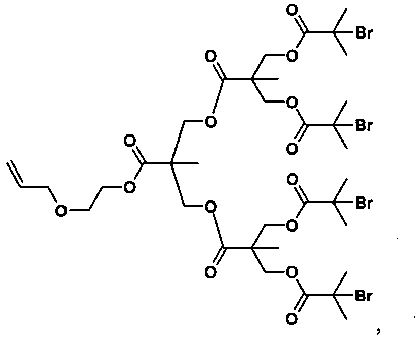 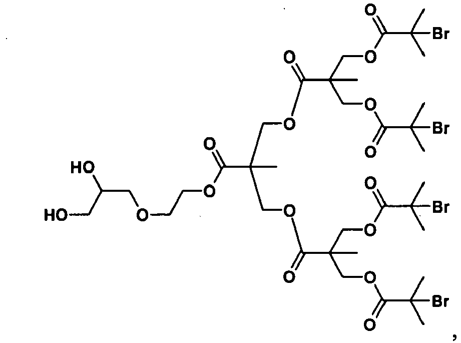 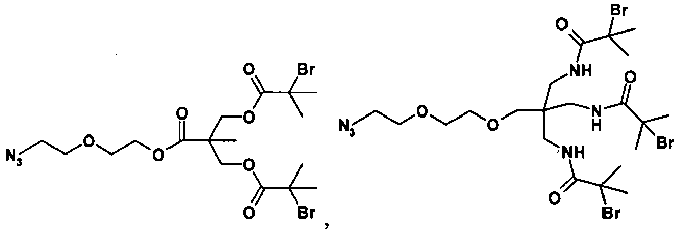 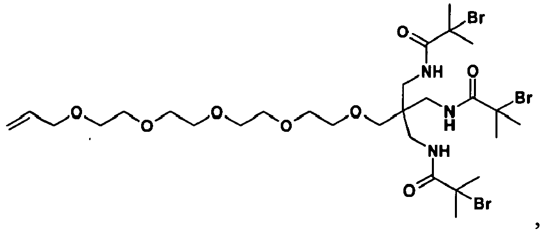 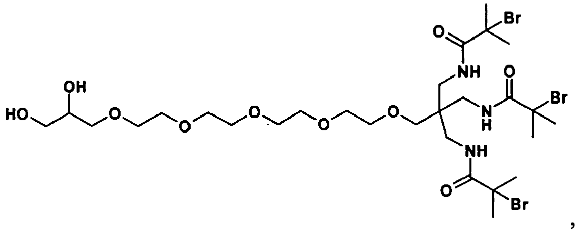 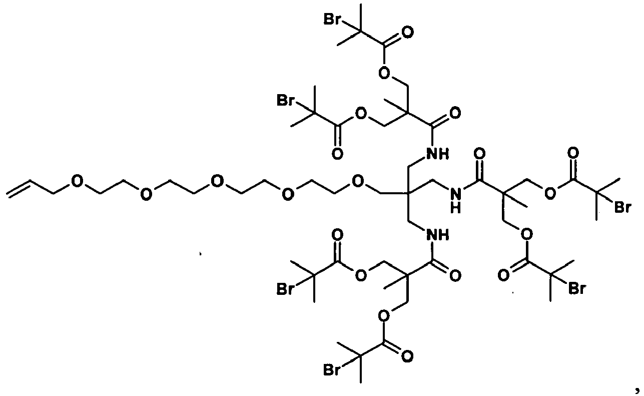 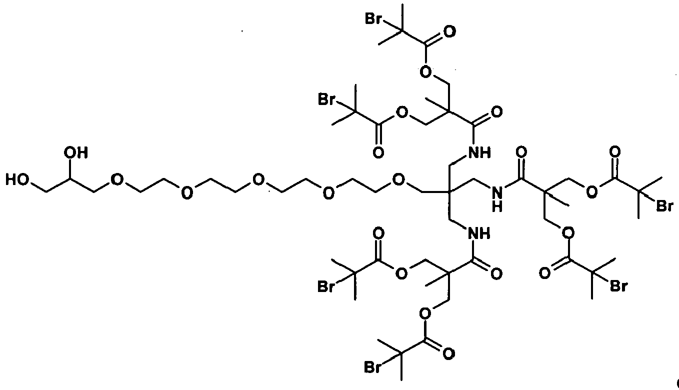 or

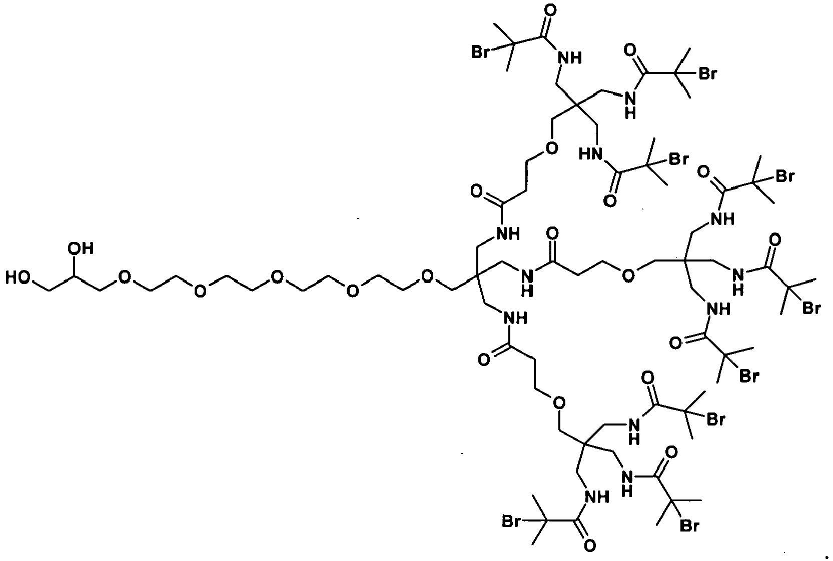

As described above, the initiator can be added to the polymerization mixture separately, or can be incorporated into another molecule, such as a monomer (hyperbranched structure) or a polymer fragment (such as graft copolymers). Initiation of the polymerization can be accomplished by heat, UV light, or other methods known to one of skill in the art.

In some embodiments, the initiator I-I' of the present invention has the formula:

(F)ᵣ-Sp¹-C-Sp²-I'

where the initiator fragment I corresponds to F-Sp¹-C-Sp². Each radical F is a functional group for reaction with a functional agent or linking group of the present invention. Radical r is from 1 to 10. Radicals Sp¹ and Sp² are spacers and can be any suitable group for forming a covalent bond, such as C₁₋₆ alkyl, aryl or heteroaryl. Radical C can be any core providing one or a plurality of points for linking to one or more spacers, Sp² (which can be the same or different), and one or more radical scavengers, I', and providing one or a plurality of points for linking to one or more spacers, Sp¹ (which can be the same or different), and one or more functional groups, F (which can be the same or different). Core C can be any suitable structure, such as a branched structure, a crosslinked structure including heteroatoms, such as silsesquiloxanes, and a linear, short polymer with multiple pendant functional groups. In addition, core C can be attached to the one or more Sp¹ and Sp² spacers by any suitable group for forming a covalent bond including, but not limited to, esters, amides, ethers, and ketones. Radical scavenger I' is a radically transferable atom or group such as, but not limited to, a halogen, Cl, Br, I, OR¹⁰, SR¹¹, SeR¹¹, OC(=O)R¹¹, OP(=O)R¹¹, OP(=O)(OR¹¹)₂, O-(R¹¹)₂, S-C(=S)N(R¹¹)₂, CN, NC, SCN, CNS, OCN, CNO, N₃, OH, O, C1-C6-alkoxy, (SO₄), PO₄, HPO₄, H₂ PO₄, triflate, hexafluorophosphate, methanesulfonate, arylsulfonate, carboxylic acid halide. R¹⁰ is an alkyl of from 1 to 20 carbon atoms or an alkyl of from 1 to 20 carbon atoms in which each of the hydrogen atoms may be replaced by a halide, alkenyl of from 2 to 20 carbon atoms, alkynyl of from 2 to 10 carbon atoms, phenyl, phenyl substituted with from 1 to 5 halogen atoms or alkyl groups with from 1 to 4 carbon atoms, aralkyl, aryl, aryl substituted alkyl, in which the aryl group is phenyl or substituted phenyl and the alkyl group is from 1 to 6 carbon atoms, and R¹¹ is aryl or a straight or branched C₁-C₂₀ alkyl group or where an N(R¹¹)₂ group is present, the two R¹¹ groups may be joined to form a 5-, 6- or 7-member heterocyclic ring. Spacer Sp¹ covalently links functional group F and core C while spacer Sp² covalently links core C and radical scavenger I'.

In other embodiments, the initiator of the present invention has the formula: 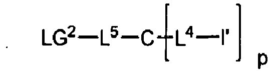 wherein each I' is independently selected from halogen, -SCN, or -NCS. L⁴ and L⁵ are each independently a bond or a linker, such that one of L⁴ and L⁵ is a linker. C is a bond or a core group. LG² is a linking group. And subscript p is from 1 to 100, wherein when subscript p is 1, C is a bond, and when subscript p is from 2 to 100, C is a core group. In some other embodiments, the initiator has the formula: 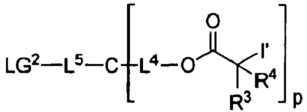 wherein each R³ and R⁴ is independently selected H, CN or C₁₋₆ alkyl.

### B. Monomers

Monomers useful for preparing the high MW polymers of the present invention include any monomer capable of radical polymerization. Typically, such monomers have a vinyl group. Suitable monomers include, but are not limited to, acrylate, methacrylate, acrylamide, methacrylamide, styrene, vinyl-pyridine, vinyl-pyrrolidone and vinyl esters such as vinyl acetate monomers. Monomers useful in the present invention include a hydrophilic group. The hydrophilic group of the present invention can be any suitable hydrophilic group. For example, the hydrophilic group can include zwitterionic groups and hydrophilic polymers. In some embodiments, each hydrophilic group includes a zwitterionic group. Zwitterion groups of the present invention include any compound having both a negative charge and a positive charge. Groups having a negative charge and suitable for use in the zwitterions of the present invention include, but are not limited to, phosphate, sulfate, other oxoanions, etc. Groups having a positive charge and suitable for use in the zwitterions of the present invention include, but are not limited to, ammonium ions. In some embodiments, the zwitterion can be phosphorylcholine. Other zwitterions useful in the present invention include those described in WO1994016748 and WO1994016749 (incorporated herein by reference). Hydrophilic polymers useful in the present invention include polyethyleneoxide, polyoxazoline, cellulose, dextran, and other polysaccharide polymers. One of skill in the art will appreciate that other hydrophilic polymers are useful in the present invention.

Other hydrophilic groups include, but are not limited to, hydroxy, amine, carboxylic acid, amide, sulfonate and phosphonate. Monomers useful in the present invention that include such hydrophilic groups include, but are not limited to, acrylamide, N-isopropylacrylamide (NiPAAM) and other substituted acrylamide, acrylic acid, and others.

Monomers, M¹, containing the zwitterionic moiety, ZW, include, but are not limited to, the following:

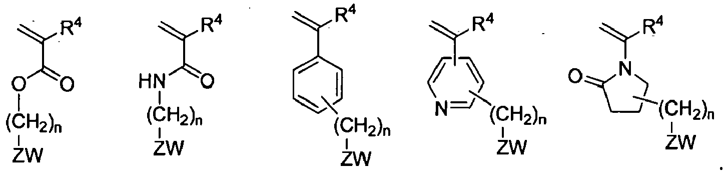

Other monomers are well-known to one of skill in the art, and include vinyl acetate and derivatives thereof.

In some embodiments, the hydrophilic group can be a zwitterionic group. In some embodiments, the monomer can be 2-(methacryloyloxyethyl)-2'-(trimethylammoniumethyl) phosphate (HEMA-PC). In some other embodiments, the monomer can be 2-(acryloyloxyethyl)-2'-(trimethylammoniumethyl) phosphate.

### C. Linkers

The high MW polymers of the present invention can also incorporate any suitable linker L. The linkers L³ provide for attachment of the functional agents to the initiator fragment I and the linkers L¹ and L² provide for attachment of the zwitterionic groups to the comonomers M¹ and M². The linkers can be cleavable or non-cleavable, homobifunctional or heterobifunctional. Other linkers can be both heterobifunctional and cleavable, or homobifunctional and cleavable.

Cleavable linkers include those that are hydrolyzable linkers, enzymatically cleavable linkers, pH sensitive linkers, disulfide linkers and photolabile linkers, among others. Hydrolyzable linkers include those that have an ester, carbonate or carbamate functional group in the linker such that reaction with water cleaves the linker. Enzymatically cleavable linkers include those that are cleaved by enzymes and can include an ester, amide, or carbamate functional group in the linker. pH sensitive linkers include those that are stable at one pH but are labile at another pH. For pH sensitive linkers, the change in pH can be from acidic to basic conditions, from basic to acidic conditions, from mildly acidic to strongly acidic conditions, or from mildly basic to strongly basic conditions. Suitable pH sensitive linkers are known to one of skill in the art and include, but are not limited to, ketals, acetals, imines or imminiums, siloxanes, silazanes, silanes, maleamates-amide bonds, ortho esters, hydrazones, activated carboxylic acid derivatives and vinyl ethers. Disulfide linkers are characterized by having a disulfide bond in the linker and are cleaved under reducing conditions. Photolabile linkers include those that are cleaved upon exposure to light, such as visible, infrared, ultraviolet, or electromagnetic radiation at other wavelengths.

Other linkers useful in the present invention include those described in U.S. Patent Application Nos. 2008/0241102 (assigned to Ascendis/Complex Biosystems) and 2008/0152661 (assigned to Mirus), and International Patent Application Nos. WO 2004/010957 and 2009/117531 (assigned to Seattle Genetics) and 01/24763, 2009/134977 and 2010/126552 (assigned to Immunogen) (incorporated in their entirety herein). Mirus linkers useful in the present invention include, but are not limited to, the following:

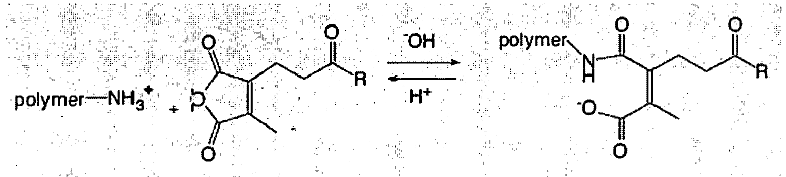

Other linkers include those described in Bioconjugate Techniques, Greg T. Hermanson, Academic Press, 2d ed., 2008 (incorporated in its entirety herein), and those described in Angew. Chem. Int. Ed. 2009, 48, 6974-6998 (Bertozzi, C.R. and Sletten, E.M) (incorporated in its entirety herein).

In some embodiments, linkers L¹, L² and L³ can have a length of up to 30 atoms, each atom independently C, N, O, S, and P. In other embodiments, the linkers L¹ and L² can be any of the following: -C₁₋₁₂ alkyl-, -C₃₋₁₂ cycloalkyl-, -(C₁₋₈alkyl)-(C₃₋₁₂ cycloalkyl)-(C₀₋₈ alkyl)-, -(CH₂)₁₋₁₂O-, (-(CH₂)₁₋₆-O-(CH₂)₁₋₆-)₁₋₁₂-, (-(CH₂)₁₋₄-NH-(CH₂)₁₋₄)₁₋₁₂-, (-(CH₂)₁₋₄-O-(CH₂)₁₋₄)₁₋₁₂-O-, (-(CH₂)₁₋₄-O-(CH₂)₁₋₄-)₁₋₁₂O-(CH₂)₁₋₁₂-, -(CH₂)₁₋₁₂-(C=O)-O-, -(CH₂)₁₋₁₂-O-(C=O)-, -(phenyl)-(CH₂)₁₋₃-(C=O)-O-, -(phenyl)-(CH₂)₁₋₃-(C=O)-NH-, -(C₁₋₆alkyl)-(C=O)-O-(C₀₋₆ alkyl)-, -(CH₂)₁₋₁₂-(C=O)-O-(CH₂)₁₋₁₂-, -CH(OH)-CH(OH)-(C=O)-O- -CH(OH)-CH(OH)-(C=O)-NH-, -S-maleimido-(CH₂)₁₋₆-, -S-maleimido-(C₁₋₃ alkyl)-(C=O)-NH-, -S-maleimido-(C₁₋₃alkyl)-(C₅₋₆cycloalkyl)-(C₀₋₃alkyl)-, -(C₁₋₃ alkyl)-(C₅₋₆cycloalkyl)-(C₀₋₃alkyl)-(C=O)-O-, -(C₁₋₃alkyl)-(C₅₋₆cycloalkyl)-(C₀₋₃ alkyl)-(C=O)-NH-, -S-maleimido-(C₀₋₃alkyl)-phenyl-(C₀₋₃alkyl)-, -(C₀₋₃ alkyl)-phenyl-(C=O)-NH-, -(CH₂)₁₋₁₂-NH-(C=O)-, -(CH₂)₁₋₁₂-(C=O)-NH-, -(phenyl)-(CH₂)₁₋₃-(C=O)-NH-, -S-(CH₂)-(C=O)-NH-(phenyl)-, -(CH₂)₁₋₁₂-(C=O)-NH-(CH₂)₁₋₁₂-, -(CH₂)₂-(C=O)-O-(CH₂)₂-O-(C=)-(CH₂)₂-(C=O)-NH-, -(C₁₋₆alkyl)-(C=O)-N-(C₁₋₆alkyl)-, acetal, ketal, acyloxyalkyl ether, -N=CH-, -(C₁₋₆ alkyl)-S-S-(C₀₋₆alkyl)-, -(C₁₋₆alkyl)-S-S-(C₁₋₆alkyl)-(C=O)-O-, -(C₁₋₆alkyl)-S-S-(C₁₋₆ alkyl)-(C=O)-NH-, -S-S-(CH₂)₁₋₃-(C=O)-NH-(CH₂)₁₋₄-NH-(C=O)-(CH₂)₁₋₃-, -S-S-(C₀₋₃ alkyl)-(phenyl)-, -S-S-(C₁₋₃-alkyl)-(phenyl)-(C=O)-NH-(CH₂)₁₋₅-, -(C₁₋₃ alkyl)-(phenyl)-(C=O)-NH-(CH₂)₁₋₅-(C=O)-NH-, -S-S-(C₁₋₃-alkyl)-, -(C₁₋₃-alkyl)-(phenyl)-(C=O)-NH-, -O-(C₁-C₆alkyl)-S(O₂)-(C₁₋₆alkyl)-O-(C=O)-NH-, -S-S-(CH₂)₁₋₃-(C=O)-, -(CH₂)₁₋₃-(C=O)-NH-N=C-S-S-(CH₂)₁₋₃-(C=O)-NH-(CH₂)₁₋₅-, -(CH₂)₁₋₃-(C=O)-NH-(CH₂)₁₋₅-(C=O)-NH-, -(CH₂)₀₋₃-(heteroaryl)-(CH₂)₀₋₃-, -(CH₂)₀₋₃-phenyl-(CH₂)₀₋₃-, -N=C(R)-, -(C₁₋₆alkyl)-C(R)=N-(C₁₋₆alkyl)-, -(C₁₋₆ alkyl)-(aryl)-C(R)=N-(C₁₋₆alkyl)-, -(C₁₋₆alkyl)-C(R)=N-(aryl)-(C₁₋₆alkyl)-, and -(C₁₋₆ alkyl)-O-P(O)(OH)-O-(C₀₋₆alkyl)-, wherein R is H, C₁₋₆ alkyl, C₃₋₆ cycloalkyl, or an aryl group having 5-8 endocyclic atoms.

In some other embodiments, linkers L¹, L² and L³ can be any of the following: -C₁-C₁₂ alkyl-, -C₃-C₁₂ cycloalkyl-, (-(CH₂)₁₋₆-O-(CH₂)₁₋₆-)₁₋₁₂-, (-(CH₂)₁₋₄-NH-(CH₂)₁₋₄)₁₋₁₂-, -(CH₂)₁₋₁₂O-, (-(CH₂)₁₋₄-O-(CH₂)₁₋₄)₁₋₁₂-O-, -(CH₂)₁₋₁₂-(CO)-O-, -(CH₂)₁₋₁₂-(CO)-NH-, -(CH₂)₁₋₁₂-O-(CO)-, -(CH₂)₁₋₁₂-NH-(CO)-, (-(CH₂)₁₋₄-O-(CH₂)₁₋₄)₁₋₁₂-O-(CH₂)₁₋₁₂-, -(CH₂)₁₋₁₂-(CO)-O-(CH₂)₁₋₁₂-, -(CH₂)₁₋₁₂-(CO)-NH-(CH₂)₁₋₁₂-, -(CH₂)₁₋₁₂-O-(CO)-(CH₂)₁₋₁₂-, -(CH₂)₁₋₁₂-NH-(CO)-(CH₂)₁₋₁₂-, -(C₃-C₁₂ cycloalkyl)-, -(C₁-C₈alkyl)-(C₃-C₁₂ cycloalkyl)-, -(C₃-C₁₂ cycloalkyl)-(C₁₋₈alkyl)-, -(C₁₋₈alkyl)-(C₃-C₁₂ cycloalkyl)-(C₁₋₈alkyl)-, and -(CH₂)₀₋₃-aryl-(CH₂)₀₋₃-.

In still other embodiments, each of linkers L¹, L² and L³ is a cleavable linker independently selected from hydrolyzable linkers, enzymatically cleavable linkers, pH sensitive linkers, disulfide linkers and photolabile linkers.

Other linkers useful in the present invention include self-immolative linkers. Useful self-immolative linkers are known to one of skill in the art, such as those useful for antibody drug conjugates. Exemplary self-immolative linkers are described in U.S. Patent No. 7,754,681.

### D. Linking Groups LG

The linkers and functional agents of the present invention can react with a linking group on the initiator fragment I to form a bond. The linking groups LG of the present invention can be any suitable functional group capable of forming a bond to another functional group, thereby linking the two groups together. For example, linking groups LG useful in the present invention include those used in click chemistry, maleimide chemistry, and NHS-esters, among others. Linking groups involved in click chemistry include, but are not limited to, azides and alkynes that form a triazole ring via the Huisgen cycloaddition process (see U.S. Patent No. 7,375,234, incorporated herein in its entirety). The maleimide chemistry involves reaction of the maleimide olefin with a nucleophile, such as -OH, -SH or -NH₂, to form a stable bond. Other linking groups include those described in Bioconjugate Techniques, Greg T. Hermanson, Academic Press, 2d ed., 2008 (incorporated in its entirety herein).

Some non-limiting examples of the reaction of the linking groups and some groups typically found or introduced into functional agents are set forth in Table I.

**Table I**

| **Illustrative Groups that may react with a linking group (LG)** | **Exemplary Reactive Linking Groups (shown as appended to -X)** | **Product Y-X** |
|---|---|---|
| Y-COOH | HO-X (hydroxyl or activated forms thereof (e.g., tresylate, mesylate etc.)) | Y-C(=O)O-X |
| Y-COOH Y-SH | HS-X (thiol) | Y-C(=O)S-X Y-S-S-X |
| Y-SH | R'-S-S-X (disulfide) | Y-S-S-X |
| Y-SH | (pyridyl)-S-S-X (dithiopyridyl) | Y-S-S-X |
| Y-NH₂ | H(O=)C-X aldehyde | Y-N=CH-X or Y-NH-CH₂-X following reduction |
| Y-NH₂ | (HO)₂HC-X aldehyde hydrate | Y-N=CH-X or Y-NH-CH₂-X following reduction |
| Y-NH₂ | (R'O)₂CH-X or | Y-N=CH-X or Y-NH-CH-X following reduction |
| | 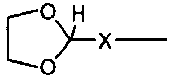 | |
| | acetal | |
| Y-NH₂ | R'OCH(OH)-X or hemiacetal | Y-N=CH-X or Y-NH-CH-X following reduction |
| Y-NH₂ | R'(O=)C-X ketone | Y-N=CR'-X or Y-NH-C(R')H-X following reduction |
| Y-NH₂ | (R'O)₂C(R')-X or | Y-N=C(R')-X or Y-NH-C(R')H-X following reduction |
| | 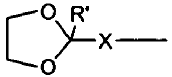 | |
| | ketal | |
| Y-NH₂ | R'OC(R')(OH)-X hemiketal | Y-N=C(R')-X or Y-NH-C(R')H-X following reduction |
| Y-NH₂ | R'(S=)C-X ketone thione (thioketone) | Y-N=C(R')-X or Y-NH-C(R')H-X following reduction |
| Y-NH₂ | (R'O)(R'S)C(R')-X or | Y-N=C(R')-X or Y-NH-C(R')H-X following reduction |
| | 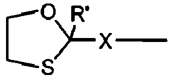 | |
| | monothioketal | |
| Y-NH₂ | R'SC(R')(SH)-X or dithiohemiketal | Y-N=C(R')-X or Y-NH-C(R')H-X following reduction |
| Y-NH₂ | (R'S)₂C(R')-X or | Y-N=C(R')-X or Y-NH-C(R')H-X following reduction |
| | 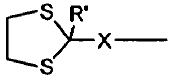 | |
| | dithioketal | |
| Y-SH | 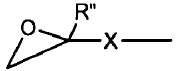 | Y-S-CH₂-C(OH)(R")-X- |
| Y-OH | epoxide (oxirane) | Y-O-CH₂-C(OH)(R")-X- |
| Y-COOH (anion) | | Y-C(=O)O-CH₂-C(OH)(R")-X- |
| Y-NHR" | | Y-NR"-CH₂-C(OH)(R")-X- |
| Y-SH | 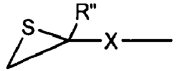 | Y-S-CH₂-C(SH)(R")-X |
| Y-OH | thioepoxide | Y-O-CH₂-C(SH)(R")-X- |
| Y-COOH (anion) | | Y-C(=O)-CH₂-C(SH)(R")-X- |
| Y-NHR" | | Y-NR"-CH₂-C(SH)(R")-X- |
| Y-SH | HO-(C=O)-X carboxyl | Y-S-(C=O)-X |
| Y-OH | | Y-O-(C=O)-X |
| Y-NHR" | | Y-NR"-C=O-X |
| Y-SH | (alcohol)-(C=O)-X carboxylic acid ester (alcohol indicates an esterified suitable alcohol leaving group *e.g*., p-nitrophenyl) | Y-S-(C=O)-X |
| Y-OH | | Y-O-(C=O)-X |
| Y-NHR" | | Y-NR"-(C=O)-X |
| Y-NH₂ | 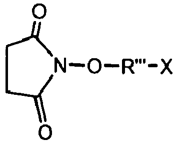 | Y-NH- R‴-X |
| | N-hydroxysuccinimide ester | |
| Y-SH | 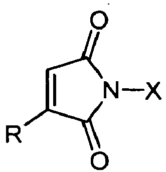 | 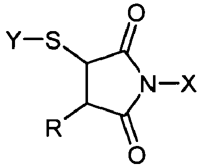 |
| | R = H, CH₃ | R = H, CH₃ |
| Y-NH₂ | 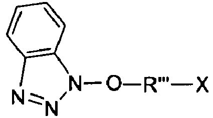 | Y-NH-R‴-X |
| | 1-benzotriazole ester | |
| Y-NH₂ | CH₃-((CH₂)₁₋₃)-O(C=NH)-X (imidoester) | Y-NH-(C=NH)-X (amidine) |
| Y-(C=NH)-O-((CH₂)₁₋₃)-CH₃ (imidoester) | H₂N-X | Y-(C=NH)-HN-X (amidine) |
| Y-COOH | H₂N-X amine | Y-(C=O)-NH-X |
| Y-(C=O)-R" | | Y-(R")C=N-X or Y-(R")CH-NH-X following reduction |
| Y-COOH | H₂N-(C=O)-NH-X urea | Y-(C=O)-NH-(C=O)-NH-X |
| Y-(C=O)-R" | | Y-(R")C=N-(C=O)-NH-X or Y-(R")CH-NH-(C=O)-NH-X following reduction |
| Y-COOH | H₂N-(C=O)-O-X carbamate | Y-(C=O)-NH-(C=O)-O-X |
| Y-(C=O)-R" | | Y-(R")C=N-(C=O)-O-X or Y-(R")CH-NH-(C=O)-O-X following reduction |
| Y-COOH | H₂N-(C=S)-NH-X thiourea | Y-(C=O)-NH-(C=S)-NH-X |
| Y-(C=O)-R" | | Y-(R")C=N-(C=S)-NH-X or Y-(R")CH-NH-(C=S)-NH-X following reduction |
| Y-COOH | H₂N-(C=S)-O-X thiocarbamate | Y-(C=O)-NH-(C=S)-O-X |
| Y-(C=O)-R" | | Y-(R")C=N-(C=S)-O-X or Y-(R")CH-NH-(C=S)-O-X following reduction |
| Y-(C=O)-R" | H₂N-HN-X | Y-(R")C=N-HN-X hydrazone |
| Y-NH-NH₂ | R"-(O=C)-X | Y-NH-N=C(R")-X hvdrazone |
| Y-NH₂ | O=C=N-X isocyanate | Y-NH-(C=O)-NH-X |
| Y-OH | | Y-O-(C=O)-NH-X |
| Y-NH₂ | S=C=N-X isothiocyanate | Y-NH-(C=S)-NH-X |
| Y-OH | | Y-O-(C=S)-NH-X |
| Y-SH | H₂C=CH-(C=O)-X or H₂C=C(CH₃)-(C=O)-X alpha-beta unsubstituted carbonyls | Y-S-CH₂CH₂-(C=O)-X |
| | | Y-S-CH₂-CH(CH₃)-(C=O)-X |
| Y-SH | H₂C=CH-(C=O)O-X | Y-S-CH₂CH₂-(C=O)O-X |
| | alpha-beta unsubstituted carboxyl | |
| Y-SH | H₂C=C(CH₃)-(C=O)-O-X | Y-S-CH₂CH(CH₃)-(C=O)O-X |
| | alpha-beta unsubstituted carboxy ls (methacrylates) | |
| Y-SH | H₂C=CH-(C=O)NH-X | Y-S-CH₂CH₂-(C=O)NH-X |
| | alpha-beta unsubstituted amides (acrylamides) | |
| Y-SH | vinylpyridine-X (2- or 4-vinylpyridine) | Y-S-CH₂-CH₂-(pyridyl)-X |
| Y-SH | H₂C=CH-SO₂-X (vinyl sulfone) | Y-S-H₂C-CH₂-SO₂-X |
| Y-SH | ClH₂C-CH₂-SO₂-L (chloroethyl sulfone) | Y-S-H₂C-CH₂-SO₂-X |
| Y-SH | (halogen)-CH₂-(C=O)-O-X | Y-S-CH₂-(C=O)-O-X |
| | (halogen)-CH₂-(C=O)-NH-X | Y-S-CH₂-(C=O)-NH-X |
| | (halogen)-CH₂-(C=O)-X (halogen is preferably I or Br) | Y-S-CH₂-(C=O)-X |
| Y-O(C=O)-CH₂-(halogen) | HS-X | Y-O(C=O)-CH₂-S-X |
| Y-NH(C=O)-CH₂-(halogen) | | Y-NH(C=O)-CH₂-S-X |
| | | Y-(C=O)-CH₂-S-X |
| Y-(C=O)-CH₂-(halogen) | | |
| (halogen is preferably I or Br) | | |
| Y-SH | (halogen)-CH₂(C=O)O-X | Y-S-CH₂(C=O)O-X |
| | (halogen)-CH₂(C=O)NH-X | Y-S-CH₂(C=O)NH-X |
| | (halogen)-CH₂(C=O)-X (halogen is preferably I or Br) | Y-S-CH₂(C=O)-X |
| Y-N₃ | HC=C-X | 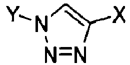 |
| Y-N₃ | 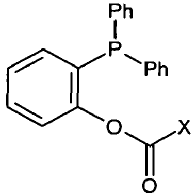 | 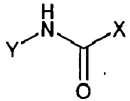 |
| Y-N₃ | 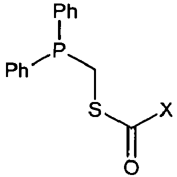 | 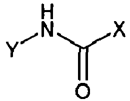 |
| Y-SH | 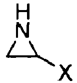 | 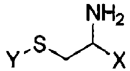 |
| Y-NH₂ | (F₃-Ph)-OC(O)-X | Y-NH-C(O)-X |

R' is C₁₋₆ alkyl, C₃₋₆ cycloalkyl, or an aryl group having 5-8 endocyclic atoms; R" is H, C₁₋₆ alkyl, C₃₋₆ cycloalkyl, or an aryl group having 5-8 endocyclic atoms; R‴ is a carbonyl derivative ^{∗}- (C=O)-, ^{∗} -(C=O)-(CH₂)₁₋₈-S-S-, ^{∗}-(C=O)-(CH₂)₁₋₈-(C=O)-O-, ^{∗}-(C=O)-(CH₂)₁₋₈-O-(C=O)-, ^{∗} - (C=O)-(CH₂)₁₋₈-(C=O)-NH- , or ^{∗}-(C=O)-(CH₂)₁₋₈-NH-(C=O)-, or alternatively, R‴ is carbonyl derivative of the form ^{∗}-(C=O)-O-(CH₂)₁₋₈-S-S-, ^{∗}-(C=O)-O-(CH₂)₁₋₈-(C=O)-O- , ^{∗}-(C=O)-O-(CH₂)₁₋₈-O-(C=O)-, ^{∗}-(C=O)-O-(CH₂)₁₋₈-(C=O)-NH-, or ^{∗}-(C=O)-O-(CH₂)₁₋₈-NH-(C=O)-, where "^{∗}" indicates the point of attachment to succinimidyl or benzotriazolyl groups;
X and Y are each the active agent, linker, monomer or initiator fragment I. -C(O)NR^{1a}R^{1b}, -NR^{1a}R^{1b}, C₁₋₆ alkyl-NR^{1a}R^{1b}, -N(R^{1a})C(O)R^{1b}, -N(R^{1a})C(O)OR^{1b}, -N(R^{1a})C(O)NR^{1a}R^{1b}, -OP(O)(OR^{1a})₂, -S(O)₂OR^{1a}, -S(O)₂NR^{1a}R^{1b}, -CN, -NO₂, cycloalkyl, heterocycloalkyl, aryl and heteroaryl

### E. Functional agents

Functional agents useful in the high MW polymers of the present invention include any biological agent or synthetic compound capable of targeting a particular ligand, receptor, complex, organelle, cell, tissue, epithelial sheet, or organ, or of treating a particular condition or disease state. In some embodiments, the bioactive agent is a drug, a therapeutic protein, a small molecule, a peptide, a peptoid, an oligonucleotide (aptamer, siRNA, microRNA), a nanoparticle, a carbohydrate, a lipid, a glycolipid, a phospholipid, or a targeting agent. Other functional agents useful in the high MW polymers of the present invention include, but are not limited to, radiolabels, contrast agents, fluorophores and dyes.

The functional agents can be linked to the initiator fragment I or the radical scavenger I', or both, of the high MW polymers. The functional agents can be linked to the initiator fragment I or the radical scavenger I' either before or after polymerization via cleavable or non-cleavable linkers described above. The functional agent can also be physisorbed or ionically absorbed to the high MW polymer instead of covalently attached.

The preparation of the high MW polymers of the present invention linked to a functional agent can be conducted by first linking the functional agent to a linking group attached to an initiator fragment and subjecting the coupled functional agent to conditions suitable for synthesis of the inventive high MW polymers. In those cases, a suitable linking group can be an initiator (*e.g*., iodinated, brominated or chlorinated compound/group) for use in ATRP reactions. Such a reaction scheme is possible where the functional agent is compatible with the polymer polymerization reactions and any subsequent workup required. However, coupling of functional agents to preformed high MW polymers can be used where the functional agent is not compatible with conditions suitable for polymerization. In addition, where cost makes the loss of an agent to imperfect synthetic yields, oftentimes encountered particularly in multistep synthetic reactions, coupling of functional agent to preformed high MW polymers of the present invention can be employed.

Where a functional agent is not compatible with the conditions employed for polymerization reactions, it can be desirable to introduce the functional agent subsequent to the polymerization reaction.

Bioactive agents, A, can be broadly selected. In some embodiments the bioactive agents can be selected from one or more drugs, vaccines, aptamers, avimer scaffolds based on human A domain scaffolds, diabodies, camelids, shark IgNAR antibodies, fibronectin type III scaffolds with modified specificities, antibodies, antibody fragments, vitamins and cofactors, polysaccharides, carbohydrates, steroids, lipids, fats, proteins, peptides, polypeptides, nucleotides, oligonucleotides, polynucleotides, and nucleic acids (*e*.*g*., mRNA, tRNA, snRNA, RNAi, microRNA, DNA, cDNA, antisense constructs, ribozymes, etc, and combinations thereof). In one embodiment, the bioactive agents can be selected from proteins, peptides, polypeptides, soluble or cell-bound, extracellular or intracellular, kinesins, molecular motors, enzymes, extracellular matrix materials and combinations thereof. In another embodiment, bioactive agents can be selected from nucleotides, oligonucleotides, polynucleotides, and nucleic acids (*e*.*g*., mRNA, tRNA, snRNA, RNAi, DNA, cDNA, antisense constructs, ribozymes *etc* and combinations thereof). In another embodiment, bioactive agents can be selected from steroids, lipids, fats and combinations thereof. For example, the bioactive agent can bind to the extracellular matrix, such as when the extracellular matrix is hyaluronic acid or heparin sulfate proteoglycan and the bioactive agent is a positively charged moiety such as choline for non-specific, electrostatic, Velcro type binding interactions. In another embodiment, the bioactive agent can be a peptide sequence that binds non-specifically or specifically.

Bioactive agents can be designed and/or selected to have a full activity (such as a high level of agonism or antagonism). Alternatively, a multifunctional bioactive agent can be selected to modulate one target protein's activity while impacting fully another.

Just as mosaic proteins contain extracellular binding domains or sub-domains (example, VEGF and Heparin Binding Epidermal Growth Factor), sequences from these binding sites can be replicated as a bioactive agent for polymer attachment. More broadly, mosaic proteins represent strings of domains of many functions (target binding, extracellular matrix binding, spacers, avidity increases, enzymatic). The set of bioactives chosen for a particular application can be assembled in similar fashion to replicate a set of desired functional activities.

Other functional agents, A, include charged species such as choline, lysine, aspartic acid, glutamic acid, and hyaluronic acid, among others. The charged species are useful for facilitating ionic attachment, to vitreous for example.

### Therapeutic Proteins and Antibodies

In one particularly useful embodiment, the functional agent is a therapeutic protein. Numerous therapeutic proteins are disclosed throughout the application such as, and without limitation, erythropoietin, granulocyte colony stimulating factor (G-CSF), GM-CSF, interferon alpha, interferon beta, human growth hormone, imiglucerase, and RANK ligand.

In one embodiment, the functional agents can be selected from specifically identified polysaccharide, protein or peptide bioactive agents, including, but not limited to: Aβ, agalsidase, alefacept, alkaline phosphatase, aspariginase, amdoxovir (DAPD), antide, becaplermin, botulinum toxin including types A and B and lower molecular weight compounds with botulinum toxin activity, calcitonins, CD1d, cyanovirin, denileukin diftitox, erythropoietin (EPO), EPO agonists, dornase alpha, erythropoiesis stimulating protein (NESP), coagulation factors such as Factor V, Factor VII, Factor VIIa, Factor VIII, B domain deleted Factor VIII, Factor IX, Factor X, Factor XII, Factor XIII, von Willebrand factor; ceredase, Fc gamma r2b, cerezyme, alpha-glucosidase, N-Acetylgalactosamine-6-sulfate sulfatase, collagen, cyclosporin, alpha defensins, beta defensins, desmopressin, exendin-4, cytokines, cytokine receptors, granulocyte colony stimulating factor (G-CSF), thrombopoietin (TPO), alpha-1 proteinase inhibitor, elcatonin, granulocyte macrophage colony stimulating factor (GM-CSF), fibrinogen, filgrastim, growth hormones human growth hormone (hGH), somatropin, growth hormone releasing hormone (GHRH), GRO-beta, GRO-beta antibody, bone morphogenic proteins such as bone morphogenic protein-2, bone morphogenic protein-6, parathyroid hormone, parathyroid hormone related peptide, OP-1; acidic fibroblast growth factor, basic fibroblast growth factor, Fibroblast Growth Factor 21, CD40 ligand, ICOS, CD28, B7-1, B7-2, TLR and other innate immune receptors, heparin, human serum albumin, low molecular weight heparin (LMWH), interferon alpha, interferon beta, interferon gamma, interferon omega, interferon tau, consensus interferon; interleukins and interleukin receptors such as interleukin-1 receptor, interleukin-2, interleukin-2 fusion proteins, interleukin-1 receptor antagonist, interleukin-3, interleukin-4, interleukin-4 receptor, interleukin-6, interleukin-8, interleukin-12, interleukin-17, interleukin-21, interleukin-13 receptor, interleukin-17 receptor; lactoferrin and lactoferrin fragments, luteinizing hormone releasing hormone (LHRH), insulin, pro-insulin, insulin analogues, amylin, C-peptide, somatostatin, somatostatin analogs including octreotide, vasopressin, follicle stimulating hormone (FSH), imiglucerase, influenza vaccine, insulin-like growth factor (IGF), insulintropin, macrophage colony stimulating factor (M-CSF), plasminogen activators such as alteplase, urokinase, reteplase, streptokinase, pamiteplase, lanoteplase, and teneteplase; nerve growth factor (NGF), trk A, trk B, osteoprotegerin, platelet-derived growth factor, tissue growth factors, transforming growth factor-1, vascular endothelial growth factor, leukemia inhibiting factor, keratinocyte growth factor (KGF), glial growth factor (GGF), T Cell receptors, CD molecules/antigens, tumor necrosis factor (TNF) (e.g., TNF-α and TNF-β), TNF receptors (e.g., TNF-α receptor and TNF-β receptor), CTLA4, CTLA4 receptor, monocyte chemoattractant protein-1, endothelial growth factors, parathyroid hormone (PTH), PTHrP, glucagon-like peptide, somatotropin, thymosin alpha 1, rasburicase, thymosin alpha 1 IIb/IIIa inhibitor, thymosin beta 10, thymosin beta 9, thymosin beta 4, alpha-1 antitrypsin, phosphodiesterase (PDE) compounds, VLA-4 (very late antigen-4), VLA-4 inhibitors, bisphosponates, respiratory syncytial virus antibody, cystic fibrosis transmembrane regulator (CFTR) gene, deoxyribonuclease (Dnase), bactericidal/permeability increasing protein (BPI), and anti-CMV antibody. Exemplary monoclonal antibodies include etanercept (a dimeric fusion protein consisting of the extracellular ligand-binding portion of the human 75 kD TNF receptor linked to the Fc portion of IgG1), abciximab, adalimumab, afelimomab, alemtuzumab, antibody to B-lymphocyte, atlizumab, basiliximab, bevacizumab, biciromab, bertilimumab, CDP-484, CDP-571, CDP-791, CDP-860, CDP-870, cetuximab, clenoliximab, daclizumab, eculizumab, edrecolomab, efalizumab, epratuzumab, fontolizumab, gavilimomab, gemtuzumab ozogamicin, ibritumomab tiuxetan, infliximab, inolimomab, keliximab, labetuzumab, lerdelimumab, olizumab, radiolabeled lym-1, metelimumab, mepolizumab, mitumomab, muromonad-CD3, nebacumab, natalizumab, odulimomab, omalizumab, oregovomab, palivizumab, pemtumomab, pexelizumab, rhuMAb-VEGF, rituximab, satumomab pendetide, sevirumab, siplizumab, tositumomab, I¹³¹tositumomab, trastuzumab, tuvirumab, visilizumab, and fragments and mimetics thereof.

In one embodiment, the bioactive agent is a fusion protein. For example, and without limitation, the bioactive component can be an immunoglobulin or portion of an immunoglobulin fused to one or more certain useful peptide sequences. For example, the bioactive agent may contain an antibody Fc fragment. In one embodiment, the bioactive agent is a CTLA4 fusion protein. For example, the bioactive agent can be an Fc-CTLA4 fusion protein. In another embodiment, the bioactive agent is a Factor VIII fusion protein. For example, the bioactive agent can be an Fc-Factor VIII fusion protein.

In one particularly useful embodiment, the bioactive agent is a human protein or human polypeptide, for example, a heterologously produced human protein or human polypeptide. Numerous proteins and polypeptides are disclosed herein for which there is a corresponding human form (i.e., the protein or peptide is normally produced in human cells in the human body). Therefore, in one embodiment, the bioactive agent is the human form of each of the proteins and polypeptides disclosed herein for which there is a human form. Examples of such human proteins include, without limitation, human antibodies, human enzymes, human hormones and human cytokines such as granulocyte colony stimulation factor, granulocyte macrophage colony stimulation factor, interferons (e.g., alpha interferons and beta interferons), human growth hormone and erythropoietin.

Other examples of therapeutic proteins which (themselves or as the target of an antibody or antibody fragment or non-antibody protein) may serve as bioactive agents include, without limitation, factor VIII, b-domain deleted factor VIII, factor VIIa, factor IX, factor X, anticoagulants; hirudin, alteplase, tpa, reteplase, tpa, tpa - 3 of 5 domains deleted, insulin, insulin lispro, insulin aspart, insulin glargine, long-acting insulin analogs, complement C5, hgh, glucagons, tsh, follitropin-beta, fsh, gm-csf, pdgh, ifn alpha2, ifn alpha2a, ifn alpha2b, inf-apha1, consensus ifn, ifn-beta, ifn-beta 1b, ifn-beta 1a, ifn-gamma (e.g., 1 and 2), ifn-lambda, ifn-delta, il-2, il-11, hbsag, ospa, murine mab directed against t-lymphocyte antigen, murine mab directed against tag-72, tumor-associated glycoprotein, fab fragments derived from chimeric mab directed against platelet surface receptor gpII(b)/III(a), murine mab fragment directed against tumor-associated antigen ca125, lysyl oxidase, LOX2, murine mab fragment directed against human carcinoembryonic antigen, cea, murine mab fragment directed against human cardiac myosin, murine mab fragment directed against tumor surface antigen psma, murine mab fragments (fab/fab2 mix) directed against hmw-maa, murine mab fragment (fab) directed against carcinoma-associated antigen, mab fragments (fab) directed against nca 90, a surface granulocyte nonspecific cross reacting antigen, chimeric mab directed against cd20 antigen found on surface of b lymphocytes, humanized mab directed against the alpha chain of the il2 receptor, chimeric mab directed against the alpha chain of the il2 receptor, chimeric mab directed against tnf-alpha, humanized mab directed against an epitope on the surface of respiratory synctial virus, humanized mab directed against her 2, human epidermal growth factor receptor 2, human mab directed against cytokeratin tumor-associated antigen anti-ctla4, chimeric mab directed against cd 20 surface antigen of b lymphocytes dornase-alpha dnase, beta glucocerebrosidase, tnf-alpha, il-2-diptheria toxin fusion protein, tnfr-lgg fragment fusion protein laronidase, dnaases, alefacept, darbepoetin alpha (colony stimulating factor), tositumomab, murine mab, alemtuzumab, rasburicase, agalsidase beta, teriparatide, parathyroid hormone derivatives, adalimumab (lgg1), anakinra, biological modifier, nesiritide, human b-type natriuretic peptide (hbnp), colony stimulating factors, pegvisomant, human growth hormone receptor antagonist, recombinant activated protein c, omalizumab, immunoglobulin e (lge) blocker, lbritumomab tiuxetan, ACTH, glucagon, somatostatin, somatotropin, thymosin, parathyroid hormone, pigmentary hormones, somatomedin, erythropoietin, luteinizing hormone, chorionic gonadotropin, hypothalmic releasing factors, etanercept, antidiuretic hormones, prolactin and thyroid stimulating hormone. And any of these can be modified to have a site-specific conjugation point (a N-terminus, or C-terminus, or other location) using natural (for example, a serine to cysteine substitution) (for example, formylaldehyde per method of Redwood Biosciences) or non-natural amino acid. Non-natural amino acid residue(s) can be selected from the group consisting of: azidonorleucine, 3-(1-naphthyl)alanine, 3-(2-naphthyl)alanine, p-ethynyl-phenylalanine, p-propargly-oxy-phenylalanine, m-ethynyl-phenylalanine, 6-ethynyl-tryptophan, 5-ethynyl-tryptophan, (R)-2-amino-3-(4-ethynyl-1H-pyrol-3-yl)propanic acid, p-bromophenylalanine, p-iodophenylalanine, p-azidophenylalanine, p-acetylphenylalanine, 3-(6-chloroindolyl)alanine, 3-(6-bromoindolyl)alanine, 3-(5-bromoindolyl)alanine, azidohomoalanine, homopropargylglycine, p-chlorophenylalanine, α-aminocaprylic acid, O-methyl-L-tyrosine, N-acetylgalactosamine-α-threonine, and N-acetylgalactosamine-α-serine.

Examples of therapeutic antibodies that may serve as bioactive agents (by themselves or fragments of such antibodies) include, but are not limited, to HERCEPTIN^{™} (Trastuzumab) (Genentech, CA) which is a humanized anti-HER2 monoclonal antibody for the treatment of patients with metastatic breast cancer; REOPRO^{™} (abciximab) (Centocor) which is an anti-glycoprotein IIb/IIIa receptor on the platelets for the prevention of clot formation; ZENAPAX^{™} (daclizumab) (Roche Pharmaceuticals, Switzerland) which is an immunosuppressive, humanized anti-CD25 monoclonal antibody for the prevention of acute renal allograft rejection; PANOREX^{™} which is a murine anti-17-IA cell surface antigen IgG2a antibody (Glaxo Wellcome/Centocor); BEC2 which is a murine anti-idiotype (GD3 epitope) IgG antibody (ImClone System); IMC-C225 which is a chimeric anti-EGFR IgG antibody (ImClone System); VITAXIN^{™} which is a humanized anti-αVβ3 integrin antibody (Applied Molecular Evolution/MedImmune); Campath; Campath 1H/LDP-03 which is a humanized anti CD52 IgG1 antibody (Leukosite); Smart M195 which is a humanized anti-CD33 IgG antibody (Protein Design Lab/Kanebo); RITUXAN^{™} which is a chimeric anti-CD2O IgG1 antibody (IDEC Pharm/Genentech, Roche/Zettyaku); LYMPHOCIDE^{™} which is a humanized anti-CD22 IgG antibody (Immunomedics); ICM3 is a humanized anti-ICAM3 antibody (ICOS Pharm); IDEC-114 is a primate anti-CD80 antibody (IDEC Pharm/Mitsubishi); ZEVALIN^{™} is a radiolabelled murine anti-CD20 antibody (IDEC/Schering AG); IDEC-13l is a humanized anti-CD40L antibody (IDEC/Eisai); IDEC-151 is a primatized anti-CD4 antibody (IDEC); IDEC-152 is a primatized anti-CD23 antibody (IDEC/Seikagaku); SMART anti-CD3 is a humanized anti-CD3 IgG (Protein Design Lab); 5G1.1 is a humanized anti-complement factor 5 (CS) antibody (Alexion Pharm); D2E7 is a humanized anti-TNF-a antibody (CATIBASF); CDP870 is a humanized anti-TNF-a Fab fragment (Celltech); IDEC-151 is a primatized anti-CD4 IgG1 antibody (IDEC Pharm/SmithKline Beecham); MDX-CD4 is a human anti-CD4 IgG antibody (Medarex/Eisai/Genmab); CDP571 is a humanized anti-TNF-a IgG4 antibody (Celltech); LDP-02 is a humanized anti-a4β7 antibody (LeukoSite/Genentech); OrthoClone OKT4A is a humanized anti-CD4 IgG antibody (Ortho Biotech); ANTOVA^{™} is a humanized anti-CD40L IgG antibody (Biogen); ANTEGREN^{™} is a humanized anti-VLA-4 IgG antibody (Elan); CAT-152, a human anti-TGF-β₂ antibody (Cambridge Ab Tech); Cetuximab (BMS) is a monoclonal anti-EGF receptor (EGFr) antibody; Bevacizuma (Genentech) is an anti-VEGF human monoclonal antibody; Infliximab (Centocore, JJ) is a chimeric (mouse and human) monoclonal antibody used to treat autoimmune disorders; Gemtuzumab ozogamicin (Wyeth) is a monoclonal antibody used for chemotherapy; and Ranibizumab (Genentech) is a chimeric (mouse and human) monoclonal antibody used to treat macular degeneration.

Other antibodies, such as single domain antibodies are useful in the present invention. A single domain antibody (sdAb, called Nanobody by Ablynx) is an antibody fragment consisting of a single monomeric variable antibody domain. Like a whole antibody, the sdAb is able to bind selectively to a specific antigen. With a molecular weight of only 12-15 kDa, single domain antibodies are much smaller than common antibodies (150-160 kDa). A single domain antibody is a peptide chain of about 110 amino acids in length, comprising one variable domain (VH) of a heavy chain antibody, or of a common IgG.

Unlike whole antibodies, sdAbs do not show complement system triggered cytotoxicity because they lack an Fc region. Camelid and fish derived sdAbs are able to bind to hidden antigens that are not accessible to whole antibodies, for example to the active sites of enzymes.

A single domain antibody (sdAb) can be obtained by immunization of dromedaries, camels, llamas, alpacas or sharks with the desired antigen and subsequent isolation of the mRNA coding for heavy chain antibodies. Alternatively they can be made by screening synthetic libraries. Camelids are members of the biological family Camelidae, the only living family in the suborder Tylopoda. Camels, dromedaries, Bactrian Camels, llamas, alpacas, vicuñas, and guanacos are in this group.

### Proteins, Peptides and Amino Acids

Proteins and peptides for use as bioactive agents as disclosed herein can be produced by any useful method including production by *in vitro* synthesis and by production in biological systems. Typical examples of *in vitro* synthesis methods which are well known in the art include solid-phase synthesis ("SPPS") and solid-phase fragment condensation ("SPFC"). Biological systems used for the production of proteins are also well known in the art. Bacteria (e.g., *E coli* and *Bacillus sp*.) and yeast (e.g., *Saccharomyces cerevisiae* and *Pichia pastoris*) are widely used for the production of heterologous proteins. In addition, heterologous gene expression for the production of bioactive agents for use as disclosed herein can be accomplished using animal cell lines such as mammalian cell lines (e.g., CHO cells). In one particularly useful embodiment, the bioactive agents are produced in transgenic or cloned animals such as cows, sheep, goats and birds (e.g., chicken, quail, ducks and turkey), each as is understood in the art. See, for example, US Patent No. 6,781,030, issued August 24, 2004, the disclosure of which is incorporated in its entirety herein by reference.

Bioactive agents such as proteins produced in domesticated birds such as chickens can be referred to as "avian derived" bioactive agents (e.g., avian derived therapeutic proteins). Production of avian derived therapeutic proteins is known in the art and is described in, for example, US Patent No. 6,730,822, issued May 4, 2004, the disclosure of which is incorporated in its entirety herein by reference.

In embodiments where the bioactive agent is a protein or polypeptide, functional groups present in the amino acids of the protein polypeptide sequence can be used to link the agent to the high MW polymer. Linkages to protein or polypeptide bioactive agents can be made to naturally occurring amino acids in their sequence or to naturally occurring amino acids that have either been added to the sequence or inserted in place of another amino acid, for example the replacement of a serine by a cysteine.

Peptides useful in the present invention also include, but are not limited to, a macrocyclic peptide, a cyclotide, an aptamer, an LDL receptor A-domain, a protein scaffold (as discussed in US Patent Number 60/514,391), a soluble receptor, an enzyme, a peptide multimer, a domain multimer, an antibody fragment multimer, and a fusion protein.

Protein or polypeptide bioactive agents may also comprise non-naturally occurring amino acids in addition to the common naturally occurring amino acids found in proteins and polypeptides. In addition to being present for the purpose of altering the properties of a polypeptide or protein, non-naturally occurring amino acids can be introduced to provide a functional group that can be used to link the protein or polypeptide directly to high MW polymer. Furthermore, naturally occurring amino acids, *e*.*g*., cysteine, tyrosine, tryptophan can be used in this way.

Non-naturally occurring amino acids can be introduced into proteins and peptides by a variety of means. Some of the techniques for the introduction of non-natural amino acids are discussed in US Patent No. 5,162,218 and US Patent No. 20080214439, the disclosure of which is incorporated in its entirety herein by reference. First, non-naturally occurring amino acids can be introduced by chemical modification of a polypeptide or protein on the amino acid side chain or at either the amino terminus or the carboxyl terminus. Non-limiting examples of chemical modification of a protein or peptide might be methylation by agents such as diazomethane, or the introduction of acetylation at an amino group present in lysine's side chain or at the amino terminus of a peptide or protein. Another example of the protein/polypeptide amino group modification to prepare a non-natural amino acid is the use of methyl 3-mercaptopropionimidate ester or 2-iminothiolane to introduce a thiol (sulfhydryl, -SH) bearing functionality linked to positions in a protein or polypeptide bearing a primary amine. Once introduced, such groups can be employed to form a covalent linkage to the protein or polypeptide.

Second, non-naturally occurring amino acids can be introduced into proteins and polypeptides during chemical synthesis. Synthetic methods are typically utilized for preparing polypeptides having fewer than about 200 amino acids, usually having fewer than about 150 amino acids, and more usually having 100 or fewer amino acids. Shorter proteins or polypeptides having less than about 75 or less than about 50 amino acids can be prepared by chemical synthesis.

The synthetic preparation methods that are particularly convenient for allowing the insertion of non-natural amino acids at a desired location are known in the art. Suitable synthetic polypeptide preparation methods can be based on Merrifield solid-phase synthesis methods where amino acids are sequentially added to a growing chain (Merrifield (1963) J. Am. Chem. Soc. 85:2149-2156). Automated systems for synthesizing polypeptides by such techniques are now commercially available from suppliers such as Applied Biosystems, Inc., Foster City, Calif. 94404; New Brunswick Scientific, Edison, N.J. 08818; and Pharmacia, Inc., Biotechnology Group, Piscataway, N.J. 08854.

Examples of non-naturally occurring amino acids that can be introduced during chemical synthesis of polypeptides include, but are not limited to: D-amino acids and mixtures of D and L-forms of the 20 naturally occurring amino acids, N-formyl glycine, ornithine, norleucine, hydroxyproline, beta-alanine, hydroxyvaline, norvaline, phenylglycine, cyclohexylalanine, t-butylglycine (t-leucine, 2-amino-3,3-dimethylbutanoic acid), hydroxy-t-butylglycine, amino butyric acid, cycloleucine, 4-hydroxyproline, pyroglutamic acid (5-oxoproline), azetidine carboxylic acid, pipecolinic acid, indoline-2-carboxylic acid, tetrahydro-3-isoquinoline carboxylic acid, 2,4-diaminobutyricacid, 2,6-diaminopimelic acid, 2,4-diaminobutyricacid, 2,6-diaminopimelicacid, 2,3-diaminopropionicacid, 5-hydroxylysine, neuraminic acid, and 3,5-diiodotyrosine.

Third, non-naturally occurring amino acids can be introduced through biological synthesis *in vivo* or *in vitro* by insertion of a non-sense codon (*e.g.*, an amber or ocher codon) in a DNA sequence (*e*.*g*., the gene) encoding the polypeptide at the codon corresponding to the position where the non-natural amino acid is to be inserted. Such techniques are discussed for example in US Patents No.: 5,162,218 and 6,964,859, the disclosures of which are incorporated in their entirety herein by reference. A variety of methods can be used to insert the mutant codon including oligonucleotide-directed mutagenesis. The altered sequence is subsequently transcribed and translated, *in vivo* or *in vitro* in a system which provides a suppressor tRNA, directed against the nonsense codon that has been chemically or enzymatically acylated with the desired non-naturally occurring amino acid. The synthetic amino acid will be inserted at the location corresponding to the nonsense codon. For the preparation of larger and/or glycosylated polypeptides, recombinant preparation techniques of this type are usually preferred. Among the amino acids that can be introduced in this fashion are: formyl glycine, fluoroalanine, 2-Amino-3-mercapto-3-methylbutanoic acid, homocysteine, homoarginine and the like. Other similar approaches to obtain non-natural amino acids in a protein include methionine substitution methods.

Where non-naturally occurring amino acids have a functionality that is susceptible to selective modification, they are particularly useful for forming a covalent linkage to the protein or polypeptide. Circumstances where a functionality is susceptible to selective modification include those where the functionality is unique or where other functionalities that might react under the conditions of interest are hindered either stereochemically or otherwise.

Other antibodies, such as single domain antibodies are useful in the present invention. A single domain antibody (sdAb, called Nanobody by Ablynx) is an antibody fragment consisting of a single monomeric variable antibody domain. Like a whole antibody, the sdAb is able to bind selectively to a specific antigen. With a molecular weight of only 12-15 kDa, single domain antibodies are much smaller than common whole antibodies (150-160 kDa). A single domain antibody is a peptide chain of about 110 amino acids in length, comprising one variable domain (VH) of a heavy chain antibody, or of a common IgG.

Unlike whole antibodies, sdAbs do not show complement system triggered cytotoxicity because they lack an Fc region. Camelid and fish derived sdAbs are able to bind to hidden antigens that are not accessible to whole antibodies, for example to the active sites of enzymes.

A single domain antibody (sdAb) can be obtained by immunization of dromedaries, camels, llamas, alpacas or sharks with the desired antigen and subsequent isolation of the mRNA coding for heavy chain antibodies. Alternatively they can be made by screening synthetic libraries. Camelids are members of the biological family Camelidae, the only living family in the suborder Tylopoda. Camels, dromedaries, Bactrian Camels, llamas, alpacas, vicuñas, and guanacos are in this group.

Peptides useful in the present invention also include, but are not limited to, a macrocyclic peptide, a cyclotide, an LDL receptor A-domain, a protein scaffold (as discussed in US Patent Number 60/514,391, incorporated in its entirety herein), a soluble receptor, an enzyme, a peptide multimer, a domain multimer, an antibody fragment multimer, and a fusion protein.

The invention also describes new ways to achieve branched polymer architectures on a bioactive surface. The concept is one of "branching points" or "proximal attachment points" on the target molecule such as to recreate an effective ≥2 arm polymer with ≥1 arm polymers attached to a localized site(s) on a target molecule. In the prior art, indiscriminate PEGylation of a protein with a non site-specific reagent (for example an NHS functionalized PEG reagent) would result in multiple PEG polymers conjugated to multiple amine groups scattered through the protein. Here, what is described is preferably a one step approach in which the target agent is modified to locate two unique conjugation sites (for example, cysteine amino acids) such that once the tertiary structure of the protein or peptide or agent is formed, the two sites will be in proximity one to the other. Then, this modified target agent is used in a conjugation reaction with a polymer containing the corresponding conjugation chemistry (for example, thiol reactive). The result is a single target agent which is conjugated with two polymers in close proximity to one another, thereby creating a branching point or "pseudo" branch. In another embodiment, the target agent would contain a single unique site, for example a free cysteine, and a tri(hetero)functional linking agent would be employed to attach ≥2 linear polymers to this single site, again creating a "pseudo" branch.

### Drugs

In another embodiment, the bioactive agents can also be selected from specifically identified drug or therapeutic agents, including but not limited to: tacrine, memantine, rivastigmine, galantamine, donepezil, levetiracetam, repaglinide, atorvastatin, alefacept, tadalafil, vardenafil, sildenafil, fosamprenavir, oseltamivir, valacyclovir and valganciclovir, abarelix, adefovir, alfuzosin, alosetron, amifostine, amiodarone, aminocaproic acid, aminohippurate sodium, aminoglutethimide, aminolevulinic acid, aminosalicylic acid, amlodipine, amsacrine, anagrelide, anastrozole, aprepitant, aripiprazole, asparaginase, atazanavir, atomoxetine, anthracyclines, bexarotene, bicalutamide, bleomycin, bortezomib, buserelin, busulfan, cabergoline, capecitabine, carboplatin, carmustine, chlorambucin, cilastatin sodium, cisplatin, cladribine, clodronate, cyclophosphamide, cyproterone, cytarabine, camptothecins, 13-cis retinoic acid, all trans retinoic acid; dacarbazine, dactinomycin, daptomycin, daunorubicin, deferoxamine, dexamethasone, diclofenac, diethylstilbestrol, docetaxel, doxorubicin, dutasteride, eletriptan, emtricitabine, enfuvirtide, eplerenone, epirubicin, estramustine, ethinyl estradiol, etoposide, exemestane, ezetimibe, fentanyl, fexofenadine, fludarabine, fludrocortisone, fluorouracil, fluoxymesterone, flutarnide, fluticazone, fondaparinux, fulvestrant, gamma-hydroxybutyrate, gefitinib, gemcitabine, epinephrine, L-Dopa, hydroxyurea, icodextrin, idarubicin, ifosfamide, imatinib, irinotecan, itraconazole, goserelin, laronidase, lansoprazole, letrozole, leucovorin, levamisole, lisinopril, lovothyroxine sodium, lomustine, mechlorethamine, medroxyprogesterone, megestrol, melphalan, memantine, mercaptopurine, mequinol, metaraminol bitartrate, methotrexate, metoclopramide, mexiletine, miglustat, mitomycin, mitotane, mitoxantrone, modafinil, naloxone, naproxen, nevirapine, nicotine, nilutamide, nitazoxanide, nitisinone, norethindrone, octreotide, oxaliplatin, palonosetron, pamidronate, pemetrexed, pergolide, pentostatin, pilcamycin, porfimer, prednisone, procarbazine, prochlorperazine, ondansetron, palonosetron, oxaliplatin, raltitrexed, rosuvastatin, sirolimus, streptozocin, pimecrolimus, sertaconazole, tacrolimus, tamoxifen, tegaserod, temozolomide, teniposide, testosterone, tetrahydrocannabinol, thalidomide, thioguanine, thiotepa, tiotropium, topiramate, topotecan, treprostinil, tretinoin, valdecoxib, celecoxib, rofecoxib, valrubicin, vinblastine, vincristine, vindesine, vinorelbine, voriconazole, dolasetron, granisetron, formoterol, fluticasone, leuprolide, midazolam, alprazolam, amphotericin B, podophylotoxins, nucleoside antivirals, aroyl hydrazones, sumatriptan, eletriptan; macrolides such as erythromycin, oleandomycin, troleandomycin, roxithromycin, clarithromycin, davercin, azithromycin, flurithromycin, dirithromycin, josamycin, spiromycin, midecamycin, loratadine, desloratadine, leucomycin, miocamycin, rokitamycin, andazithromycin, and swinolide A; fluoroquinolones such as ciprofloxacin, ofloxacin, levofloxacin, trovafloxacin, alatrofloxacin, moxifloxicin, norfloxacin, enoxacin, gatifloxacin, gemifloxacin, grepafloxacin, lomefloxacin, sparfloxacin, temafloxacin, pefloxacin, amifloxacin, fleroxacin, tosufloxacin, prulifloxacin, irloxacin, pazufloxacin, clinafloxacin, and sitafloxacin; aminoglycosides such as gentamicin, netilmicin, paramecin, tobramycin, amikacin, kanamycin, neomycin, and streptomycin, vancomycin, teicoplanin, rampolanin, mideplanin, colistin, daptomycin, gramicidin, colistimethate; polymixins such as polymixin B, capreomycin, bacitracin, penems; penicillins including penicllinase-sensitive agents like penicillin G, penicillin V; penicillinase-resistant agents like methicillin, oxacillin, cloxacillin, dicloxacillin, floxacillin, nafcillin; gram negative microorganism active agents like ampicillin, amoxicillin, and hetacillin, cillin, and galampicillin; antipseudomonal penicillins like carbenicillin, ticarcillin, azlocillin, mezlocillin, and piperacillin; cephalosporins like cefpodoxime, cefprozil, ceftbuten, ceftizoxime, ceftriaxone, cephalothin, cephapirin, cephalexin, cephradrine, cefoxitin, cefamandole, cefazolin, cephaloridine, cefaclor, cefadroxil, cephaloglycin, cefuroxime, ceforanide, cefotaxime, cefatrizine, cephacetrile, cefepime, cefixime, cefonicid, cefoperazone, cefotetan, cefmetazole, ceftazidime, loracarbef, and moxalactam, monobactams like aztreonam; and carbapenems such as imipenem, meropenem, and ertapenem, pentamidine isetionate, albuterol sulfate, lidocaine, metaproterenol sulfate, beclomethasone diprepionate, triamcinolone acetamide, budesonide acetonide, salmeterol, ipratropium bromide, flunisolide, cromolyn sodium, and ergotamine tartrate; taxanes such as paclitaxel; SN-38, and tyrphostines. Bioactive agents may also be selected from the group consisting of aminohippurate sodium, amphotericin B, doxorubicin, aminocaproic acid, aminolevulinic acid, aminosalicylic acid, metaraminol bitartrate, pamidronate disodium, daunorubicin, levothyroxine sodium, lisinopril, cilastatin sodium, mexiletine, cephalexin, deferoxamine, and amifostine in another embodiment.

Other bioactive agents useful in the present invention include extracellular matrix targeting agents, functional transport moieties and labeling agents. Extracellular matrix targeting agents include, but are not limited to, heparin binding moieties, matrix metalloproteinase binding moieties, lysyl oxidase binding domains, negatively charged moieties or positively charged moieties and hyaluronic acid. Functional transport moieties include, but are not limited to, blood brain barrier transport moieties, intracellular transport moieties, organelle transport moieties, epithelial transport domains and tumor targeting moieties (folate, other). In some embodiments, the targeting agents useful in the present invention target anti-TrkA, anti A-beta (peptide 1-40, peptide 1-42, monomeric form, oligomeric form), anti-IGF1-4, agonist RANK-L, anti-ApoE4 or anti-ApoAl, among others.

### Diagnostic agents

Diagnostic agents useful in the high MW polymers of the present invention include imaging agents and detection agents such as radiolabels, fluorophores, dyes and contrast agents.

Imaging agent refers to a label that is attached to the high MW polymer of the present invention for imaging a tumor, organ, or tissue in a subject. The imaging moiety can be covalently or non-covalently attached to the high MW polymer. Examples of imaging moieties suitable for use in the present invention include, without limitation, radionuclides, fluorophores such as fluorescein, rhodamine, Texas Red, Cy2, Cy3, Cy5, Cy5.5, Cy7 and the AlexaFluor (Invitrogen, Carlsbad, CA) range of fluorophores, antibodies, gadolinium, gold, nanomaterials, horseradish peroxidase, alkaline phosphatase, derivatives thereof, and mixtures thereof.

Radiolabel refers to a nuclide that exhibits radioactivity. A "nuclide" refers to a type of atom specified by its atomic number, atomic mass, and energy state, such as carbon 14 (¹⁴C). "Radioactivity" refers to the radiation, including alpha particles, beta particles, nucleons, electrons, positrons, neutrinos, and gamma rays, emitted by a radioactive substance. Radionuclides suitable for use in the present invention include, but are not limited to, fluorine 18 (¹⁸F), phosphorus 32 (³²P), scandium 47 (⁴⁷SC), cobalt 55 (⁵⁵Co), copper 60 (⁶⁰Cu), copper 61 (⁶¹Cu), copper 62 (⁶²Cu), copper 64 (⁶⁴Cu), gallium 66 (⁶⁶Ga), copper 67 (⁶⁷Cu), gallium 67 (⁶⁷Ga), gallium 68 (⁶⁸Ga), rubidium 82 (⁸²Rb), yttrium 86 (⁸⁶Y), yttrium 87 (⁸⁷Y), strontium 89 (⁸⁹Sr), yttrium 90 (⁹⁰Y), rhodium 105 (¹⁰⁵Rh), silver 111 (¹¹¹Ag), indium 111 (¹¹¹In), iodine 124 (¹²⁴I), iodine 125 (¹²⁵I), iodine 131 (¹³¹I), tin 117m (^{117m}Sn), technetium 99m (^{99m}Tc), promethium 149 (¹⁴⁹Pm), samarium 153 (¹⁵³Sm), holmium 166 (¹⁶⁶Ho), lutetium 177 (¹⁷⁷Lu), rhenium 186 (¹⁸⁶Re), rhenium 188 (¹⁸⁸Re), thallium 201 (²⁰¹Tl), astatine 211 (²¹¹At), and bismuth 212 (²¹²Bi). As used herein, the "m" in ^{117m}Sn and ^{99m}Tc stands for meta state. Additionally, naturally occurring radioactive elements such as uranium, radium, and thorium, which typically represent mixtures of radioisotopes, are suitable examples of radionuclides. ⁶⁷Cu, ¹³¹I, ¹⁷⁷Lu, and ¹⁸⁶Re are beta- and gamma-emitting radionuclides. ²¹²Bi is an alpha- and beta-emitting radionuclide. ²¹¹At is an alpha-emitting radionuclide. ³²P, ⁴⁷Sc, ⁸⁹Sr, ⁹⁰Y, ¹⁰⁵Rh, ¹¹¹Ag, ^{117m}Sn, ¹⁴⁹Pm, ¹⁵³Sm, ¹⁶⁶Ho, and ¹⁸⁸Re are examples of beta-emitting radionuclides. ⁶⁷Ga, ¹¹¹In, ^{99m}Tc, and ²⁰¹Tl are examples of gamma-emitting radionuclides. ⁵⁵Co, ⁶⁰Cu, ⁶¹Cu, ⁶²Cu, ⁶⁶Ga, ⁶⁸Ga, ⁸²Rb, and ⁸⁶Y are examples of positron-emitting radionuclides. ⁶⁴Cu is a beta- and positron-emitting radionuclide. Imaging and detection agents can also be designed into the polymers of the invention through the addition of naturally occurring isotopes such as deuterium, ¹³C, or ¹⁵N during the synthesis of the initiator, linkers, linking groups, comonomers.

Contrast agents useful in the present invention include, but are not limited to, gadolinium based contrast agents, iron based contrast agents, iodine based contrast agents, barium sulfate, among others. One of skill in the art will appreciate that other contrast agents are useful in the present invention.

### Nanoparticles

The functional agents can also include nanoparticles. Nanoparticles useful in the present invention include particles having a size ranging from 1 to 1000 nm. Nanoparticles can be beads, metallic particles or can in some cases be micelles and in some other be liposomes. Other nanoparticles include carbon nanotubes, quantum dots and colloidal gold. Nanoparticles can be packed with diagnostic and/or therapeutic agents.

Those skilled in the art will also recognize that the invention can be used to enable coincident detection of more than one agent of the same or different type. Also, the use of flexible linker chemistries can also be used to witness the loss of one fluorescent label, for example as the molecule is taken up into the cell and into a low pH environment.

### Conjugates

The polymers of the present invention can be linked to a variety of functional agents described above to form a conjugate. In some embodiments, the present invention provides a conjugate including at least one polymer having a polymer arm having a plurality of monomers each independently selected from the group consisting of acrylate, methacrylate, acrylamide, methacrylamide, styrene, vinyl-pyridine, vinyl-pyrrolidone and vinyl esters such as vinyl acetate, wherein each monomer includes a hydrophilic group, an initiator fragment linked to a proximal end of the polymer arm, wherein the initator moiety is suitable for radical polymerization, and an end group linked to a distal end of the polymer arm. The conjugate of the present invention also includes at least one functional agent having a bioactive agent or a diagnostic agent, linked to the initiator fragment or the end group.

The bioactive agent of the conjugate of the present invention can include a drug, an antibody, an antibody fragment, a single domain antibody, an avimer, an adnectin, diabodies, a vitamin, a cofactor, a polysaccharide, a carbohydrate, a steroid, a lipid, a fat, a protein, a peptide, a polypeptide, a nucleotide, an oligonucleotide, a polynucleotide, or a nucleic acid. The diagnostic agent of the conjugate can be a radiolabel, a contrast agent, a fluorophore or a dye. In some embodiments, at least two polymers are linked to the functional agent. In some embodiments, at least two polymers are linked to the functional agent via proximal reactive groups on the functional agent to create a pseudo-branched structure. In other embodiments, the conjugate includes at least two functional agents attached to the polymer.

### IV. Preparation of Zwitterion/Phosphoryl-Containing High MW polymers

The high MW polymers of the present invention can be prepared by any means known in the art. In some embodiments, the present invention provides a process for preparing a high MW polymer of the present invention, the process including the step of contacting a mixture of a first monomer and a second monomer with an initiator, I¹, under conditions sufficient to prepare a high MW polymer via free radical polymerization, wherein the first monomer comprises a phosphorylcholine, and each of the second monomer and initiator independently comprise at least one of a functional agent or a linking group for linking to the functional agent.

The mixture for preparing the high MW polymers of the present invention can include a variety of other components. For example, the mixture can also include catalyst, ligand, solvent, and other additives. In some embodiments, the mixture also includes a catalyst and a ligand. Suitable catalysts and ligands are described in more detail below.

Any suitable monomer can be used in the process of the present invention, such as those described above.

The high MW polymers of the present invention can be prepared by any suitable polymerization method, such as by living radical polymerization. Living radical polymerization, discussed by Odian, G. in Principles of Polymerization, 4th , Wiley-Interscience John Wiley & Sons: New York, 2004, and applied to zwitterionic polymers for example in US 6,852,816. Several different living radical polymerization methodologies can be employed, including Stable Free Radical Polymerization (SFRP), Radical Addition-Fragmentation Transfer (RAFT), and Nitroxide-Mediated Polymerization (NMP). In addition, Atom Transfer Radical Polymerization (ATRP), provides a convenient method for the preparation of the high MW polymers of the invention.

The preparation of polymers via ATRP involves the radical polymerization of monomers beginning with an initiator bearing one or more halogens. The halogenated initiator is activated by a catalyst (or a mixture of catalysts when CuBr₂ is employed) such as a transition metal salt (CuBr) that can be solubilized by a ligand (*e*.*g*., bipyridine or PMDETA). RAFT polymerization uses thiocarbonylthio compounds, such as dithioesters, dithiocarbamates, trithiocarbonates, and xanthates, to mediate the polymerization process via a reversible chain-transfer process. Other "living" or controlled radical processes useful in the preparation of the inventive random copolymers include NMP.

### Initiators

Initiators useful for the preparation of the high MW polymers of the present invention include any initiator suitable for polymerization via radical polymerization. In some embodiments, the initiators are suitable for atom transfer radical polymerization (ATRP), such as those described above. Other useful initiators include those for nitroxide mediated radical polymerization (NMP), or reversible addition-fragmentation-termination (RAFT or MADIX) polymerization. Still other techniques to control a free-radical polymerization process can be used, such as the use of iniferters, degenerative transfer or telomerization process. Moreover, the initiators useful in the present invention include those having at least one branch point, such as those described above. In other embodiments, the initiators are useful for controlled radical polymerization.

High MW polymers of the present invention having complex architectures including branched compounds having multiple polymer arms including, but not limited to, comb and star structures. Comb architectures can be achieved employing linear initiators bearing three or more halogen atoms, preferably the halogens are chlorine, bromine, or iodine atoms, more preferably the halogens are chlorine or bromine atoms. Star architectures can also be prepared employing compounds bearing multiple halogens on a single carbon atom or cyclic molecules bearing multiple halogens. In some embodiments compounds having star architecture have 3 polymer arms and in other embodiments they have 4 polymer arms. See initiators described above.

### Catalysts and Ligands

Catalysts for use in ATRP or group radical transfer polymerizations may include suitable salts of Cu¹⁺, Cu²⁺, Fe²⁺, Fe³⁺, Ru²⁺, Ru.³⁺, Cr²⁺, Cr³⁺, Mo²⁺, Mo.³⁺, W²⁺, W³⁺, Mn²⁺, Mn²⁺, Mn⁴⁺, Rh³⁺, Rh⁴⁺, Re²⁺, Re³⁺, Co¹⁺, Co.²⁺, Co³⁺, V²⁺, V³⁺, Zn.¹⁺, Zn²⁺, Ni²⁺, Ni³⁺, Au¹⁺, Au²⁺, Ag¹⁺ and Ag²⁺. Suitable salts include, but are not limited to: halogen, C₁ - C₆ -alkoxy, sulfates, phosphate, triflate, hexafluorophosphate, methanesulphonate, arylsulphonate salts. In some embodiments the catalyst is a chloride, bromide salts of the above-recited metal ions. In other embodiments the catalyst is CuBr, CuCl or RuCl₂.

In some embodiments, the use of one or more ligands to solubilize transition metal catalysts is desirable. Suitable ligands are usefully used in combination with a variety of transition metal catalysts including where copper chloride or bromide, or ruthenium chloride transition metal salts are part of the catalyst. The choice of a ligand affects the function of catalyst as ligands not only aid in solubilizing transition metal catalysts in organic reaction media, but also adjust their redox potential. Selection of a ligand is also based upon the solubility and separability of the catalyst from the product mixture. Where polymerization is to be carried out in a liquid phase soluble ligands/catalyst are generally desirable although immobilized catalysts can be employed. Suitable ligands include those pyridyl groups (including alkyl pyridines *e.g*., 4.4. dialkyl-2,2' bipyridines) and pyridyl groups bearing an alkyl substituted imino group, where present, longer alkyl groups provide solubility in less polar monomer mixtures and solvent media. Triphenyl phosphines and other phosphorus ligands, in addition to indanyl, or cyclopentadienyl ligands, can also be employed with transition metal catalysts (*e.g*., Ru⁺²-halide or Fe⁺²-halide complexes with triphenylphosphine, indanyl or cyclopentadienyl ligands).

An approximately stoichiometric amount of metal compound and ligand in the catalyst, based on the molar ratios of the components when the metal ion is fully complexed, is employed in some embodiments. In other embodiments the ratio between metal compound and ligand is in the range 1:(0.5 to 2) or in the range 1:(0.8 to 1.25).

Generally, where the catalyst is copper, bidentate or multidentate nitrogen ligands produce more active catalysts. In addition, bridged or cyclic ligands and branched aliphatic polyamines provide more active catalysts than simple linear ligands. Where bromine is the counter ion, bidentate or one-half tetradentate ligands are needed per Cu⁺¹. Where more complex counter ions are employed, such as triflate or hexafluorophosphate, two bidentate or one tetradentate ligand can be employed. The addition of metallic copper can be advantageous in some embodiments particularly where faster polymerization is desired as metallic copper and Cu⁺² may undergo redox reaction to form Cu⁺¹. The addition of some Cu⁺² at the beginning of some ATRP reactions can be employed to decrease the amount of normal termination.

In some embodiments, the amount of catalyst employed in the polymerization reactions is the molar equivalent of the initiator that is present. Since catalyst is not consumed in the reaction, however, it is not essential to include a quantity of catalyst as high as of initiator. The ratio of catalyst to each halogen contained in the initiator, based on transition metal compound in some embodiments is from about 1:(1 to 50), in other embodiments from about 1:(1 to 10), in other embodiments from about 1:(1 to 5), and in other embodiments from 1:1.

### Polymerization Conditions

In some embodiments, the living radical polymerization process of the invention is preferably carried out to achieve a degree of polymerization in the range of 3 to about 2000, and in other embodiments from about 5 to about 500. The degree of polymerization in other embodiments is in the range 10 to 100, or alternatively in the range of about 10 to about 50. The degree of polymerization in group or atom transfer radical polymerization technique, is directly related to the initial ratio of initiator to monomer. Therefore, in some embodiments the initial ratios of initiator to monomer are in the range of 1:(3 to about 2,000) of about 1:(5 to 500), or about 1:(10 to 100), or about 1:(10 to 50).

Polymerization reactions are typically carried out in the liquid phase, employing a single homogeneous solution. The reaction may, however, be heterogeneous comprising a solid and a liquid phase (*e.g*., a suspension or aqueous emulsion). In those embodiments where a non-polymerizable solvent is employed, the solvent employed is selected taking into consideration the nature of the zwitterionic monomer, the initiator, the catalyst and its ligand; and in addition, any comonomer that can be employed.

The solvent may comprise a single compound or a mixture of compounds. In some embodiments the solvent is water, and in other embodiments water is present in an amount from about 10% to about 100% by weight, based on the weight of the monomers present in the reaction. In those embodiments where a water insoluble comonomer is to be polymerized with a zwitterionic monomer, it can be desirable to employ a solvent or co-solvent (in conjunction with water) that permits solubilization of all the monomers present. Suitable organic solvents include, without limitation, formamides (*e*.*g*., N,N'-dimethylformamide), ethers (*e.g.*, tetrahydrofuran), esters (ethyl acetate) and, most preferably, alcohols. In some embodiments where a mixture of water and organic solvent is to be employed, C₁-C₄ water miscible alkyl alcohols (methanol, ethanol, propanol, isopropanol, butanol, isobutanol, and tertbutanol) are useful organic solvents. In other embodiments, water and methanol combinations are suitable for conducting polymerization reactions. The reaction may also be conducted in supercritical solvents such as CO₂.

As noted above, in some embodiments it is desirable to include water in the polymerization mixture in an amount from about 10% to about 100% by weight based on the weight of monomers to be polymerized. In other embodiments the total non-polymerizable solvent is from about 1% to about 500% by weight, based on the weight of the monomers present in the reaction mixture. In other embodiments, the total non-polymerizable solvent is from about 10% to about 500% by weight or alternatively from 20% to 400%, based on the weight of the monomers present in the reaction mixture. It is also desirable in some cases to manipulate the solubility of an input reagent, such as initiator or monomer, for example by modifying temperature or solvent or other method so as to modify the reaction conditions in a dynamic fashion.

In some embodiments, contact time of the zwitterionic monomer and water prior to contact with the initiator and catalyst are minimized by forming a premix comprising all components other than the zwitterionic monomer and for the zwitterionic monomer to be added to the premix last.

The polymerization reactions can be carried out at any suitable temperature. In some embodiments the temperature can be from about ambient (room temperature) to about 120° C. In other embodiments the polymerizations can be carried out at a temperature elevated from ambient temperature in the range of about 60° to 80° C. In other embodiments the reaction is carried out at ambient (room temperature).

In some embodiments, the compounds of the invention have a polydispersity (of molecular weight) of less than 1.5, as judged by gel permeation chromatography. In other embodiments the polydispersities can be in the range of 1.2 to 1.4. In still other embodiments, the polydispersities can be less than 1.2.

A number of workup procedures can be used to purify the polymer of interest such as precipitation, fractionation, reprecipitation, membrane separation and freeze-drying of the polymers.

### Non-Halogenated Polymer Terminus

In some embodiments, it can be desirable to replace the halogen, or other initiator fragment **I**', with another functionality. A variety of reactions can be employed for the conversion of the aliphatic halogen. In some embodiments, the conversion of the aliphatic halogen can include reaction to prepare an alkyl, alkoxy, cycloalkyl, aryl, heteroaryl or hydroxy group. Halogens can also be subject to an elimination reaction to give rise to an alkene (double bond). Other methods of modifying the halogenated terminus are described in Matyjaszewski et al. Prog. Polym. Sci. 2001, 26, 337, incorporated by reference in its entirety herein.

### Attachment of Functional agents

The coupling of functional agents to the high MW polymers of the present invention can be conducted employing chemical conditions and reagents applicable to the reactions being conducted. Exemplary methods are described in Bioconjugate Techniques, Greg T. Hermanson, Academic Press, 2d ed., 2008 (incorporated in its entirety herein). Other bioconjugation techniques are described in Bertozzi et al. Angewandte Chemie 2009, 48, 6974, and Gauthier et al. Chem. Commun. 2008, 2591, each incorporated by reference in its entirety herein.

Where, for example, the coupling requires the formation of an ester or an amide, dehydration reactions between a carboxylic acid and an alcohol or amine may employ a dehydrating agent (*e.g*., a carbodiimide such as dicyclohexylcarbodimide, DCC, or the water soluble agent 1-ethyl-3-(3-dimethyllaminopropyl)carbodiimide hydrochloride, EDC). Alternatively, N-hydroxysuccinimide esters (NHS) can be employed to prepare amides. Reaction to prepare amides employing NHS esters are typically conducted near neutral pH in phosphate, bicarbonate, borate, HEPES or other non-amine containing buffers at 4° to 25° C. In some embodiments, reactions employing EDC as a dehydrating agent, a pH of 4.5-7.5 can be employed; in other embodiments, a pH of 4.5 to 5 can be employed. Morpholinoethanesulfonic acid, MES, is an effective carbodiimide reaction buffer.

Thiol groups can be reacted under a variety of conditions to prepare different products. Where a thiol is reacted with a maleimide to form a thioether bond, the reaction is typically carried out at a pH of 6.5-7.5. Excess maleimide groups can be quenched by adding free thiol reagents such as mercaptoethanol. Where disulfide bonds are present as a linkage, they can be prepared by thiol-disulfide interchange between a sulfhydryl present in the bioactive group and an X functionality which is a disulfide such as a pyridyl disulfide. Reactions involving pyridyl disulfides can be conducted at pH 4 - pH 5 and the reaction can be monitored at 343 nm to detect the released pyridine-2-thione. Thiol groups may also be reacted with epoxides in aqueous solution to yield hydroxy thioethers. A thiol may also be reacted at slightly alkaline pH with a haloacetate such as iodoacetae to form a thioether bond.

The reaction of guanido groups (*e.g.*, those of an arginine in a protein or polypeptide of interest) with a glyoxal can be carried out at pH 7.0-8.0. The reaction typically proceeds at 25° C. The derivative, which contains two phenylglyoxal moieties per guanido group, is more stable under mildly acidic conditions (below pH 4) than at neutral or alkaline pHs, and permits isolation of the linked materials. At neutral or alkaline pH values, the linkage decomposes slowly. Where an arginine residue of a protein or polypeptide is reacted with a phenylglyoxal reagent, about 80% of the linkage will hydrolyze to regenerate the original arginine residue (in the absence of excess reagent) in approximately 48 hours at 37° at about pH 7.

Imidoester reactions with amines are typically conducted at pH of 8-10, and preferably at about pH 10. The amidine linkage formed from the reaction of an imidoester with an amine is reversible, particularly at high pH.

Haloacetals can be reacted with sulfhydryl groups over a broad pH range. To avoid side reactions between histidine residues that can be present, particularly where the sulfhydryl group is present on a protein or polypeptide, the reaction can be conducted at about pH 8.3.

Aldehydes can be reacted with amines under a variety of conditions to form imines. Where either the aldehyde or the amine is immediately adjacent to an aryl group the product is a Schiff base that tends to be more stable than where no aryl group is present. Conditions for the reaction of amines with aldehydes to form an imine bond include the use of a basic pH from about pH 9 to about pH 11 and a temperature from about 0° C to room temperature, over 1 to 24 hours. Alternatively, where preferential coupling to the N-terminal amine of a protein is desired, lower pHs from about 4-7 can be employed. Buffers including borohydride and tertiary amine containing buffers are often employed for the preparation of imines. Where it is desired imine conjugates, which are hydrolytically susceptible, can be reduced to form an amine bond which is not hydrolytically susceptible. Reduction can be conducted with a variety of suitable reducing agents including sodium borohydride or sodium cyanoborohydride.

The reaction conditions provided above are intended to provide general guidance to the artisan. The skilled artisan will recognize that reaction conditions can be varied as necessary to promote the attachment of the functional agent to the high MW polymers of the present invention and that guidance for modification of the reactions can be obtained from standard texts in organic chemistry. Additional guidance can be obtained from texts such as Wong, S.S., "Chemistry of Protein Conjugation and Cross-Linking," (CRC Press 1991), which discuss related chemical reactions.

Different recombinant proteins have been shown to conjugate successfully to a wide variety of polymers of the present invention of different sizes and architectures via different conjugation chemistries. Many lessons have been learned during the course of process development efforts (conjugation, downstream processing, analytical development) and some unique features of the technology are described below. The conjugate refers exclusively to protein or other therapeutic agents conjugated covalently to the polymers of the present invention.

In the area of conjugation reactions, low polymer molar excess ratios of 1 - 2 fold are useful in order to obtain good conjugation efficiency. In order to achieve low polymer molar excess and yet maintain good conjugation efficiency (>20%), protein concentration should be much higher than the normally acceptable concentration of 1 - 2 mg/ml. The concentration that can be achieved for any one particular protein used will depend on the stability and biophysical properties of that protein. Exemplary ranges include 5 - 10 mg/ml, 10 - 15 mg/ml, 15 - 20 mg/ml, 20 - 25 mg/ml, 25 - 30 mg/ml, 30 - 50 mg/ml, 50 - 100 mg/mL, >100 mg/ml.

On the other side of the reaction, a major challenge is the concentration of polymer which is also required to be at a very high level for optimal conjugation efficiencies, a normal concentration being upwards of 100 mg/ml. Interestingly, the polymers of this invention demonstrate extreme solubility with low viscosity even at concentrations in excess of 500 mg/ml. This feature makes it possible to manipulate the conjugation reaction such as mixing very easily whereas with other polymers such as PEG at such a concentration the solution is too viscous to be handled. The use of a variety of devices to improve mixing further improves the process. For example, an ultrasonic bath with temperature control can be used for initial mixing in order to facilitate polymer solubilization and in turn improve conjugation efficiency. Alternative ultrasonic devices such as VialTweeter from HielscherUltrasonic GmbH improve the efficiency with which ultrasonic energy is delivered compared with an ultrasonic bath. From a theoretical point of view, the ultrasonic wave creates an oscillation wave that facilitates the interaction between polymer and protein. This translates into higher and better conjugation efficiency. The addition of a temperature controlled mechanism such as a cooling system protects heat labile proteins in this system. To scale up such a process to large industrial scale (e.g. kilogram or greater scale), other instrumentation such as the resonant acoustic mixing technology developed by Resodyn is useful. In fact, this type of mixer has been successfully used to solubilize highly viscous polymers and fluids with viscosity over 1,000 cP. The polymers of this invention at the highest practical concentration are just a fraction of such a viscosity level and therefore render the resonant acoustic mixing technology particularly attractive. Additional advantages of such technology include non-invasive and fully concealable character as well as fast mixing time. These properties make it highly desirable for protein pharmaceutics generally and for combination with the technology of this invention specifically.

Undesirable poly-PEGylated conjugation byproducts have long been an issue in the industry which increases the cost of goods during manufacturing while also increasing regulatory complexity and product approval hurdles. Interestingly, many different purified conjugates derived from all the polymers of this invention and which have been tested always result in an equal molar ratio between protein and polymer. This is a unique and highly desirable feature as compared to other polymer and conjugation technologies.

In the area of downstream processing, as described previously, the preferred polymers of this invention are net charge neutral due to their zwitterionic nature. Therefore, they do not interact with anion or cation ion exchange resins under any chromatographic conditions including wide ranges of pH and ionic strength. In other words, the free polymer will flow through any ion exchanger irrespective of pH and ionic strength. However, upon conjugation to different proteins, the chromatographic behavior of the conjugate is dictated by the protein. Due to the presence of the polymer shielding effect and altered charge of the protein during the conjugation chemistry, the interaction of the conjugate with the ion exchange resin is weakened as compared to the native protein. This property is observed for basic and acidic proteins that interact with cation and anion exchanger resins, respectively. These are also highly desirable properties from a manufacturing point of view as they allow for the design of a highly efficient, simple, cost-effective, and orthogonal purification method for separation of conjugate from the product releated contaminants which include: unreactive free polymer, unreacted free proteins and aggregates; and process contaminants such as endotoxin, conjugation reactants and additives. A single ion exchange chromatographic step is sufficient.

For example, for an acidic protein conjugate where the conjugation reaction is carried out at low ionic strength (e.g. 0-20mM NaCl) with buffer pH higher than the pI of the protein, upon completion of the conjugation reaction, the contents of the conjugation reaction vessel can be applied directly over the anion exchanger resin (e.g. Q type IEX resin) where the unreacted free polymer will flow through the resin, the column can then be chased and washed with low ionic strength buffer at the same pH similar to the conjugation reaction. The bound fraction can then by eluted stepwise with increasing salt concentrations. The first protein fraction is the pure conjugate as it binds more weakly to the ion exchange resin as compared to the native protein and other contaminants such as aggregates and endotoxin. A step gradient is highly desirable as this minimizes the potential risk that the native protein will leach out from the column. For example, using a strong anion exchange resin, a cytokine polymer conjugate will elute around 30-60mM NaCl at pH 7 while the native cytokine will not elute until 100mM or higher; under such conditions, the dimeric and aggregated form of the cytokine typically elutes at 200mM NaCl or higher; and finally the endotoxin elutes at an even higher salt concentration.

For a basic protein conjugate, the separation is accomplished using a cation exchanger (e.g. SP type IEX resin) at low ionic strength (e.g. 0-20mM NaCl) with buffer pH lower than the pI of the protein. In this process, the unreacted free polymer will still be in the flow through fraction together with endotoxin and other negatively charged contaminants while the conjugate and free unreacted protein remain bound to the column. By increasing the ionic strength of the elution buffer, the first protein fraction eluted is the conjugate due to the weaker interaction with the IEX resin as compared to the native protein. A typical Fab' conjugate will elute at 30-60mM NaCl while the native Fab' will elute at 100-200mM NaCl.

The experience with purifying many different protein conjugates including both acidic protein conjugates (such as cytokines and scaffold-based multi-domain based proteins) and basic protein conjugates (such as Fab') show that the ionic strength required for conjugate elution is largely independent of polymer size (even greater than one million daltons) and architecture (multi-armed architectures). This is a highly desirable feature of the platform technology that enables the design of a generic manufacturing process where major process development efforts are not required with changes in polymers and to some extent therapeutic agents.

From the manufacturing point of view, the above described downstream purification process has the following advantages:
1. Highly scalable;
2. Amenable to current commercial production processes as the resins are available commercially and the required instrumentation is already at industrial standard;
3. The sample technique can be used for both In Process Analytics (IPA) as well as scale up production;
4. Development of a generic process is feasible;
5. Cost effective due to its single step nature and orthogonal design;
6. Excellent recovery (current process yields are upwards of 80%).

In the area of analytical development, the zwitterionic nature of the polymers of this invention has two impacts on development of SDS-PAGE analysis of conjugates. Firstly, SDS-PAGE analysis has long been a ubiquitous and convenient method for protein analysis, in that it provides a fast, high resolution, high throughput and low cost method for semiquantitative protein characterization. However, the net charge neutral property and also the large hydrodynamic radius of the polymer means that the polymer migrates poorly or (for very large size polymers) almost not at all into a polyacrylamide matrix even with as low as a 4% gel. Secondly, the polymers of this invention are not stainable by Coomassie Blue type stains, potentially due to their net charge neutral property which prevents the Coomassie Blue dye from interacting with the polymer. However, once the protein is conjugated to the polymer, the conjugate becomes stainable. These are two undesirable properties for most protein biochemists at first glance; however, the combination of these two properties allows for the design of a highly desirable and unique technique that enables quick and easy analysis of conjugation efficiency directly from the reaction mixture without further purification. In this technique, the conjugation reaction mixture is loaded onto the SDS-PAGE gel and separated as per standard protocol. Then the gel is stained with Coomassie Blue and then destained according to the standard protocol. The presence of the conjugate will display Coomassie blue stained bands close to the loading well while the smaller protein migrates at its molecular weight and will display concomitant reduction in band intensity as compared to a control reaction without polymer. It is therefore very easy to distinguish those reactions with inefficient conjugation as the polymer alone will not display any staining at the high molecular weight region of the gel. It should be noted that such a technique for conjugation reaction analysis is impossible for PEGylation reaction as both the PEG polymer and PEGylated proteins stain by Coomassie Blue and migrate at a very similar position in the gel, especially the very large PEG polymers; in addition, PEG polymers display the highly undesirable property of distorting the migration pattern of SDS-PAGE gels. This latter problem is not observed for the polymers of this invention, as the net charge neutral property of the unreacted free polymer renders them unlikely to enter the gel matrix (whereas only the conjugate and unconjugated free protein will do so).

Another interesting property of the polymers of this invention is that they do not have UV 280nm absorbance due to the absence of an aromatic group. However, they do absorb at 220nm. There is at least 10x lower absorbance for the polymer when compared with an equal mass concentration of protein solution. This is very useful when trying to identify the presence of conjugate in the conjugation reaction mixture using different chromatographic methods such as size exclusion or IEX analysis. By comparing the UV280/UV220 ratio, it is very easy to identify the presence of conjugate as the ratio increases dramatically. The same technique can be used for both analytical scale and production scale monitoring of product elution.

### V. Compositions

The present invention includes and provides for pharmaceutical compositions comprising one or more compounds of the invention and one or more pharmaceutically acceptable excipients. The compounds of the invention may be present as a pharmaceutically acceptable salt, prodrug, metabolite, analog or derivative thereof, in the pharmaceutical compositions of the invention. As used herein, "pharmaceutically acceptable excipient" or "pharmaceutically acceptable carrier" is intended to include any and all solvents, dispersion media, coatings, antibacterial and antifungal agents, isotonic and absorption delaying agents, and the like, compatible with pharmaceutical administration.

Pharmaceutically acceptable carriers for use in formulating the high MW polymers of the present invention include, but are not limited to: solid carriers such as lactose, terra alba, sucrose, talc, gelatin, agar, pectin, acacia, magnesium stearate, stearic acid and the like; and liquid carriers such as syrups, saline, phosphate buffered saline, water and the like. Carriers may include any time-delay material known in the art, such as glyceryl monostearate or glyceryl distearate, alone or with a wax, ethylcellulose, hydroxypropylmethylcellulose, methylmethacrylate or the like.

Other fillers, excipients, flavorants, and other additives such as are known in the art may also be included in a pharmaceutical composition according to this invention. The use of such media and agents for pharmaceutically active substances is well known in the art. Except insofar as any conventional media or agent is incompatible with the active compound, use thereof in the compositions of the invention is contemplated. Supplementary active compounds can also be incorporated into the compositions of the present invention.

The pharmaceutical preparations encompass all types of formulations. In some embodiments they are parenteral (including subcutaneous, intramuscular, intravenous, intradermal, intraperitoneal, intrathecal, intraventricular, intracranial, intraspinal, intracapsular, and intraosseous) formulations suited for injection or infusion (*e*.*g*., powders or concentrated solutions that can be reconstituted or diluted as well as suspensions and solutions). Where the composition is a solid that requires reconstitution or a concentrate that requires dilution with liquid media, any suitable liquid media may be employed. Preferred examples of liquid media include, but are not limited to, water, saline, phosphate buffered saline, Ringer's solution, Hank's solution, dextrose solution, and 5% human serum albumin.

Where a compound or pharmaceutical composition comprising a high MW polymer of the present invention is suitable for the treatment of cell proliferative disorders, including but not limited to cancers, the compound or pharmaceutical composition can be administered to a subject through a variety of routes including injection directly into tumors, the blood stream, or body cavities.

While the pharmaceutical compositions may be liquid solutions, suspensions, or powders that can be reconstituted immediately prior to administration, they may also take other forms. In some embodiments, the pharmaceutical compositions may be prepared as syrups, drenches, boluses, granules, pastes, suspensions, creams, ointments, tablets, capsules (hard or soft) sprays, emulsions, microemulsions, patches, suppositories, powders, and the like. The compositions may also be prepared for routes of administration other than parenteral administration including, but not limited to, topical (including buccal and sublingual), pulmonary, rectal, transdermal, transmucosal, oral, ocular, and so forth. Needle free injection devices can be used to achieve subdermal, subcutaneous and/or intramuscular administration . Such devices can be combined with the polymers and conjugates of this invention to administer low (<20 cP), medium (20 - 50 cP), and high (>100 cP) viscosity formulations.

In some embodiments, the pharmaceutical compositions of the present invention comprise one or more high MW polymers of the present invention.

Other pharmaceutical compositions of the present invention may comprise one or more high MW polymers of the present invention that function as biological ligands that are specific to an antigen or target molecule. Such compositions may comprise a high MW polymer of the present invention, where the bioactive agent is a polypeptide that comprises the amino acid sequence of an antibody, or an antibody fragment such as a FAb₂ or FAb' fragment or an antibody variable region. Alternatively, the compound may be a high MW polymer and the polypeptide may comprise the antigen binding sequence of a single chain antibody. Where a bioactive agent present in a high MW polymer of the present invention functions as a ligand specific to an antigen or target molecule, those compounds may also be employed as diagnostic and/or imaging reagents and/or in diagnostic assays.

The amount of a compound in a pharmaceutical composition will vary depending on a number of factors. In one embodiment, it may be a therapeutically effective dose that is suitable for a single dose container (*e*.*g*., a vial). In one embodiment, the amount of the compound is an amount suitable for a single use syringe. In yet another embodiment, the amount is suitable for multi-use dispensers (*e*.*g*., containers suitable for delivery of drops of formulations when used to deliver topical formulations). A skilled artisan will be able to determine the amount a compound that produces a therapeutically effective dose experimentally by repeated administration of increasing amounts of a pharmaceutical composition to achieve a clinically desired endpoint.

Generally, a pharmaceutically acceptable excipient will be present in the composition in an amount of about 0.01% to about 99.999% by weight, or about 1% to about 99% by weight. Pharmaceutical compositions may contain from about 5% to about 10%, or from about 10% to about 20%, or from about 20% to about 30%, or from about 30% to about 40%, or from about 40% to about 50%, or from about 50% to about 60%, or from about 60% to about 70%, or from about 70% to about 80%, or from about 80% to about 90% excipient by weight. Other suitable ranges of excipients include from about 5% to about 98%, from about from about 15 to about 95%, or from about 20% to about 80% by weight.

Pharmaceutically acceptable excipients are described in a variety of well known sources, including but not limited to "Remington: The Science & Practice of Pharmacy", 19th ed., Williams & Williams, (1995) and Kibbe, A. H., Handbook of Pharmaceutical Excipients, 3rd Edition, American Pharmaceutical Association, Washington, D.C., 2000.

### VI. Methods

The high MW polymers of the present invention are useful for treating any disease state or condition. The disease state or condition can be acute or chronic.

Disease states and conditions that can be treated using the high MW polymers of the present invention include, but are not limited to, cancer, autoimmune disorders, genetic disorders, infections, inflammation, neurologic disorders, and metabolic disorders.

Cancers that can be treated using the high MW polymers of the present invention include, but are not limited to, ovarian cancer, breast cancer, lung cancer, bladder cancer, thyroid cancer, liver cancer, pleural cancer, pancreatic cancer, cervical cancer, testicular cancer, colon cancer, anal cancer, bile duct cancer, gastrointestinal carcinoid tumors, esophageal cancer, gall bladder cancer, rectal cancer, appendix cancer, small intestine cancer, stomach (gastric) cancer, renal cancer, cancer of the central nervous system, skin cancer, choriocarcinomas; head and neck cancers, osteogenic sarcomas, fibrosarcoma, neuroblastoma, glioma, melanoma, leukemia, and lymphoma.

Autoimmune diseases that can be treated using the high MW polymers of the present invention include, but are not limited to, multiple sclerosis, myasthenia gravis, Crohn's disease, ulcerative colitis, primary biliary cirrhosis, type 1 diabetes mellitus (insulin dependent diabetes mellitus or IDDM), Grave's disease, autoimmune hemolytic anemia, pernicious anemia, autoimmune thrombocytopenia, vasculitides such as Wegener's granulomatosis, Behcet's disease, rheumatoid arthritis, systemic lupus erythematosus (lupus), scleroderma, systemic sclerosis, Guillain-Barre syndromes, Hashimoto's thyroiditis spondyloarthropathies such as ankylosing spondylitis, psoriasis, dermatitis herpetiformis, inflammatory bowel diseases, pemphigus vulgaris and vitiligo.

Some metabolic disorders treatable by the high MW polymers of the present invention include lysosomal storage disorders, such as mucopolysaccharidosis IV or Morquio Syndrome, Activator Deficiency/GM2 Gangliosidosis, Alpha-mannosidosis, Aspartylglucosaminuria, Cholesteryl ester storage disease, Chronic Hexosaminidase A Deficiency, Cystinosis, Danon disease, Fabry disease, Farber disease, Fucosidosis, Galactosialidosis, Gaucher Disease, GM1 gangliosidosis, hypophosphatasia, I-Cell disease/Mucolipidosis II, Infantile Free Sialic Acid Storage Disease/ISSD, Juvenile Hexosaminidase A Deficiency, Krabbe disease, Metachromatic Leukodystrophy, Mucopolysaccharidoses disorders such as Pseudo-Hurler polydystrophy/Mucolipidosis IIIA, Hurler Syndrome, Scheie Syndrome, Hurler-Scheie Syndrome, Hunter syndrome, Sanfilippo syndrome, Morquio, Hyaluronidase Deficiency, Maroteaux-Lamy, Sly Syndrome, Mucolipidosis I/Sialidosis, Mucolipidosis, and Mucolipidosis, Multiple sulfatase deficiency, Niemann-Pick Disease, Neuronal Ceroid Lipofuscinoses, Pompe disease/Glycogen storage disease type II, Pycnodysostosis, Sandhoff disease, Schindler disease, Salla disease/Sialic Acid Storage Disease, Tay-Sachs/GM2 gangliosidosis and Wolman disease.

Conjugates of the invention and compositions (e.g., pharmaceutical compositions) containing conjugates of the invention can be used to treat a variety of conditions. For example, there are many conditions for which treatment therapies are known to practitioners of skill in the art in which functional agents, as disclosed herein, are employed. The invention contemplates that the conjugates of the invention (e.g., phosphorylcholine containing polymers conjugated to a variety of functional agents) and compositions containing the conjugates of the invention can be employed to treat such conditions and that such conjugates provide for an enhanced treatment therapy relative to the same functional agent not coupled to a phosphorylcholine containing polymer.

Therefore, the invention contemplates the treatment of a condition known to be treatable by a certain bioactive agent by treating the condition using the same certain bioactive agent conjugated to a phosphorylcholine containing polymer.

Another aspect of the present invention relates to methods of treating a condition responsive to a biological agent comprising administering to a subject in need thereof a therapeutically effective amount of a compound of the invention or of a pharmaceutically acceptable composition of the invention as described above. Dosage and administration are adjusted to provide sufficient levels of the bioactive agent(s) to maintain the desired effect. The appropriate dosage and/or administration protocol for any given subject may vary depending on various factors including the severity of the disease state, general health of the subject, age, weight, and gender of the subject, diet, time and frequency of administration, drug combination(s), reaction sensitivities, and tolerance/response to therapy. Therapeutically effective amounts for a given situation can be determined by routine experimentation that is within the skill and judgment of the clinician.

The pharmaceutical compositions described herein may be administered singly. Alternatively, two or more pharmaceutical compositions may be administered sequentially, or in a cocktail or combination containing two high MW polymers of the present invention or one high MW polymer of the present invention and another bioactive agent. Other uses of bioactive agents set forth herein may be found in standard reference texts such as the Merck Manual of Diagnosis and Therapy, Merck & Co., Inc., Whitehouse Station, NJ and Goodman and Gilman's The Pharmacological Basis of Therapeutics, Pergamon Press, Inc., Elmsford, N.Y., (1990).

This invention describes the modification of hematology related proteins such as Factor VIII, Factor VII, Factor IX, Factor X and proteases such as serine proteases of native sequence or mutein sequence and of native function or altered (for example via phage display, reference Catalyst Biosciences of South San Francisco with technology to alter specificity of binding of an existing enzyme). US Patent 7,632,921 is included in its entirety herein. Modification of the enzyme to allow for site-specific conjugation of a functionalized polymer is disclosed. The use of flexible chemistries between the polymer and the enzyme is disclosed, such that the protein can be released *in vivo* in the proper setting, for example to enable close to a zero order release profile. A target product profile for a next generation Factor VIII could involve a covalent conjugate of recombinant FVIII or recombinant B-domain deleted FVIII to which an extended form, multi-arm zwitterion-containing polymer of greater than 50 kDa molecular weight is attached to a site-specific amino acid such as a cysteine. The clinical pharmacology of the conjugate would demonstrate unparalled water structuring to shield the conjugate from clearance and immune systems. The conjugate would demonstrate greater than a 50 hour elimination half life in humans (preferably greater than 80 hours). The conjugate would demonstrate a 2x (preferably 4x) increased half-life versus a 60 kDa PEG-BDD FVIII with the same bioactivity. The conjugate as used in patients would show clinically insignificant antibody formation. The biopharmaceutical conjugate would be used both prophylactically (once weekly or less frequent) and for on demand treatment of patients with Hemophilia. It would also be used as rescue therapy for patients with existing FVIII neutralizing antibodies, for example from prior FVIII biopharmaceutical therapy. The drug would enable a liquid formulation for IV and/or subcutaneous administration and with high stability, high concentration, and low viscosity. Active ingredient could be in the range of 250 to 2,000 IU composed of 30 to 250 microgram of polymer drug conjugate in a nominal volume ideally of 0.4ml. The cost of the polymer would be low, and the conjugation efficiency of the polymer to the FVIII or BDD FVIII protein would be very high, for example upwards of 75%. Such a product and product profile would make use of the extreme biocompatibility of the polymer and as transferred onto the protein. Specifically, the extreme biocompatibility would manifest itself with very tight water binding, extreme solubility, very high concentration, very low viscosity, and extreme stability. Technically, this translates into a >2x (or ideally >4x) increased elimination half-life versus PEGylation or its equivalent technologies, extremely low or no immunogenicity, high concentration, and room temperature stable liquid formulations. Product profile benefits include less frequent dosing, lower dose for same Area Under the Curve, effective safe treatment for naive patients, rescue therapy for patients with neutralizing antibodies, at home subcutaneous administration, pre-filled syringe/autoinjector with room temperature storage, higher gauge (lower diameter) syringe needles, lower injection volumes, and longer shelf lives. On the manufacturing front, single pot synthesis, very high polymer molecular weights, complex architectures, and low cost to manufacture are achievable. Furthermore, high efficiency conjugation of polymer to drug is possible. These manufacturing benefits can translate into cheaper, more available medicines and higher gross margins.

This invention describes attaching high MW zwitterion-containing polymers to multimers of recombinant modified LDL receptor class A domains or relevant consensus sequences as described in US patent application 60/514,391 assigned to Avidia. Those skilled in the art will understand that the avimers can be lysine depleted and then lysines and/or other amino acids added to the N- and/or C- termini for site-specific attachment of a functionalized polymer. An N-terminal lysine is preferably the second amino acid (after methionine) and can drive relative site specific conjugation of an amine-driven initiator such as a-functionalized polymer containing an aldehyde or acetal group. Those skilled in the art will also know the benefit of avimer compositions with relatively hydrophilic amino acids and low pI and high stability, such that pH can be driven very low in the conjugation reaction such as to preferentially conjugate to the amine of the lysine rather than multi-point attachments that also conjugate to N-terminal amine group or other amine groups present in the protein. The therapeutic can have one polymer conjugated to the N- terminus and another conjugated to the C- terminus via a C-terminal lysine (an effective branched structure). Such an avimer can also be made in mammalian systems with an extra N- or C- terminal cysteine added for site specific conjugation with a thiol-reacting functionalized polymer. The polymer's functional group can also contain tissue targeting elements. The chemistry attaching the polymer to the avimer can be flexible such that it breaks *in vivo,* for example in serum or in a pH responsive manner, etc. Monomers and multimers composed of other domains of interest used similarly include EGF domains, Notch/LNR domains, DSL domains, Anato domains, integrin beta domains or such other domains as described in the referenced patent family.

This invention also describes the attachment of high MW zwitterion-containing polymers to peptides and synthetic peptides and especially longer synthetic peptides with multiple domains. A big problem with multiple domain peptides is that they are unstable and also have very rapid clearance. The attachment of a highly biocompatible zwitterion-containing polymer such as those described in this invention solves these problems. The polymer increases the stability and also increases the *in vivo* residence time. This enables simple linear (unstructured) peptides as drugs, for example modules of around twenty amino acids per functional module in series of two, three, four or more modules with the goal to achieve avidity benefit or multifunctionality benefit. Each module could also have a bit of structure ('constrained' peptide like) or each module could actually be a knotted peptide domain such as a cysteine knot or macrocyclic element. The key is they are made synthetically and can be strung together with a site specific moiety for polymer conjugation at N- terminal or C-terminal (or both) or with the polymer conjugation point in the middle, which attachment point can be a site specific amino acid that is a natural amino acid or a non-natural amino acid. In a sense, this is a synthetic avimer with preferential properties. All of the amino acids could be synthetic, as well. Such a peptide plus the polymers of this invention describe a novel and powerful drug format of the future.

Those skilled in the art will understand that the breadth of application of the high molecular weight polymers of this invention is very broad. A partial list of therapeutic modalities that can benefit from conjugation of such polymers consists of: avimer (LDL receptor A-domain scaffold), adnectin (fibronectin type III scaffold), Ablynx (camelid, llama-ids), NAR's (shark), one-arm and/or single domain antibodies from all species (rat, rabbit, shark, llama, camel, other), diabodies, other multi-domain based proteins such as multimers of modified fibronectin domains, antibody fragments (scFv monomer, scFv dimers as agonists or antagonists), Fab's, Fab'-2's, soluble extracellular domains (sTNFR1, for example, or soluble cMet receptor fragment), combination with Amunix XTEN which comprises a hydrophilic amino acid string of up to 1,500 amino acids made as part of the open reading frame, oligonucleotides such as aptamers, microRNA, siRNA, whole antibodies (conjugated to Fc- region ; conjugated to non-Fc regions), Fc-fusions (conjugated to Fc-region; conjugated to fused protein), the use of such polymers as a replacement for the CovX antibody backbone (where high molecular weight polymer is conjugated directly to the peptide itself), more broadly the attachment of the polymers of this invention even to a full-length natural or mutein antibody (CovX body, Peptibody, humanized or other antibody, the new Zyngenia platform from Carlos Barbas where peptides are conjugated to different locations on the antibody to create modular multifunctional drugs on top of an antibody backbone). Also the many domain structures as outlined in US Patent Application 60/514,391 are included in their entirety herein. Of particular interest are conjugates for binding to and inhibiting cell-surface targets, in which setting the large size, extended form architectures, and slow off rates of the polymer conjugates described in this invention can have a particularly advantageous biological effect.

This invention describes conjugates for ophthalmic and preferentially intravitreal or subconjunctival administration that have an intravitreal mean terminal half live of greater than 10 days as measured by physical presence of active conjugate. The active conjugate can also contain two functional agents, covalently attached proximally at one end of the polymer. In this case the two functional agents could be aptamers to VEGF and PDGF for the treatment of wet and dry age-related macular degeneration.

This invention contemplates conjugation of the high MW polymers of the invention to GLP-1, soluble TACI receptor, BAFF as well as inhibitors of BAFF, insulin and its variants, IL-12 mutein (functional anti-IL-23 equivalent), anti-IL-17 equivalent, FGF21 and muteins, RANK ligand and its antagonists, factor H and fusion proteins for inhibiton of alternative complement (Taligen), inhibitors of the immune synapse, activators of the immune synapse, inhibitors of T- cell and/or B /cell costimulatory pathways, activators or inhibitors of neuronal cells and/or their supporting matrix cells, extracellular matrix enzymes such as lysyl oxidase or metalloproteinase/metalloproteases, activators or inhibitors of regulatory T cells or antibody producing cells, as protectors of cells from inflammatory or clearance processes such as binding to beta cells of the pancreas and thereby exerting a protective function for the cell to prolong their lifespan in the body (that is, the repairing the biocompatibility by binding to them for cells or tissues or proteins in the body that can benefit from a biocompatibility boost to reduce clearance and/or their involvement in localized or generalized inflammatory processes either active or passive), for treating genetic diseases, to chaperone an existing but mis-folded protein, for stimulating the co-localization of two soluble or cell-surface entities such as bringing together a cell-surface inhibitor module (ITIM) to a cell-surface activating module (ITAM) to inhibit a cell type such as a mast cell.

This invention contemplates using the polymers of the invention for mediating cell-penetration. For example, conjugation of the polymers of this invention through their initiator structure or end termini to one or more protein-derived peptides and amphipathic peptides either secondary and primary (Current Opinion in Biotechnology, 2006, 17, 638-642). Those skilled in the art will also recognize the possibility to combine with the stapled peptide technology which adds hydrocarbon moieties to peptides to facilitate cell penetration.

This invention contemplates the combination of these inventions with other drug delivery technologies, such as PLGA. Just as PEG's hydrophilic nature improved a number of PLGA properties, the high MW polymer technology of the current invention should further improve this. For example, increased drug loading as a percent of total mass (current biopharmaceutical state of the art <20% but generally less than 10%), also generally burst % is >5%. Enhanced water binding of the polymers of the current invention drives the solubility and drives higher loading and better *in vivo* performance of PLGA loaded with biopharmaceutical-polymer conjugate.

This invention contemplates conjugates that demonstrate lower immunogenicity for a particular drug-polymer conjugate (so lower new incidence of neutralizing antibodies). It also contemplates shielding, masking, or de-immunizing. Not that existing neutralizing antibodies are removed but that the drug-polymer conjugate can be given to patients who already have or have had an antibody response either natively or because the particular patient was previously treated with an immunogenic biopharmaceutical drug and developed antibodies. In this latter patient set, the present invention contemplates the ability to 'rescue' such patients and re-enable them to receive therapy. This is useful, for example, with Factor VIII because patients can be kept on Factor VIII therapy (rather than fail it and then they move to a Factor VII therapy, for example). These immune system shielding aspects of the present technology also enable drugs to be formulated for subcutaneous or needle-free injection where local dendritic and other innate and adaptive immune cell populations increase the incidence of immunogenicity. To the extent that drug-polymer conjugates of the present invention decrease *de novo* immunogenicity and hide existing neutralizing antibodies, then the technology enables subcutaneous dosing and avoids antibody interactions and therefore expands the eligible patient base and also will decrease incidence of injection related adverse events such as anaphylaxis.

The present invention allows the possibility to include different populations of polymer conjugate to the same or different therapeutic moieties to be combined into a single formulation. The result is to carefully tailor the desired *in vivo* and *in vitro* properties. For example, take a single therapeutic moiety and conjugate to it either in a single pot or separate pots two polymers of different size, architecture. The two populations will behave differently *in vivo.* One population can be smaller or contain less branched polymers. The second population can be larger, more branched architectures. The conjugate with the smaller polymers will be cleared more quickly. This is great as a loading dose or as a bolus specifically for example to clear existing cytokines (say with the conjugation of an anti-TNF or an anti-IL-6 scFv as the drug moiety) from the serum. The conjugate with the larger polymers will be cleared more slowly and clear *de novo* produced TNFa or IL-6, for example. This can be done with different ratios of the populations, for example 1:1 or 2:1 or 10:1 or 100:1, etc. The conjugated therapeutic moiety is the same, but there are different end properties as a result of the different polymers conjugated and is another way to impact biology. Another example would be with insulin or other agonistic proteins where the goal is to have a single injection that has both bolus aspect (quick activity) and also a basal (prolonged) aspect. For Factor VIII, one population of conjugated Factor VIII can have hydrolyzable linker between the polymer and the enzyme and so the enzyme comes off quickly. The second population could have a stable linker and so provide for the longer duration (chronic, prophylaxis) aspect.

The present invention can create conjugates such that after IV and/or SC injection, a zero order kinetics of release is achieved. The duration of release (1 month, 2 months, 3 months, 4 months, 6 months, 12 months) will depend on the size and architecture and linker chemistry of the polymer. This can be functionally equivalent to a medical device or pump that releases a constant amount of drug from a geographically localized reservoir. In the case of this invention, the drug will not be physically contained. Rather it will be in continuous circulation or by virtue of targeting be enriched in a particular tissue, but it is engineered such that onset is similar to or equivalent to zero order kinetics with linear release and minimal burst and equivalent of 100% loading.

Those skilled in the art will recognize that the present invention allows for the introduction of break points or weak points in the polymers and initiators such that larger polymer structures and/or conjugates will break down over time into smaller pieces that are readily and quickly cleared by the body. First order examples include a sensitive linker between initiator and drug, ester bonds anywhere (initiator, polymer backbone, monomers). Such weak points can break passively (for example by means of hydrolysis) or actively (by means of enzymes). Other approaches to drive breakdown or clearance can involve the use of protecting groups or prodrug chemistries such that over time, a change in exposed chemistry takes place which exposed chemistry drives destruction or targets the conjugate of released polymer to the kidney or liver or other site for destruction or clearance.

### VII. Examples

### Example 1. Preparation of N-(2-hydroxyethyl)-exo-3,6-euoxy-1,2,3,6-tetrahydrophthalimide

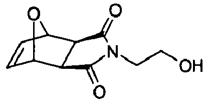

A 100-ml round-bottom flask equipped with a stir bar was charged with 50 ml ethanol and 2.0 grams of exo-3,6-epoxy-1,2,3,6-tetrahydrophthalic anhydride. The stirring mixture was cooled with an ice water bath, and a solution of 0.73 grams of ethanolamine in 20 ml of ethanol was added drop wise over 10 minutes. The reaction was heated at reflux for 4 hours, then refrigerated overnight. Filtration and rinsing with ethanol yielded 0.73 grams of the desired product as a white crystalline solid. The filtrate was concentrated and chilled again to obtain a second crystal crop. ¹H NMR (400 MHz, CDCl₃): δ = 2.90 (s, 2H, CH), 3.71 (m, 2H, OCH₂), 3.77 (t, *J*=5.0 Hz, NCH₂), 5.29 (t, *J*=1.0 Hz, 2H, OCH), 6.53 (t, *J*=1.0 Hz, 2H, CH=CH).

### Example 2. Preparation of isopropylidene-2,2-bis(hydroxymethyl)propionic acid

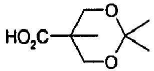

A 100 ml round-bottom flask equipped with a stir bar was charged with 50 ml of acetone, 13.8 ml of 2,2-dimethoxypropane, 10 grams of 2,2-bis(hydroxymethyl)propionic acid, and 0.71 grams p-toluenesulfonic acid monohydrate. The mixture was stirred for two hours at ambient temperature, then neutralized with 1 ml of 2M ammonia in methanol. The solvent was evaporated and the mixture dissolved in dichloromethane, then extracted twice with 20 ml of water. The organic phase was dried over magnesium sulfate and evaporated to give 10.8 grams of the product as a white crystalline solid. ¹H NMR (400 MHz, CDCl₃): δ = 1.20 (s, 3H, CH₃CC=O), 1.43 (s, 3H, CH₃), 1.46 (s, 3H, CH₃), 3.70 (d, *J*=12.4 Hz, 2H, OCH₂), 4.17 (d, *J*=12.4 Hz, 2H, OCH₂).

### Example 3. Preparation of N,N-dimethylpyridinium p-toluenesulfonate (DPTS)

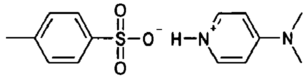

A solution of 1.9 grams of p-toluenesulfonic acid monohydrate in 10 ml benzene was dried by azeotropic distillation using a Dean-Stark trap, then 3.42 grams of 4-dimethylaminopyridine were added. Much solid formed, and an additional 25 ml of benzene were required to mobilize the reaction, which stirred slowly as it cooled to room temperature. The resulting solid was isolated by filtration, washed with 10 ml of benzene, and dried to yield 7.88 grams of the product as a white solid.

### Example 4. Preparation of protected maleimide bromopropionate initiator

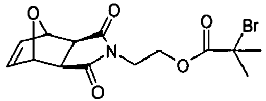

A 100-ml round-bottom flask equipped with a stir bar was charged with 50 ml tetrahydrofuran, 2 grams of N-(2-hydroxyethyl)-exo-3,6-epoxy-1,2,3,6-tetrahydrophthalimide, and 2.0 ml triethylamine. The stirring mixture was cooled to 0 degrees, and a solution of 1.18 ml of 2-bromoisobutyryl bromide in 5 ml tetrahydrofuran was added drop wise over 30 minutes. The reaction was allowed to stir on ice for 3 hours followed by room temperature overnight. Concentration of the reaction mixture gave an oily residue, which was purified by silica gel flash chromatography with 30-50% ethyl acetate in hexane, giving 1.96 grams of the desired product as a white powder. ¹H NMR (400 MHz, CDCl₃): δ = 1.89 (s, 6H, CH₃), 2.87 (s, 2H, CH), 3.82 (t, *J*=5.4 Hz, 2H, NCH₂), 4.33 (t, *J*=5.4 Hz, 2H, OCH₂), 5.27 (t, *J*=1.0 Hz, 2H, OCH), 6.51 (t, *J*=1.0 Hz, 2H, CH_{vinyl}).

### Example 5. Preparation of protected maleimide bis(bromopropionate) initiator

Protected maleimide isopropylidene acid.

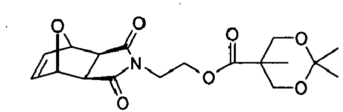

A solution of 2.00 grams of N-(2-hydroxyethyl)-exo-3,6-epoxy-1,2,3,6-tetrahydrophthalimide and 1.67 grams of isopropylidene-2,2-bis(hydroxymethyl)propionic acid in 30 ml of dry dichloromethane, together with 563 mg of DPTS was treated drop wise with a solution of 2.37 grams of N,N'-dicyclohexylcarbodiimide in 10 ml of dry dichloromethane. Much solid began to form as the reaction mixture was stirred at ambient temperature overnight. The reaction was filtered, and the precipitate was washed with a small amount of dichloromethane. The combined organic layers were concentrated to give a clear oil containing a small amount of solid. This oil was subjected to flash column chromatography on silica gel, using first 20-100% ethyl acetate in hexane. The fractions containing the desired product were combined and concentrated to give 3.17 grams of the final product as a white solid. ¹H NMR (400 MHz, CDCl₃): δ = 1.19 (s, 3H, CH₃CC=OO), 1.37 (s, 3H, CH₃), 1.41 (s, 3H, CH₃), 1.55 (s, 6H, (CH₃)₂C), 2.86 (s, 2H, C=OCHCHC=O), 3.58 (d, *J*=12Hz, CH₂O), 3.78 (t, *J*=5.4Hz, CH₂CH₂O), 4.14 (d, *J*=12H, CH₂O), 4.30 (t, *J*=5.4Hz, CH₂CH₂O), 5.27 (t, 2H, CHOCH), 6.51 (s, 2H, CH=CH).

Protected maleimide diol.

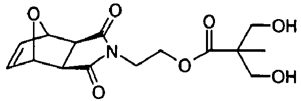

A solution of the isopropylidene compound from above in 50 ml of methanol was treated with 1.0 grams of Dowex 50W×8-100 ion exchange resin (H⁺ form) and the reaction was stirred at room temperature overnight, at which time the reaction appeared complete by tlc (silica gel, ethyl acetate). The mixture was filtered, and the solid resin was washed with a small amount of methanol. The combined organics were concentrated and placed under high vacuum to give 1.55 grams of a slightly cloudy oil, which was used in the next reaction without further purification.

Protected maleimide bis(bromopropionate) initiator.

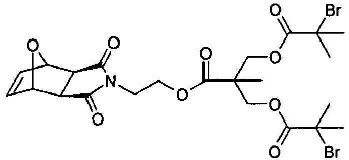

A solution of the crude product from above in 40 ml of anhydrous tetrahydrofuran (THF), together with 1.45 ml of triethylamine was cooled in an ice water bath, and a solution of 1.23 ml of 2-bromoisobutyryl bromide in 20 ml of anhydrous THF was added drop wise over a few minutes. The reaction was stirred in the cold for 30 minutes, then allowed to warm to room temperature over 6 hours. Another 600 µl of triethylamine were added, followed by another 0.5 ml of 2-bromoisobutyryl bromide. The reaction was acidic by pH paper, so another 200 µl of triethylamine were added to bring the pH of the solution to 9. The reaction was stirred overnight, concentrated, and the residue was partitioned between 50 ml of dichloromethane and 50 ml of water. The organic layer was dried over sodium sulfate, filtered and concentrated to give an oil. This was subjected to flash column chromatography on silica gel, first with 20%, then 30% and finally 40% ethyl acetate in hexane. The fractions containing product were combined and concentrated to give 1.63g of an oil which solidified to a white solid. ¹H NMR (400 MHz, CDCl₃): δ = 1.32 (s, 3H, CH₃CC=O), 1.91 [s, 12H, (CH₃)₂CBr], 2.90 (s, 2H, CHC=O), 3.78 (t, 2H, NCH₂CH₂O), 4.28 (t, 2H, NCH₂CH₂O), 4.31 (app q, 4H, CH₂OC=O), 5.30 (s, 2H, CHOCH), 6.52 (s, 2H, CH=CH).

### Example 6. Preparation of N-[2-(2-hydroxyethoxy)ethyl]-exo-3,6-epoxy-1,2,3,6-tetrahydrophthalimide

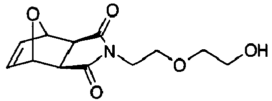

A 250 ml round-bottom flask equipped with a stir bar was charged with 100 ml methanol and 20 grams of exo-3,6-epoxy-1,2,3,6-tetrahydrophthalic anhydride. The stirring mixture was cooled to 0 degrees, and a solution of 0.73 grams 2-(2-aminoethoxy)ethanol in 40 ml of methanol was added drop wise over 45 minutes. The reaction was stirred at room temperature for 2 hours, then heated at gentle reflux overnight. The solution was concentrated and the product was dissolved in 100 ml of dichloromethane, then washed with 100 ml brine. The organic layer was dried over sodium sulfate, concentrated, and purified by passage through a silica gel plug with 100 ml dichloromethane and 100 ml ethyl acetate. ¹H NMR (400 MHz, CDCl₃): δ = 2.90 (s, 2H, CH), 3.49 (m, 2H, OCH₂), 3.59 (m, 4H, OCH₂), 3.65 (m, 2H, NCH2), 5.15 (t, *J*=0.8 Hz, 2H, OCH), 6.55 (t, *J*=0.8 Hz, 2H, CH=CH).

### Example 7. Preparation of bis 2,2-[(2-bromoisobutyryl)hydroxymethyl]propionic acid

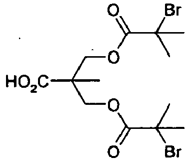

A 500 ml round-bottom flask equipped with a stir bar was charged with 200 ml of dichloromethane, 8.0 grams of 2,2-bis(hydroxymethyl)propionic acid, and 33.5 ml of triethylamine. The stirring mixture was cooled to 0 degrees, and a solution of 14.7 ml of 2-bromoisobutyryl bromide in 30 ml of dichloromethane was added drop wise over 30 minutes. The reaction was allowed to stir on ice for 1.5 hours, then allowed to warm to room temperature overnight. The precipitate was brought into solution with additional dichloromethane and the mixture was washed with 400 ml of 0.5 N hydrochloric acid and dried over anhydrous sodium sulfate. Concentration of the reaction mixture gave an oily residue, which was purified by flash chromatography on silica gel using 30-40% ethyl acetate in hexane containing 1% acetic acid, giving 27.4 grams of the desired product as a white waxy solid. ¹H NMR (400 MHz, CD₃OD): δ = 1.33 (s, 3H, CCH₃), 1.90 (s, 12H, (CH₃)₂CBr), 4.30 (d, *J*=5.4 Hz, 2H, NCH₂), 4.39 (d, *J*=5.4 Hz, 2H, OCH₂).

### Example 8. Preparation of protected maleimide extended bis(bromopropionate) initiator

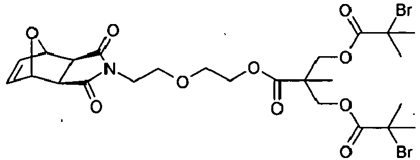

A 250 ml round-bottom flask equipped with a stir bar was charged with 100 ml dichloromethane, 1.0 grams of N-[2-(2-hydroxyethoxy)ethyl]-exo-3,6-epoxy-1,2,3,6-tetrahydrophthalimide, 2.5 grams of the dibromo acid from Example 7, 0.5 grams of dimethylaminopyridine, and 0.35 grams DPTS. Nitrogen was bubbled through the solution briefly, and 1.6 grams DCC was added slowly. The reaction was allowed to stir at room temperature overnight. Filtration and evaporation gave a pink oily residue, which was purified by silica gel flash chromatography. ¹H NMR (400 MHz, CD₃OD): δ = 1.34 (s, 3H, CH₃), 1.90 (s, 6H, CH₃), 2.94 (s, 2H, CH), 3.64 (m, 6H, OCH₂), 4.22 (t, *J*=5.4 Hz, 2H, NCH₂), 4.35 (app q, 4H, OCH₂), 5.15 (t, *J*=1.0 Hz, 2H, OCH), 6.54 (t, *J*=1.0 Hz, 2H, CH=CH).

### Example 9. Preparation of acetal bis(bromopropionate) initiator

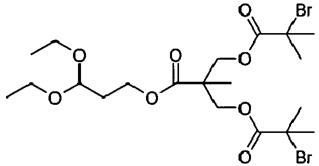

To a solution of 1.03 grams of 3,3-diethoxy-1-propanol and 3.0 grams of 2,2-bis(2-bromoisobutyryloxymethyl)propionic acid in 50 ml of dichloromethane, together with 817 mg of N,N-dimethylpyridinium p-toluenesulfonate, was treated with 1.58 grams of N,N'-dicyclohexylcarbodiimide, and the reaction was stirred at ambient temperature overnight. The reaction was filtered, and the precipitate was washed with a small amount of dichloromethane. The combined organics were concentrated, and the residue was subjected to flash column chromatography on silica gel with 10-20% ethyl acetate in hexane. The fractions containing the desired product were combined and concentrated to give 2.87 grams of a clear, colorless oil. This material was still not pure by ¹H NMR, so it was again subjected to flash column chromatography on silica gel using dichloromethane. The appropriate fractions were combined and concentrated to give 2.00 grams of the desired product as a viscous, clear oil. ¹H NMR (400 MHz, CDCl₃): δ = 1.20 (t, 6H, CH₃CH₂O), 1.34 (s, 3H, CH₃CC=O), 1.92 [s, 12H, (CH₃)₂CBr], 1.98 (app q, 2H, CHCH₂CH₂), 3.50 (m, 2H, OCH₂CH₃), 3.66 (m, 2H, OCH₂CH₃), 4.24 (t, 2H, CH₂CH₂OC=O), 4.37 (app q, 4H, CH₂OC=OCBr), 4.60 (t, 1H, O-CH-O).

### Example 10. Preparation of vinyl bis(bromopropionate) initiator 1

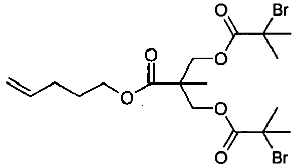

A 100 ml round-bottom flask equipped with a stir bar was charged with 30 ml of dichloromethane, 86 milligrams of 4-penten-1-ol, 432 milligrams of the dibromo acid from Example 7, and 88 milligrams of DPTS. Nitrogen was bubbled through the solution briefly, and 169 µl of N,N'-diisopropylcarbodiimide was added slowly. The reaction was allowed to stir at room temperature overnight, then another 0.1 grams DPTS was added and the reaction was again stirred overnight. Filtration and evaporation gave an oily residue, which was purified by flash chromatography on silica gel using 20-40% ethyl acetate in hexane. The solvent was removed from the first product to come off the column, yielding 0.13 grams of the desired product as a colorless oil. ¹H NMR (400 MHz, CD₃OD): δ = 1.34 (s, 3H, CH₃), 1.77 (m, 2H, CH₂CH₂CH₂), 1.90 (s, 12H, CH₃), 2.15 (q, *J*=7.2 Hz, 2H, CHCH₂CH₂), 4.16 (t, *J*=6.4 Hz, 2H, OCH₂), 4.36 (app. q, 4H, CCH₂O), 5.02 (m, 2H, CH₂=CH), 5.82 (m, 1H, CH₂=CH).

### Example 11. Preparation of vinyl bis(bromopropionate) initiator 2

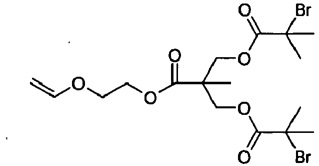

A 100 ml round-bottom flask equipped with a stir bar was charged with 25 ml dichloromethane, 370 milligrams of ethylene glycol monovinyl ether, 432 milligrams of the dibromo acid from Example 7, and 590 grams of DPTS. The flask was flushed with nitrogen, and 681 µl ofN,N'-diisopropylcarbodiimide was added slowly. The reaction was allowed to stir at room temperature overnight. The mixture was filtered and then dried onto silica gel for flash chromatography using 5-10% ethyl acetate in hexane, yielding the product as a colorless oil. ¹H NMR (400 MHz, CDCl₃): δ = 1.36 (s, 3H, CH₃), 1.92 (s, 12H, CH₃), 3.90 (app q, *J*=5.4 Hz, 2H, NCH₂CH₂O), 4.05 (dd, 1H, *J*=2.4, 6.8 Hz, =CH), 4.19 (dd, *J*=2.4, 14.4 Hz, 1H, =CH), 4.39 (m, 2H, NCH₂CH₂O), 4.40 (app q, 4H, OCH₂), 6.45 (dd, 1H, *J*=6.8, 14.4 Hz, =CHO).

### Example 12. Preparation of Boc-amino bis(maleimide) initiator

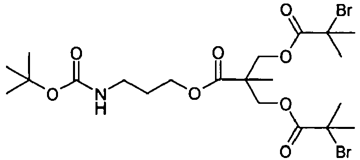

A solution of 2.19 grams of N-Boc-3-amino-1-propanol and 5.20 grams of 2,2-bis(2-bromoisobutyryloxymethyl)propionic acid in 50 ml of dichloromethane, together with 350 mg of DPTS, was treated with 3.0 grams of N,N'-dicyclohexylcarbodiimide and the reaction was stirred at ambient temperature overnight. The reaction mixture was filtered, and the precipitate was washed with a small amount of dichloromethane. Concentration gave a residue, which was subjected to flash column chromatography on silica gel with 5-20% ethyl acetate in hexane. The appropriate fractions were combined and concentrated to give an oil containing a little solid residue. This material was taken up in ethyl acetate and filtered. Concentration again gave an oil still containing a little solid, so the material was again taken up in ethyl acetate, filtered, and concentrated to give the desired product as a clear oil. ¹H NMR (400 MHz, CDCl₃): δ = 4.8 (br s, 1H, NH), 4.37 (app q, 4H, CH₂OC=OCBr), 4.22 (t, 2H, CH₂CH₂OC=O), 3.20 (app q, 2H, NHCH₂), 1.92 [s, 12H, (CH₃)₂CBr], 1.85 (t, 2H, CH₂CH₂CH₂), 1.43 (s, 9H, (CH₃)₃O), 1.35 (s, CH₃CC=O).

### Example 13. Preparation of protected maleimide 4-ol

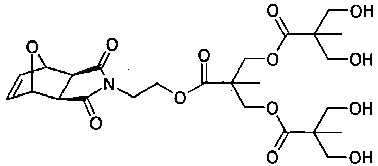

A 100 ml round-bottom flask equipped with a stir bar was charged with 30 ml of dichloromethane, 1.6 grams of the diol from Example 7, 1.71 grams of isopropylidene-2,2-bis(hydroxymethyl)propionic acid, and 0.5 grams of DPTS. Nitrogen was bubbled through the solution briefly, 1.70 ml of N,N'-diisopropylcarbodiimide was added slowly, and the reaction was allowed to stir at room temperature overnight. Filtration and evaporation gave an oily residue, which was purified by flash chromatography on silica gel using 10-40% ethyl acetate in hexane. A second purification by flash chromatography on silica gel using 2% methanol in dichloromethane yielded about 2 grams of colorless oil. This oil was dissolved in 25 ml of methanol and stirred for 60 hours at room temperature with Dowex 50WX8-100 resin (H⁺ form). The reaction was filtered, concentrated, then passed through a silica gel plug with 150 ml of 15% methanol in dichloromethane. Evaporation yielded 1.3 grams of a nearly colorless hard foam. ¹H NMR (400 MHz, CDCl₃): δ = 1.13 (s, 6H, CH₃), 1.25 (s, 3H, CH₃), 2.96 (s, 2H, CHC=ON), 3.57-3.65 (m, 8H, CH₂OH), 3.64 (t, *J*=2.8 Hz, 2H, CH₂CH₂OC=O),4.22 (app q, 4H, C(CH₃)CH₂OC=O₁), 4.22 (t, *J*=2.8 Hz, CH₂CH₂OC=O), 5.21 (t, *J*=0.8 Hz, CHOCH), 6.55 (t, *J*=0.8 Hz, CH=CH).

### Example 14. Preparation of protected maleimide tetra(bromopropionate) initiator

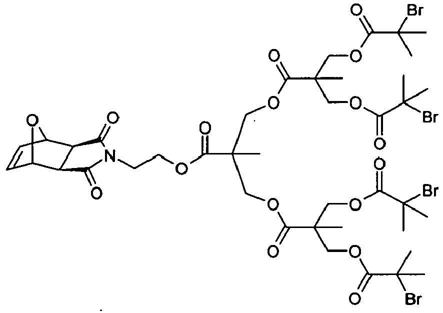

A 100 ml round-bottom flask equipped with a stir bar was charged with 20 ml of dichloromethane, 0.55 grams of the tetraol from Example 13, and 1.69ml of triethylamine. The stirring mixture was cooled to 0 degrees, and a solution of 0.99 ml of 2-bromoisobutyryl bromide in 10 ml dichloromethane was added drop wise. The reaction was allowed to stir at room temperature overnight, then washed with 50 ml of half-saturated sodium bicarbonate. Concentration of the reaction mixture gave an oily brown residue, which was purified by flash chromatography on silica gel with 40% ethyl acetate in hexane. The brown residue was dissolved in methanol and treated with charcoal to remove color, yielding 0.68 grams of the desired product as a light brown oil. ¹H NMR (400 MHz, CDCl₃): δ = 1.26 (s, 3H, CH₃CC=O), 1.34 (s, 6H, CH₃CC=O), 1.90 (s, 24H, (CH₃)₂CBr), 2.95 (s, 2H, CH), 3.78 (t, *J*=5 Hz, 2H, NCH₂), 4.25 (m, 6H, OCH₂C (4H) and OCH₂CH₂N (2H)), 4.35 (app q, 8H, OCH₂), 5.23 (t, *J*=1 Hz, 2H, CHOCH), 6.55 (t, *J*=1 Hz, 2H, CH=CH).

### Example 15. Preparation of high molecular weight zwitterionic polymers

A representative protocol to produce high molecular weight, tailor-made hydrophilic polymers of the zwitterionic monomer, 2-methacryloyloxyethyl phosphorylcholine (HEMA-PC), using a "living" controlled free radical process, atom transfer radical polymerization (ATRP), is as follows.

The following initiators were used:
PMC2M1 (from Example 4) 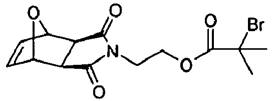
PMC2M2 (from Example 5) 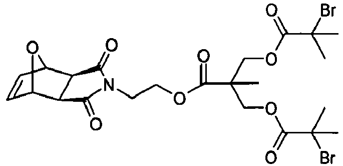
PMC2M4 (from Example 14)

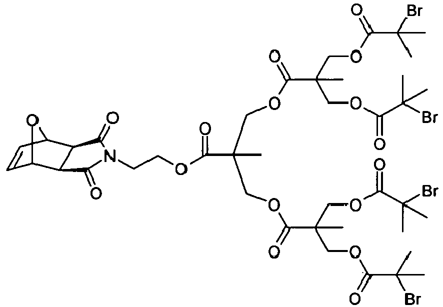

The initiator and the ligand (2,2'-bipyridyl) were introduced into a Schlenk tube. Dimethyl formamide or dimethylsulfoxide was introduced drop wise so that the weight percent of initiator and ligand was approximately 20%. The resultant solution was cooled to -78°C using a dry ice/acetone mixture, and was degassed under vacuum for 10min. The tube was refilled under nitrogen and the catalyst (CuBr unless otherwise indicated), kept under nitrogen, was introduced into the Schlenck tube (the Molar ratio of bromine/catalyst/ligand was kept at 1/1/2). The solution became dark brown immediately. The Schlenk tube was sealed and kept at -78°C. The solution was purged by applying a vacuum/nitrogen cycle three times. A solution of HEMA-PC was prepared by mixing a defined quantity of monomer, kept under nitrogen, with 200proof degassed ethanol. The monomer solution was added drop wise into the Schlenk tube and homogenized by light stirring. The temperature was maintained at -78°C. A thorough vacuum was applied to the reaction mixture for at least 10 to 15 min. until bubbling from the solution ceased. The tube was then refilled with nitrogen and warmed to room temperature. The solution was stirred, and as the polymerization proceeded, the solution became viscous. After 3 to 8 hours, the reaction was quenched by direct exposure to air in order to oxidize Cu (I) to Cu (II), the mixture became blue-green in color, and was passed through a silica column in order to remove the copper catalyst. The collected solution was concentrated by rotary evaporation and the resulting mixture was either precipitated with tetrahydrofuran or dialyzed against water followed by freeze drying to yield a free-flowing white powder.

Data from several polymerization reactions are shown in the following table.

| Sample | Initiator | Initiator (µmol) | Monomer (g) | Catalyst (µmol) | Ligand (µmol) | Ethanol (ml) | GPC (g/mol) | GPC (PDI) | Monomer Conversion (¹HNMR) |
|---|---|---|---|---|---|---|---|---|---|
| 1 | PMC2M1 | 10.4 | 1.05 | 10.4 | 21.0 | 4.0 | 54000 | 1.22 | 83% |
| 2 | PMC2M1 | 10.5 | 2.11 | 10.5 | 21.0 | 8.0 | 110000 | 1.38 | 97% |
| 3 | PMC2M2 | 10.2^{∗} | 1.14 | 22.6 | 45.0 | 3.7^{∗∗} | 50000 | 1.15 | 92% |
| 4 | PMC2M2 | 4.87 | 0.97 | 9.75 | 19.4 | 4.0 | 100000 | 1.16 | 98% |
| 5 | PMC2M2 | 4.87 | 3.03 | 9.75 | 19.4 | 9.2 | 198000 | 1.11 | 70% |
| 6 | PMC2M4 | 5.85 | 1.17 | 23.3 | 47.0 | 4.0 | 91300 | 1.06 | 93% |
| 7 | PMC2M4 | 4.72 | 2.21 | 18.8 | 38.0 | 8.0 | 176650 | 1.16 | 87% |

| | | | | | | | | | |
|---|---|---|---|---|---|---|---|---|---|
| ^{∗} CuCl ^{∗∗} Isopropanol/ethanol (2/1, v/v) | | | | | | | | | |

The peak molecular weight (g/mol) and polydispersity (PDI) were determined by gel permeation chromatography (GPC) on a Shodex OHpak SB-806M HQ column calibrated with poly(ethylene oxide) standards.

### Example 16. Deprotection of furan-protected maleimide functionalized polymers using retro Diels-Alder reaction

Polymers from Example 15 were dissolved in ethanol (20 to 50 % w/w) in a round bottom flask. Ethanol was slowly removed by rotary evaporation to make a thin film on the wall of the flask. The reaction vessel was placed in an oil bath at a temperature of at least 110°C for 90 min. under vacuum and then cooled to room temperature.

Deprotection of the maleimide functional group was monitored by ¹HNMR (400MHz, d-methanol):

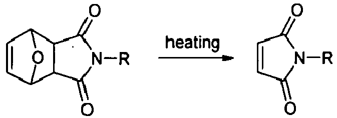

Before deprotection: δ(ppm):5.2 (2H, -CH-O-CH-) and 6.6 (2H, -CH=CH-). After deprotection: δ(ppm): 6.95 (2H, -CO-CH=CH-CO-).

### Example 17. Pepsin digestion of human IgG and purification of F(ab')2 fragments

Whole human IgG was purchased from Innovative Research, Jackson Immunochem, and/or Rockland Laboratories for use in the production of F(ab')2 antibody fragments for conjugation to the functionalized polymers of Example 15. The IgG was digested using immobilized pepsin (Thermo Scientific) following pH adjustment to 4.5 with sodium acetate buffer either by dialysis or by using a PD-10 desalting column (GE Healthcare). Following pH adjustment, a 0.5 ml quantity of immobilized pepsin was washed three times with sodium acetate buffer, pH 4, and resuspended in a final volume of 0.5 ml. 1 ml of IgG was added to the immobilized pepsin at a concentration of 10 mg/ml and placed on a rocker/shaker at 37°C. The digestion was allowed to proceed for four hours. After four hours, a 40 µL sample was removed and analyzed by HPLC using a Shodex Protein KW-802.5 column with a PBS mobile phase. The IgG peak was resolved from the F(ab')2 peak and the progression of the digestion was determined based on the percent digested. Immobilized pepsin is a proteolytic enzyme used to generate F(ab')2 antibody fragments by removing only the Fc domains beyond the hinge regions. This results in F(ab')2 fragments composed of two antibody-binding Fab' fragments connected by a covalent disulfide bond in the hinge region.

Following digestion of IgG to F(ab')2, the samples were centrifuged to separate the gel of the immobilized pepsin from the digested antibody fragments and the resin was washed three times. The rinses were combined with the original supernatant. The F(ab')2 antibody fragments were purified from the Fc fragments using a Superdex 200 HR 10/30 column (GE Healthcare) and PBS. The purified F(ab')2 eluted first followed by Fc fragments. The purified F(ab')2 was stored at 2-8°C.

### Example 18. Conjugation of maleimide functionalized polymers to Fab' fragments

Fab' fragments were produced from the F(ab')2 preparation of Example 17 by reduction of the disulfide bonds using sodium borohydride at a final concentration of 15 mM in solution. The F(ab')2 preparation was diluted with PBS containing 4 mM EDTA and an equal volume of sodium borohydride in the same buffer was added and the mixture placed on a stir plate at room temperature. The reaction was allowed to proceed for 1-1.5 hours at room temperature and the progress of the reduction was monitored by HPLC using a Shodex Protein KW-802.5 column and PBS as the mobile phase. The reduction was considered complete when greater than 90% of the F(ab')2 had been consumed. Immediately following disulfide reduction, the sample pH was adjusted down to approximately 4-5 with 0.1 N HCl. After adjusting the pH of the solution, the sample was mixed for an additional 10 minutes and then the pH was adjusted up to 6.5-7.5 using 0.1 N NaOH. While stirring, a 10-molar excess of a maleimide functionalized polymer from Example 16 was added to the mixture and incubated at room temperature. A sample was removed at time zero for analysis by HPLC and again at 1 and 2 hours in order to monitor the progress of the reaction. A Waters Alliance 2695 HPLC system 2695 was equipped with a Waters 2996 Photodiode Detector and a Shodex Protein KW-803 column with a PBS mobile phase. The conjugation efficiency was monitored at 220 nm and 280 nm. After 2 hours, the samples were purified using an AKTA Prime Plus (GE Healthcare) and a Superdex 200 HR 10/30 preparative size exclusion column. The elution buffer used was PBS. The polymer conjugated Fab' eluted first followed by the free polymer and unreacted Fab'. The fractions collected were analyzed using the Shodex Protein KW-803 column with PBS mobile phase. The fractions containing the purified Fab' conjugate were combined and concentrated using Vivaspin 2 (3000 MWCO) filters from Sartorius.

### Example 19. Conjugation of anti-VEGF aptamer to 200 kDa maleimide functionalized polymer

Anti-VEGF aptamer (Agilent, Boulder, CO) containing a terminal amine was conjugated to the maleimide functionalized polymer of Example 15 (Sample 5) following deprotection according to Example 16. Traut's Reagent was used to convert the terminal amine into a thiol as follows. Aptamer (5.4 mg) was dissolved in 500 µl of 0.1 M Sodium Bicarbonate Buffer, pH=8.0. In a separate vial, 7.2 mg of 2-Iminothiolane HCl (Traut's Reagent, Sigma) was dissolved in 3.6 ml of purified water to yield a 2 mg/ml solution. A 100 µl quantity of the 2-Iminothiolane HCl was added to the aptamer mixture and stirred at room temperature for one hour. The aptamer sample containing the Traut's reagent was passed over a PD-10 desalting column to remove any unreacted 2-Iminothiolane and the final buffer was exchanged to PBS containing 4 mM EDTA. A small portion of the aptamer sample containing the terminal thiol group was mixed at room temperature with a stir bar and 14.0 mg of maleimide functionalized polymer was added to the reaction, stirring constantly. A 60 µl sample was removed at time 0 for analysis by HPLC using a KW-803 column, PBS mobile phase and a flow rate of 1 ml/min. Samples were monitored at wavelengths of 220 and 280 nm as well as by refractive index detection. Aliquots were removed and tested after 2 hours and again after stirring at 4°C overnight.

The aptamer conjugate was purified using an isocratic gradient on a Superdex 200 HR 10/30(GE Healthcare) with phosphate buffer as the eluent. The purified conjugate eluted first followed by the unreacted polymer and residual aptamer.

### Example 20. PLGA microsphere preparation using polymer-aptamer conjugate

The polymer-aptamer conjugate from Example 19 was formulated into an oil-in-oil solvent mixture with poly(lactic-co-glycolic) acid (PLGA) microspheres. Polymer-aptamer conjugate (20 mg) was suspended in a solution of 100 mg/ml PLGA in 0.1% chloroform in dichloromethane at room temperature. The suspended conjugate was mixed with poly(diemethyl) siloxane to produce a homogeneous dispersion of the microspheres. The mixture was transferred to a flask containing heptane and stirred for 3 hours at room temperature. The resulting microspheres were isolated and collected using a 0.2 micron filter and dried under vacuum overnight.

### Example 21. Conjugation of mutein Factor VIII to 50, 100, and 200 kDa maleimide functionalized polymer (2-armed polymer) and to 100 and 200 kDa functionalized polymer (4-armed polymer)

Site specific conjugation of BDD Factor VIII with cysteine mutein (US Patent 7,632,921) was reduced using either immobilized Tris (2-carboxyethyl)phosphine (TCEP) or dithiothrietol (DTT) to release the "cap". Following reduction, the reducing agent, immobilized TCEP, was removed through centrifugation, or when using DTT, removal was accomplished using a PD-10 desalting column (GE Healthcare). The reduced cysteine on BDD Factor VIII was treated with between a 1 and a10-fold molar excess of the maleimide functionalized polymers from Example 16 with molecular weights of 50-200 kDa (2-arm) or 100-200 kDa (4-arm) for up to 2 hours at room temperature or overnight at 4°C. The final conjugated BDD Factor VIII samples were purified using anion exchange chromatography using a sodium chloride gradient. The conjugated mutein was separated from the unreacted Factor VIII and free maleimide functionalized polymer. Fractionated samples were analyzed by SEC HPLC and SDS-PAGE for confirmation. All fractions containing the conjugated mutein of Factor VIII were combined and buffer exchanged using PD-10 desalting columns into the final formulation in sodium phosphate buffer. In certain instances, depending on the molecular weight of the maleimide functionalized polymer used in the conjugation reactions, further purification was required using SEC to separate conjugated Factor VIII from unreacted species.

### Example 22. Conjugation of scFV to 50-200 kDa maleimide functionalized polymers

scFv fragments modified with c-terminal protected cysteines were diluted with PBS containing 4 mM EDTA and an equal volume of sodium borohydride in the same buffer was added. The mixture was placed on a stir plate at room temperature. Alternately, the reduction was carried out using immobilized TCEP at a pH range of 6-7. The reaction was allowed to proceed for 0.5-2 hours at room temperature and the progress of the reduction was monitored by HPLC using a Shodex Protein KW-802.5 column and PBS as the mobile phase. Immediately following disulfide reduction, samples were reacted while stirring with a 10-molar excess of a maleimide functionalized polymer from Example 16 at room temperature. A sample was removed at time zero for analysis by HPLC and again at 1 and 2 hours in order to monitor the progress of the reaction. A Waters Alliance 2695 HPLC system 2695 was equipped with a Waters 2996 Photodiode Detector and a Shodex Protein KW-803 column with a PBS mobile phase. The conjugation efficiency was monitored at 220 nm and 280 nm. After 2 hours, the samples were purified using an AKTA Prime Plus (GE Healthcare) and a Superdex 200 HR 10/30 preparative size exclusion column. The elution buffer used was PBS. The polymer conjugated scFv eluted first followed by the free polymer and unreacted Fab'. The fractions collected were analyzed using the Shodex Protein KW-803 column with PBS mobile phase. The fractions containing the purified scFv conjugate were combined and concentrated using Vivaspin 2 (3000 MWCO) filters from Sartorius.

### Example 23. Synthesis of bis 2,2-[(2-bromoisobutyryloxy)methyl]propionic acid, 3-hydroxypropyl ester

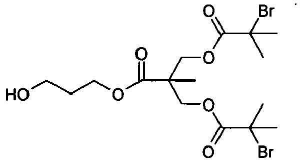

A solution of 4.40 grams of 1,3-propanediol and 5.00 grams of bis 2,2-[(2-bromoisobutyryloxy) methyl]propionic acid (from Example 7) in 50 ml of dry acetonitrile, together with 500 mg of DPTS, was treated with 2.86 grams of DCC, and the reaction was stirred at room temperature overnight. The reaction was then filtered, and the filtrate was concentrated to give an oil containing some solid. This was purified by flash column chromatography on silica gel with 30% ethyl acetate in hexane, and the product containing fractions were combined and concentrated to give 1.75 grams of the product as a clear, colorless oil. ¹H NMR (400 MHz, CDCl₃): δ = 1.35 (s, 3H, CCH₃), 1.92 (s and overlapping m, 14H, (CH₃)₂CBr and CH₂CH₂CH₂), 3.71 (app q, *J*=6 Hz, 2H, HOCH₂), 4.31 (t, *J*=6 Hz, 2H, CH₂OC=O), 4.37 (app q, 4H, CH₂OC=OCBr).

### Example 24. Synthesis of bis 2,2-[(2-bromoisobutyrytoxy)methyl]propionic acid, 3-oxopropanol ester

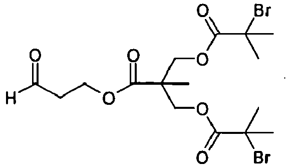

A solution of 1.01 grams of bis 2,2-[(2-bromoisobutyryloxy)methyl]propionic acid, 3-hydroxypropyl ester (from Example 23) in 25 ml of dichloromethane was treated with 1.75 grams of Dess-Martin periodinane [Org. Synth. Coll. Vol. X, 696 (2004)] and the reaction was stirred at room temperature for 30 minutes, at which time the reaction appeared to be complete by tlc (silica gel, 30% ethyl acetate in hexane). The reaction was filtered and concentrated, and the residue was subjected to flash column chromatography on silica gel with 30% ethyl acetate in hexanes to give 730 mg of the desired aldehyde product as a clear, colorless oil, which was protected from light and stored in the refrigerator under a nitrogen-filled glove box. ¹H NMR (400 MHz, CDCl₃): δ = 1.33 (s, 3H, CCH₃), 1.92 (s, 12H, (CH₃)₂CBr), 2.83 (t, *J*=6.4 Hz, 2H, HC=OCH₂), 4.34 (app q, 4H, OCH₂), 4.48 (t, J=6.4 Hz, HC=OCH₂CH₂), 9.79 (br s, 1H, CHO).

### Example 25. Bis 2,2-[(2-bromoisobutyrytoxy)methyl]propionic acid, N-hydroxysuccinimide ester

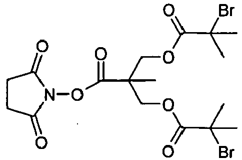

A solution of 500 mg of bis 2,2-[(2-bromoisobutyryloxy)methyl]propionic acid (from Example 7) and 133 mg of N-hydroxysuccinimide in 5 ml of dichloromethane was treated with 286 mg of DCC, and the reaction was stirred at room temperature for 1.5 hr, at which time the reaction appeared to be complete by tlc (silica gel, 30% ethyl acetate in hexane). The reaction was filtered and concentrated, and the residue was subjected to flash column chromatography on silica gel with 30% ethyl acetate in hexane. The product containing fractions were combined and concentrated to give 518 mg of the desired NHS ester as a clear, colorless oil. ¹H NMR (400 MHz, CDCl₃): δ = 1.55 (s, 3H, CCH₃), 1.95 (s, 12H, (CH₃)₂CBr), 2.84 (broad s, 4H, O=CCH₂CH₂C=O), 4.49 (s, 4H, CH₂OC=OCBr).

### Example 26. Preparation of high molecular weight aldehyde and NHS ester functionalized zwitterionic polymers

A representative protocol to produce high molecular weight, tailor-made hydrophilic polymers of the zwitterionic monomer, 2-methacryloyloxyethyl phosphorylcholine (HEMA-PC), using a "living" controlled free radical process, atom transfer radical polymerization (ATRP), is as follows.

The following initiators were used:
NHSM2 (from Example 25) 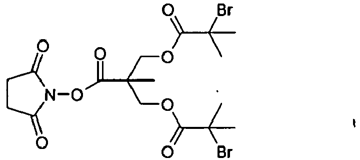
AlC2M2 (from Example 24)

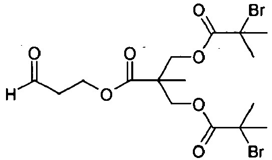

The initiator and the ligand (2,2'-bipyridyl) were introduced into a Schlenk tube. Dimethyl formamide or dimethylsulfoxide was introduced drop wise so that the weight percent of initiator and ligand was approximately 20%. The resultant solution was cooled to -78°C using a dry ice/acetone mixture, and was degassed under vacuum for 10min. The tube was refilled under nitrogen and the catalyst (CuBr unless otherwise indicated), kept under nitrogen, was introduced into the Schlenck tube (the Molar ratio of bromine/catalyst/ligand was kept at 1/1/2). The solution became dark brown immediately. The Schlenk tube was sealed and kept at -78°C. The solution was purged by applying a vacuum/nitrogen cycle three times. A solution of HEMA-PC was prepared by mixing a defined quantity of monomer, kept under nitrogen, with 200proof degassed ethanol. The monomer solution was added drop wise into the Schlenk tube and homogenized by light stirring. The temperature was maintained at -78°C. A thorough vacuum was applied to the reaction mixture for at least 10 to 15 min. until bubbling from the solution ceased. The tube was then refilled with nitrogen and warmed to room temperature. The solution was stirred, and as the polymerization proceeded, the solution became viscous. After 3 to 8 hours, the reaction was quenched by direct exposure to air in order to oxidize Cu (I) to Cu (II), the mixture became blue-green in color, and was passed through a silica column in order to remove the copper catalyst. The collected solution was concentrated by rotary evaporation and the resulting mixture was either precipitated with tetrahydrofuran or dialyzed against water followed by freeze drying to yield a free-flowing white powder.

Data from the polymerization reactions are shown in the following table.

| Sample | Initiator | Initiator (µmol) | Monomer (g) | Catalyst (µmol) | Ligand (µmol) | Ethanol (ml) | GPC (g/mol) |
|---|---|---|---|---|---|---|---|
| 1 | NHSM2 | 13.5 | 2.03 | 27.0 | 54.1 | 8.0 | 81250 |
| 2 | AlC2M2 | 13.5 | 2.03 | 27.0 | 54.1 | 8.0 | 83000 |

### Example 27. Conjugation of human growth hormone to 75 kDa aldehyde functionalized polymer

A sample of Human Growth Hormone (hGH) at a concentration of 10 mg/ml in phosphate buffer was prepared. In a separate flask, sodium cyanoborohydride was weighed at 100 mM concentration and diluted in 10 ml of sodium phosphate buffer, pH6. This was used immediately after diluting with PBS. An equal volume of sodium cyanoborohydride in solution was added to the reaction mixture containing the aldehyde functionalized polymer from Example 26 and hGH. The reaction was mixed at room temperature or at 4 °C overnight. The percent conjugation of the reaction was monitored by HPLC using a Shodex Protein KW-803 column and PBS as the mobile phase.

The samples were purified using the AKTA Prime Plus (GE Healthcare) and the Superdex 200 HR 10/30 preparative size exclusion column. The elution buffer used was PBS. The conjugated hGH eluted first followed by the free aldehyde functionalized polymer and unreacted hGH. The fractions collected were analyzed by HPLC using a Shodex Protein KW-803 column with PBS mobile phase. The fractions containing the purified hGH conjugate were combined and concentrated using Vivaspin 2 (3000 MWCO) filters from Sartorius.

### Example 28. Conjugation of Hematide to 75 kDa NHS ester functionalized polymer

A solution of Hematide at a concentration between 1-10 mg/ml was buffer exchanged to 0.1 M sodium borate buffer, pH9, using a PD-10 desalting column (GE Healthcare). The NHS ester functionalized polymer from Example 26 was added in 10 Molar excess to the constantly stirring samples of Hematide at room temperature. The reactions proceeded at room temperature for 2 hours or overnight at 4° C. Samples for determining the degree of conjugation were analyzed by HPLC using a Shodex KW-803 column and PBS mobile phase. Aliquots of samples were pulled at time zero and 1 and 2 hours after conjugation. At the end of two hours or after overnight, 1 M glycine was added to quench the reaction.

The samples were purified using an AKTA Prime Plus (GE Healthcare) and a Superdex 200 HR 10/30 preparative size exclusion column. The elution buffer used was PBS. The NHS ester functionalized polymer conjugated to Hematide eluted first followed by free polymer, unreacted Hematide, and other small molecules. The fractions collected were analyzed by HPLC using a Shodex Protein KW-803 column with PBS mobile phase. The fractions containing the purified Hematide conjugate were combined and concentrated using Vivaspin 2 (3000 MWCO) filters from Sartorius.

### Example 29. High pressure polymerization of HEMA-PC

Polymerization of HEMA-PC monomer under high pressure was performed in a glass-lined, stainless steel pressure vessel. The ratio HEMA-PC/2-arm protected maleimide initiator (from Example 8/CuBr/ bipyridyl ranged from 500-10000/1/2/4; T=22°C in ethanol; [HEMA-PC]0=0.86M in ethanol with DMF (1-1.5%w/w in ethanol). The pressure ranged from 1 bar to 6kbar.

### Example 30. Preparation of N-[2-(2-hydroxyethoxy)ethyl]-exo-3,6-epoxv-1,2,3,6- tetrahydrophthalimide, isopropylidene-2,2-bis(hydroxymethyl)propionate

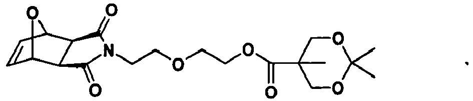

A solution of 11.0 grams of N-[2-(2-hydroxyethoxy)ethyl]-exo-3,6-epoxy-1 ,2,3,6-tetrahydrophthalimide and 8.22 grams of isopropylidene-2,2-bis(hydroxymethyl)propionic acid in 250 ml of dichloromethane, together with 1.3 grams of DPTS and 5.24 grams of DMAP was treated with 12.9 grams of DCC, and the reaction was stirred overnight. The reaction was filtered and concentrated to give a residue, which was subjected to flash column chromatography in two portions on silica gel with 40 - 50% ethyl acetate in hexane to give the desired product as a clear oil.

### Example 31. Preparation of N-[2-(2-hydroxyethoxy)ethyl]-exo-3,6-epoxy-1,2,3,6- tetrahydrophthalimide, 2,2-bis(hydroxymethyl)propionate

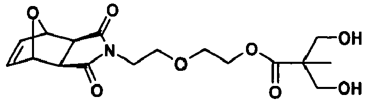

The product from above was dissolved in 100 ml of methanol and treated with 2.0 grams of Dowex 50Wx8-100 ion exchange resin (H⁺ form) and the reaction was stirred at room temperature overnight. The reaction was filtered and concentrated to give the desired product as an oil which was used without further purification. NMR (CD₃OD): δ 6.546 (t, 2H, CH=CH, *J*=0.8 Hz), 5.158 (t, 2H, CH-O, *J*=0.8 Hz), 4.180 (m, 2H, CH₂-CH₂-O-C=O, *J*= 4.9 Hz), 3.63 (m, 10H, N-CH₂ and N-CH₂-CH2 and CH₂-CH₂-O-C=O and CH₂-OH), 2.936 (s, 2H, CH-CH), 1.147 (s, 3H, CH₃).

### Example 32. Preparation of N-[2-(2-6ydroxyethoxy)ethyl]-exo-3,6-epoxy-1,2,3,6- tetrahydrophthalimide, 2,2-bis-[2,2-bis(2-bromoisobutyryloxymethyl) propionyloxymethyl] propionate initiator

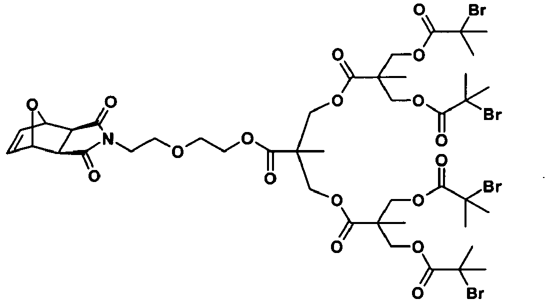

To a solution of 1.5 grams of the diol from the previous step and 3.72 grams of 2,2-bis[(2-bromoisobutyryloxy)methyl]propionic acid in 50 ml of dichloromethane, together with 500 mg of DPTS and 810 mg of DMAP, was treated with 1.40 grams of diisopropylcarbodiimide, and the reaction was stirred at room temperature overnight. The reaction was concentrated and the residue was chromatographed several times on silica gel with 40% ethyl acetate in hexane. The appropriate fractions in each case were combined and concentrated to give the desired product as an oil. NMR (CD₃OD): δ 6.55 (t, 2H, CH=CH, *J*=0.8 Hz), 5.17 (t, 2H, CH-O, *J*=0.8 Hz), 3.34 (m, 12H, CCH₂), 4.23 (m, 2H, CH₂-CH₂-O-C=O, *J*= 4.7 Hz), 3.68 (m, 2H, N-CH₂, *J*=4.7 Hz), 3.64 (app q, 4H, N-CH₂-CH₂ and CH₂-CH₂-O-C=O), 2.95 (s, 2H, CH-CH), 1.907 (s, 24H, Br-C-CH₃), 1.34 (s, 6H, CH₃), 1.308 (s, 3H, CH₃).

### Example 33. Preparation of N-(3-propionic acid)-exo-3,6-epoxy-3,6-dimethyl-1,2,3,6- tetrahydrophthalimide, ester with 2,2-bis[(2-bromoisobutyryloxy)methyl] propionic acid, 3-hydroxypropyl ester initiator

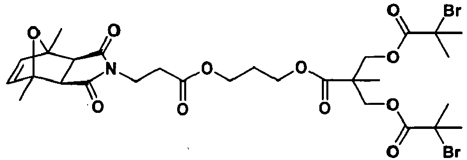

A solution of 738 mg of 2,2-bis[(2-bromoisobutyryloxy)methyl]propionic acid, 3-hydroxypropyl ester and 399 mg of N-(3-propionic acid)-exo-3,6-epoxy-3,6-dimethyl-1,2,3,6-tetrahydrophthalimide in 20 ml of dry acetonitrile, together with 50 mg of DPTS and 100 mg of DMAP, was treated with 375 mg of DCC and the reaction was stirred at room temperature overnight. The reaction was filtered to give a residue, which was subjected to flash column chromatography on silica gel with 30 - 40% ethyl acetate in hexane. The appropriate fractions were combined and concentrated to give 1.02 grams of the desired product as a clear oil. By ¹H NMR, it appeared that about 10% of the product had already undergone retro Diels-Alder reaction. NMR (CDCl₃): δ 6.19 (s, 2H, CH=CH), 4.37 (app q, 4H, CCH₂O, *J*=10.9, 29.7 Hz), 4.23 (t, 2H,CH₂CH₂O, *J*=6.3 Hz), 4.15 (t, 2H, CH₂CH₂O, *J*=6.3 Hz), 3.62 (t, 2H, NCH₂, *J*=7.4 Hz), 3.22 (s, 2H, CHC=O), 2.48 (t, 2H, CH₂C=O, *J*=7.4 Hz), 2.00 (m, 2H, CH₂CH₂CH₂, *J*=6.3 Hz), 1.92 (s, 12H, Br-C (CH₃)₂), 1.78 (s, 6H, CH₃), 1.35(s, 3H,CH₃).

### Example 34. Preparation of N-(3-Propionic acid, t-butyl ester)-2,2-Bis[(2- bromoisobutyrytoxy) methyl] propionamide

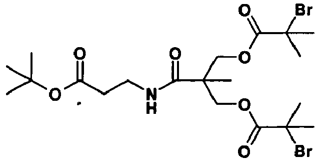

A solution of 1.00 grams of b-alanine t-butyl ester hydrochloride in 50 ml of dichloromethane was treated with 25 ml of saturated aqueous sodium bicarbonate, and the mixture was stirred for 15 minutes. The layers were separated, and the organics were dried over sodium sulfate. To this solution was added 2.38 grams of 2,2-bis[(2-bromoisobutyryloxy]methyl)propionic acid , followed by 1.92 ml of diisopropylethylamine and 2.1 grams of HBTU, and the reaction was stirred at room temperature overnight. The reaction mixture was then diluted with another 50 ml of dichloromethane, washed with 2 × 50 ml of water, and dried over sodium sulfate. Filtration and concentration gave an oil, which was subjected to flash column chromatography with 20 - 25% ethyl acetate in hexane. The appropriate fractions were combined and concentrated to give 730 mg of a white solid. NMR (CDCl₃): δ 6.70 (t, 1H,NH, *J*=5.4 Hz), 4.33 (app q, 4H, CH₂O, *J*=*16.3,* 11.4 Hz), 3.51 (q, 2H, NCH₂, *J*=6.0 Hz), 2.46 (t, 2H, CH₂CO, *J*=6.0 Hz), 1.93 (s, 12H, Br-C(CH₃)₂), 1.45 (s, 9H, C(CH₃)₃), 1.33 (s, 3H, CH₃).

### Example 35. Preparation of 2,2-Bis[(2-bromoisobutyryloxy)methyl]propionic acid, 2-hydroxyethyl ester initiator

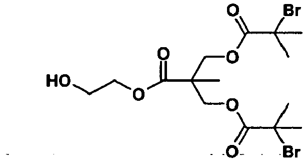

A solution of 4.32 grams of 2,2-bis[(2-bromoisobutyryloxy]methyl)propionic acid and 12.41 grams of ethylene glycol in 50 ml of dichloromethane, together with 883 mg of DPTS was treated with 1.39 grams of diisopropylcarbodiimide, and the reaction was stirred at room temperature overnight. The reaction mixture was concentrated, then partitioned between 150 ml of ethyl acetate and 70 ml of water. The organic layer was concentrated, and the residue was subjected to flash column chromatography on silica gel with 20% - 40% ethyl acetate in hexane. The appropriate fractions were combined and concentrated to give 2.7 grams of the desired product as a clear oil. NMR (CD₃OD): δ 4.38 (app q, 4H, CCH₂, *J*=11.2, 30.2 Hz), 4.20 (t, 2H, CH₂OH, *J*=5.0 Hz), 3.75 (t, 2H, CH₂CH₂OH, *J*=5.0 Hz), 1.90 (s, 12H, Br-CCH₃), 1.36 (s, 3H,CH₃).

### Example 36. Preparation of 2,2-Bis[(2-bromoisobutyryloxy)methyl]propionic acid, 3-hydroxypropyl ester initiator

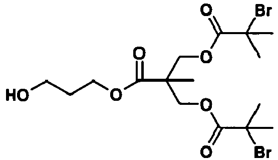

A solution of 5.31 grams of 2,2-bis[(2-bromoisobutyryloxy)methyl]propionic acid and 4.68 grams of 1,3-propanediol in 80 ml of dichloromethane and 20 ml of acetonitrile was treated with 1.0 grams of DPTS, followed by 3.0 grams of DCC, and the reaction was stirred at room temperature for 2 hours. The reaction was then filtered, concentrated and the residue was subjected to flash column chromatography on silica gel with 30% ethyl acetate in hexane. The appropriate fractions were combined and concentrated to give a clear oil, which was not quite pure. Rechromatography on silica gel with 10 - 15% acetone in hexane gave the desired product as a clear, colorless oil. NMR (CDCl₃): δ 4.38 (app q, 4H, CCH₂O, *J*=11.2 Hz), 4.31 (t, 2H, CH₂CH₂O, *J*=6.3 Hz), 3.71 (q, 2H, CH₂OH, *J*=5.9 Hz), 1.92 (s, 12H, Br-C(CH₃)₂), 1.9 (m, 2H, CH₂CH₂CH₂), 1.35 (s, 3H, CH₃).

### Example 37. 2,2-Bis[(2-bromoisobutyryloxy-)methyl]propionicacid, 11-hydroxy-3,6,9- trioxaundecanoate initiator

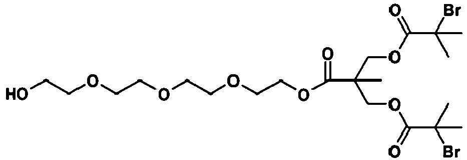

A solution of 1.86 grams of 2,2-bis[(2-bromoisobutyryloxy)methyl]propionic acid and 4.18 grams of tetraethylene glycol in 50 ml of dichloromethane, together with 250 mg of DPTS, was treated with 1.15 grams of DCC and the reaction was stirred at room temperature overnight. The reaction was filtered and the filtrate was diluted with 50 ml of dichloromethane and washed with 20 ml of water. The organics were dried over sodium sulfate, filtered and concentrated to give a residue, which was subjected to flash column chromatography on silica gel first with 50 - 70% ethyl acetate in hexane. The appropriate fractions were combined, filtered and concentrated to give 1.19 grams of the desired product as a clear, colorless oil. NMR (CDCl₃): δ 4.38 (app q, 4H, CCH₂O, *J*=31.8, 11.2 Hz), 4.31 (t, 2H, CH₂CH₂OC=O, *J*=5.0 Hz), 3.6 - 3.73 (m, 14H,CH₂O), 2.46 (t, 1H, OH, *J*=6.3 Hz), 1.92 (s, 12H, Br-C(CH₃)₂), 1.35 (s, 3H, CH₃).

### Example 38. Preparation of 2,2-Bis[(2-bromoisobutyryloxy)methyl]propionic acid. 11-hydroxy-3.6.9-trioxaundecanoate, NHS carbonate initiator

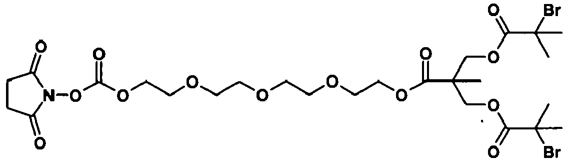

A solution of 630 grams of the above hydroxyl compound and 1.28 grams of disuccinimidyl carbonate in 3 ml of dry acetonitrile was treated with 610 mg of DMAP and the reaction was stirred at room temperature. The reaction was still heterogeneous, so 4 ml of dry THF were added, and after 2 hours the reaction turned yellow and became homogeneous, but contained several spots on tlc (silica gel, 50% ethyl acetate in hexane). The reaction was concentrated to give a residue which was subjected to flash column chromatography on silica gel with 50 - 60% ethyl acetate in hexane. Two fractions were isolated, and the fraction with a lower rf was concentrated to give 260 mg of the desired product as a clear oil. NMR (CDCl₃): δ 4.47 (m, 2H,CH₂O(C=O)O), 4.37 (app q, 4H, CCH₂O, *J*=11.2, 31.6 Hz), 4.30 (m, 2H, CH₂CH₂O(C=O)C), 3.79 (m, 2H, CH₂CH₂O(C=O)C), 3.71 (t, 2H, CH₂CH₂O(C=O)O, J=5.0 Hz), 3.67 (s, 4H,CH₂O), 3.65 (s, 4H, CH₂O), 2.84 (s, 4H,CH2C=O), 1.92(s, 12H, Br-C (CH₃)₂), 1.35(s, 3H,CH₃).

### Example 39. Preparation of 2,2-Bis[(2-bromoisobutyryloxy)methyl]propionic acid, solketal ester initiator

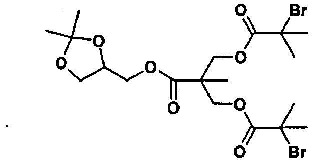

A solution of 918 mg of solketal and 3.0 grams of 2,2-bis[(2-bromoisobutyryloxy) methyl]propionic acid, together with 200 mg of DPTS was treated with 2.15 grams of DCC and the reaction was stirred at room temperature overnight. The reaction was filtered to give a residue, which was subjected to flash column chromatography on silica gel with 10% ethyl acetate in hexane. The appropriate fractions were combined and concentrated to give 1.85 grams of the desired product as a clear, colorless oil. NMR (CDCl₃): δ 4.38 (app q, 4H,CCH₂O), 4.32 (m, 1H, OCH), 4.19 (m, 2H, CHCH₂OC=O), 4.07 (d of d, 1H, OCH₂CH, *J*=6.7, 8.6 Hz), 3.76 (d of d, 1H, OCH₂CH, *J*=5.7, 8.6 Hz), 1.92 (s, 12H, Br-C(CH₃)₂), 1.43 (s, 3H, (CH₃)₂CO), 1.36 (s, 3H, CH₃), 1.35 (s, 3H, (CH₃)₂CO).

### Example 40. Preparation of 2,2-Bis[(2-bromoisobutyryloxy)methyl]propionic acid, 2,3-dihydroxypropyl ester initiator

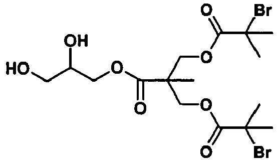

A solution of 1.0 grams of the previous ketal in 50 ml of methanol was treated with 750 mg of Dowex 50Wx8-100 and the reaction was stirred overnight. The reaction was then filtered, concentrated, and the residue was subjected to flash column chromatography on silica gel with 20 - 40% ethyl acetate in hexane. The appropriate fractions were combined and concentrated to give 630 mg of the desired product as a clear, colorless oil. NMR (CDCl₃+D₂O): δ 4.40 (app q of d, 4H,CCH₂O, *J*=2.8, 11.5, 30.2 Hz), 4.24 (app q of d, 2H, CHCH₂OC=O, *J*=4.5, 6.6, 11.5 Hz), 3.96 (m, 1H, CH), 3.66 (app q of d, 2H, HOCH₂CH, *J*=3.8, 5.6, 11.5, 37.9 Hz), 1.92 (s, 12H, Br-C(CH₃)₂), 1.37 (s, 3H, CH₃).

### Example 41. Preparation of 2,2-Bis[(2-bromoisobutyryloxy)methyl]propionic acid, 2-(2,3-dihydroxypropoxy)ethyl ester initiator

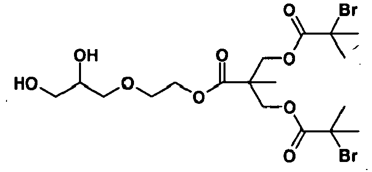

To a solution of 1.5 grams of 2-[(2-bromoisobutyryloxy)methyl]-2-hydroxymethylpropionic acid, 2-(allyloxy)ethyl ester in 15 ml of water and 15 ml of t-butanol was added 2.86 grams (3 eq) of potassium ferricyanide, 1.20 grams (3 eq) of potassium carbonate, 7.5 mg of potassium osmate dehydrate, 11 mg of quinuclidine, and 276 mg (1 eq) of methanesulfonamide, and the reaction mixture was stirred at room temperature overnight. The reaction appeared to be complete by TLC (silica gel, 50% ethyl acetate in hexane), so the reaction was poured into 100 ml of water, then extracted with 100 ml of dichloromethane. The combined organics were dried over sodium sulfate, filtered and concentrated to give an oily residue, which was subjected to flash column chromatography on silica gel with 30 - 40% ethyl acetate in hexane. The appropriate fractions were combined, treated with decolorizing carbon, filtered and concentrated to give 850 mg of the desired product as a nearly colorless oil. NMR (CDCl₃): δ 4.39 (app q of d, 4H, CCH₂O, *J*=4.1, 11.1, 3.0, 37.6 Hz), 4.31(t, 2H, OCH₂CH₂OC=O, *J*=4.7 Hz), 3.87 (m, 1H, CH-OH), 3.54 - 3.77 (m, 2H,CH₂-OH), 3.72(m, 2H, OCH₂CH), 3.58(app t, 2H, OCH₂CH₂OC=O), 2.68 (d, 1H, CH-OH, *J*=*5.1* Hz), 2.15 (app t, 1H, CH₂-OH, *J*=6.1 Hz), 1.92 (s, 12H, Br-C(CH₃)₂), 1.36 (s, 3H, CH₃).

### Example 42. 2,2-Bis[(2-bromoisobutyryloxy)methyl]propionic acid. 12-(allyloxy)-3,6,9,12-tetraoxadodecanoate initiator

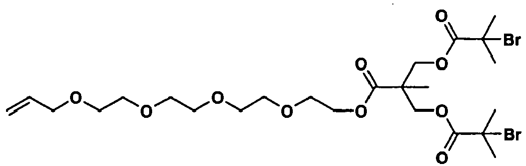

To a solution of 1.60 g of 2,2-bis[(2-bromoisobutyryloxy)methyl]propionic acid and 870 mg of 12-(allyloxy)-3,6,9,12-tetraoxadodecane in 30 ml of dry acetonitrile, together with 218 mg of DPTS and 362 mg of DMAP, was added 917 mg of DCC and the reaction was stirred at room temperature overnight. The mixture was then filtered and concentrated, and the residue was subjected to flash column chromatography on silica gel first with 50 - 60% ethyl acetate in hexanes, and the product containing fractions were combined and concentrated to give 1.35 grams of the desired product as a clear, colorless oil. NMR (CDCl₃): δ 5.87-5.97 (m, 1H, CH₂CH=CH₂), 5.28 (dq, 1H, H-CH=CH), 5.18 (dq, 1H, H-CH=CH), 4.37 (app q, CH₂OC=O), 4.30 (dd, 2H, CH₂CH₂OC=O), 4.02 (d, 2H, CH₂=CHCH₂), 3.60-3.72 (m, 14H, CH₂CH₂OCH₂), 1.92 (s, 12H, Br-C (CH₃)₂), 1.35 (s, 3H, CH₃).

### Example 43. Preparation of 2,2-Bis[(2-bromoisobutyryloxy)methyl]propionic acid, 12-(2,3-dihydroxypropoxy)-3,6,9,12-tetraoxadodecyl ester initiator

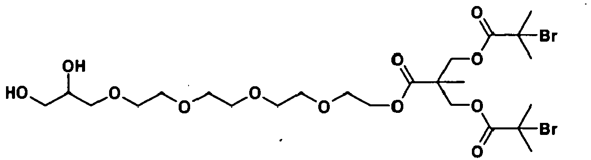

To a mixture of 1.29 grams of 2,2-bis[(2-bromoisobutyryloxy)methyl]propionic acid, 12-(allyloxy)-3,6,9,12-tetraoxadodecyl ester in 15 ml of water and 15 ml of t-butanol was added 1.98 grams (3 eq) of potassium ferricyanide, 829 mg (3 eq) of potassium carbonate, 8 mg of potassium osmate dehydrate, 11 mg of quinuclidine, and 190 mg (1 eq) of methanesulfonamide, and the reaction mixture was stirred at room temperature overnight. The reaction appeared to be complete by TLC (silica gel, 50% ethyl acetate in hexane), so the reaction was poured into 50 ml of water, then extracted with 100 ml of dichloromethane. The combined organics were dried over sodium sulfate, filtered and concentrated to give an oily residue, which was subjected to flash column chromatography on silica gel with 5% methanol in dichloromethane. The product containing fractions were combined and treated twice with two small spatulafuls of activated carbon, filtering between treatments. Filtration and concentration gave a light gray oil containing a small amount of solid, so it was taken up in ethyl acetate and filtered, then concentrated to give 1.06 grams of the desired product as a light gray oil, still containing a tiny amount of solid. NMR (CDCl₃): δ 4.38 (app q, 4H, CCH₂OC=O), 4.30 (t, 2H, CH₂CH₂OC=O, *J*=5.0 Hz), 3.85(p, 1H, CHOH, *J*=5 Hz), 3.71 (t, 2H, OCH₂CHOH, *J*= 4.8 Hz), 3.72 - 3.55 (m, 16H, OCH₂CH₂O and CH₂OH), 3.12 (s, 1H, CHOH), 2.37 (s, 1H, CH₂OH), 1.92 (s, 12H, Br-C(CH₃)₂), 1.35 (s, 3H, CH₃).

### Example 44. Preparation of 2,2,5-Trimethyl-1,3-dioxane-5-carboxylic acid. 2-(allyloxy)ethyl ester

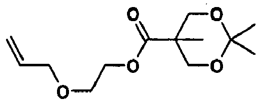

A solution of 1.4 grams of ethylene glycol monoallyl ether and 2.35 grams of 2,2,5-trimethyl-1,3-dioxane-5-carboxylic acid in 25 ml of anhydrous THF was treated with 500 mg of 4-dimethylaminopyridinium p-toluenesulfonate (DPTS) and 1.44 grams of dimethylaminopyridine (DMAP), followed by the addition of 3.38 grams of dicyclohexylcarbodiimide, and the reaction was stirred at room temperature for 3 days. The reaction mixture was filtered and concentrated to give a semisolid residue, which was subjected to flash column chromatography on silica gel with 20% ethyl acetate in hexane. The product containing fractions were combined, concentrated and filtered to give 2.83 grams (81%) of a clear oil containing a small amount of solid. ¹H NMR (400 MHz, CDCl₃): δ = 1.23 (s, 3H, C=OCCH₃), 1.39 (s, 3H, CH₃), 1.43 (s, 3H, CH₃), 3.66 (m, 4H), 4.02 (dd, 2H, CH₂=CHCH₂), 4.20 (d, 2H), 4.31 (t, 2H, C=OOCH₂), 5.18 (dd, 1H, =CH), 5.28 (dd, 1H, =CH), 5.89 (m, =CHCH₂).

### Example 45. 2,2-Bis(hydroxymethyl)propionic acid, 2-(allyloxy)ethyl ester

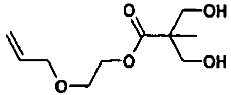

A solution of 2.72 grams of 2,2,5-trimethyl-1,3-dioxane-5-carboxylic acid, 2-(allyloxy)ethyl ester in 50 ml of methanol was treated with 1.0 gram of Dowex 50W-X8 resin (H+ form) and the reaction was stirred at room temperature overnight. The reaction was filtered, and the filtrate was concentrated to give an oil, which was subjected to flash column chromatography on silica gel with 5% methanol in dichloromethane. The product containing fractions were combined and concentrated to give 2.23 grams of the product as a clear, light yellow oil. ¹H NMR (400 MHz, CDCl₃): δ = 5.84-5.94 (ddt, 1H, H₂C=CHCH₂), 5.28 (dq, 1H, HHC=CHCH₂), 5.22 (dq, 1H, HHC=CHCH₂), 4.36 (app t, 2H, OCH₂CH₂), 4.02 (dt, 2H, H₂C=CHCH₂), 3.86 (dd, 2H, CH₂OH), 3.74 (dd, 2H, CH₂OH), 3.68 (app t, 2H, OCH₂CH₂), 2.90 (br d, 2H, OH), 1.11 (s, CH₃).

### Example 46. Preparation of 2,2-Bis[(2-bromoisobutyrytoxy)methyl]propionic acid, 2-(allyloxy)ethyl ester initiator

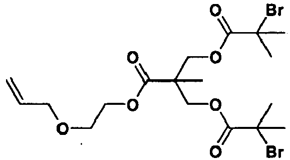

A solution of 1.2 grams of allyloxyethanol, 5.0 grams of 2,2-bis(2-bromoisobutyryloxymethyl) propionic acid and 690 mg of DPTS in 100 ml of dichloromethane was stirred at room temperature as 2.86 grams of DCC were added as a solution in a small amount of dichloromethane. The reaction was stirred at room temperature overnight, then filtered and concentrated to give an oil. This was subjected to flash chromatography on silica gel with 10% ethyl acetate in hexane. The appropriate fractions were combined and concentrated to give a clear oil, which was not sufficiently pure. This oil was again subjected to flash chromatography on silica gel with 3 - 4% ethyl acetate in hexane. The product containing fractions were combined and concentrated to give 2.78 grams of the desired product as a clear, colorless oil. NMR (CDCl₃): δ 5.89 (m, 1H, CH₂CH=CH₂), 5.28 (d of q, 1H, H-CH=CH, *J*=17.2, 1.7 Hz), 5.20 (d of q, 1H, H-CH=CH, *J*=10.5, 1.5 Hz), 4.38 (app q, 4H, CH₂OC=O), 4.31 (t, 2H, OCH₂, *J*=4.7 Hz), 4.01 (d of t, 2H, OCH₂, *J*=5.6, 1.5 Hz), 3.65 (t, 2H, OCH₂, J=4.7 Hz), 1.91 (s, 12H, Br-C (CH₃)₂), 1.35 (s, 3H, CH₃).

### Example 47. 2,2-Bis-[2,2-bis(2-bromoisobutyrytoxymethyl)propionyloxymethy)] propionic acid, 2-(allyloxy)ethyl ester initiator

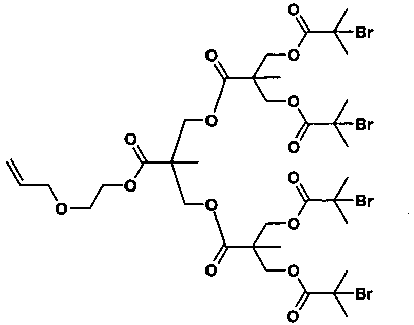

A solution of 2.42 grams of 2-[(2-bromoisobutyryloxy)methyl]-2-hydroxymethylpropionic acid, 2-(allyloxy)ethyl ester and 1.73 grams of 2,2-[bis-(2-bromoisobutyryloxy)methyl] propionic acid in 25 ml of dry acetonitrile, together with 200 mg of DPTS and 580 mg of DMAP, was treated with 1.03 grams of DCC, and the reaction was stirred at room temperature overnight. By TLC (silica gel, 30% ethyl acetate in hexane) it appeared that the reaction was incomplete, so another 812 mg of 2,2-[bis-(2-bromoisobutyryloxy)methyl]propionic acid and 400 mg of DCC were added, and the reaction was again stirred at room temperature overnight. The reaction mixture was filtered and concentrated, and the residue was subjected to flash column chromatography on silica gel first with 20%, and then with 30% ethyl acetate in hexanes. The product containing fractions were combined and concentrated to give 1.27 grams of the desired compound as a clear, colorless oil. NMR (CDCl₃): δ 5.88 (m, 1H, CH₂CH=CH₂), 5.28 (d of q, 1H, H-CH=CH, J=17.4, 1.6 Hz), 5.20 (d of q, 1H, H-CH=CH, *J*=10.3, 1.3 Hz), 4.24 - 4.44 (m, 14H, CH₂OC=O), 4.01 (d, 2H, CH₂=CHCH₂, *J*=5.6), 3.65 (t, 2H, CH₂CH₂OCH₂, J=4.7 Hz), 1.91 (s, 24H, Br-C (CH₃)₂), 1.33 (s, 6H, CH₃), 1.30 (s, 3H, CH₃).

### Example 48. Preparation of 2,2-Bis-[2,2-Bis[(2-Bromoisobutyryloxy) propionyloxymethyl]propionic acid], 2-[(2,3-dihydroxy)propoxy]ethyl ester initiator

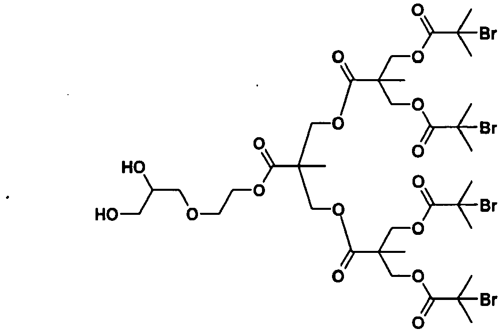

To a mixture of 1.21 grams of 2,2-bis[(2-bromoisobutyryloxy)methyl]propionic acid, 2-(allyloxy)ethyl ester in 15 ml of water and 15 ml of t-butanol was added 1.14 grams (3 eq) of potassium ferricyanide, 480 mg (3 eq) of potassium carbonate, 7.5 mg of potassium osmate dehydrate, 11 mg of quinuclidine, and 110 mg (1 eq) of methanesulfonamide, and the reaction mixture was stirred at room temperature overnight. The reaction appeared to be complete by tlc (silica gel, 50% ethyl acetate in hexane), so the reaction was poured into 50 ml of water, then extracted with 100 ml of dichloromethane, followed by another 50 ml of dichloromethane. The combined organics were dried over sodium sulfate, filtered and concentrated to give an oily residue, which was subjected to flash column chromatography on silica gel with 50% ethyl acetate in hexane, and the product containing fractions were combined and concentrated to give 620 mg of the desired product as a clear, colorless oil. NMR (CDCl₃): δ 4.28-4.41 (m, 14H, CCH₂OC=O), 3.86 (m, 1H, CH₂CHOHCH₂), 3.69-3.75 (m, 3H), 3.56-3.65 (m, 3H), 2.78 (dd, 1H, OH), 2.23 (app t, 1H, OH), 1.92 (s, 24H, CH₃CBr), 1.34 (s, 6H, CH₃), 1.31 (s, 3H, CH₃).

### Example 49. Preparation of 2,2-bis[(2-bromoisobutyryloxy)methyl]propionic acid, (2-azidoethoxy)ethyl ester initiator

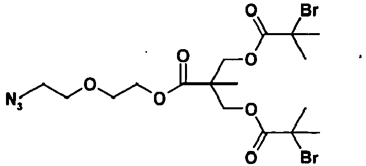

To a solution of 3.30 grams of 2,2-bis[(2-bromoisobutyryloxy)methyl]propionic acid and 1.0 gram of 2-(2-azidoethoxy)ethanol in 20 mL of dry acetonitrile, together with 225 mg of DPTS, was added 1.89 grams of DCC and the reaction was stirred at room temperature overnight. The reaction was filtered and concentrated to give a residue, which was subjected to flash column chromatography on silica gel with 10 - 15% ethyl acetate in hexane. The appropriate fractions were combined and concentrated to give 2.06 grams of the desired product as a clear, colorless oil. NMR (CDCl₃): δ 4.39 (app q, 4H, CCH₂O, *J*=11.1, 33.8 Hz), 4.31 (t, 2H, OCH₂CH₂OC=O, *J*=5 Hz), 3.72 (t, 2H, CH₂N₃, *J*=5 Hz), 3.67 (t, 2H, CH₂CH₂N₃, *J*=5 Hz), 3.38 (t, 2H, OCH₂CH₂OC=O, *J*=5 Hz), 1.92 (s, 12H, Br-C(CH₃)₂), 1.36 (s, 3H, CH₃).

### Example 50. Preparation of 3,5-bis-(2-bromoisobutyryloxy) benzaldehyde

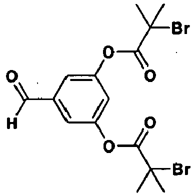

A solution of 1 .0 gram of 3,5-dihydroxybenzaldehyde and 4.0 ml (4 eq) of triethylamine in 20 ml of dichloromethane was cooled with an ice-water bath, and a solution of 3.35 grams of 2-bromoisobutyryl bromide in 5 ml of dichloromethane was added dropwise over a few minutes as much solid formed. The reaction was stirred at room temperature for 1.5 hr, at which time the reaction appeared to be complete by TLC (silica gel, 30% ethyl acetate in hexane). The reaction was washed with 25 ml of water, then concentrated to give a residue, which was subjected to flash column chromatography on silica gel with 10% ethyl acetate in hexane. The appropriate fractions were combined, treated with a small amount of decolorizing carbon, filtered and concentrated to give 2.2 grams of an oil, which crystallized in the refrigerator to give a white solid. ¹H NMR (400 MHz, CDCl₃): δ = 2.08 (s, 12H, CH₃), 7.29 (t, 1H, J=2.4 Hz, ArH), 7.61 (d, J=2.4 Hz, 2H, ArH), 10.0 (s, 1H, CHO).

### Example 51. Preparation of 7-(13-allyloxy-2,5,8,11-tetraoxatridecyl)-2,4,9-triphenyl-1,3,5-triazatricyclof3.3.1.13,7]decane

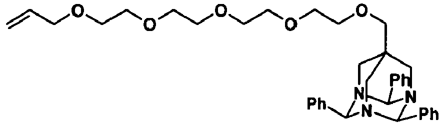

A solution of 870 mg of 11-allyloxy-3,6,9-trioxaundecan-1-ol methanesulfonate and 1.01 grams of 2,4,9-triphenyl-1,3,5-triazatricyclo[3.3-1.13,7]decane-7-methanol (WO2000/037658) in 10 ml of dry THF was treated with 410 mg of sodium hydride (60% in oil) and the reaction was heated at 80°C for 20 hours. The reaction was then quenched carefully by the addition of a few ml of water, poured into 20 ml of sat NaCl, then extracted with 3 × 10 ml of dichloromethane. The organics were dried over sodium sulfate, filtered and concenrated to give a residue, which was subjected to flash chromatography on silica gel with 25-35% ethyl acetate in hexane. The appropriate fractions were combined and concentrated to give 920 mg of the desired product as a colorless oil. NMR (DMSO-d₆): δ 7.70-7.82 (m, 6H, PhH), 7.26-7.51 (m, 9H, PhH), 3.69-3.75 (m, 3H), 3.56-3.65 (m, 3H), 2.78 (dd, 1H, OH), 2.23 (app t, 1H, OH), 1.92 (s, 24H, CH₃CBr), 1.34 (s, 6H, CH₃), 1.31 (s, 3H, CH₃).

### Example 52. Preparation of 1-Amino-15-allyloxy-2,2-bis(aminomethyl)-4,7,10,13-tetraoxapentadecane trihydrochloride

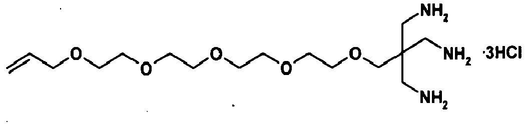

The triazaadamantane compound from the previous reaction was taken up in 20 ml of ethanol and 4 ml of ether, then treated with 2 ml of concentrated hydrochloric acid. The reaction was mixed and then left to stand at 4°C for 1.5 hours. Then 30 ml of ether were added and the mixture was cooled again for another 30 minutes. Then added 100 ml of ether and the solid product was recovered by filtration, washed with ether and dried under vacuum to give 564 mg of the product as a white solid. NMR (DMSO-d₆): δ 7.75 (m, 6H, CCH), 7.44 (m, 6H, CCHCH), 7.30 (m, 3H, CCHCHCH), 5.86 (m, 1H, CH₂=CH), 5.70 (s, 1H, NCH (equatorial)), 5.250 (s, 2H, NCH(axial)), 5.23 (d of q, 1H, CH₂=CH), 5.11 (d of q, 1H, CH₂=CH), 3.93 (d of t, 2H, CH-CH₂-O), 3.55-3.25 (m, 16H, OCH₂CH₂O), 3.26 (m, 2H, NCH₂), 3.19 (d, 2H, NCH₂), 2.88 (s, 2H, NCH₂), 2.719 (s, 2H, CCH₂O).

### Example 53. Preparation of N-(2-Bromo-2-methylpropionyl)-1-Amino-15-allyloxy-2,2-bis[N-(2-bromo-2-methylpropionyl)aminomethyl]-4,7,10,13-tetraoxapentadecane initiator

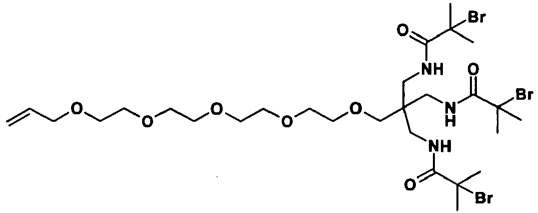

The triamine hydrochloride from the previous procedure was taken up in 25 ml of dichloromethane, the solution was cooled with and ice water bath, and treated with 1.35 ml of triethylamine, followed by the addition of 0.46 ml of 2-bromoisobutyryl bromide. The reaction was then stirred as it was allowed to warm to room temperature over 2 hours. The reaction mixture was then washed with 3 × 10 ml of 1N HCl, 2 × 10 mL of sat NaHCO₃, 10 ml of sat NaCl, and dried over magnesium sulfate. The solution was filtered and concentrated to give a residue, which was flushed through a plug of silica gel with ethyl acetate. Concentration gave 989 mg of the desired product as a viscous oil. NMR (DMSO-d₆): δ 8.004 (t, 3H, NH), 5.87 (m, 1H, CH), 5.23 (d of q, 1H, CH₂=CH), 5.12 (d of q, 1H, CH₂=CH), 3.93 (d of t, 2H, CH₂-CH), 3.6 - 3.45 (m, 16H, OCH₂CH₂O), 3.289 (s, 2H, CCH₂O), 3.12 (d, 6H, CCH₂N), 1.907 (s, 18H, CH₃).

### Example 54. Preparation of N-(2-Bromo-2-methylpropionyl)-1-Amino-15-(2,3-dihydroxypropyl)-2,2-bis[N-(2-bromo-2-methylpropionyl)aminomethyl]-4,7,10,13-tetraoxapentadecane initiator

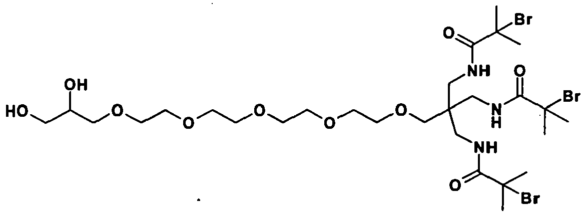

To a mixture of 350 mg of the alkene from the previous procedure in 5 ml of t-butanol and 5 ml of water was added 433 mg (3 eq) of potassium ferricyanide, 182 mg (3 eq) of potassium carbonate, 42 mg (1 eq) of methanesulfonamide, 7.5 mg of quinuclidine, and 4 mg of potassium osmate dihydrate, and the solution was stirred at room temperature overnight. The reaction appeared to be complete by TLC (silica gel, 5% methanol in dichloromethane), so 50 ml of water were added and the solution was extracted with 50 ml of dichloromethane, followed by another 2 × 25 ml of dichloromethane. The combined extracts were dried over sodium sulfate, concentrated, and the dark gray residue was subjected to flash column chromatography on silica gel with 2-5% methanol in dichloromethane. The appropriate fractions were combined and concentrated to give 310 mg of the desired dihydroxy compound as a light gray oil. NMR (CDCl₃): δ 7.91 (t, 3H, NH), 3.88 (m, 1H, HOCH₂CHOHCH₂), 3.55-3.72 (complex m, 21H), 3.35 (s, 1H, OCH₂C(CH₂)₃), 3.19 (d, 6H, *J*=6.4 Hz, CH₂NH), 1.99 (s, 18H, CH₃).

### Example 55. Preparation of 7-(7-Azido-2,5-dioxaheptyl)-2,4,9-triphenyl-1,3,5- triazatricyclo[3.3.1.13,7] decane

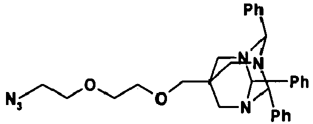

To a solution of 1.1 grams of 2,4,9-triphenyl-1,3,5-triazatricyclo[3.3.1.13,7]decane-7-methanol (WO2000/037658) and 585 mg of 2-(2-azidoethoxy)ethyl methanesulfonate in 15 ml of anhydrous THF was added 224 mg of NaH (60% in oil), and the solution was heated at 70°C overnight. Another 245 mg of NaH and 600 mg of 2-(2-azidoethoxy)ethyl methanesulfonate were added, and heating was again continued overnight. The reaction mixture was cooled, diluted with 25 ml of water, and extracted with 50 ml of dichloromethane. The organic layer was washed with saturated NaCl, dried over sodium sulfate, filtered and concentrated to give a residue. This material was subjected to flash column chromatography on silica gel with 10 - 25% ethyl acetate in hexane. The appropriate fractions were combined and concentrated to give 1.15 grams of the desired product as an oil, which was not completely pure, but used in the next reaction without further purification. NMR(DMSO) extremely complex.

### Example 56. Preparation of 1-Amino-9-azido-2,2-bis(aminomethyl)-4,7-dioxanonane trihydrochloride

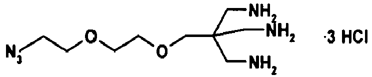

A solution of 1 .15 grams of the triazaadamantane compound from the previous procedure in 20 ml of ethanol and 4 ml of ether was cooled with an ice water bath, and 3 ml of concentrated HCl were added. Solid product began to form immediately, and the reaction was allowed to stand in the cold for 10 minutes. Another 30 ml of ether were added, and the reaction was refrigerated overnight. The reaction mixture was diluted with another 100 ml of ether, and the solid product was isolated by filtration, washed with more ether and dried under vacuum to give 800 mg of the product as a white solid.

### Example 57. Preparation of N-(2-Bromo-2-methylpropionyl)-1-Amino-9-azido-2,2-bis[N-(2-bromo-2-methylpropionyl)aminomethyl]-4.7-dioxanonane initiator

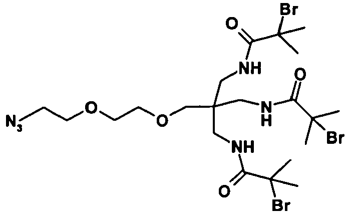

A solution of 800 mg of the trihydrochloride salt from the previous procedure in 25 ml of dichloromethane was cooled with an ice water bath, then treated with 3.5 ml of triethylamine. To this mixture was added dropwise 1.07 ml of 2-bromoisobutyryl bromide, and the reaction was stirred while warming to room temperature over 2 hours. The mixture was then washed with 3 × 10 ml of 1N HCl, 2 × 10 ml of saturated NaHCO3, and with 10 ml of saturated NaCl, then dried over magnesium sulfate. Filtration and concentration gave a residue, which was subjected to flash column chromatography on silica gel with 20-30% ethyl acetate in hexane. The appropriate fractions were combined and concentrated to give 630 mg of the desired product as an oil. NMR(CDCl₃): δ 7.76 (t, 3H, NH, J=6.3 Hz), 3.68 (m, 4H, OCH₂CH₂O), 3.63 (m, 2H, N₃CH₂CH₂O), 3.40 (t, 2H, N₃CH₂, J=5.0 Hz), 3.37 (s, 2H, CCH₂O), 3.19 (d, 6H, CCH₂N, J=6.8 Hz), 1.99 (s, 18H, CH₃).

### Example 58. 13-Allyloxy-2,5,8,11-tetraoxatridecyl 6-arm initiator

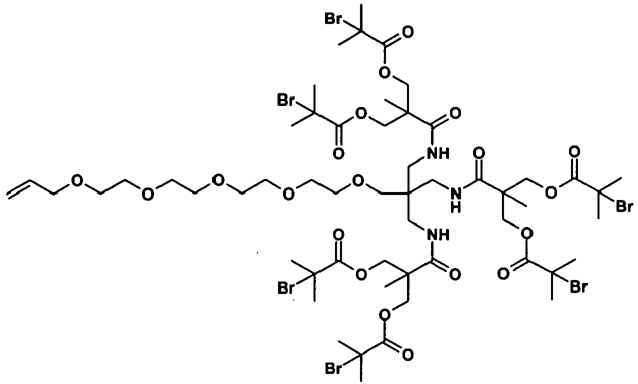

To a solution of 0.9 grams of 1-amino-15-allyloxy-2,2-bis(aminomethyl)-4,7,10,13-tetraoxapentadecane trihydrochloride and 3.89 grams of 2,2-bis[(2-bromoisobutyryloxy]methyl)propionic acid in 25 ml of dichloromethane, together with 530 mg of DPTS and 890 mg of DMAP, was added 2.7 grams of DCC and the reaction was stirred at room temperature overnight. The reaction was filtered and concentrated, and the residue was subjected to flash column chromatography on silica gel with 50-70% ethyl acetate in hexane. The appropriate fractions were combined and concentrated to give 1.9 grams of the desired product as a viscous oil. NMR (CDCl₃): δ 7.78 (t, 3H, NH, *J*=6.5 Hz), 5.91 (m, 1H, CH), 5.27 (d of q, 1H, CH₂=CH, *J*=17.4, 1.6 Hz), 5.18 (d of q, 1H, CH₂=CH, *J*=10.4, 1.4 Hz), 4.38 (app q, 12H, CH₂OC=O), 4.01 (d of t, 2H, CH-CH₂, J=5.7, 1.4 Hz), 3.61 (two m, 16H, OCH₂CH₂O), 3.30 (s, 2H, CCH₂O), 3.14 (d, 6H, CH₂N, *J*=6.1 Hz), 1.92 (d, 36H, BrC(CH₃)₂, *J*=1.2 Hz), 1.38 (s, 9H, CH₃).

### Example 59. 13-(2,3-Dihydroxypropyl)-2.5.8.11-tetraoxatridecyl 6-arm initiator

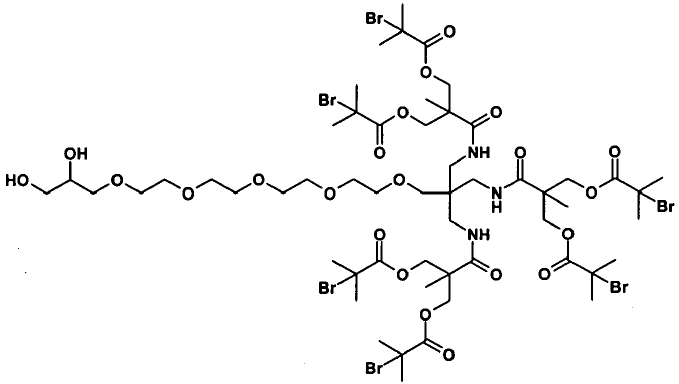

To a mixture of 1.0 gram of the alkene from the previous procedure in 10 ml of water and 10 ml of t-butanol was added 638 mg (3 eq) of potassium ferricyanide, 268 mg (3 eq) of potassium carbonate, 10 mg of potassium osmate dehydrate, 12 mg of quinuclidine, and 61 mg (1 eq) of methanesulfonamide, and the reaction mixture was stirred at room temperature overnight. The reaction was poured into 50 ml of water, then extracted with 50 ml of dichloromethane, followed by another 25 ml of dichloromethane. The combined organics were dried over sodium sulfate, filtered and concentrated to give an oily residue, which was subjected to flash column chromatography on silica gel with 2-4% methanol in dichloromethane, and the product containing fractions were combined and concentrated to give 417 mg of the desired product as a viscous oil. NMR (CDCl₃): δ 7.78 (t, 3H, NH, J=6.0 Hz), 4.39 (app q, 12H, CH₂OC=O), 3.86 (broad s, 1H, OH-CH), 3.62 (m, 20H, OCH₂CH₂O and OHCHCH₂O and OH-CH₂), 3.27 (s, 2H, CCH₂O), 3.13 (s, 6H, NCH₂), 2.40 (s, 2H, OH), 1.92 (s, 36H, BrC(CH₃)₂), 1.38 (s, 9H, CH₃).

### Example 60. Preparation of 2-(Acryloyloxyethyl-2'-(trimethylammonium)ethyl phosphate, inner salt

### 1^{st} intermediate

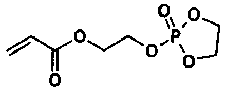

A solution of 1 1.6 grams of 2-hydroxyethylacrylate and 14.0 ml of triethylamine in 100 ml of dry acetonitrile, under a nitrogen atmosphere, was cooled to -20°C, and a solution of 14. 2 grams of 2-chloro-2-oxo-1,3,2-dioxaphospholane in 10 ml of dry acetonitrile was added dropwise over about 30 minutes. The reaction was stirred in the cold for 30 minutes, then filtered under a nitrogen atmosphere. The precipitate was washed with 10 ml of cold acetonitrile, and the filtrate was used directly in the next reaction.

### 2-(Acryloyloxyethyl-2'-(trimethylammonium)ethyl phosphate, inner salt

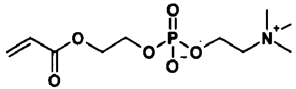

To the solution from the previous procedure was added 14.0 ml of trimethylamine (condensed using a dry ice-acetone condenser under nitrogen), the reaction mixture was sealed into a pressure vessel, and stirred at 65°C for 4 hours. The reaction mixture was allowed to stir while cooling to room temperature, and as it reached about 30°C, a solid began to form. The vessel was then placed in a 4°C refrigerator overnight. Strictly under a nitrogen atmosphere, the solid was recovered by filtration, washed with 20 ml of cold dry acetonitrile, then dried under a stream of nitrogen for 15 minutes. The solid was then dried under high vacuum overnight to give 12.4 grams of product as a white solid. NMR (CDCl₃): δ 6.41 (dd, 1H, *J*=1.6, 17.2 Hz, vinyl CH), 6.18 (dd, 1H,*J*=10.6, 17.2 Hz, vinyl CH), 5.90 (dd, 1H, *J*=1.6, 10.4 Hz, vinyl CH), 4.35 (m, 2H), 4.27 (m, 2H), 4.11 (m, 2H), 3.63 (m, 2H), 3.22 (s, 9H, N(CH₃)₃).

### Example 61. Preparation of 4-Pentyn-1-ol, NHS ester

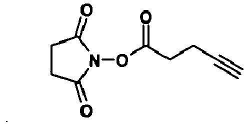

A solution of 1 .02 grams of 4-pentynoic acid and 1.20 grams of N-hydroxysuccinimide in 20 ml of dry acetonitrile was treated with 300 mg of DPTS, followed by 2.8 grams of DCC, and the reaction was stirred at room temperature overnight. The reaction was filtered and concentrated to give a residue, which was subjected to flash column chromatography on silica gel with 30% ethyl acetate in hexane. The product containing fractions were combined and concentrated to give a 1.62 grams of the desired product as a white solid. NMR(CDCl₃): δ 2.89 (d of d, 2H, CH₂C=O, *J*=7.9, 6.4 Hz), 2.85 (s, 4H, O=CCH₂CH₂C=O), 2.62 (app d of d of d, 2H, CHCCH₂, J=8.6, 6.9, 2.7 Hz), 2.06 (t, 1H, CH, J=2.7 Hz).

### Example 62. Preparation of N-Propargylmaleimide

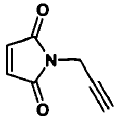

A solution of 1.08 grams of propargylamine hydrochloride in 50 ml of saturated sodium bicarbonate was cooled with an ice water bath, and 2.0 grams of N-carboethoxymaleimide were added portionwise over a few minutes. The reaction was stirred in the cold for 30 min., then while warming to room temperature over 25 min. The reaction was then extracted with 3 × 25 ml of dichloromethane, which was dried over sodium sulfate, filtered and concentrated. The residue was taken up in 10 ml of ethyl acetate and heated at 50°C for two hours to complete the cyclization. The reaction was concentrated and the residue was which was subjected to flash column chromatography on silica gel with 30% ethyl acetate in hexane. A second chromatography as before gave 1.24 g of the product as a very light yellow oil. NMR(CDCl₃): δ 6.77 (s, 2H, CHC=O), 4.30 (d, 2H, NCH₂, *J*=2.4 Hz), 2.22 (t, 1H, CCH, *J*=2.5 Hz).

### Example 63. Preparation of 5-Hexyn-1-al

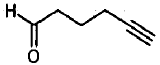

A solution of 694 mg of 5-hexyn-1-ol in 20 ml of dichoromethane was treated at room temperature with 3.0 grams of Dess-Martin periodinane, and the solution was stirred at room temperature for 2 hr. The reaction was filtered and the filtrate was concentrated to give a residue, which was subjected to flash column chromatography on silica gel with ethyl acetate in hexane. Concentration of the appropriate fractions gave the product as a very light yellow oil. NMR(CDCl₃): δ 9.81 (t, 1H, CH=O, *J*=2.6 Hz), 2.61 (t of d, 2H, CH₂CH=O, *J*=7.1, 1.2 Hz), 2.28 (t of d, 2H, CCH₂, *J*=7.1, 2.6 Hz), 1.99 (t, 1H, CCH, *J*=2.6 Hz), 1.86 (p, 2H, CCH₂CH₂, *J*=7.0 Hz).

### Example 64. Preparation of Bis [2,2-(2-bromoisobutyryl)hydroxymethyl] propionic acid, 3,6,9,12-tetraoxapentadec-14-yn-1-ol ester

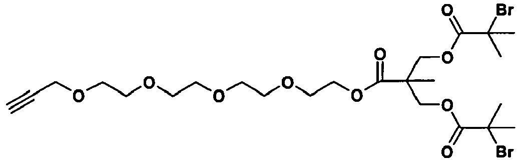

A 100-ml round-bottom flask equipped with a stir bar was charged with 30 ml of dry acetonitrile, 3.0 grams of bis[2,2-(2-bromoisobutyryl)hydroxymethyl]propionic acid and 1.63 grams of 3,6,9,12- tetraoxapentadec-14-yn-1-ol. To the solution was added 300 mg of DPTS, followed by 1.86 grams (1.3 eq) of DCC and the reaction mixture was allowed to stir at room temperature overnight. Filtration and concentration of the reaction mixture gave a residue, which was purified by flash chromatography on silica gel with 20-50% ethyl acetate in hexane. The appropriate fractions were combined and concentrated to give 1.82 grams of the desired product as a clear oil containing a small amount of solid. ¹H NMR (400 MHz, CDCl₃): δ = 1.35 (s, 3H, CH₃CC=O), 1.92 (s, 12H, (CH₃)₂CBr), 2.43 (t, *J*=2.4, 1H, CCH), 3.64-3.72 (m, 14H, OCH₂CH₂O), 4.21 (d, 2H, *J*=2.4, HCCCH₂), 4.30 (app q, 2H, OCH₂CH₂OC=O), 4.34 (dd, 2H, CH₂OC=OCBr).

### Example 65. Preparation of 3,6,9,12-Tetraoxapentadec-14-yn-1-amine

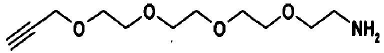

A solution of 3.5 grams of 3,6,9,12-tetraoxapentadec-14-yn-1-ol, 1-methanesulfonate in 50 mL of concentrated aqueous ammonia was stirred and heated at 100 °C in a pressure vessel for 2 hours. The vessel was then cooled, and the reaction was concentrated to give a yellow oil. To this was added 20 ml of absolute ethanol and the solution was reconcentrated to give a yellow oil, which was subjected to chromatography on silica gel with 7% methanol in dichloromethane. The appropriate fractions were combined and concentrated to give 2.24 grams of the desired product as a yellow oil. ¹H NMR (400 MHz, CDCl₃): δ = 2.54 (t, 1H, *J*=2.4, CCH), 3.23 (app t, 2H, CH₂NH₂), 3.66 (m, 8H, OCH₂CH₂O), 3.74 (m, 4H, OCH₂CH₂O), 3.86 (app t, 2H, CH₂CH₂NH₂), 4.26 (d, *J*=2.4, 2H, CH₂CCH).

### Example 66. Preparation of 7-Allyloxymethyl-2,4,9-triphenyl-1,3,5-triazatricyclo [3.3.1.13,7] decane

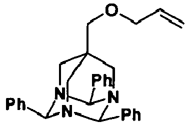

A mixture of 50 ml of DMSO and 2.8 grams of powdered KOH was stirred at room temperature for 10 minutes, then 4.0 grams of 2,4,9-triphenyl-1,3,5-triazatricyclo [3.3.1.13,7] decane-7-methanol were added, quickly followed by 1.46 grams (1.2 eq) of allyl bromide. The reaction mixture was stirred at room temperature for 3 hours, then partitioned between 100 ml of ether and 100 ml of water. The aqueous layer was extracted with another 3 × 50 ml of ether, and the combined organics were dried over sodium sulfate. Filtration and concentration gave a solid foam, which was subjected to flash chromatography on silica gel with 5% ethyl acetate in hexane. The appropriate fractions were combined and concentrated to give 3.51 grams (80%) of the desired product as a crushable yellow foam. ¹H NMR (400 MHz, CDCl₃): δ = 2.68 (s, 2H, NCH₂ adjacent to equatorial phenyls), 2.92 (s, 2H, CCH₂), 3.28 (d, *J*=13.4 Hz, 2H, NCH₂ between axial and equatorial phenyls), 3.51 (d, *J*=13.4 Hz, 2H, NCH₂ nearest axial phenyl), 3.73 (d of t, *J*=1.5, 5.4 Hz, 2H, CHCH₂O), 5.04 (d of q, *J*=1.5, 10.4 Hz, 1H, CH₂=CH), 5.07 (d of q, *J*=1.7, 17.2 Hz, 1H, CH₂=CH), 5.42 (s, 2H, NCH axial), 5.65 (s, 1H, NCH equatorial), 5.71 (m, *J*=5.4, 10.4, 17.2 Hz, 1H, CH₂=CH), 7.2 - 7.9 (m, 15H, phenyl).

### Example 67. Preparation of 2,2-Bis(aminomethyl)-4-oxahept-6-enylamine trihvdrochloride

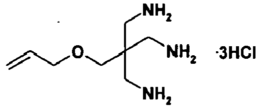

A solution of 3.51 grams of 7-allyloxymethyl-2,4,9-triphenyl-1,3,5-triazatricyclo [3.3.1.13,7] decane in 30 ml of tetrahydrofuran was treated with 30 ml of 1N HCl, and the reaction was stirred at room temperature for 30 minutes. The THF was removed on the rotovap, and the aqueous residue was extracted with 3 × 25 ml of ether. The aqueous layer was concentrated to dryness, 20 ml of methanol were added, and the solution was again concentrated to dryness. The resulting white solid was placed under high vacuum overnight to give 2.10 grams (93%) of the desired product as a white solid. ¹H NMR (400 MHz, D₂O): δ = 3.34 (s, 6H, CH₂NH₃), 3.76 (s, 2H, OCH₂C(CH₂)₃), 4.11 (m, 2H, CH₂=CHCH₂), 5.28-5.39 (m, 2H, CH₂=CHCH₂), 5.92-6.03 (m, CH₂=CHCH₂).

### Example 68. Preparation of N-(2-Bromo-2-methylisobutyryl)-2,2-bis[N-(2-bromo-2- methylpropionyl)aminomethyl]-4-oxahept-6-enyl amine

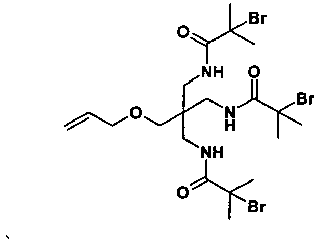

A mixture of 2.10 grams of 2,2-bis(aminomethyl)-4-oxa-hept-6-ylamine trihydrochloride in 250 ml of dichloromethane was treated with 10 ml of triethylamine, then cooled with an ice water bath. To this solution was added 5.77 grams of 2-bromoisobutyryl bromide dropwise over a few minutes. The ice bath was removed and the solution was stirred for 2 hours. The reaction mixture was extracted with 100 ml of water and the organic layer was dried over sodium sulfate. Filtration and concentration gave a residue, which was subjected to flash chromatography on silica gel with 2-6% ethyl acetate in dichloromethane. The appropriate fractions were combined and concentrated to give 3.66 grams (79%) of the desired product as a white solid. ¹H NMR (400 MHz, CDCl₃): δ = 1.99 (s, 18H, CH₃), 3.20 (d, 6H, *J*=6.8, CH₂NH₂), 3.34 (s, 2H, OCH₂C(CH₂)₃), 3.99 (m, 2H, CH₂=CHCH₂), 5.19-5.30 (m, 2H, CH₂=CHCH₂), 5.87-5.97 (m, 1H, CH₂=CHCH₂), 7.72 (app t, *J*=6.8, 3H, NH).

### Example 69. Preparation of N-(2-Bromo-2-methylpropionyl)-2,2-bis[N-(2-bromo-2-methylpropionyl) aminomethyl]-4-oxa-6,7-dihydroxyheptyl amine

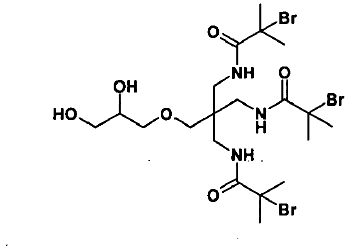

A solution of 5.39 grams of N-(2-bromo-2-methylpropionyl)-2,2-bis[N-(2-bromo-2-methylpropionyl)aminomethyl]-4-oxahept-6-enyl amine in 60 ml of t-butanol and 60 ml of water was treated with 8.8 grams (3 eq) of potassium ferricyanide, 3.68 grams (3 eq) of potassium carbonate, 850 mg (1 eq) of methanesulfonamide, 160 mg of quinuclidine, and 130 mg of potassium osmate dehydrate, and the reaction was stirred at room temperature for 4 hours. The mixture was partitioned between 150 ml of ethyl acetate and 150 ml of water, and the aqueous layer was extracted with another 2 × 30 ml of ethyl acetate. The combined organics were dried over sodium sulfate, filtered and concentrated to give a semisolid residue. This was subjected to flash chromatography on silica gel with 2-4% methanol in dichloromethane, and the appropriate fractions were combined and concentrated to give 5.33 grams of the desired product as a gray foam.

### Example 70. N-(2-Bromo-2-methylpropionyl)-6-amino-5,5-bis[N-(2-bromo-2- methylpropionyl) aminomethyl]-3-oxahexanal

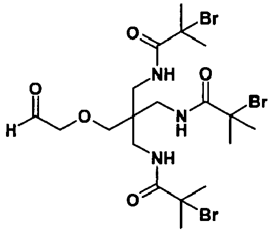

To a solution of 5.33 g of N-(2-bromo-2-methylpropionyl)-2,2-bis[N-(2-bromo-2-methylpropionyl)aminomethyl]-4-oxa-6,7-dihydroxyheptyl amine in 200 ml of THF and 50 ml of water was added 3.5 grams of sodium metaperiodate, and the reaction was stirred at room temperature for 3 hours, then concentrated to remove most of the THF. The residue was partitioned between 100 ml of ethyl acetate and 50 ml of water, and the aqueous was washed with 25 ml of ethyl acetate. The combined organics were washed with 50 ml of sat NaCl and dried over sodium sulfate. Filtration and concentration gave a gray residue, which was subjected to flash chromatography on silica gel with 50% ethyl acetate in hexane, and the appropriate fractions were combined and concentrated to give 3.87 grams of the desired product as a nearly white solid. ¹H NMR (400 MHz, CDCl₃): δ = 2.00 (s, 18H, CH₃), 3.19 (d, 6H, *J*=6.8, CH₂NH), 3.31 (s, 2H, OCH₂C(CH₂)₃), 4.32 (s, 2H, CHOCH₂), 8.01 (app t, *J*=6.8, 3H, NH), 9.70 (s, 1H, CHO).

### Example 71. Preparation of N-(2-Bromo-2-methylpropionyl)-5,5-bis[N-(2-bromo-2-methylpropionyl)aminomethyl]-3-oxa-6-aminohexanoic acid

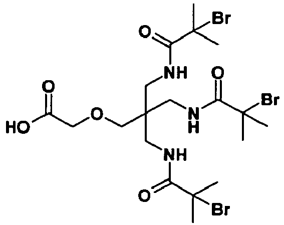

A solution of chromic acid (Jones reagent) was prepared by dissolving 2.55 grams of chromium trioxide in 2.2 ml of conc sulfuric acid, cooled with an ice bath, and carefully diluting the mixture to 10 ml with water. A 7 ml aliquot of this reagent was cooled with an ice water bath, and a solution of 3.67 grams of N-(2-bromo-2-methylpropionyl)-2,2-bis[N-(2-bromo-2-methylpropionyl)aminomethyl]-4-oxa-6-oxohexyl amine in 20 ml of acetone was added dropwise over 5 minutes. The reaction was stirred in the cold for 20 minutes, then partitioned between 200 ml of ethyl acetate and 200 ml of water. The aqueous layer was extracted with another 25 ml of ethyl acetate and the combined organics were washed with 25 ml of saturated NaCl and dried over sodium sulfate. The solution was filtered and concentrated to give a thick dark oil. This was subjected to flash column chromatography on silica gel with 2% methanol in dichloromethane containing 0.1% acetic acid. The appropriate fractions were combined and concentrated to give 3.58 grams of the desired product as a foam. ¹H NMR (400 MHz, CDCl₃): δ = 2.01 (s, 18H, CH₃), 3.21 (d, 6H, *J*=2.8, CH₂NH), 3.36 (s, 2H, OCH₂C(CH₂)₃), 4.13=2 (s, 2H, CH₂CO₂H), 8.15 (app t, *J*=2.8, 3H, NH).

### Example 72. Preparation of N-Boc-β-alanine, N-hydroxysuccinimide ester

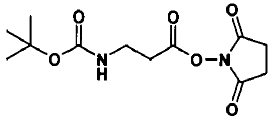

A solution of 8.0 grams of N-Boc-β-alanine and 5.0 grams of N-hydroxysuccinimide, together with 100 mg of DPTS, in 80 ml of anhydrous acetonitrile was treated with 10.5 grams of DCC, and the reaction was stirred at room temperature overnight. The mixture was filtered and the precipitate was washed with acetonitrile. The filtrate was concentrated to give an oil, which was subjected to flash chromatography on silica gel with 30-40% ethyl acetate in hexane to give the desired product as a white solid. ¹H NMR (400 MHz, CDCl₃): δ = 1.45 (s, 9H, C(CH₃)₃), 5.93 (m, 6H, NHS, CH₂COON), 3.53 (app q, 2H, NHCH₂), 5.2 (br s, 1H, NH).

### Example 73. Preparation of 3,6,9,12-Tetraoxa-14-ynpentadecanal

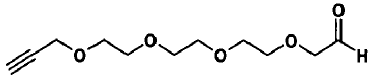

To a solution of 1 .0 gram of **3,6,9,12-tetraoxapentadec-14-yn-1-ol** and 67 mg of TEMPO in 5 ml of dichloromethane was added 1.52 grams of iodobenzene diacetate and the reaction was stirred at room temperature overnight. The reaction was concentrated to give a yellow oil, which was subjected to flash chromatography on silica gel with 50-100% ethyl acetate in hexane. The appropriate fractions were combined and concentrated to give 300 mg of the product as a clear, colorless oil. ¹H NMR (400 MHz, CDCl₃): δ = 2.44 (t, 1H, *J*=2.4, CCH), 3.65-3.77 (m, 12H, OCH₂CH₂O), 4.17 (d, *J*=0.8, 2H, CH₂CHO), 4.21 (d, 2H,*J*=2.4, CH₂CCH), 9.74= (s, 1H, CHO).

### Example 74. Preparation of 7-Azidooxy-2,5-dioxaheptyl 6-arm initiator

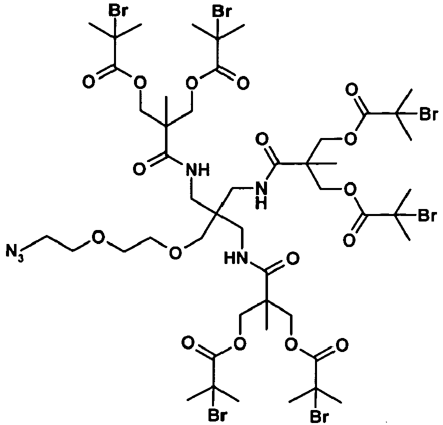

A solution of 800 mg of 1-Amino-9-azido-2,2-bis(aminomethyl)-4,7-dioxanonane trihydrochloride, 3.89 g of bis[2,2-(2-bromoisobutyryl)hydroxymethyl]propionic acid, 530 mg of DPTS, and 890 mg of dimethylaminopyridine in dichloromethane was treated with 2.7 g N,N'-dicyclohexylcarbodiimide and stirred overnight at room temperature. The reaction mixture was filtered, concentrated, and purified by silica gel flash chromatography with 50% ethyl acetate in hexane to give 2.1 g of the desired product. ¹H NMR (400 MHz, CDCl₃): δ = 1.38 (s, 9H, CH₃), 1.92 (s, 36H, CH₃), 3.15 (d, *J*=6.6 Hz, 6H, CH₂NH), 3.32 (s, 2H, OCH₂C), 3.42 (t, *J*=5.2 Hz, 2H, N₃CH₂), 3.60 (m, 2H, OCH₂CH₂O), 3.66 (m, 2H, OCH₂CH₂O), 3.69 (t, *J*=5.2 Hz, 2H, N₃CH₂), 4.38 (dd, *J*=11.1, 17.0 Hz, 12H, CCH₂O), 7.57 (broad t, *J*=6.6 Hz, NH₂).

### Example 75. Preparation of N-(2-Bromo-2-methylpropionyl)-5,5-bis[N-(2-bromo-2- methylpropionyl)aminomethyl]-3-oxa-6-aminohexanoic acid, N-hydroxysuccinimidyl ester

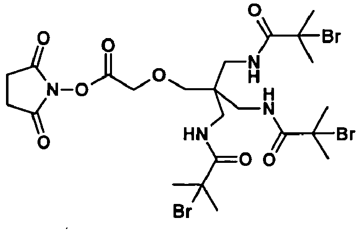

A solution of 64.5 mg N-hydroxysuccinimide, 358 mg of N-(2-Bromo-2-methylpropionyl)-5,5-bis[N-(2-bromo-2-methy]propionyl)aminomethyl]-3-oxa-6-aminohexanoic acid, and 26 mg of DPTS was treated with 300 mg N,N'-dicyclohexylcarbodiimide and stirred overnight at room temperature. The reaction mixture was filtered, concentrated, and purified by silica gel flash chromatography with 50% ethyl acetate in hexane to give 270 mg of the desired product as a white powder. ¹H NMR (400 MHz, CDCl₃): δ = 1.99 (s, 18H, CH₃), 2.87 (s, 4H, CH₂CO), 3.17 (d, *J*=6.6 Hz, 6H, CH₂NH), 3.39 (s, 2H, CCH₂O), 4.51 (s, 2H, OCH₂CO), 7.86 (t, *J*=6.6 Hz, 3H, NH).

### Example 76. Preparation of N-(3,7,10,13-tetraoxapentadec-14-ynyl)-3-methylmaleimide

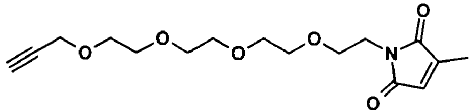

A 346 mg aliquot of 3,6,9,12-tetraoxapentadec-14-yn-1-amine was added slowly to 224 mg of citraconic anhydride powder with stirring under nitrogen. An exothermic reaction took place, producing a tan solid. The resulting mixture was heated to 120 °C for 6 hours, then allowed to cool to room temperature. The product was isolated by silica gel flash chromatography with 50% ethyl acetate in hexane, yielding 160 mg of pure product as a clear, colorless oil. ¹H NMR (400 MHz, CDCl₃): δ = 2.08 (d, 3H, CH₃), 2.43 (t, 1H, *J*=2.4 Hz, CHCCH₂), 3:58-3:72 (m, 18H, CH₂CH₂O), 4.20 (d, *J*=2.4 Hz, 2H, CCH₂O), 6.32 (q, *J*=1.8 Hz, 1H, CHCO).

### Example 77. Preparation of N-(3,7,10,13-tetraoxapentadec-14-ynyl) maleimide

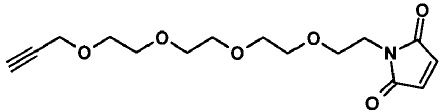

A 1.15 g aliquot of 3,6,9,12-tetraoxapentadec-14-yn-1-amine was added slowly to 660 mg of powdered maleic anhydride with stirring under nitrogen. The mixture was then heated to 120 °C for 6 hours, then allowed to cool to room temperature. The product was isolated by silica gel flash chromatography with 50% ethyl acetate in hexane.

### Example 78. Preparation of 7-Propargyloxymethyl-2,4,9-triphenyl-1,3,5-triazatricyclo[3.3.1.13,7]decane

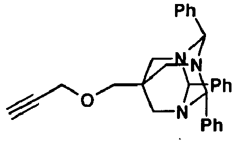

A solution of 3.0 g of 2,4,9-triphenyl-1,3,5-triazatricyclo[3.3.1.13,7] decane-7-methanol (WO2000/037658) and 850 mg of potassium hydroxide in 20 ml of dimethylsulfoxide was treated with 1.46 g of 80% propargyl bromide solution and the reaction was stirred overnight at room temperature. The mixture was partitioned between 100 ml each of water and diethyl ether and the aqueous layer was extracted twice with 50 ml ether. The combined organics were washed with 20 ml water, then dried, filtered, and concentrated. The residue was subjected to silica gel flash chromatography in 5% ethyl acetate in hexane to yield 0.9 g of the desired product.

### Example 79. Preparation of 2,2-Bis(aminomethyl)-4-oxahept-6-ynylamine trihydrochloride

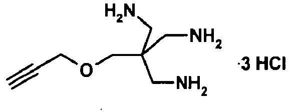

A 900 mg sample of [the propargyl adamantane product from the previous procedure] in 10 ml of tetrahydrofuran was treated with 10 ml of IN aqueous hydrochloric acid and stirred at room temperature for 30 minutes. The tetrahydrofuran was then removed by rotary evaporation at room temperature and the resulting aqueous solution was extracted with 3 × 25 ml of ether. The aqueous layer was carefully concentrated, dissolved in 20 ml methanol, and concentrated again to yield 568 mg of the desired product as a dark brown powder.

### Example 80. Preparation of N-(2-Bromo-2-methylisobutyryl)-2,2-bis[N-(2-bromo-2-methylpropionyl)aminomethyl]-4-oxahept-6-ynylamine

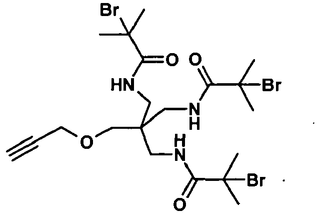

A 580 mg sample of [the propargyl triamine from the previous procedure] was suspended in 25 ml dichloromethane with 2.5 ml triethylamine and stirred on ice. Then 1.43 g of bromoisobutyryl bromide were added dropwise and the reaction stirred for 2 hours as it gradually warmed to room temperature. The mixture was washed with 3 × 10 ml 1N hydrochloric acid, 2 × 10 ml saturated sodium bicarbonate, and 10 ml saturated sodium chloride. The organic phase was dried over anhydrous magnesium sulfate, filtered, and concentrated, and the residue subjected to silica gel flash chromatography with 5% ethyl acetate in dichloromethane to yield 700 mg of the desired product. ¹H NMR (400 MHz, CDCl₃): δ = 1.99 (s, 18H, CH₃), 2.46 (t, 1H, *J*=2.4 Hz, CCH), 3.18 (d, *J*=6.7 Hz, 6H, CH₂NH), 3.39 (s, 2H, CH₂O), 4.18 (d, *J*=2.4 Hz, CHCCH₂O), 7.73 (t, *J*=6.7 Hz, 3H, NH).

### Example 81. Preparation of 1-Azido-2.2-bis(azidomethyl)-4,7,10,13,16-pentaoxanonadec-18-ene

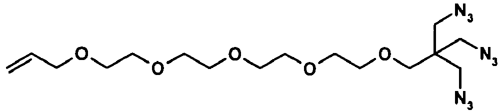

A solution of 530 mg of pentaerythritol triazide in 10 ml tetrahydrofuran was treated with 380 mg sodium hydride (60% dispersion). When the bubbling subsided, 1.24 g of 3,6,9,12-tetraoxapentadec-14-en-1-ol, 1-methanesulfonate was added, and the reaction stirred overnight at 70-80 °C. The mixture was allowed to cool and a few drops of water were added to quench any remaining sodium hydride, then most of the THF was removed by concentration. The residue was partitioned between 50 ml each water and dichloromethane. The aqueous phase was extracted twice with 25 ml dichloromethane, and-the combined organics (100 ml) were washed twice with 25 ml saturated sodium chloride. The organic phase was dried over anhydrous magnesium sulfate, filtered, and concentrated, and the residue subjected to silica gel flash chromatography with 10-50% ethyl acetate in hexane to separate two closely spaced spots. The final yield was 260 mg of clear, colorless oil. ¹H NMR (400 MHz, CDCl₃): δ = 3.34 (s, 2H, CCH₂O), 3.35 (s, 6H, CH₂N₃), 3.59 - 3.68 (m, 16H, OCH₂CH₂O), 4.03 (d oft, *J*=1.4, 5.6 Hz, 2H, CHCH₂O), 5.18 (d ofq, *J*=1.4, 10.4 Hz, 1H, CH₂=CH), 5.28 (d of q, *J*=1.6, 17.3 Hz, 1H, CH₂=CH), 5.92 (m, *J*=5.6, 10.4, 17.2 Hz, 1H, CH).

### Example 82. Preparation of 2,5,8,11,14-Pentaoxaheptadec-16-enyl 9-arm click-based initiator

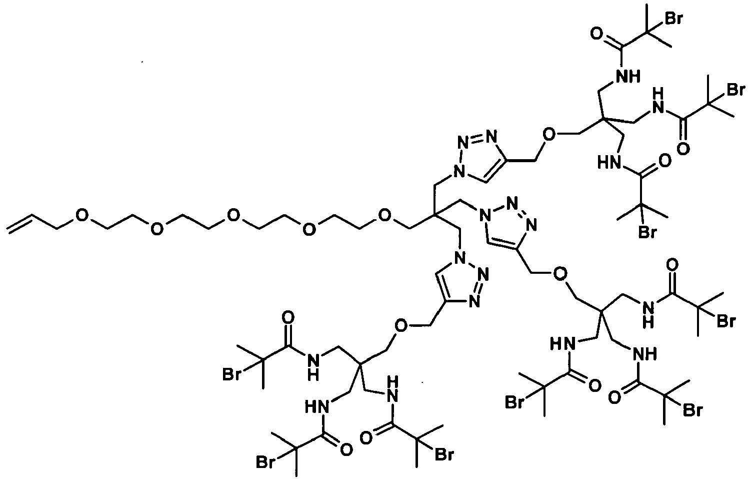

To a degassed solution of allyl tetraethylene glycol triazide (120mg, 0.28mmol) in 3 ml of absolute ethanol was added 253 µl of a solution of PMDETA in DMF (100mg/ml) (25.3mg, 0.146mmol) followed by 700 mg of the 3-arm alkyne derivative (1.1 mmol, 4 equivalents vs. mol of initiator) dissolved in 3 ml of ethanol. The mixture was degassed by 3 quick vacuum - nitrogen cycles. Then 21mg of CuBr (0.146 mmol, 0.5 equivalents, or 0.17 Cu per azide) were added to the reaction mixture. The reaction was quickly degassed and left to proceed overnight under nitrogen with stirring at room temperature. Silica gel flash chromatography yielded the desired product.

### Example 83. Preparation of 16,17-Dihydroxy-2,5,8.11,14-pentaoxaheptadecyl 9-arm click-based initiator

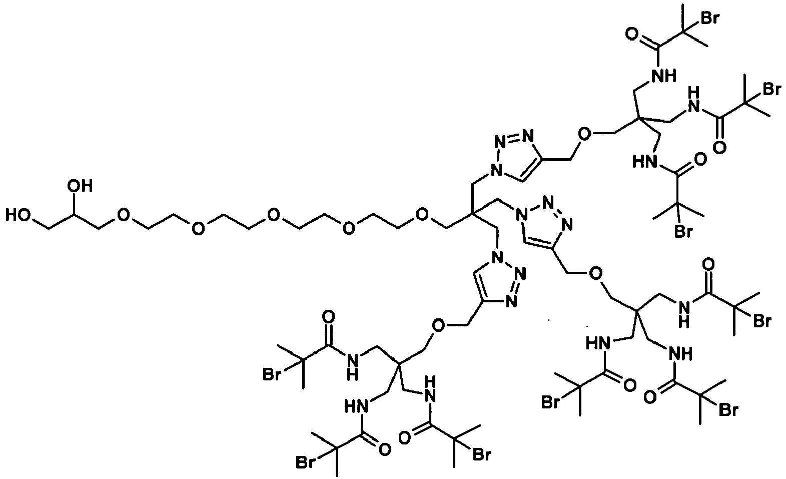

A round-bottomed flask equipped with a stirbar was charged with 15 ml water, 15 ml t-butanol, 456 mg of the allyl tetraethylene glycol triazole from the previous procedure, 198 mg potassium ferricyanide, 83 mg potassium carbonate, 19 mg methanesulfonamide, 1 mg quinuclidine, and 1 mg potassium osmate dihydrate and stirred overnight at room temperature. The reaction mixture was partitioned between 100 ml each of water and dichloromethane. The aqueous layer was extracted twice more with 25 ml dichloromethane, and the organic layers were combined, dried over anhydrous magnesium sulfate, filtered, and concentrated. The residue was subjected to silica gel flash chromatography using 5% methanol in dichloromethane to give the desired product.

### Example 84. Preparation of 2,5,8,11,14-Pentaoxaheptadee-16-enyl 9-arm amide-based initiator

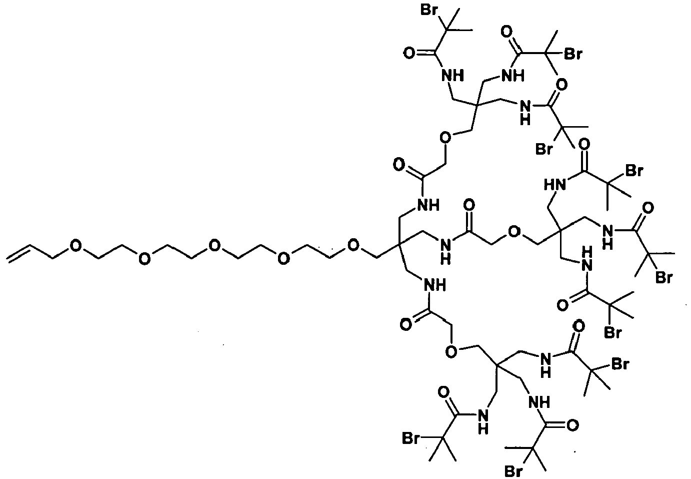

A solution of 1-amino-15-allyloxy-2,2-bis(aminomethyl)-4,7,10,13-tetraoxapentadecane trihydrochloride in acetonitrile, together with 6 eq of triethylamine, was allowed to react with a solution of 3 eq of N-(2-bromo-2-methylpropionyl)-5,5-bis[N-(2-bromo-2-methylpropionyl)aminomethyl]-3-oxa-6-aminohexanoic acid, N-hydroxysuccinimidyl ester in acetonitrile, and the mixture was stirred overnight. The reaction mixture was concentrated to give a residue, which was taken up in dichloromethane and washed with 1N HCl, followed by saturated sodium chloride, then dried over sodium sulfate. Filtration and concentration gave a residue, which was purified by flash chromatography on silica gel with mixtures of ethyl acetate in hexane to give the desired product.

### Example 85. Preparation of propargyl tetraethylene glycol iodoacetamide

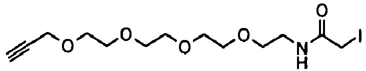

Iodoacetic anhydride (8.8 mmol, 3.11 g) was added to a stirred solution of propargyl tetraethylene glycol amine (8 mmol, 1.85 g) and N,N-Diisopropylethylamine (8 mmol, 1.39 g) in dry acetonitrile (20 ml). After 90 minutes, the mixture was concentrated. The residue was dissolved in 100 ml ethyl acetate and washed three times with 100 ml water followed by 50 ml saturated sodium chloride. The organics were dried over anhydrous sodium sulfate and concentrated, and the residue subjected to silica gel flash chromatography with 30-40% ethyl acetate in hexane.

### Example 86. Preparation of propargyl tetraethylene glycol bromoacetamide

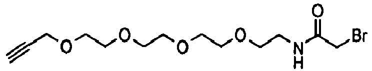

A round-bottomed flask equipped with stirbar was charged with propargyl tetraethylene glycol amine (8 mmol, 1.85 g), bromoacetic acid (12 mmol, 1.67 g), dimethylaminopyridine (9.6 mmol, 1.17 g), 4-Dimethylaminopyridinium 4-toluenesulfonate (2.4 mmol, 0.71 g), and dichloromethane (20 ml). Nitrogen was bubbled through the stirring mixture for 10 minutes, then N,N-Dicyclohexylcarbodiimide (15.6 mmol, 3.22 g) was added. After stirring overnight at room temperature, the mixture was filtered, concentrated, and subjected to silica gel flash chromatography with 40% ethyl acetate in hexane.

### Example 87. Preparation of N-(2-Bromo-2-methylisobutyryl)-2,2-bis[N-(2-bromo-2-methylpropionyl)aminomethyl]-3-amino-1-propanol

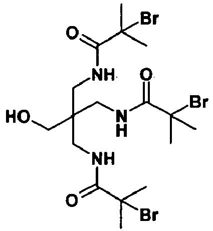

A solution of 2.00 grams of 2,2-aminomethyl-3-amino-1-propanol trihydrochloride (WO2000/037658) and 9.33 ml of triethylamine in 200 ml of dichloromethane was cooled with an ice water bath, and 9.33 ml of 2-bromoisobutyryl bromide were added dropwise. The reaction mixture was allowed to stir while warming to room temperature over 3 hours. The solution was then washed with 3 x 50 ml of IN HCl, 3 x 50 ml of saturated sodium bicarbonate, and 50 ml of saturated sodium chloride. The solution was then dried over anhydrous magnesium sulfate, filtered and concentrated to give 4.67 grams of the desired product as a white solid. This material could be further purified by silica gel chromatography with 30-50% ethyl acetate in hexane. ¹H NMR (400 MHz, DMSO-d₆): δ = 1.91 (s, 18H, CH₃), 3.05 (d, 6H, *J*=6.4 Hz, CH₂N), 3.21 (d, 2H, *J*=4.4 Hz, CH₂OH), 8.17 (t, *J*=6.4 Hz, 3H, NH).

### Example 88. Preparation of N-t-Butytoxycarbonyl-2,2-[bis(2-bromo-2-methylpropionyloxy)methyl]-O-(2-bromo-2-methylpropionyl)-ethanolamine

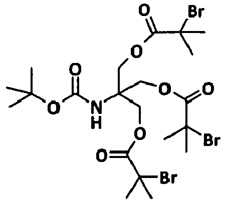

A solution 3.50 grams of N-Boc tris(hydroxymethyl)aminomethane (J. Fluorine Chem. 2007, 128, 179) in 100 mL of dichloromethane, together with 11 mL (5 eq) of triethylamine was cooled with an ice-water bath, and 6.2 mL (3.2 eq) of 2-bromoisobutyryl bromide were added dropwise. The reaction was stirred in the cold for 3 hours, then examined by tlc (silica gel, 30% ethyl acetate in hexane). The reaction was not yet complete, so another 3 grams of 2-bromoisobutyryl bromide were added dropwise. After stirring for another hour, the reaction was filtered and the precipitate was washed with a small amount of dichloromethane. The combined organics were washed with 50 mL of saturated sodium bicarbonate, then dried over sodium sulfate. Filtration and concentration gave a residue, which was subjected to flash chromatography on silica gel with 10-30% ethyl acetate in hexane. The product containing fractions were concentrated to a volume of about 50 mL, and another 200 mL of hexane was then added with cooling and stirring. Over about 2 hours, much solid product crystallized from the mixture. This was recovered by filtration and air-dried to give 7.1 grams (67%) of the desired product as a white crystalline solid. ¹H NMR (400 MHz, CDCl₃): δ = 1.43 (s, 9H, Boc), 1.95 (s, 18H, (CH₃)₂CBr), 4.54 (s, 6H, CH₂O), 4.8 (br s, 1H, NH).

### Example 89. Preparation of 2,2-[Bis(2-bromo-2-methylpropionyloxy)methyl]-O-(2-bromo-2-methylpropionyl)ethanolamine trifluoroacetate

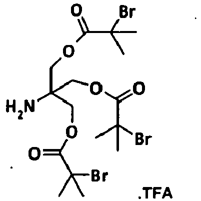

A solution of 6.0 grams ofN-t-butyloxycarbonyl-2,2-[bis(2-bromo-2-methylpropionyloxy) methyl]-O-(2-bromo-2-methylpropionyl)-ethanolamine in 40 ml of dichloromethane was treated with 10 ml of trifluoroacetic acid and the reaction was stirred at room temperature for 1 hr. The reaction was then concentrated and 20 ml of hexane were added. The mixture was again concentrated, then placed under high vacuum to give 6.14 grams of the desired product as a white solid.

### Example 90. Preparartion of 2,2-[Bis(2-bromo-2-methylpropionyloxy)methyl]-O-(2-bromo-2-methylpropionyl)ethanolamine half amide with digylcolic anhydride

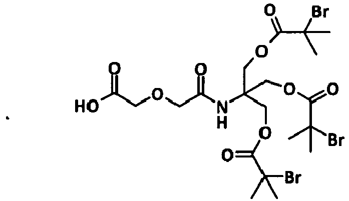

A mixture of 5.03 grams of 2,2-[bis(2-bromo-2-methylpropionyloxy)methyl]-O-(2-bromo-2-methylpropionyl)ethanolamine trifluoroacetate in 50 ml of acetonitrile was treated with 2.0 ml (2 eq) of triethylamine, whereupon the reaction immediately became homogeneous. A 50 mg portion of DMAP was added, followed by 860 mg (1 eq) of diglycolic anhydride, and the reaction was stirred at room temperature for 3 hr. The reaction was then concentrated and the residue was dissolved in 100 ml of dichloromethane, and washed with 2 x 50 ml of IN HCl, followed by 50 ml of saturated sodium chloride. The organics were dried over sodium sulfate, filtered and concentrated to give a residue, which was subjected to flash chromatography on silica gel with 50% ethyl acetate in hexane. The appropriate fractions were combined and concentrated to give the desired product.

### Example 91. Preparation of 2,2-[Bis(2-bromo-2-methylpropionyloxy)methyl]-O-(2-bromo-2-methylpropionyl)ethanolamine half amide with digylcolic anhydride, NHS ester

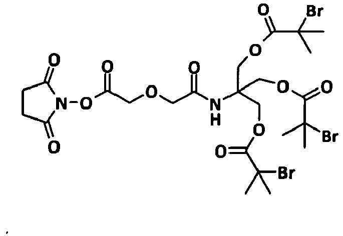

A solution of 2.5 grams of 2,2-[bis(2-bromo-2-methylpropionyloxy)methyl]-O-(2-bromo-2-methylpropionyl)ethanolamine, half amide with digylcolic anhydride in 30 ml of anhydrous acetonitrile, together with 500 mg of N-hydroxysuccinimide and 85 mg of DPTS, was treated with 900 mg of DCC and the reaction was stirred at room temperature overnight. The mixture was then filtered and the filtrate was concentrated to give a residue, which was subjected to flash chromatography on silica gel with 50% ethyl acetate in hexane. The appropriate fractions were combined and concentrated to give the desired product.

### Example 92. Preparation of 2,5,8,11,14-Pentaoxaheptadec-16-enyl 9-arm diglycolic acid-based initiator

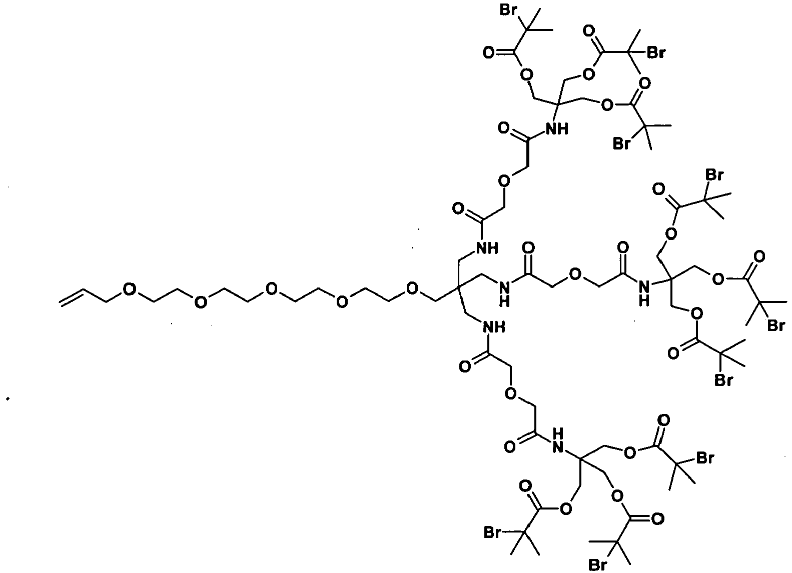

A solution of 1-amino-15-allyloxy-2,2-bis(aminomethyl)-4,7,10,13-tetraoxapentadecane trihydrochloride in acetonitrile, together with 6 eq of triethylamine, was reacted with a solution of 2,2-[bis(2-bromo-2-methylpropionyloxy)methyl]-O-(2-bromo-2-methylpropionyl)ethanolamine half amide with digylcolic anhydride, NHS ester and the reaction was stirred at room temperature overnight. The reaction mixture was then concentrated and the residue was dissolved in 100 ml of dichloromethane, and washed with 2 x 50 ml of IN HCl, followed by 50 ml of saturated sodium chloride. The organics were dried over sodium sulfate, filtered and concentrated to give a residue, which was subjected to flash chromatography on silica gel with ethyl acetate in hexane. The appropriate fractions were combined and concentrated to give the desired product.

### Example 93. Preparation of 2-Methyl-2-hydroxymethyl-4,7,10,13,16-pentaoxa-18-enylnonadecanol

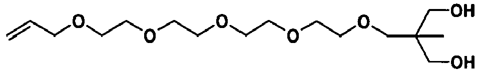

A solution of 3.6 grams of 5-hydroxymethyl-2,2,5-trimethyl-1,3-dioxane in 100 ml of anhydrous THF was cooled with an ice water bath and treated with 2.7 grams of NaH (60% in oil). After the bubbling subsided, 7.0 grams of 3,6,9,12-tetraoxapentadec-14-en-1-ol methanesulfonate were added and the reaction was stirred at 70 °C for 2 hours. The reaction was cooled, 3 ml of water were added carefully, and the reaction mixture was partitioned between 100 ml of water and 100 ml of ether. The aqueous layer was extracted with another 2 x 50 ml of ether and the combined organics were dried over sodium sulfate. Filtration and concentration gave a yellow oil, which was subjected to flash chromatography on silica gel with 10-15% acetone in hexane to give 5.62 grams of the desired acetonide product as a clear oil. A 4.84 gram portion of this oil was taken up in 50 mL of methanol and treated with 1.0 grams of Dowex 50Wx8 resin (H+ form) and the reaction was stirred at room temperature overnight. The mixture was then filtered and the filtrated concentrated to give 4.30 grams of the desired product as a clear, nearly colorless oil.

### Example 94. Preparation of 2-Methyl-2-hydroxymethyl-4,7,10,13,16-pentaoxa-18-enylnonadecanol, mono ester with bis 2,2-[(2-bromoisobutyryl)hydroxymethyl] propionic acid

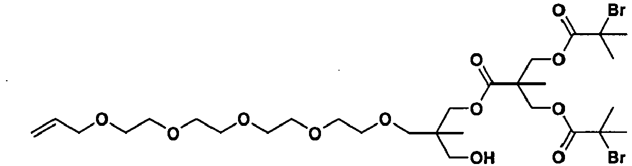

A sample of 2-methyl-2-hydroxymethyl-4,7,10,13,16-pentaoxa-18-enylnonadecanol in anhydrous acetonitrile was treated with 1 eq of bis 2,2-[(2-bromoisobutyryl)hydroxymethyl] propionic acid, a catalytic amount of DPTS and 1.2 eq of DCC and the reaction was stirred at room temperature. Filtration and concentration gave an oil, which was purified by flash chromatography on silica gel with ethyl acetate in hexane to give the desired compound.

### Example 95. Preparation of 2,5,8,11,14-Pentaoxaheptadec-16-enyl5-arm hybrid initiator

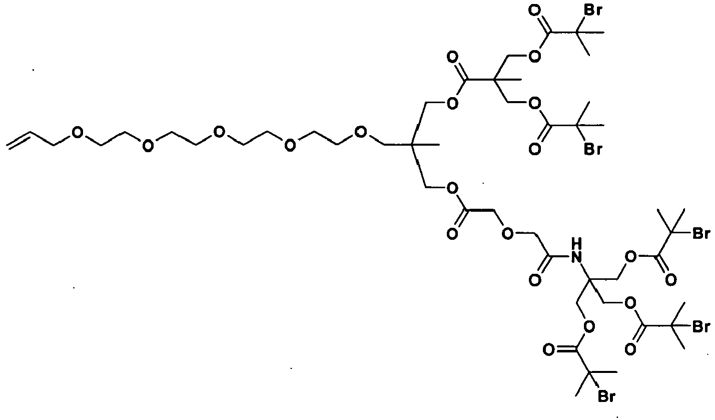

A solution of 2-methyl-2-hydroxymethyl-4,7,10,13,16-pentaoxa-18-enylnonadecanol, mono ester with bis 2,2-[(2-bromoisobutyryl)hydroxymethyl]propionic acid in anhydrous acetonitrile was treated with 1 eq of 2,2-[bis(2-bromo-2-methylpropionyloxy)methyl]-O-(2-bromo-2-methylpropionyl)ethanolamine half amide with digylcolic anhydride, a catalytic amount of DPTS and 1.2 eq of DCC and the reaction was stirred at room temperature. Filtration and concentration gave an oil, which was purified by flash chromatography on silica gel with ethyl acetate in hexane to give the desired 5-arm initiator.

### Example 96. Preparation of protected maleimide 8-arm initiator

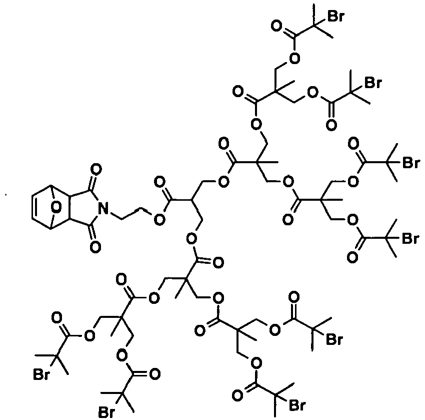

A round-bottomed flask equipped with stirbar was charged with the protected maleimide tetraol (1 mmol, 543 mg), bis(bromo) acid (4.5 mmol, 1.94 g), dimethylaminopyridine (3.6 mmol, 440 mg), 4-dimethylaminopyridinium 4-toluenesulfonate (0.9 mmol, 265 mg), and dichloromethane (20 ml). Nitrogen was bubbled through the stirring mixture for 10 minutes, then N,N-dicyclohexylcarbodiimide (5.85 mmol, 1.21 g) was added. After stirring overnight at room temperature, the mixture was filtered, concentrated, and subjected to silica gel flash chromatography with 40% ethyl acetate in hexane.

### Example 97. Preparation of protected maleimide 12-arm initiator

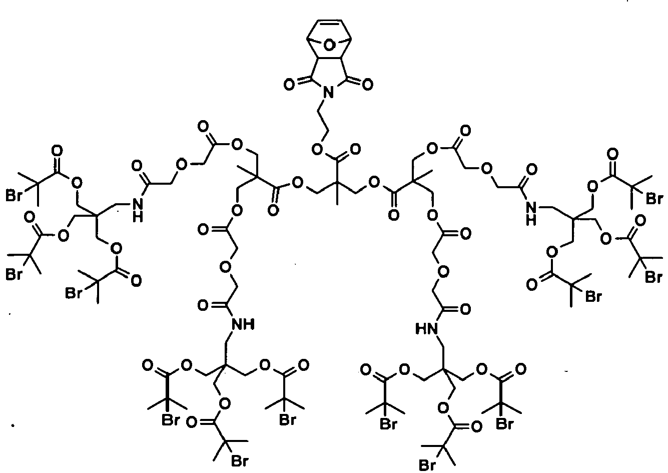

A round-bottomed flask equipped with stirbar was charged with the protected maleimide tetraol (1 mmol, 543 mg), 3-arm half amide acid (4.5 mmol, 3.14 g), dimethylaminopyridine (3.6 mmol, 440 mg), 4-dimethylaminopyridinium 4-toluenesulfonate (0.9 mmol, 265 mg), and dichloromethane (20 ml). Nitrogen was bubbled through the stirring mixture for 10 minutes, then N,N-dicyclohexylcarbodiimide (5.85 mmol, 1.21 g) was added. After stirring overnight at room temperature, the mixture was filtered, concentrated, and subjected to silica gel flash chromatography with 40% ethyl acetate in hexane.

### Example 98. Preparation of high molecular weight zwitterionic polymers

An example 2-arm polymer synthesized using the NHSM2 initiator

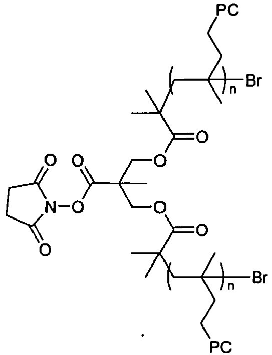

A representative protocol to produce high molecular weight, tailor-made hydrophilic polymers of the zwitterionic monomer, 2-methacryloyloxyethyl phosphorylcholine (HEMA-PC), using a "living" controlled free radical process, atom transfer radical polymerization (ATRP), is as follows.

The following initiators were used:
PMC2M1 (from Example 4) 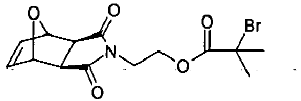
PMC2M2 (from Example 5) 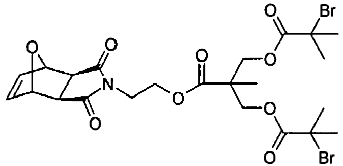
PMC2EOM2 (from Example 8) 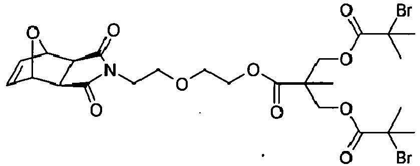
PMC2M4 (from Example 14) 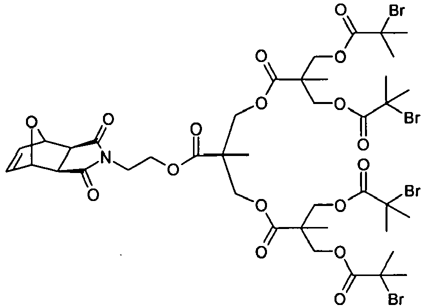
PMC2EOM4 (from Example 32) 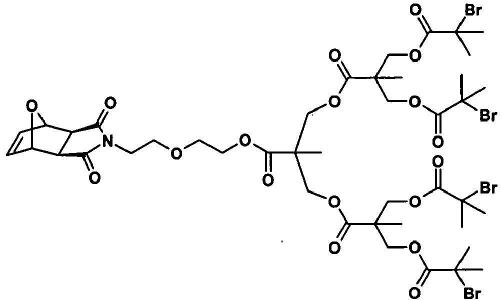
NHSM2 (from Example 25) 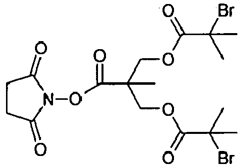
NHSEO4M2 (from Example 38) 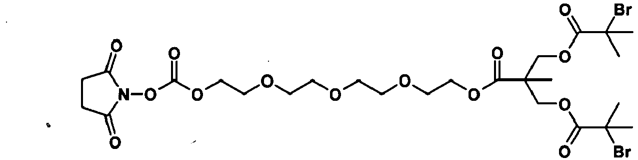
N3C2EOM2 (from Example 49) 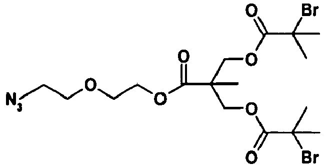
N3EOM6 (from Example 74) 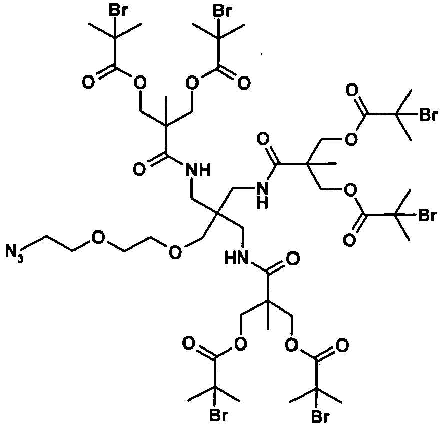
AlC2M2 (from Example 24) 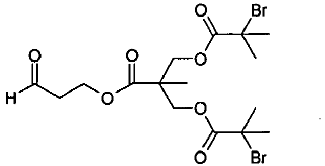
BAlM2 (from Example 50) 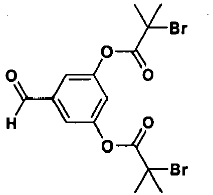
AcC2M2 (from Example 9) 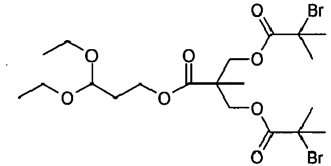
DC1M2 (from Example 40) 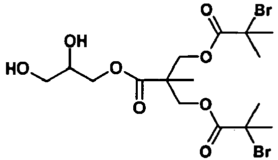
DC1EOM2 (from Example 41) 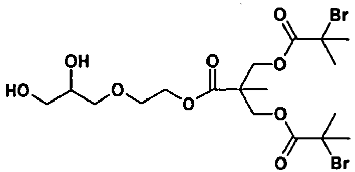
DC1EO4M2 (from Example 43) 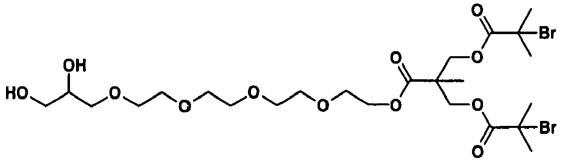
DC1EOM4 (from Example 48) 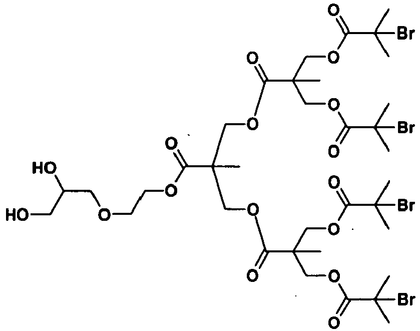
DC1EO4M6 (from Example 59) 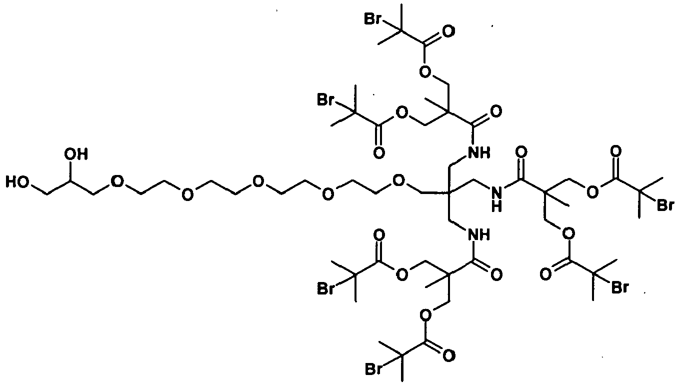
AKC1EO4M2 (from Example 64) 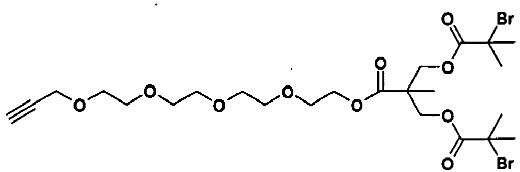
AEC1EO4M9 (from Example 92)

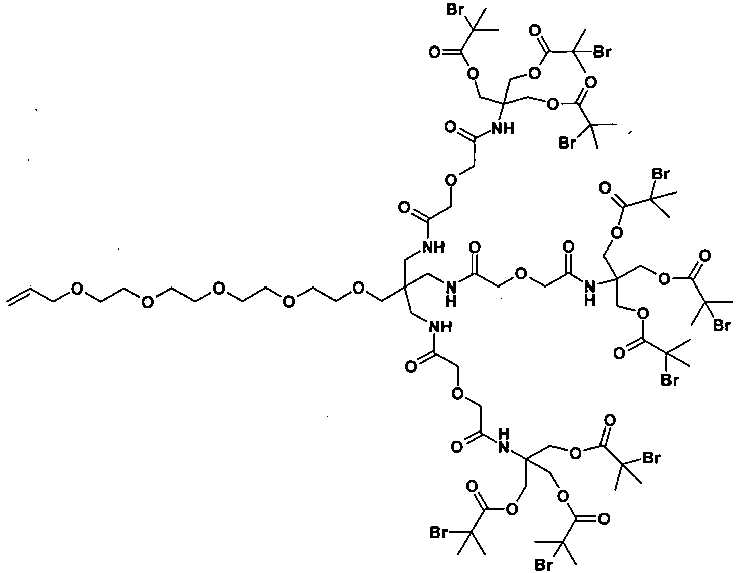

The initiator and ligand (2,2'-bipyridyl unless otherwise indicated) were introduced into a Schlenk tube. Dimethyl formamide or dimethylsulfoxide was introduced drop wise so that the total weight percent of both initiator and ligand did not exceed 20%. In the event that initiators or ligands were oils, or the quantities involved were below the accuracy limit of the balance, the reagents were introduced as solutions in dimethyl formamide (100 mg/ml). The resultant solution was cooled to -78°C using a dry ice/acetone mixture, and was degassed under vacuum until no further bubbling was seen. The mixture remained homogeneous at this temperature. The tube was refilled under nitrogen and the catalyst (CuBr unless otherwise indicated), kept under nitrogen, was introduced into the Schlenck tube. The solution became dark brown immediately. The Schlenk tube was sealed and kept at -78°C and the solution was purged immediately by applying a vacuum. Care was taken to ensure that the monomer, HEMA-PC, was kept as a dry solid under inert conditions at all times until ready for use. A solution of HEMA-PC was freshly prepared by mixing a defined quantity of monomer, under nitrogen, with 200proof degassed ethanol. The monomer solution was added drop wise into the Schlenk tube and homogenized by light stirring. Unless otherwise indicated, the ratio of monomer (g)/ethanol (ml) was 0.255. The temperature was maintained at -78°C. A thorough vacuum was applied to the reaction mixture for at least 10 to 15 min. until bubbling from the solution ceased. The mixture stayed homogeneous at this temperature, i.e. with no precipitation of any reaction ingredients (such as initiator or ligand) thus avoiding premature or unwanted polymerization. The tube was refilled with nitrogen, and the vacuum-nitrogen cycle was repeated twice. The tube was then refilled with nitrogen and warmed to room temperature (25°C). As the polymerization proceeded, the solution became viscous. After some time (defined in the table below), the reaction was quenched by direct exposure to air causing the mixture to become blue-green in color, and was passed through a silica column in order to remove the copper catalyst. The collected solution was concentrated by rotary evaporation and the resulting mixture was purified by careful precipitation into tetrahydrofuran followed by thorough washing with diethyl ether, or by dialysis against water. Polymer was collected as a white fluffy powder (following freeze drying if dialyzed against water) and placed under vacuum at room temperature.

Data from several polymerization reactions are shown in the following table.

| Sample | Initiator | Initiator (10⁻⁵ mol) | Monomer (g) | Catalyst (10⁻⁵ mol) | Ligand (10⁻⁵ mol) | Time (h) | MALS (Mn kDa) | MALS (Mp kDa) | MALS (PDI) | Monomer Conversion (¹HNMR %) |
|---|---|---|---|---|---|---|---|---|---|---|
| Maleimide (protected maleimide precursor) series | | | | | | | | | | |
| 1 | PMC2M2 | 2.05 | 2.046 | 4.08 | 8.20 | 8 | 103 | 121 | 1.15 | 95 |
| 2 | PMC2M2 | 1.35 | 2.028 | 2.70 | 5.40 | 8 | 158 | 183.2 | 1.15 | 93 |
| 3 | PMC2M2 | 2.48 | 2.486 | 4.97 | 9.90 | 8 | 119.1 | 135 | 1.15 | 97 |
| 4 | PMC2M1 | 2.03 | 1.529 | 2.03 | 4.07 | 8 | 91.6 | 93.3 | 1.15 | 98 |
| 5 | PMC2M2 | 2.00 | 3.993 | 3.99. | 7.97 | 7½ | 175.2 | 202.8 | 1.15 | 96 |
| 6 | PMC2M2 | 0.33 | 1.000 | 0.69 | 1.32 | 6½ | 196.2 | 240.7 | 1.2 | 85 |
| 7 | PMC2M2 | 0.55 | 2.065 | 1.10 | 2.20 | 21 | 289.9 | 351.2 | 1.25 | 90 |
| 8 | PMC2M2 | 0.26 | 2.095 | 0.52 | 1.04 | 20½ | 348.6 | 415.9 | 1.25 | 50 |
| 9³ | PMC2M2 | 2.82 | 2.829 | 5.65 | 11.3 | 8 | 110.2 | 123.3 | 1.1 | 95 |
| 10³ | PMC2M2 | 1.33 | 4.529 | 2.66 | 5.32 | 19 | 317.3 | 372.9 | 1.15 | 98 |
| 11 | PMC2M2 | 1.33 | 4.012 | 2.66 | 5.32 | 15½ | 270.3 | 314 | 1.15 | 96 |
| 12³ | PMC2M2 | 0.49 | 3.026 | 0.97 | 1.94 | 16 | 414.3 | 517.8 | 1.25 | 70 |
| 13⁴ | PMC2M2 | 0.80 | 6.016 | 1.60 | 3.20 | 24 | 531.3 | 692 | 1.3 | 94 |
| 14 | PMC2M2 | 1.50 | 4.280 | 2.98 | 5.96 | 20 | 248.7 | 296.6 | 1.2 | 90 |
| 15 | PMC2M2 | 1.00 | 1.000 | 1.99 | 3.99 | 8 | 99.3 | 117.2 | 1.15 | 94 |
| 16 | PMC2M1 | 2.00 | 2.000 | 2.00 | 3.99 | 17 | 122.5 | 145.8 | 1.15 | 97 |
| 17 | PMC2M2 | 2.89 | 2.893 | 5.77 | 11.5 | 8 | 99.47 | 117.6 | 1.15 | 97 |
| 18 | PMC2M1 | 1.01 | 2.023 | 1.00 | 2.01 | 26½ | 190.7 | 253 | 1.3 | 90 |
| 19 | PMC2M2 | 2.98 | 1.493 | 5.96 | 11.9 | 5 | 76.8 | 76.1 | 1.1 | 96 |
| 20 | PMC2M4 | 1.48 | 1.494 | 5.94 | 11.9 | 5 | 128.5 | 130.9 | 1.1 | 94 |
| 21 | PMC2M2 | 2.05 | 2.058 | 4.10 | 8.22 | 8 | 128.8 | 136.3 | 1.1 | 94 |
| 22 | PMC2M2 | 0.76 | 2.202 | 1.53 | 3.06 | 20 | 292.1 | 328.8 | 1.15 | 95 |
| 23³ | PMC2M4 | 1.04 | 2.094 | 4.16 | 8.34 | 6 | 198.5 | 214.9 | 1.1 | 91 |
| 24 | PMC2EOM2 | 1.07 | 1.076 | 2.14 | 4.29 | 7 | 148 | 155.4 | 1.1 | 93 |
| 25 | PMC2M2 | 1.46 | 1.468 | 2.92 | 5.85 | 8 | 148 | 157.4 | 1.1 | 93 |
| 26¹ | PMC2M2 | 1.70 | 1.704 | 3.39 | 6.8 | 8 | 94.8 | 100.7 | 1.1 | 98 |
| 27 | PMC2M2 | 0.90 | 0.897 | 1.78 | 3.58 | 8 | 104.5 | 115.2 | 1.1 | 93 |
| 28 | PMC2M2 | 1.06 | 1.060 | 2.16 | 4.23 | 8 | 53.4 | 55.4 | 1.1 | 85 |
| 29 | PMC2M4 | 0.26 | 2.180 | 1.03 | 2.04 | 41 | 300 | 340 | 1.1 | 35 |
| 30² | PMC2M2 | 0.99 | 0.994 | 1.98 | 3.96 | 8 | 520 | 830 | 1.7 | 70 |
| 31⁴ | PMC2M2 | 0.40 | 2.370 | 0.78 | 1.58 | 39 | 350 | 429 | 1.15 | 56 |
| 32 | PMC2M4 | 0.50 | 2.020 | 2.01 | 4.03 | 18 | 402 | 445 | 1.15 | 98 |
| 33⁴ | PMC2M4 | 0.37 | 2.203 | 1.46 | 2.93 | 38 | 550 | 640 | 1.15 | 96 |
| 34⁴ | PMC2M4 | 0.38 | 2.256 | 1.49 | 3.00 | 38 | 625 | 670 | 1.15 | 98 |
| 35⁴ | PMC2M4 | 0.54 | 2.181 | 2.17 | 4.35 | 16 | 400 | 465 | 1.2 | 98 |
| 36⁴ | PMC2M4 | 0.67 | 2.336 | 2.66 | 5.33 | 16 | 404 | 445 | 1.15 | 98 |

| Aldehyde series | | | | | | | | | | |
|---|---|---|---|---|---|---|---|---|---|---|
| 37 | AlC2M2 | 1.00 | 1.500 | 1.99 | 3.99 | 6½ | 117.3 | 145 | 1.2 | 90 |
| 38 | A1C2M2 | 10.0 | 1.000 | 20.0 | 39.9 | 2 | 18.99 | 19.54 | 1.1 | 95 |
| 39 | A1C2M2 | 10.0 | 1.000 | 20.0 | 39.9 | 2 | 18.64 | 18.96 | 1.1 | >99 |
| 40 | AlC2M2 | 1.00 | 1.000 | 2.00 | 3.99 | 4½ | 132.3 | 157 | 1.15 | >99 |
| 41 | A1C2M2 | 2.17 | 1.300 | 4.35 | 8.70 | 7 | 52.32 | 58.57 | 1.15 | 99 |
| 42 | AlC2M2 | 1.51 | 1.517 | 3.02 | 6.05 | 7½ | 89.43 | 104.7 | 1.1 | 96 |
| 43 | AlC2M2 | 1.90 | 1.142 | 3.80 | 7.61 | 7 | 78 | 81.1 | 1.1 | 97 |
| 44 | AlC2M2 | 4.17 | 1.045 | 8.36 | 16.7 | 15 | 33.3 | 36.2 | 1.1 | >99 |
| 45 | BAlM2 | 20.0 | 1.000 | 40.0 | 80.0 | 1½ | 10.32 | 10.2 | 1.1 | >99 |
| 46 | BA1M2 | 2.17 | 1.300 | 4.35 | 8.70 | 8 | 60 | 62.0 | 1.1 | 98 |
| 47 | BA1M2 | 1.51 | 1.517 | 3.02 | 6.05 | 8 | 94 | 98.9 | 1.1 | 91 |
| 48 | BA1M2 | 1.89 | 1.133 | 3.79 | 7.58 | 7 | 86.2 | 82.4 | 1.1 | 95 |
| 49 | BA1M2 | 4.13 | 1.035 | 8.27 | 16.5 | 15 | 32.9 | 30.7 | 1.1 | >99 |

| NHS Series | | | | | | | | | | |
|---|---|---|---|---|---|---|---|---|---|---|
| 50 | NHSM2 | 0.50 | 1.500 | 1.00 | 1.99 | 22 | 159.3 | 204 | 1.2 | 93 |
| 51 | NHSM2 | 1.00 | 1.500 | 1.99 | 3.99 | 6½ | 117.7 | 144.7 | 1.15 | 85 |
| 52 | NHSM2 | 2.97 | 1.487 | 5.93 | 11.8 | 5 | 59.9 | 58 | 1.1 | 90 |
| 53 | NHSM2 | 0.50 | 1.000 | 1.00 | 1.99 | 21 | 160.3 | 186.6 | 1.1 | 80 |
| 54 | NHSE04M2 | 1.31 | 1.320 | 2.62 | 5.27 | 8 | 110 | 118 | 1.1 | 94 |

| Aldehyde (diol precursor) series | | | | | | | | | | |
|---|---|---|---|---|---|---|---|---|---|---|
| 55 | DClM2 | 1.75 | 1.049 | 3.50 | 7.01 | 7 | 89.5 | 87.7 | 1.1 | 95 |
| 56 | DC1EOM2 | 2.92 | 1.752 | 5.85 | 11.7 | 7 | 79.3 | 85.6 | 1.1 | 95 |
| 57 | DC1EOM2 | 0.73 | 1.467 | 1.46 | 2.92 | 7½ | 148.1 | 162.9 | 1.1 | 92 |
| 58 | DC1E04M2 | 1.55 | 1.550 | 3.10 | 6.20 | 8 | 112.9 | 121.8 | 1.1 | >99 |
| 59 | DC1EOM2 | 0.94 | 2.071 | 1.88 | 3.76 | 24 | 240 | 260 | 1.1 | - |
| 60 | DC1EOM2 | 0.38 | 3.050 | 0.76 | 1.51 | 23 | 330 | 390 | 1.2 | 70 |
| 61⁴ | DC1EOM2 | 1.03 | 2.07 | 2.06 | 4.12 | 19 | 135 | 155 | 1.1 | >99 |
| 62⁴ | DC1EOM2 | 0.34 | 2.096 | 0.69 | 1.34 | 24 | 244 | 300 | 1.2 | 56 |
| 63 | DC1EOM2 | 1.05 | 2.099 | 2.09 | 4.19 | 19 | 185 | 213 | 1.1 | 90 |
| 64 | DC1EOM2 | 0.98 | 2.052 | 1.95 | 3.9 | 19 | 230 | 258 | 1.1 | 94 |
| 65³ | DC1EOM2 | 0.38 | 3.074 | 0.76 | 1.53 | 23 | 420 | 498 | 1.2 | 91 |
| 66³ | DC1EOM2 | 0.396 | 1.970 | 0.78 | 1.57 | 22 | 330 | 380 | 1.15 | 63 |
| 67³ | DC1EOM2 | 0.38 | 2.146 | 0.76 | 1.52 | 21 | 435 | 510 | 1.15 | 82 |
| 68⁴ | DC1EOM4 | 0.54 | 2.173 | 2.16 | 4.33 | 18 | 435 | 470 | 1.1 | 98 |
| 69⁴ | DC1EOM4 | 0.26 | 1.584 | 1.05 | 2.10 | 20 | 580 | 660 | 1.15 | 96 |
| 70⁴ | DC1EOM4 | 0.59 | 2.126 | 2.35 | 4.71 | 18 | 405 | 433 | 1.15 | 99 |
| 71⁴ | DC1EOM4 | 0.40 | 2.168 | 1.60 | 3.20 | 20 | 516 | 570 | 1.15 | 96 |
| 72 | DC1EO4M2 | 0.41 | 2.033 | 0.80 | 1.46 | 116 | 337 | 378 | 1.15 | 80 |
| 73 | DC1EOM4 | 1.14 | 4.101 | 4.53 | 9.00 | 18 | 395 | 435 | 1.15 | 99 |
| 74 | DC1EOM4 | 0.75 | 4.066 | 3.00 | 5.99 | 20 | 533 | 617 | 1.2 | 97 |
| 75 | DC1EOM2 | 1.04 | 2.085 | 2.07 | 4.16 | 19 | 200 | 227 | 1.1 | 93 |
| 76 | DC1EO4M6 | 0.52 | 1.036 | 3.10 | 6.21 | 21 | 232.5 | 243.2 | 1.1 | 99 |
| 77 | DC1EO4M2 | 3.97 | 0.994 | 7.94 | 15.8 | 15½ | 34.4 | 36.4 | 1.1 | 99 |
| 78 | DC1EOM2 | 4.00 | 1.009 | 8.06 | 16.1 | 16 | 38 | 38.8 | 1.1 | >99 |
| 79 | DClM2 | 4.00 | 1.011 | 8.08 | 16.1 | 16 | 33.5 | 33.5 | 1.1 | 98 |
| 80 | DC1EO4M6 | 1.95 | 3.904 | 11.6 | 23.4 | 21 | 241 | 254 | 1.1 | 99 |
| 81 | DC1EO4M6 | 0.26 | 1.021 | 1.52 | 3.06 | 90 | 410.7 | 467.4 | 1.15 | >99 |
| 82 | DC1EO4M6 | 0.95 | 3.662 | 5.70 | 11.4 | 20 | 452 | 470 | 1.1 | 99 |
| 83 | DC1EO4M6 | 1.70 | 2.033 | 10.2 | 20.4 | 21 | 151 | 152 | 1.1 | >99 |

| Azido series | | | | | | | | | | |
|---|---|---|---|---|---|---|---|---|---|---|
| 84 | N3C2EOM2 | 4.21 | 1.055 | 8.43 | 16.8 | 15 | 35.8 | 35.9 | 1.1 | >99 |
| 85⁵ | N3EO2M6 | 0.78 | 1.947 | 4.67 | 9.34 | 15 | 336 | 336 | 1.12 | 99 |
| 86⁵ | N3EO2M6 | 1.00 | 1.997 | 6.30 | 12.6 | 16½ | 226.9 | 240.6 | 1.1 | >99 |
| 87 | N3EO2M6 | 1.73 | 2.000 | 10.1 | 20.1 | 21 | 149.4 | 156.9 | 1.1 | >99 |
| 88⁵ | N3EO2M6 | 0.56 | 2.006 | 3.30 | 6.50 | 20 | 477.4 | 480 | 1.2 | >99 |
| 89 | N3C2EOM2 | 2.05 | 2.049 | 4.09 | 8.18 | 7½ | 114.5 | 125.9 | 1.1 | 91 |

| Aldehyde (acetal precursor) series | | | | | | | | | | |
|---|---|---|---|---|---|---|---|---|---|---|
| 90 | AcC2M2 | 1.81 | 1.082 | 3.61 | 7.24 | 7 | 88.6 | 92.6 | 1.05 | 96 |

| Alkyne series | | | | | | | | | | |
|---|---|---|---|---|---|---|---|---|---|---|
| 91 | AKC1EO4M2 | 4.19 | 1.048 | 8.36 16.7 15 48.9 | | | | 50.8 | 1.06 | >99 |

| Alkene (thiol reactive) series | | | | | | | | | | |
|---|---|---|---|---|---|---|---|---|---|---|
| 92 | AEC1EO4M9 | 1.73 | 2.000 | 15.57 | 31.14 | 21 | 150 | 160 | 1.1 | >99 |
| 93 | AEC1EO4M9 | 0.56 | 2.006 | 5.04 | 10.08 | 20 | 470 | 480 | 1.2 | 99 |

| | | | | | | | | | | |
|---|---|---|---|---|---|---|---|---|---|---|
| Methanol/water solvent (75/25) v/v ²Ethanol/glycerol solvent (50/50) v/v ³Monomer (g)/solvent (ml) 0.33 ⁴Monomer (g)/solvent (ml) 0.40 ⁵Monomer (g)/solvent (ml) 0.50 | | | | | | | | | | |

The peak molecular weight (Mp), number molecular weight (Mn) and polydispersity (PDI) were determined/derived by multi-angle light scattering.

### Example 99. Further preparations of high molecular weight zwitterionic polymers

An example 3-arm polymer synthesized using the DC1EO4NM3 initiator

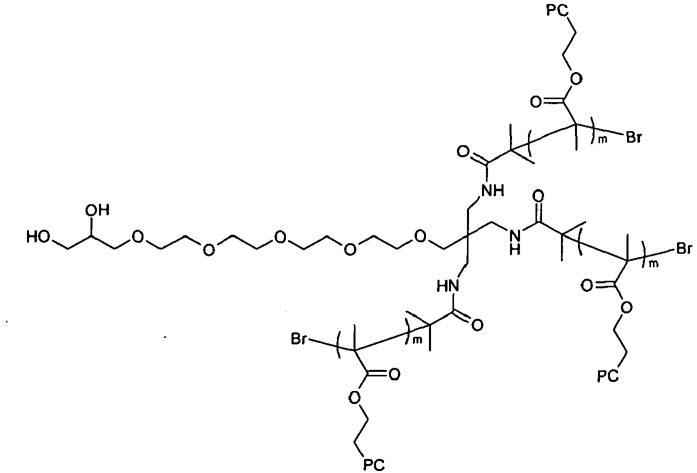

An alternative representative protocol to produce high molecular weight, tailor-made hydrophilic polymers of the zwitterionic monomer, 2-methacryloyloxyethyl phosphorylcholine (HEMA-PC), using a "living" controlled free radical process, atom transfer radical polymerization (ATRP), is as follows.

The following initiators were used:
HOC1NM3 (from Example 87) 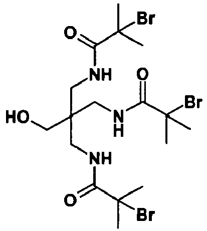
DC1EO4NM3 (from Example 54) 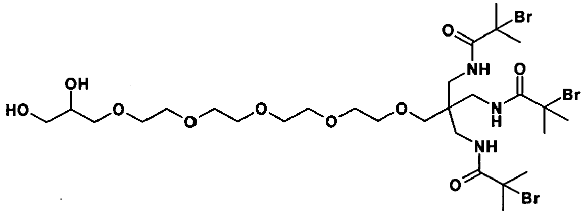
N3EO2NM3 (from Example 57)

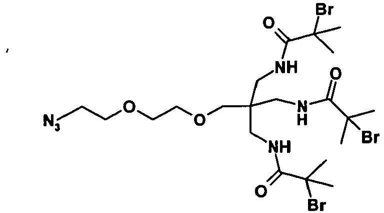

The initiator and ligand (2,2'-bipyridyl unless otherwise indicated) were introduced into a Schlenk tube. Dimethyl formamide or dimethylsulfoxide was introduced drop wise so that the total weight percent of both initiator and ligand did not exceed 20%. In the event that initiators or ligands were oils, or the quantities involved were below the accuracy limit of the balance, the reagents were introduced as solutions in dimethyl formamide (100 mg/ml). The resultant solution was cooled to -78°C using a dry ice/acetone mixture, and was degassed under vacuum until no further bubbling was seen. The mixture remained homogeneous at this temperature. The tube was refilled under nitrogen and the catalyst (CuBr unless otherwise indicated), kept under nitrogen, was introduced into the Schlenck tube. The solution became dark brown immediately. The Schlenk tube was sealed and kept at -78°C and the solution was purged immediately by applying a vacuum. Care was taken to ensure that the monomer, HEMA-PC, was kept as a dry solid under inert conditions at all times until ready for use. A solution of HEMA-PC was freshly prepared by mixing a defined quantity of monomer, kept under nitrogen, with 200proof degassed ethanol. A degassed solution of CuBr₂ in dimethyl formamide (100 mg/ml) was added to the solution of HEMA-PC under nitrogen in the ratio of halide/CuBr/CuBr₂ of 1/0.9/0.1 for reaction times up to 24 hours and 1/0.75/0.25 for reaction times longer than 24 hours. The resulting solution was added drop wise into the Schlenk tube and homogenized by light stirring. Unless otherwise indicated, the ratio of monomer (g)/ethanol (ml) was 0.50. The temperature was maintained at -78°C. A thorough vacuum was applied to the reaction mixture for at least 10 to 15 min. until bubbling from the solution ceased. The mixture stayed homogeneous at this temperature, i.e. with no precipitation of any reaction ingredients (such as initiator or ligand) thus avoiding premature or unwanted polymerization. The tube was refilled with nitrogen, and the vacuum-nitrogen cycle was repeated twice. The tube was then refilled with nitrogen and warmed to room temperature (25°C). As the polymerization proceeded, the solution became viscous. After some time (defined in the table below), the reaction was quenched by direct exposure to air causing the mixture to become blue-green in color, and was passed through a silica column in order to remove the copper catalyst. The collected solution was concentrated by rotary evaporation and the resulting mixture was purified by careful precipitation into tetrahydrofuran followed by thorough washing with diethyl ether, or by dialysis against water. Polymer was collected as a white fluffy powder (following freeze drying if dialyzed against water) and placed under vacuum at room temperature.

Data from several polymerization reactions are shown in the following table.

| Sample | Initiator | Initiator (10⁻⁵ mol) | Monomer (g) | CuBr (10⁻⁵ mol) | Ligand (10⁻⁵ mol) | Time (h) | MALS (Mn kDa) | MALS (Mp kDa) | MALS (PDI) | Monomer Conversion (¹HNMR %) |
|---|---|---|---|---|---|---|---|---|---|---|
| Aldehyde (diol precursor) series | | | | | | | | | | |
| 1 | DC1EO4NM3 | 0.32 | 1.931 | 0.49 | 1.92 | 137 | 366.7 | 432 | 1.15 | 57 |
| 2 | DC1EO4NM3 | 0.98 | 1.966 | 1.47 | 5.89 | 62 | 156 | 180 | 1.15 | 60 |
| 3 | DC1EO4NM3 | 0.34 | 2.065 | 0.77 | 2.06 | 63 | 547 | 624 | 1.15 | 95 |
| 4 | DC1EO4NM3 | 0.61 | 2.136 | 1.36 | 3.66 | 40 | 359 | 406 | 1.15 | 99 |
| 5 | DC1EO4NM3 | 0.99 | 1.975 | 2.21 | 5.91 | 50 | 292 | 329 | 1.15 | 96 |
| 6 | DC1EO4NM3 | 0.34 | 2.021 | 1.00 | 2.01 | 50 | 498 | 585 | 1.15 | 96 |
| 7 | DC1EO4NM3 | 1.15 | 4.040 | 2.57 | 6.92 | 40 | 331 | 367 | 1.15 | >99 |
| 8 | DC1EO4NM3 | 1.53 | 2.027 | 3.45 | 9.22 | 48 | 175.7 | 186 | 1.1 | 99 |
| 9¹ | DC1EO4NM3 | 1.17 | 2.072 | 3.17 | 7.05 | 24 | 254 | 274 | 1.1 | 99 |
| 10¹ | DC1EO4NM3 | 1.58 | 2.084 | 3.55 | 9.47 | 62 | 269.7 | 286.5 | 1.15 | 99 |
| 11¹ | DC1EO4NM3 | 1.16 | 4.088 | 2.60 | 6.99 | 62 | 434.8 | 511.1 | 1.2 | 97 |
| 12 | DC1EO4NM3 | 0.93 | 3.585 | 1.98 | 5.4 | 92 | 393 | 452 | 1.2 | 93 |
| 13¹ | DC1EO4NM3 | 1.44 | 3.619 | 6.04 | 8.64 | 48 | 265 | 322 | 1.2 | 81 |

| Hydroxyl series | | | | | | | | | | |
|---|---|---|---|---|---|---|---|---|---|---|
| 14 | HOC1NM3 | 0.28 | 1.122 | 0.42 | 1.67 | 20 | 134 | 140 | 1.15 | 25 |
| 15 | HOC1NM3 | 0.53 | 2.141 | 0.79 | 3.18 | 133 | 387 | 415 | 1.15 | 93 |
| 16 | HOC1NM3 | 0.40 | 1.123 | 0.89 | 2.39 | 20 | 124.9 | 127.1 | 1.15 | 40 |
| 17 | HOC1NM3 | 0.40 | 1.034 | 0.89 | 2.39 | 20 | 194.1 | 209 | 1.1 | 55 |
| 18 | HOC1NM3 | 0.40 | 1.021 | 1.08 | 2.39 | 20 | 279.3 | 312.7 | 1.2 | 95 |

| Azido series | | | | | | | | | | |
|---|---|---|---|---|---|---|---|---|---|---|
| 19 | N3EO2NM3 | 0.88 | 3.099 | 1.97 | 5.30 | 64 | 393.7 | 422.6 | 1.1 | 94 |
| 20 | N3EO2NM3 | 0.48 | 1.192 | 1.28 | 2.86 | 20 | 115 | 116 | 1.1 | 65 |
| 21 | N3EO2NM3 | 0.41 | 1.013 | 0.85 | 2.42 | 64 | 169.7 | 178 | 1.1 | 41 |
| 22 | N3EO2NM3 | 0.40 | 0.994 | . 1.07 | 2.38 | 64 | 323.8 | 374.5 | 1.18 | 94 |
| 23 | N3EO2NM3 | 0.79 | 1.989 | 1.78 | 4.76 | 46 | 56 | 52 | 1.1 | 12 |
| 24 | N3EO2NM3 | 0.82 | 2.048 | 2.20 | 4.90 | 46 | 146 | 154 | 1.15 | 37 |
| 25 | N3EO2NM3 | 0.80 | 2.006 | 2.16 | 4.80 | 22 | 324.9 | 349.7 | 1.15 | 91 |
| 26 | N3EO2NM3 | 0.80 | 1.994 | 2.15 | 4.77 | 46 | 342.1 | 379.2 | 1.15 | 99 |
| 27 | N3EO2NM3 | 0.80 | 2.007 | 2.45 | 4.80 | 15 | 315.1 | 379.5 | 1.25 | 90 |
| 28 | N3EO2NM3 | 0.80 | 2.002 | 2.15 | 4.79 | 22 | 333.7 | 358 | 1.11 | 94 |
| 29 | N3EO2NM3 | 0.80 | 2.002 | 2.30 | 4.80 | 22 | 323 | 360 | 1.15 | 95 |
| 30 | N3EO2NM3 | 1.09 | 2.029 | 2.95 | 6.57 | 22 | 277 | 292 | 1.15 | 99 |
| 31 | N3EO2NM3 | 1.00 | 2.005 | 2.70 | 6.01 | 22 | 286.5 | 306.3 | 1.1 | 97 |
| 32 | N3EO2NM3 | 1.23 | 2.045 | 3.53 | 7.42 | 22 | 242.4 | 267.6 | 1.15 | 94 |
| 33 | N3EO2NM3 | 1.24 | 1.926 | 3.54 | 7.45 | 22 | 233 | 289 | 1.25 | 99 |

| | | | | | | | | | | |
|---|---|---|---|---|---|---|---|---|---|---|
| ¹Monomer (g)/solvent (ml) 0.33 The ratio of halide/CuBr/CuBr₂ was 1/0.9/0.1 for reaction times up to 24 hours and 1/0.75/0.25 for reaction times longer than 24 hours | | | | | | | | | | |

The peak molecular weight (Mp), number molecular weight (Mn) and polydispersity (PDI) were determined/derived by multi-angle light scattering.

### Example 100. Preparation of high molecular weight PEG polymers

A representative protocol to produce high molecular weight, tailor-made hydrophilic polymers of the hydrophilic monomer, poly (ethylene glycol) methyl ether methacrylate, MW 475 (HEMA-PEG475), using a "living" controlled free radical process, atom transfer radical polymerization (ATRP), is essentially the same as the protocol outlined in Example 98 with the following differences. The monomer (HEMA-PEG 475 ) was dissolved in 200 proof and the solution degassed using the freeze-pump-thaw technique (3 cycles). The resulting degassed mixture was introduced under nitrogen at -78°C into a degassed solution of initiator, ligand and CuBr. The resulting mixture was degassed at -78°C, allowed to thaw, and placed under nitrogen at room temperature.

| Sample | Initiator | Initiator (10⁻⁵ mol) | Monomer (g) | CuBr (10⁻⁵ mol) | Ligand (10⁻⁵ mol) | Time (h) | MALS (Mn kDa) | MALS (Mp kDa) | MALS (PDI) | Monomer Conversion (¹HNMR %) |
|---|---|---|---|---|---|---|---|---|---|---|
| Aldehyde (diol precursor) series | | | | | | | | | | |
| 1 | DC1EO4M6 | 0.52 | 1.036 | 3.10 | 3.31 | 116 | 384.4 | 383.2 | 1.54² | 100 |
| 2¹ | DC1EO4M6 | 1.05 | 1.997 | 6.30 | 12.6 | 16½ | 192 | 190 | 1.1³ | 80 |
| 3¹ | DC1EO4M6 | 0.56 | 1.997 | 3.34 | 6.69 | 20 | 916 | 700 | 1.64² | 90 |
| 4 | DC1EO4M2 | 1.05 | 2.052 | 2.00 | 4.20 | 43 | 119 | 121.7 | 1.02³ | 53 |

| Azido series | | | | | | | | | | |
|---|---|---|---|---|---|---|---|---|---|---|
| 5¹ | N3EO2M6 | 1.05 | 1.997 | 6.30 | 12.6 | 16½ | 261.6 | 244.3 | 1.2³ | 95 |
| 6 | N3EO2M6 | 3.32 | 0.998 | 19.9 | 39.9 | 7 | 42.4 | 38 | 1.07³ | 91 |
| 7¹ | N3EO2M6 | 1.05 | 1.833 | 6.30 | 12.6 | 16½ | 231 | 211 | 1.28³ | >99 |

| | | | | | | | | | | |
|---|---|---|---|---|---|---|---|---|---|---|
| ¹Monomer(g)/solvent(ml) 0.50 ²Higher PDI due to heterogeneous polymerization due to freezing of mixture at -78°C ³Lower PDI due to addition of ethylene glycol cosolvent (prevents freezing at -78°C) | | | | | | | | | | |

### Example 101. Preparation of high molecular weight acrylamide polymers

A representative protocol to produce high molecular weight, tailor-made hydrophilic polymers of the hydrophilic monomers, N,N-dimethyl acrylamide (DMA), acrylamide (AM) or N-isopropylacrylamide (NIPAM), using a "living" controlled free radical process, atom transfer radical polymerization (ATRP), is essentially the same as the protocol outlined in Example 99 with the following differences. The ligand used was tris[2-dimethylamino)ethyl]amine (Me6TREN) and 3.3 mol×10⁻⁵ were added in Samples 1 and 2, and 1.5 mol×10⁻⁵ to all other Samples and the solvent was water. The ratio of halide/CuBr/CuBr₂/Me6TREN was 1/0.75/0.25/1 in each case. Following addition of the catalyst, the vessel was sealed and placed at 0°C. An aqueous solution of acrylamide derivative, DMA, AM or NIPAM, was degassed using the freeze-pump-thaw technique (3 cycles) and introduced in the Schlenk tube containing the initiator, the ligand and the catalysts via canula under nitrogen. The vessel was sealed and the reaction allowed to proceed at 4°C. After some time, the reaction was quenched by direct exposure to air. The blue-green reaction mixture was passed through a short plug of silica gel to remove the copper catalyst. The collected solution was concentrated by lyophilization.

| Sample | Initiator | Initiator (10⁻⁵ mol) | Monomer (g) | CuBr (10⁻⁵mol) | Ligand (10⁻⁵ mol) | Time (h) | MALS (Mn kDa) | MALS (Mp kDa) | MALS (PDI) | Monomer Conversion (¹HNMR %) |
|---|---|---|---|---|---|---|---|---|---|---|
| Aldehyde (diol precursor) series | | | | | | | | | | |
| 1 | DC1EO4M6 | 0.52 | 1.036¹ | 3.10 | 3.30 | 0 | 644.9 | 622 | 1.35 | 98 |
| 2 | DC1EO4M6 | 0.52 | 1.036² | 3.10 | 3.30 | 2 | 548.6 | 620.8 | 1.45 | 98 |

| Hydroxyl series | | | | | | | | | | |
|---|---|---|---|---|---|---|---|---|---|---|
| 3 | HOC1NM3 | 0.52 | 1.036² | 1.39 | 1.50 | 2 | 321.2 | 388.6 | 1.25 | 55 |
| 4 | HOC1NM3 | 0.52 | 1.036² | 1.24 | 1.50 | 2 | 294.5 | 340.7 | 1.2 | 60 |
| 5 | HOC1NM3 | 0.52 | 1.036¹ | 1.16 | 1.50 | 1 | 302.6 | 318.5 | 1.1 | 77 |
| 6 | HOC1NM3 | 0.52 | 1.036² | 1.16 | 1.50 | 2 | 186.7 | 211.2 | 1.15 | 50 |
| 7 | HOC1NM3 | 0.52 | 1.036³ | 1.16 | 1.50 | 6 | 300 | 320 | 1.2 | 81 |

| | | | | | | | | | | |
|---|---|---|---|---|---|---|---|---|---|---|
| ¹AM monomer ²DMA monomer ³NIPAM monomer The ratio of halide/CuBr/CuBr₂/Me6TREN was 1/0.75/0.25/1 in each case | | | | | | | | | | |

### Example 102. Generation of aldehyde functional groups from diol precursors following polymerization of diol functionalized initiators

A large excess of sodium periodate dissolved in distilled water was added to a solution of diol functionalized polymer in distilled water (10wt. %). The reaction was allowed to proceed at room temperature for 90 minutes in the dark.

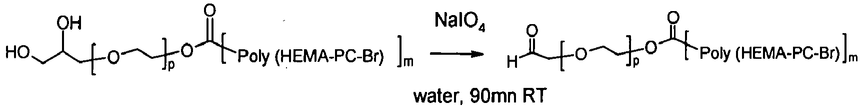

The reaction was quenched with an aqueous solution of glycerol (1.5X vs. NaIO₄) to remove any unreacted sodium periodate. The mixture was stirred at room temperature for 15 minutes and placed in a dialysis bag (MWCO 14 to 25 kDa) and purified by dialysis at room temperature for one day. Water was then removed by lyophilization and the polymer collected as a dry powder. Quantification of aldehyde functionality was by binding of Cy5.5 hydrazide fluorescent dye (GE Healthcare).

### Example 103. Attachment of N-propargyl maleimide and 5-hexyn-1-al to azido functionalized polymers

The following reagents were attached to azido functionalized polymers:
N-propargyl maleimide (from Example 62) 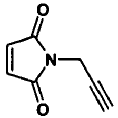
5-hexyn-1-al (from Example 63)

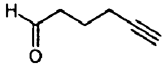

To a degassed solution of azido functionalized polymer in 200proof ethanol was added an excess of alkyne derivative (1.2 equivalents per azido group) followed by the ligand N,N,N',N",N"-pentamethyldiethylenetriamine (PMDETA) which was introduced as a stock solution in DMF (100mg/ml). The mixture was degassed by 3 vacuum/nitrogen cycles. Copper bromide (I) was added to the reaction mixture typically in a ratio of 0.2 to 1 vs. azido group. The ratio of CuBr/PMDETA was 1/1. The reaction was degassed again and stirred overnight at room temperature.

The following polymers were used (Samples 1, 2, 8, 9 and 10 from Example 98; Sample 4 from Example 100; and Samples 3, 5, 6 and 7 from Example 99):

| Sample | Polymer Initiator | Monomer | Alkyne | Mp (kDa) | PDI |
|---|---|---|---|---|---|
| 1 | N3C2EOM2 | HEMA-PC | 5-hexyn-1-al | 35.9 | 1.1 |
| 2 | N3C2EOM2 | HEMA-PC | 5-hexyn-1-al | 126 | 1.1 |
| 3 | N3EONM3 | HEMA-PC | 5-hexyn-1-al | 430 | 1.1 |
| 4 | N3EO2M6 | HEMA-PEG475 | 5-hexyn-1-al | 244 | 1.2 |
| 5 | N3EO2NM3 | HEMA-PC | N-propargyl maleimide | 154 | 1.1 |
| 6 | N3EO2NM3 | HEMA-PC | N-propargyl maleimide | 263 | 1.15 |
| 7 | N3EO2NM3 | HEMA-PC | N-propargyl maleimide | 422 | 1.1 |
| 8 | N3EO2M6 | HEMA-PC | N-propargyl maleimide | 150 | 1.15 |
| 9 | N3EO2M6 | HEMA-PC | N-propargyl maleimide | 242 | 1.15 |
| 10 | N3EO2M6 | HEMA-PC | N-propargyl maleimide | 465 | 1.2 |

### Example 104. Conjugation of recombinant human monoclonal Fab' to maleimide functionalized polymers

The following maleimide functionalized polymers (from Example 98 following deprotection according to Example 16) were used:

| | | **Polymer** | | **Conjugate** | |
|---|---|---|---|---|---|
| Sample | No. of Arms | Mp (kDa) | PDI | Mp (kDa) | PDI |
| 1 | 2 | 126.5 | 1.133 | 177 | 1.17 |
| 2 | 2 | 293 | 1.22 | 412 | 1.15 |
| 3 | 2 | 643 | 1.35 | 964 | 1.20 |
| 4 | 4 | 446 | 1.22 | 723 | 1.19 |
| 5 | 4 | 661 | 1.23 | 1079 | 1.16 |

Conjugation of recombinant human Fab' (molecular weight 50 kDa) was carried out in 10 mM sodium acetate at pH 5 containing 2mM EDTA with 10x molar excess of TCEP and 5-10 fold molar excess of maleimide functionalized polymer. The final Fab' concentration in the reaction mixture was 1-2mg/ml and the reaction was carried out in the dark at room temperature for 5 hrs followed by overnight at 4°C with gentle mixing using a rocking table. The resulting Fab'-polymer conjugates were purified using ion exchange chromatography on a MacroCap SP (MSP) column from GE Healthcare using 20mM Tris pH 7.4 as binding buffer. In general, the conjugation reaction (containing approx. 5 mg protein) was diluted 4 fold into binding buffer and loaded onto a 2 ml MSP column by gravity flow. The column was washed with at least 10 column volumes (CV) of binding buffer. Elution of conjugate was achieved by eluting the column with binding buffer containing 40-50mM NaCl for at least 10 CV. The fractions collected were concentrated with an Amicon Ultrafree concentrator with a 10 kDa MW cutoff membrane, and buffer exchanged into binding buffer containing 0.5M NaCl and further concentrated to a final protein concentration of at least 1mg/ml. The final conjugate was sterile filtered with a 0.22 micron filter and stored at 4°C before use. The final protein concentration was determined using OD280nm with a Fab' extinction coefficient of 1.46 (1mg/ml solution in a 10mm path length cuvette). The conjugate concentration was then calculated by including the MW of the polymer in addition to the Fab'.

MW of the conjugate was analyzed using a Shodex 806MHQ column with a Waters 2695 HPLC system equipped with a 2996 Photodiode Array Detector and a Wyatt miniDAWN Treos multi angle light scattering detector. The PDI and Mp were calculated using the ASTRA Software that was associated with the Wyatt MALS detector and the data are presented in the table above. In addition, in all cases the stoichiometry of the conjugates was shown to be 1 to 1 between Fab' and polymer.

### Example 105. Conjugation of recombinant human cytokine to aldehyde functionalized polymers

The following aldehyde functionalized polymers (from Examples 98 and 99 following oxidation according to Example 102 except where otherwise indicated) were used:

| | | **Polymer** | | **Conjugate** | |
|---|---|---|---|---|---|
| Sample | No. of Polymer Arms | Mp (kDa) | PDI | Mp (kDa) | PDI |
| 1¹ | 2 | 36 | 1.102 | NA | NA |
| 2¹ | 2 | 130 | 1.055 | NA | NA |
| 3² | 2 | 78 | 1.05 | 109 | 1.04 |
| 4³ | 2 | 84 | 1.05 | 113 | 1.04 |
| 5 | 2 | 220 | 1.11 | 260 | 1.05 |
| 6 | 2 | 357 | 1.16 | 389 | 1.05 |
| 7 | 3 | 160 | 1.15 | 194 | 1.12 |
| 8 | 3 | 274 | 1.16 | 298 | 1.12 |
| 9 | 4 | 434 | 1.12 | 392 | 1.18 |
| 10 | 4 | 606 | 1.18 | 503 | 1.10 |
| 11 | 6 | 152 | 1.06 | 173 | 1.08 |
| 12 | 6 | 249 | 1.08 | 255 | 1.07 |
| 13 | 6 | 456 | 1.1 | 422 | 1.10 |

| | | | | | |
|---|---|---|---|---|---|
| Polymers from Example 103 (aldehyde attached via click chemistry) ²DC1M2 initiator (i.e. no spacer) ³DC1EOM2 initiator (i.e. ethylene oxide spacer) | | | | | |

Conjugation of a 22 kDa recombinant human cytokine with a pI of 5.02 was performed in 10mM Hepes buffer at pH 7 containing 40mM sodium cyanoborohydride. The final protein concentration was 1-1.5mg/ml in the presence of 6-7 fold molar excess of polymer dissolved in the conjugation buffer. The reaction was carried out at room temperature or 4°C overnight in the dark with gentle mixing using a rocking table.

The conjugation efficiency was monitored using two methods: (i) a semiquantitative method using SDS-PAGE analysis and (ii) a quantitative method using analytical size exclusion chromatography (SEC) with a ProPac SEC-10 column, 4x300mm from Dionex Corporation.

Purification of the resulting cytokine-polymer conjugates was carried out using an anion exchange Q Sepharose HP (QHP) column from GE Healthcare. In general, the conjugation reaction (containing approx. 1 mg protein) was diluted at least 4 fold with QHP wash buffer containing 20 mM Tris pH 7.5 and loaded onto a 2ml QHP column by gravity flow. The column was washed with at least 10 column volumes (CV) of wash buffer. Elution of conjugate was achieved by eluting the column with wash buffer containing 40-50mM NaCl for at least 5 CV. The fractions collected were concentrated with an Amicon Ultrafree concentrator with a 10 kDa MW cutoff membrane, buffer exchanged into 1xPBS pH 7.4 and further concentrated to a final protein concentration of at least 1mg/ml. The final conjugates were sterile filtered with a 0.22 micron filter and stored at 4°C before use. The final protein concentration was determined using OD277nm with the cytokine extinction coefficient of 0.81 (1mg/ml solution in a 10mm pathlength cuvette). The conjugate concentration was then calculated by including the MW of the polymer in addition to the protein.

Characterization of the cytokine-polymer conjugates was performed with the following assays: (i) MW of the conjugate was analyzed using a Shodex 806MHQ column with a Waters 2695 HPLC system equipped with a 2996 Photodiode Array Detector and a Wyatt miniDAWN Treos multi angle light scattering detector. The PDI and Mp were calculated using the ASTRA Software that was associated with the Wyatt MALS detector and the data are presented in the table above. In addition, in all cases the stoichiometry of the conjugates was shown to be 1 to 1 between protein and polymer; (ii) SDS-PAGE analysis using Coomassie Blue stain. The presence of the high MW conjugate and the lack of free protein under both non-reducing and reducing conditions provided a good indication that the protein was covalently conjugated to the polymers. In addition, there was no sign of noncovalent association between the protein and the polymers nor the presence of intermolecular disulfide bond mediated protein aggregation in the purified protein-polymer conjugate preparations.

A very important difference was observed between Samples 3 and 4. Sample 3 was constructed from a polymer which was made using the DC1M2 initiator which has no spacer between the terminal functional group and the initiator core. Sample 4 was constructed from a polymer which was made using the DC1EOM2 initiator which has a single ethylene oxide spacer between the terminal functional group and the initiator core. Conjugation efficiency for Sample 4 was 5 times higher than for Sample 3 indicating the importance of spacer chemistry in influencing functional group reactivity.

### Example 106. Conjugation of recombinant human multi-domain protein to aldehyde functionalized polymers

The following aldehyde functionalized polymers (from Examples 98 and 99 following oxidation according to Example 102) were used:

| | | **Polymer** | | **Conjugate** | |
|---|---|---|---|---|---|
| Sample | No. of Arms | Mp (kDa) | PDI | Mp (kDa) | PDI |
| 1 | 3 | 278.3 | 1.154 | 313.6 | 1.083 |
| 2 | 6 | 240.2 | 1.059 | 261.2 | 1.065 |

Conjugation of a 21 kDa recombinant human multi-domain protein with a pI of 4.77 was performed in 10mM Hepes buffer at pH 7 containing 40mM sodium cyanoborohydride. The final protein concentration was 1-1.5mg/ml in the presence of 6-7 fold molar excess of polymer dissolved in the conjugation buffer. The reaction was carried out at room temperature or 4°C overnight in the dark with gentle mixing using a rocking table.

The conjugation efficiency was monitored using two methods: (i) a semiquantitative method using SDS-PAGE analysis and (ii) a quantitative method using analytical size exclusion chromatography (SEC) with a ProPac SEC-10 column, 4x300mm from Dionex Corporation.

Purification of the resulting protein-polymer conjugates was carried out using an anion exchange Q Sepharose HP (QHP) column from GE Healthcare. In general, the conjugation reaction (containing approx. 1 mg protein) was diluted at least 4 fold with QHP wash buffer containing 20 mM Tris pH 7.5 and loaded onto a 2ml QHP column by gravity flow. The column was washed with at least 10 column volumes (CV) of wash buffer. Elution of conjugate was achieved by eluting the column with wash buffer containing 40-50mM NaCl for at least 5 CV. The fractions collected were concentrated with an Amicon Ultrafree concentrator with a 10 kDa MW cutoff membrane, buffer exchanged into 1xPBS pH 7.4 and further concentrated to a final protein concentration of at least 1mg/ml. The final conjugates were sterile filtered with a 0.22 micron filter and stored at 4°C before use. The final protein concentration was determined using OD280nm with the domain protein extinction coefficient of 1.08 (1mg/ml solution in a 10mm pathlength cuvette). The conjugate concentration was then calculated by including the MW of the polymer in addition to the protein.

Characterization of the protein-polymer conjugates was performed with the following assays: (i) MW of the conjugate was analyzed using a Shodex 806MHQ column with a Waters 2695 HPLC system equipped with a 2996 Photodiode Array Detector and a Wyatt miniDAWN Treos multi angle light scattering detector. The PDI and Mp were calculated using the ASTRA Software that was associated with the Wyatt MALS detector and the data are presented in the table above. In addition, in all cases the stoichiometry of the conjugates was shown to be 1 to 1 between protein and polymer; (ii) SDS-PAGE analysis using Coomassie Blue stain. The presence of the high MW conjugate and the lack of free protein under both non-reducing and reducing conditions provided a good indication that the protein was covalently conjugated to the polymers. In addition, there was no sign of noncovalent association between the protein and the polymers nor the presence of intermolecular disulfide bond mediated protein aggregation in the purified protein-polymer conjugate preparations.

### Example 107. Conjugation of recombinant human cytokine and recombinant human multi-domain protein to aldehyde functionalized HEMA-PEG polymers

A 6-arm azido functionalized HEMA-PEG475 polymer with a molecular weight of 312.9 kDa was made according to the procedure in Example 100. The aldehyde functional group was introduced by attaching 5-hexyn-1-al to the azido functional group according to the procedure in Example 103. This polymer was conjugated to the 22 kDa recombinant cytokine and the 21 kDa recombinant human multi-domain protein generally according to the procedures in Examples 105 and 106 respectively with the following differences. Following overnight incubation at room temperature under inert conditions in the dark, the reactions were quenched by addition of 20 mM Tris pH 7.5, and the samples chromatographed using weak anion exchange chromatography (Shodex DEAE-825 column) using a Waters HPLC system equipped with a solvent delivering module capable of gradient formation and a UV detector for chromatogram trace detection. 15µl of each sample was applied to the column at a flow rate of 1ml/min followed by a 5 min isocratic wash in buffer A (20 mM Tris pH 7.4) followed by a linear gradient of 80% buffer (buffer A containing 0.5M NaCl) over a course of 9min. The salt gradient was maintained at 80% for 2 minutes before ramped down back to 100% buffer A for column regeneration. In the course of the chromatographic separation, protein peak fractions, detected by OD220nm, were manually collected for further analysis by SDS-PAGE . Three major peaks were collected. The first peak eluted at 1.8-3min during the initial isocratic wash, this fraction being equivalent to the unconjugated free polymer due to the fact that the polymer being charge neutral flowed through the column; the second peak was the weakly-bound conjugate fraction that eluted early in the salt gradient; and the last fraction which eluted later in the gradient corresponded to the unconjugated free protein. The 3 fractions were collected and concentrated with an Amicon Ultrafree 4 with 10K MWCO concentrator. The concentrated fractions were further analyzed with SDS-PAGE followed by Coomassie Blue stain, and by SEC-MALS as described in the previous referenced Examples, and the data is shown in the following table:

| | | **Polymer** | | **Conjugate** | | |
|---|---|---|---|---|---|---|
| Sample | No. of Arms | Mp (kDa) | PDI | Mp (kDa) | PDI | Protein Used |
| 1 | 6 | 312.9 | 1.396 | 337.2 | 1.256 | Cytokine |
| 2 | 6 | 312.9 | 1.396 | 334.4 | 1.289 | Domain Protein |

### Example 108. Preparation of N-2-Bromoisobutyryl-β-alanine t-butyl ester

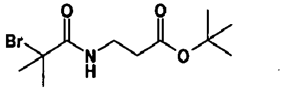

A mixture of 1.92 grams of t-butyl-β-alaninate hydrochloride in 25 ml of dichloromethane was cooled with an ice water bath, and 25 ml of IN NaOH were added, followed by 2.53 grams of 2-bromoisobutyryl bromide. The reaction was stirred in the cold for 15 minutes, then the layers were separated and the organics were dried over sodium sulfate. Filtration and concentration gave an oil, which was subjected to flash chromatography on silica gel with 40% ethyl acetate in hexane. The appropriate fractions were combined and concentrated to give 2.78 grams of the desired product as a clear oil. ¹H NMR (400 MHz, CDCl₃): δ = 1.47 (s, 9H, Boc), 1.94 (s, 6H, (CH₃)₂CBr), 2.48 (t, 6H, *J*=6, CH₂C=O), 3.50 (app q, 2H, *J*=6, CH₂NH).

### Example 109. Preparation of N-2-Bromoisobutyryl-β-alanine 2-(diphenylphosphino) phenyl ester

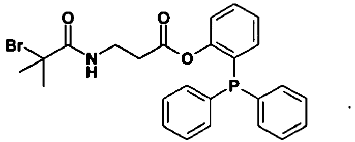

A solution of 2.78 grams of N-2-bromoisobutytyl-β-alanine t-butyl ester in 5 ml of formic acid was stirred at room temperature overnight. The reaction was then concentrated to give an oil, which was partitioned between 50 ml of ether and 50 ml of water. The organic layer was dried over sodium sulfate, filtered and concentrated to give 1.66 grams of a white solid. This solid was taken up in 20 mL of anhydrous acetonitrile, and 1.94 grams of (2-hydroxyphenyl)diphenylphosphine were added, followed by 200 mg of DPTS and 1.88 grams of DCC. The reaction was stirred at room temperature for 2 hours, at which time the reaction appeared to be complete by tlc (silica gel, 50% dichloromethane in hexane). The reaction was filtered and concentrated to give an oil, which was subjected to flash chromatography on silica gel with 10-20% acetone in hexane to give the desired product as a viscous oil. ¹H NMR (400 MHz, CDCl₃): δ = 1.95 (s, 6H, (CH₃)₂CBr), 2.55 (t, 2H, *J*=6, CH₂C=O), 3.44 (app q, 2H, *J*=6, CH₂NH), 6.85 (m, 1H, PhH), 7.15 (m, 2H, PhH), 7.25-7.42 (m, 12H, PhH).

Compounds of this type can be used to introduce functional groups in "traceless" Staudinger ligations (J. Am. Chem. Soc. 2006, 128, 8820) with azido polymers.

### Example 110. Preparation of 3-Maleimidopropionic acid, (2-diphenylphosphino)phenyl ester

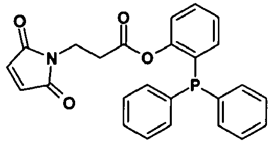

A solution of 3-maleimidopropionic acid (J. Am. Chem. Soc. 2005, 127, 2966), together with 1 eq of 2-(hydroxyphenyl)diphenylphosphine (Catalysis Today 1998, 42, 413) in anhydrous acetonitrile was treated with a catalytic amount of DPTS, followed by 1.2 eq of DCC and the reaction was stirred at room temperature until completion. The reaction was filtered and the filtrate was concentrated to give a residue, which was purified by flash chromatography on silica gel with ethyl acetate in hexane to give the desired product.

### Example 111. Preparation of 9-Hydroxy-4,7-dioxanonanoic acid, 2-(hydroxyphenyl) diphenylphosphino ester

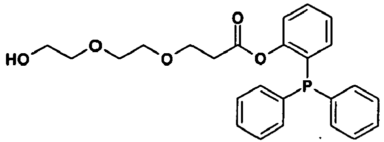

A solution of 9-t-butyldiphenylsilyloxy-4,7-dioxanonanoic acid, 2-(hydroxyphenyl)diphenylphosphino ester in THF was treated with tetrabutylammonium fluoride and the reaction was stirred at room temperature. Concentration gave a residue, which was partitioned between ethyl acetate and water. The organics were dried over sodium sulfate and used in the next reaction without further purification.

### Example 112. Preparation of 9-Oxo-4,7-dioxanonanoic acid, 2-(hydroxyphenyl) diphenylphosphino ester

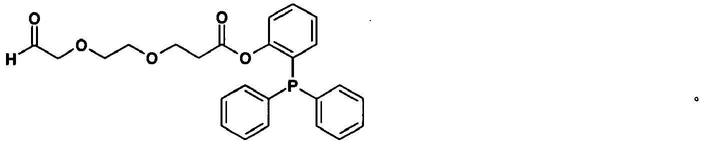

A sample of 9-hydroxy-4,7-dioxanonanoic acid, 2-(hydroxyphenyl) diphenylphosphino ester was oxidized with Dess-Martin periodinane to afford the corresponding aldehyde, which was purified by silica gel chromatography using ethyl acetate in hexane.

### Example 113. Preparation of N-Boc-β-alanine, 2-(hydroxyphenyl)diphenylphosphino ester

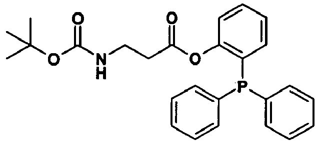

A solution of N-Boc- β-alanine in anhydrous acetonitrile, together with 1 eq of 2-(hydroxyphenyl)diphenylphosphine was treated with a catalytic amount of DPTS, followed by 1.2 eq of DCC and the reaction was stirred at room temperature until completion. The reaction was filtered and the filtrate was concentrated to give a residue, which was purified by flash chromatography on silica gel with ethyl acetate in hexane to give the desired product.

### Example 114. Preparation of N-Iodoacetyl-β-alanine, 2-(hydroxyphenyl) diphenylphosphino ester

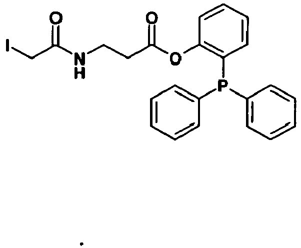

A solution of N-Boc-β-alanine, 2-(hydroxyphenyl)diphenylphosphino ester in dichloromethane was treated with trifluoroacetic acid, and upon completion the reaction was concentrated to give a residue, which was reconcentrated with hexane to remove as much of the TFA as possible. This residue was taken up in dichloromethane, treated with 6 eq of triethylamine, and iodoacetic anhydride was added. The reaction mixture was washed with water, dried over sodium sulfate, and concentrated to give a residue, which was subjected to flash chromatography with ethyl acetate in hexane to give the desired product.

### Example 115. Preparation of Pentanedioic acid, mono 2-(hydroxyphenyl) diphenylphosphino ester

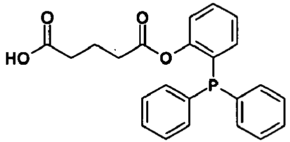

A solution of 2-(hydroxyphenyl)diphenylphosphine in dichloromethane was treated with 0.1 eq of DMAP and 2 eq of triethylamine, followed by 1.0 eq of glutaric anhydride. The reaction was heated at gentle reflux overnight, then washed with IN HCl and saturated sodium chloride, and dried over sodium sulfate. Filtration and concentration gave the crude acid, which was used in the next reaction without further purification.

### Example 116. Preparation of Pentanedioic acid, half 2-(hydroxyphenyl) diphenylphosphino ester, half N-hydroxysuccinimide ester

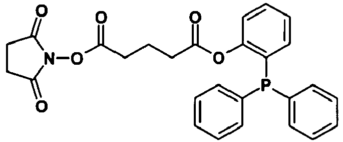

A solution of pentanedioic acid, mono 2-(hydroxyphenyl)diphenylphosphino ester in dry acetonitrile was treated with a catalytic amount of DPTS, followed by 1.2 eq of DCC. The reaction was filtered and concentrated to give a residue, which was subjected to flash chromatography with ethyl acetate in hexane to give the desired product.

### Example 117. Preparation of N-(3-Hydroxy-4-carbomethoxy)benzyl-bis 2,2-[(2-bromoisobutyryl) hydroxymethyl] propionamide

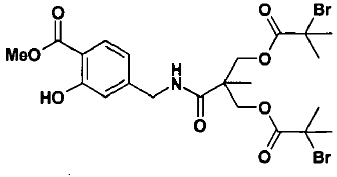

A sample of bis 2,2-[(2-bromoisobutyryloxy)methyl]propionic acid, N-hydroxysuccinimide ester was allowed to react with methyl 4-(aminomethyl)-2-hydroxybenzoate (US 6,156,884) in the presence of triethylamine, and the product was isolated by flash chromatography on silica gel with ethyl acetate in hexane.

### Example 118. Preparation of N-(3-Hydroxy-4-hydroxyaminocarbonyl)benzyl-bis 2,2-[(2-bromoisobutyryl)hydroxymethyl] propionamide

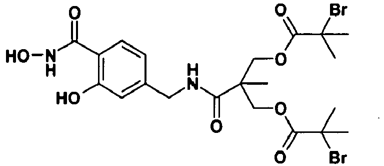

The product from the previous step was treated with hydroxylamine hydrochloride under basic conditions to afford the corresponding hydroxamic acid.

Polymers prepared using this initiator may be used in coupling reactions with phenylboronic acid -containing conjugation reagents such as 3-maleimidophenylboronic acid moieties (see US 6,156,884 and references therein each of which are incorporated in their entirety herein). Below is depicted the structure of the product formed from the conjugation reaction between the polymer from the hydroxamic acid-containing initiator and 3-maleimidophenylboronic acid. This polymer is now ready to conjugate with biomolecules containing a free thiol.

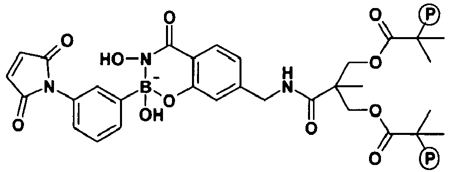

Essentially any functional group can be incorporated, and other example bioconjugation groups that can be employed in this strategy beside maleimide are bromoacetamide, iodoacetamide, hydrazide, carboxylic acid, dithiopyridyl, N-hydroxysuccinimidyl ester, imido ester, amino and thiol moieties (see table, US 6,156,884).

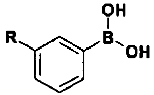

Where R=

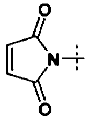

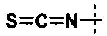

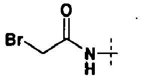

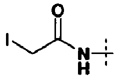

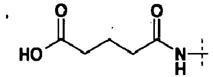

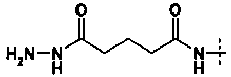

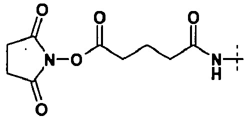

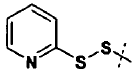

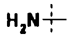

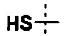

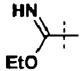

### Example 119. Conjugation of Macugen aptamer to aldehyde functionalized polymers

Macugen is an anti-angiogenic medicine for the treatment of neovascular (wet) age-related macular degeneration (AMD). It is a covalent conjugate of an oligonucleotide of twenty-eight nucleotides in length (aptamer) that terminates in a pentylamino linker, to which two 20 kDa monomethoxy polyethylene glycol (PEG) units are covalently attached via the two amino groups on a lysine residue. In the current embodiment, the Macugen aptamer with free amino group was used for conjugation to aldehyde functionalized polymers using the protocol outlined in Example 105 with the following differences. Conjugation to an aptamer with the polymers of this invention creates conjugates with high stability, low viscosity, and beneficial in vivo properties such as long residence time as well as being a base for exploring microRNA and RNAi delivery.

20mg/ml aptamer stock solution was prepared in Hepes buffer at pH 7, and then mixed with sodium cyanoborohydride reducing agent to result in a final concentration of 33mM. This solution was then used to dissolve a the following series of aldehyde functionalized polymers (also used in Example 105):

| | No. of Arms | MW (kDa) |
|---|---|---|
| 1 | 3 arm | 160 |
| 2 | 3 arm | 274 |
| 3 | 3 arm | 460 |
| 4 | 6 arm | 250 |
| 5 | 2 arm | 450 |

The final molar excess ratio of polymer to aptamer was 2-2.5 fold and the final aptamer concentration was 4.4-8.9 mg/ml. The conjugation mixture was incubated in a 22-23°C water bath overnight, samples were analyzed using a Shodex DEAE-825 anion exchange column connected to a Waters 2695 solvent delivery system equipped with a 2669 PDA for wavelength monitoring of the elution profile. To analyze the conjugation reaction, 2µl of the reaction mixture was diluted 10× with 20mM Tris pH 7.5 (buffer A), then applied to the column and chased at a flow rate of 1 ml/min followed by a 5 min isocratic wash in buffer A followed by a linear gradient of 80% buffer (buffer A containing 0.5M NaCl) over a course of 9 min. The salt gradient was maintained at 80% for 2 minutes before ramping down to 100% buffer A for column regeneration. Three major peaks were detected by OD220nm: the first peak was eluted at 2.2 min during the initial isocratic wash, this fraction equivalent to the unconjugated free polymer (the polymer being charge neutral remains unbound to the column); the second peak was a weakly-bound conjugate fraction at 5.4 min that eluted early in the salt gradient; and the last peak eluted later in the gradient at 13.6 min and corresponded to the unconjugated free aptamer. Both the conjugate peak and free aptamer peak show OD254nm absorbance, indicating the presence of oligonucleotide. The 5.4 min peak was detected in all the polymer containing reactions at both 254nm and 220nm trace but not in the control reaction where no polymer was added, which further supports that this is indeed the conjugate peak.

### Example 120. Preparation of 2,5,8,11,14-Pentaoxa-15,16-dihydroxyheptadecenyl 9-arm amide-based initiator

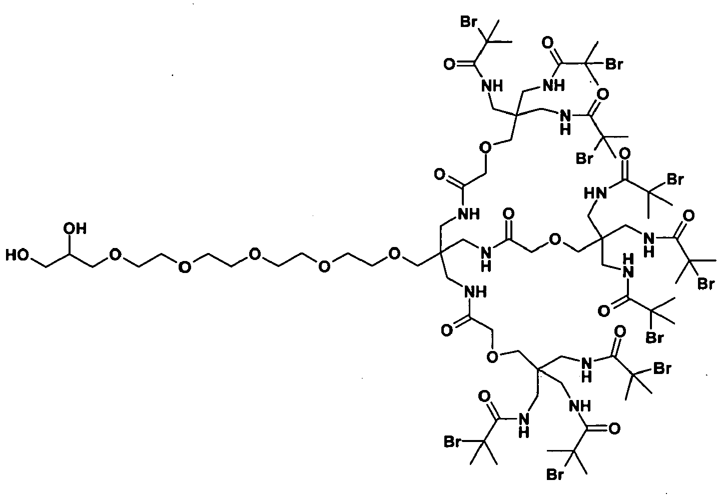

A solution of the product from the previous step in 15 ml water, 15 ml t-butanol, 3 eq of potassium ferricyanide, 3 eq of potassium carbonate, 1 eq of methanesulfonamide, 10 mg of quinuclidine, and 7 mg of potassium osmate dihydrate was stirred overnight at room temperature. The reaction mixture was partitioned between 100 ml each of water and dichloromethane. The aqueous layer was extracted twice more with 25 ml dichloromethane, and the organic layers were combined, dried over anhydrous magnesium sulfate, filtered, and concentrated. The residue was subjected to silica gel flash chromatography using methanol in dichloromethane to give the desired product.

Although the foregoing invention has been described in some detail by way of illustration and example for purposes of clarity of understanding, one of skill in the art will appreciate that certain changes and modifications can be practiced within the scope of the appended claims. In addition, each reference provided herein is incorporated by reference in its entirety to the same extent as if each reference was individually incorporated by reference.

Embodiments of the invention are described in the following numbered clauses:
1. A polymer comprising
   at least two polymer arms each comprising a plurality of monomers each independently selected from the group consisting of acrylate, methacrylate, acrylamide, methacrylamide, styrene, vinyl-pyridine, vinyl-pyrrolidone and vinyl-ester, wherein each monomer comprises a hydrophilic group;
   an initiator fragment linked to a proximal end of the polymer arm, wherein the initator moiety is suitable for radical polymerization; and
   an end group linked to a distal end of the polymer arm,
   wherein at least one of the initiator fragment and the end group comprises a functional agent or a linking group.
2. The polymer of clause 1, wherein each hydrophilic group comprises a zwitterionic group.
3. The polymer of clause2, wherein each zwitterionic group comprises phosphorylcholine.
4. The polymer of clause3, wherein the monomer comprises 2-(acryloyloxyethyl)-2' -(trimethylammoniumethyl) phosphate.
5. The polymer of clause 3, wherein the monomer comprises 2-(methacryloyloxyethyl)-2'-(trimethylammoniumethyl) phosphate (HEMA-PC).
6. The polymer of clause 1, wherein the initiator fragment is linked to the proximal end of from 2 to about 100 polymer aims.
7. The polymer of clause6, wherein the polymer has a polydispersity index of less than about 2.0.
8. The polymer of clause 6, wherein the initiator fragment is linked to the proximal end of 2, 3, 4, 5, 6, 8, 9 or 12 polymer arms.
9. A conjugate comprising:
   at least one polymer comprising:
      at least two polymer arms each comprising a plurality of monomers each independently selected from the group consisting of acrylate, methacrylate, acrylamide, methacrylamide, styrene, vinyl-pyridine, vinyl-pyrrolidone and vinyl-ester, wherein each monomer comprises a hydrophilic group,
      an initiator fragment linked to a proximal end of the polymer arm, wherein the initator moiety is suitable for radical polymerization, and
      an end group linked to a distal end of the polymer arm; and
   at least one functional agent comprising a bioactive agent or a diagnostic agent, linked to the initiator fragment or the end group.
10. The conjugate of clause9, wherein the bioactive agent is selected from the group consisting of a drug, an antibody, an antibody fragment, a single domain antibody, an avimer, an adnectin, diabodies, a vitamin, a cofactor, a polysaccharide, a carbohydrate, a steroid, a lipid, a fat, a protein, a peptide, a polypeptide, a nucleotide, an oligonucleotide, a polynucleotide, and a nucleic acid.
11. The conjugate of clause 9, wherein the diagnostic agent is selected from the group consisting of a radiolabel, a contrast agent, a fluorophore and a dye.
12. The conjugate of clause9, wherein at least two polymers are linked to the functional agent.
13. The conjugate of clause14, wherein at least two polymers are linked to the functional agent via proximal reactive groups on the functional agent to create a pseudo-branched structure.
15. The conjugate ofclause9, wherein the conjugate comprises at least two functional agents attached to the polymer.
16. A polymer of the formula: 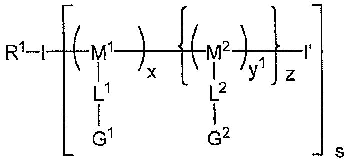 wherein
   R¹ is selected from the group consisting of H, L³-A¹, LG¹ and L³-LG¹;
   each M¹ and M² is independently selected from the group consisting of acrylate, methacrylate, acrylamide, methacrylamide, styrene, vinyl-pyridine, vinyl-pyrrolidone and vinyl-ester;
   each of G¹ and G² is each independently a hydrophilic group;
   each I and I' is independently an initiator fragment, such that the combination of I-I' is an initiator, I¹, for the polymerization of the polymer of Formula I via radical polymerization;
   alternatively, each I' is independently selected from the group consisting of H, halogen and C₁₋₆ alkyl;
   each of L¹, L² and L³ is independently a bond or a linker;
   each A¹ is a functional agent;
   each LG¹ is a linking group;
   subscripts x and y¹ are each independently an integer of from 1 to 1000;
   each subscript z is independently an integer of from 0 to 10; and
   subscript s is an integer of from 2 to 100.
17. The polymer of clause 16, wherein the polymer has the formula: 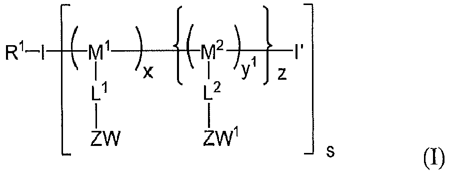 wherein
   R¹ is selected from the group consisting of H, L³-A¹, LG¹ and L³-LG¹;
   each M¹ and M² is independently selected from the group consisting of acrylate, methacrylate, acrylamide, methacrylamide, styrene, vinyl-pyridine, vinyl-pyrrolidone and vinyl-ester;
   each of ZW and ZW¹ is independently a zwitterionic moiety;
   each I and I' is independently an initiator fragment, such that the combination of I-I' is an initiator, I¹, for the polymerization of the polymer of Formula I via radical polymerization;
   alternatively, each I' is independently selected from the group consisting of H, halogen and C₁₋₆ alkyl;
   each of L¹, L² and L³ is a linker;
   each A¹ is a functional agent;
   each LG¹ is a linking group;
   subscripts x and y¹ are each independently an integer of from 1 to 1000;
   each subscript z is independently an integer of from 0 to 10; and
   subscript s is an integer of from 2 to 100.
18. The polymer of clause 16, wherein each hydrophilic group comprises a zwitterionic group.
19. The polymer of clause 16, wherein each hydrophilic group comprises phosphorylcholine.
20. The polymer of clause 16, wherein subscript s is 2, 3, 4, 5, 6, 8, 9 or 12.
21. The polymer of clause 16, wherein the polymer has the formula: 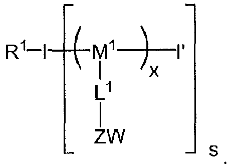
22. The polymer of clause 16, wherein the polymer has the formula: 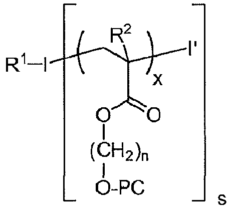 wherein
   R² is selected from the group consisting of H and C₁₋₆ alkyl; and
   PC is phosphorylcholine.
23. The polymer of clause 16, wherein the initiator I¹ has the formula: 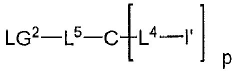 wherein
   each I' is independently selected from the group consisting of halogen, -SCN, and -NCS;
   L⁴ and L⁵ are each independently a bond or a linker, such that one of L⁴ and L⁵ is a linker;
   C is a bond or a core group;
   LG² is a linking group; and
   subscript p is from 1 to 20, wherein when subscript p is 1, C is a bond, and when subscript p is from 2 to 20, C is a core group.
24. The polymer ofclause 23, wherein each of the initiators I¹ is of the formula: 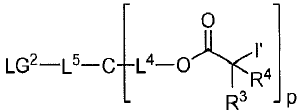 wherein each R³ and R⁴ is independently selected from the group consisting of H, CN and C₁₋₆ alkyl.
25. The polymer ofclause23, wherein each of the initiators I¹ is independently selected from the group consisting of: 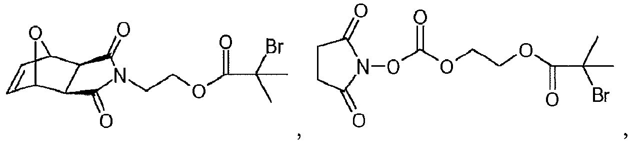 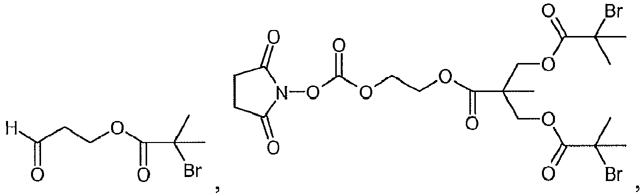 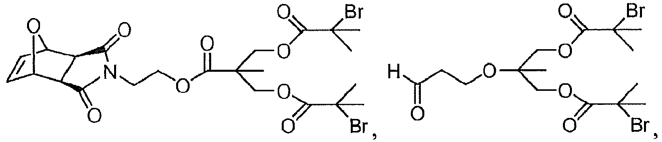 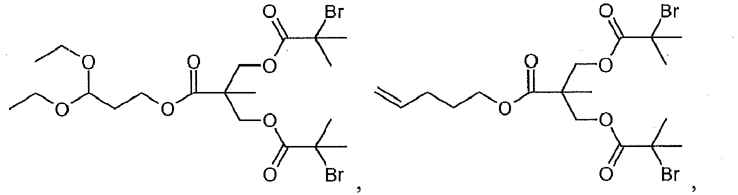 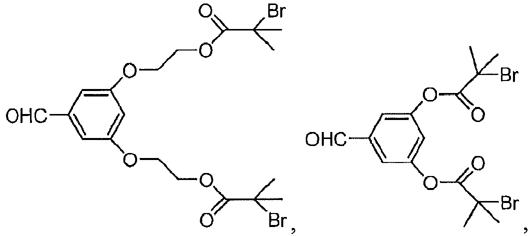 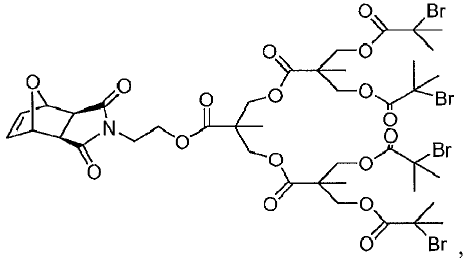 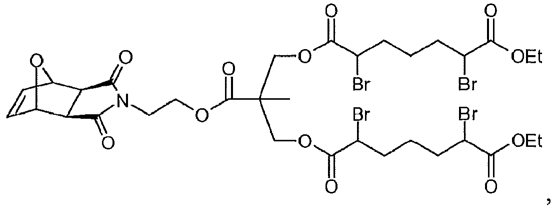 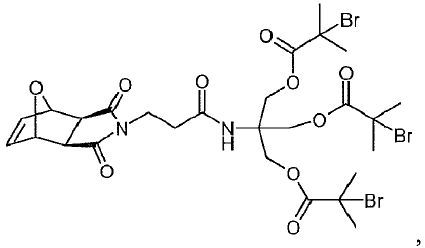 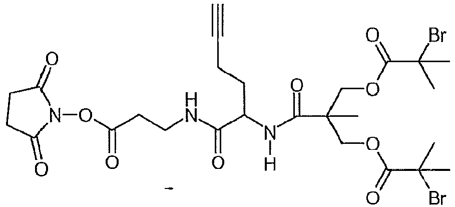 and 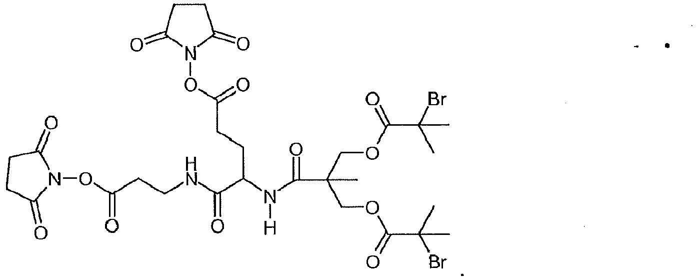
26. The polymer of clause 16, having the formula selected from the group consisting of: 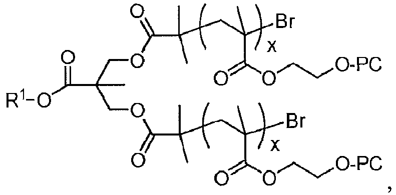 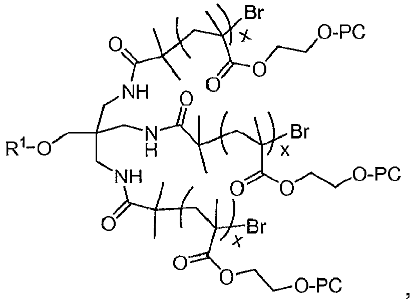 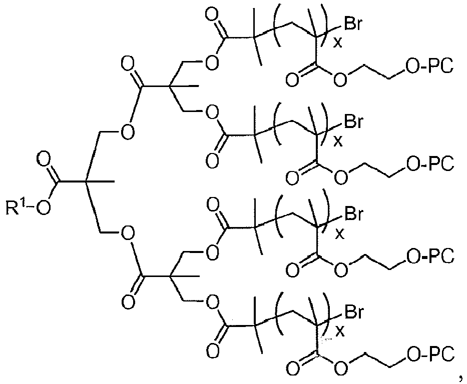 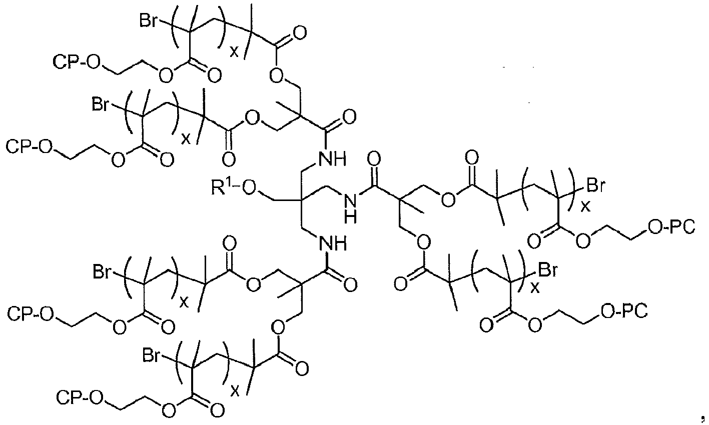 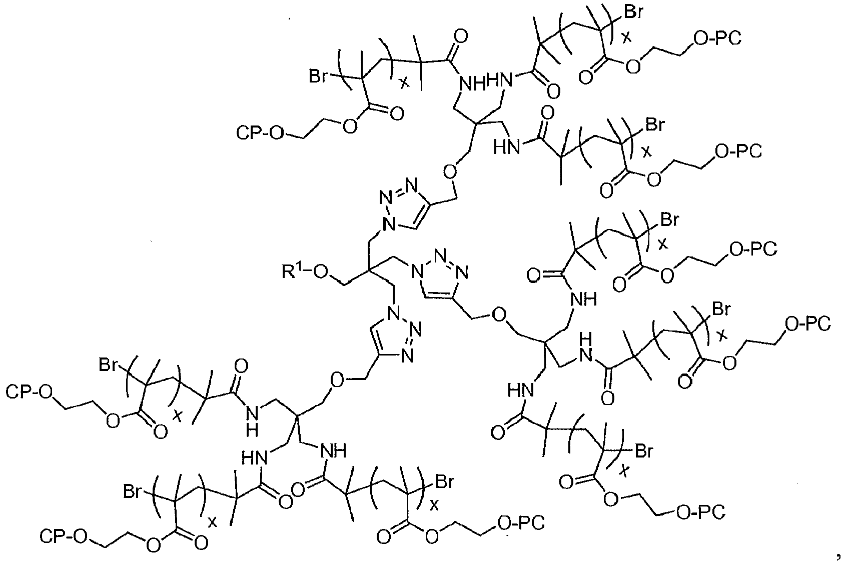 and 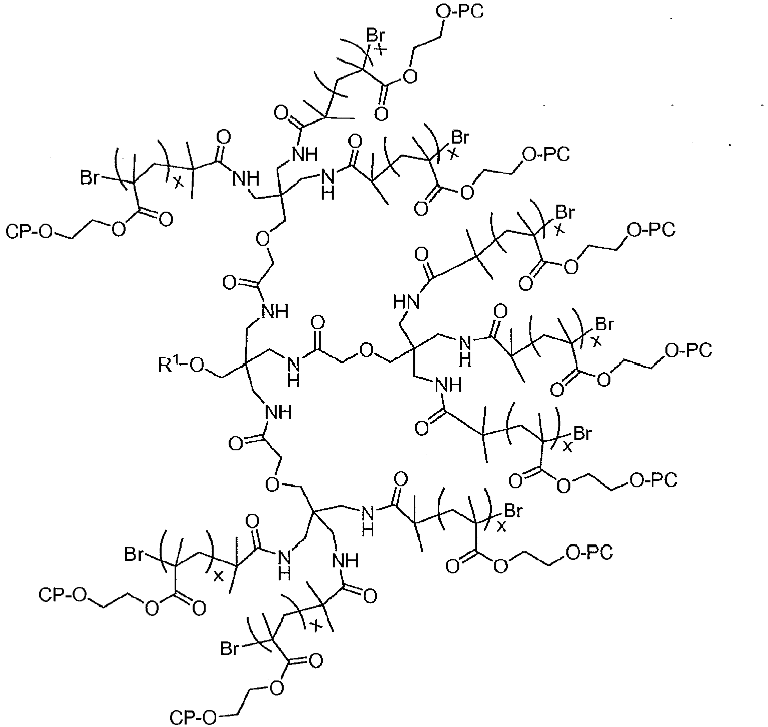 wherein PC is phosphorylcholine.
27. The polymer of clause26, wherein
   R¹ is selected from the group consisting of L³-A¹, LG¹ and L³-LG¹;
   A¹ is selected from the group consisting of a drug, an antibody, an antibody fragment, a single domain antibody, an avimer, an adnectin, diabodies, a vitamin, a cofactor, a polysaccharide, a carbohydrate, a steroid, a lipid, a fat, a protein, a peptide, a polypeptide, a nucleotide, an oligonucleotide, a polynucleotide, a nucleic acid. a radiolabel, a contrast agent, a fluorophore and a dye;
   L³ is -(CH₂CH₂O)₁₋₁₀-; and
   LG¹ is selected from the group consisting of maleimide, acetal, vinyl, allyl, aldehyde, -C(O)O-C₁₋₆ alkyl, hydroxy, diol, ketal, azide, alkyne, carboxylic acid, and succinimide.
28. The polymer of clause27, wherein each LG¹ is independently selected from the group consisting of:
   hydroxy, carboxy, vinyl, vinyloxy, allyl, allyloxy, aldehyde, azide, ethyne, propyne, propargyl, -C(O)O-C₁₋₆ alkyl, 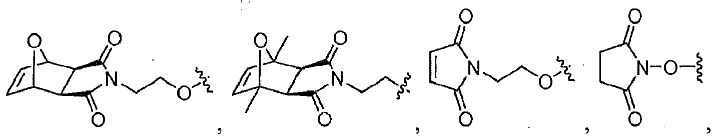 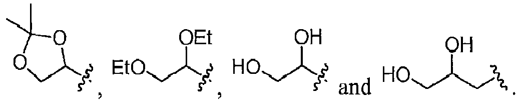
29. The polymer of clause 16, having the formula selected from the group consisting of: 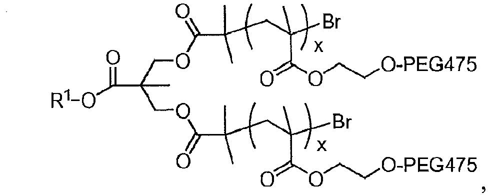 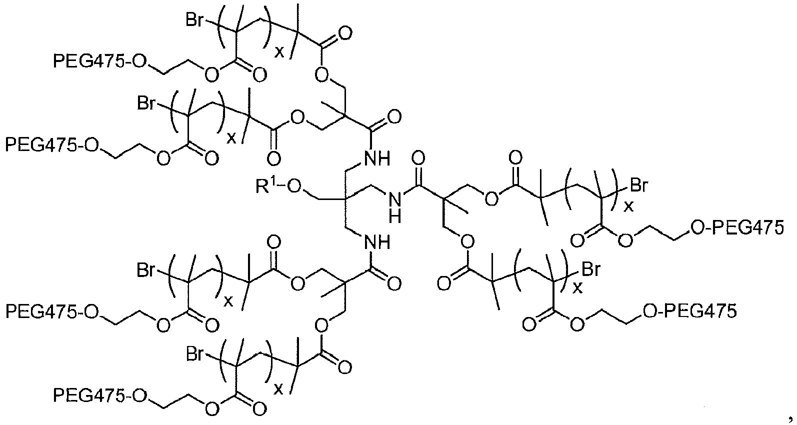 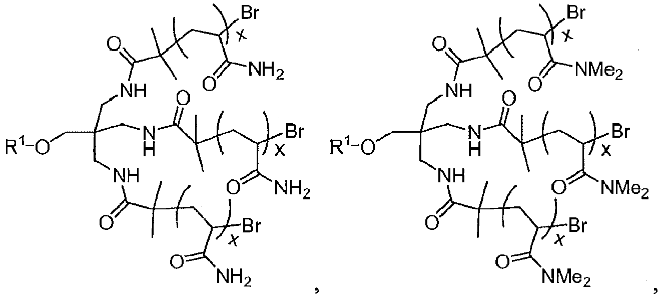 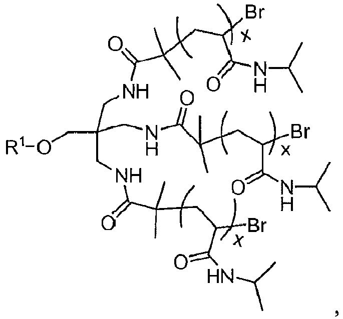 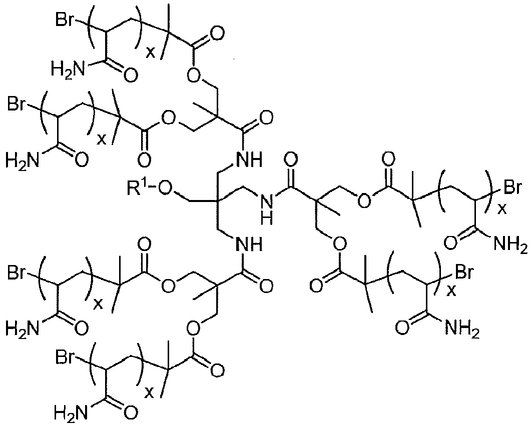 and 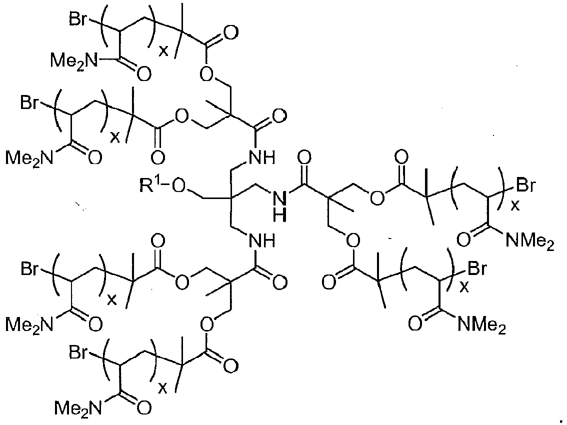
30. A polymer comprising
   a polymer arm independently comprising a plurality of monomers each independently selected from the group consisting of acrylate, methacrylate, acrylamide, methacrylamide, styrene, vinyl-pyridine, vinyl-pyrrolidone and vinyl-ester, wherein each monomer comprises a hydrophilic group;
   an initiator fragment linked to a proximal end of the polymer arm, wherein the initator moiety is suitable for radical polymerization; and
   an end group linked to a distal end of the polymer arm,
   wherein at least one of the initiator fragment and the end group comprises a functional agent or a linking group,
   and wherein the polymer has a peak average molecular weight of from about 50kD to about 1,500kD, as measured by light scattering.

## Claims

1. A polymer comprising
at least two polymer arms each comprising a plurality of monomers each independently selected from the group consisting of methacrylate, acrylate, acrylamide, methacrylamide, styrene, vinyl pyridine, vinyl pyrrolidone and vinyl ester, wherein each monomer comprises a hydrophilic group;
an initiator fragment linked to a proximal end of each polymer arm, wherein the initiator moiety is suitable for radical polymerization; and
an end group linked to a distal end of each polymer arm,
wherein at least one of the initiator fragment and the end group comprises a linking group, and the polymer has a peak molecular weight of greater than 50 kDa as measured by multi-angle light scattering and a polydispersity index of less than 1.5 as determined by gel permeation chromatography.

2. The polymer of claim 1, wherein each hydrophilic group comprises a zwitterionic group.

3. The polymer of claim 2, wherein each zwitterionic group comprises phosphorylcholine.

4. The polymer of claim 1, wherein the polymer has a polydispersity of less than 1.2.

5. The polymer of claim 1, wherein the polymer has 2, 3, 4, 5, 6, 8, 9, or 12 polymer arms.

6. A polymer of the formula: 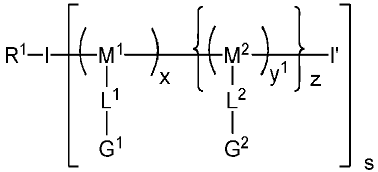 wherein
R¹ is selected from the group consisting of H, LG¹ and L³-LG¹;
each M¹ and M² is independently selected from the group consisting of methacrylate, acrylate, acrylamide, methacrylamide, styrene, vinyl pyridine, vinyl pyrrolidone and vinyl ester;
each of G¹ and G² is each independently a hydrophilic group;
each I and I' is independently an initiator fragment, such that the combination of I-I' is an initiator, I¹, for the polymerization of the M¹ and M² via radical polymerization;
alternatively, each I' is independently selected from the group consisting of H, halogen and C₁₋₆ alkyl;
each of L¹, L² and L³ is independently a bond or a linker;
each LG¹ is a linking group;
subscripts x and y¹ are each independently an integer of from 1 to 1000;
each subscript z is independently an integer of from 0 to 10; and
subscript s is an integer of from 5 to 100,
wherein the polymer has a peak molecular weight of greater than 50 kDa as measured by multi-angle light scattering and a polydispersity index of less than 1.5 as determined by gel permeation chromatography.

7. The polymer of claim 6, wherein each hydrophilic group comprises a phosphorylcholine

8. The polymer of claim 6 wherein the polymer has the formula: 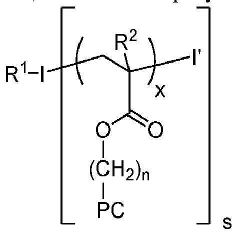 wherein
R² is selected from the group consisting of H and C₁₋₆ alkyl;
subscript n is an integer of from 1 to 12; and
PC is phosphorylcholine.

9. The polymer of claim 6, wherein the initiator I¹ has the formula: 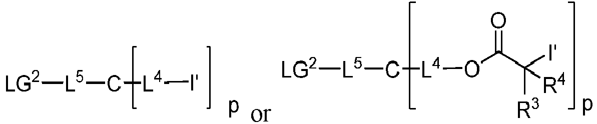 wherein
each I' is independently selected from the group consisting of halogen, -SCN, and -NCS;
L⁴ and L⁵ are each independently a bond or a linker, such that one of L⁴ and L⁵ is a linker;
C is a core group;
LG² is a linking group; and
subscript p is from 2 to 20; and
each R³ and R⁴ is independently selected from the group consisting of H, CN, and C₁₋₆ alkyl.

10. The polymer of claim 6, having the formula selected from the group consisting of: 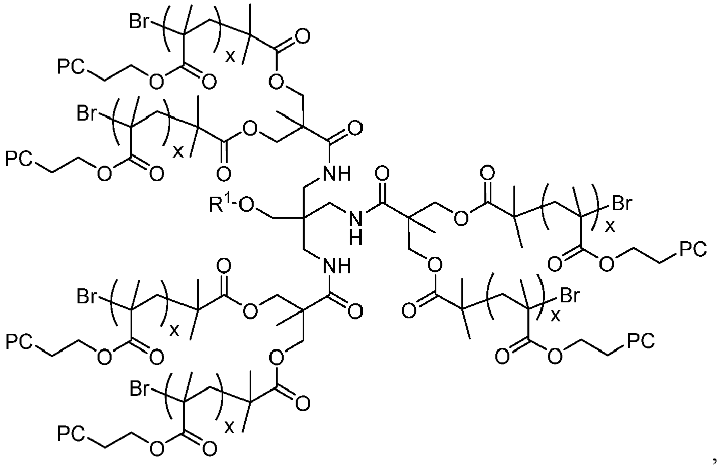 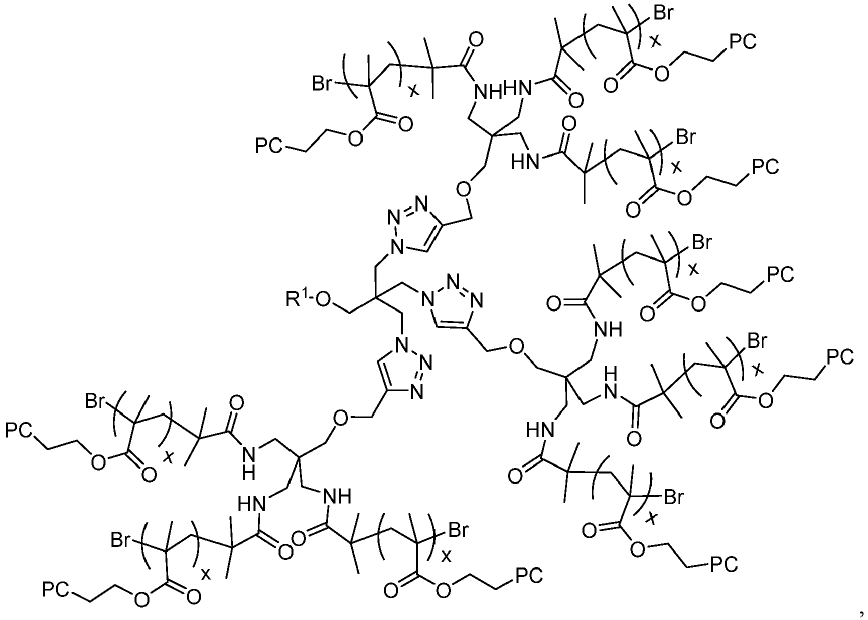 and 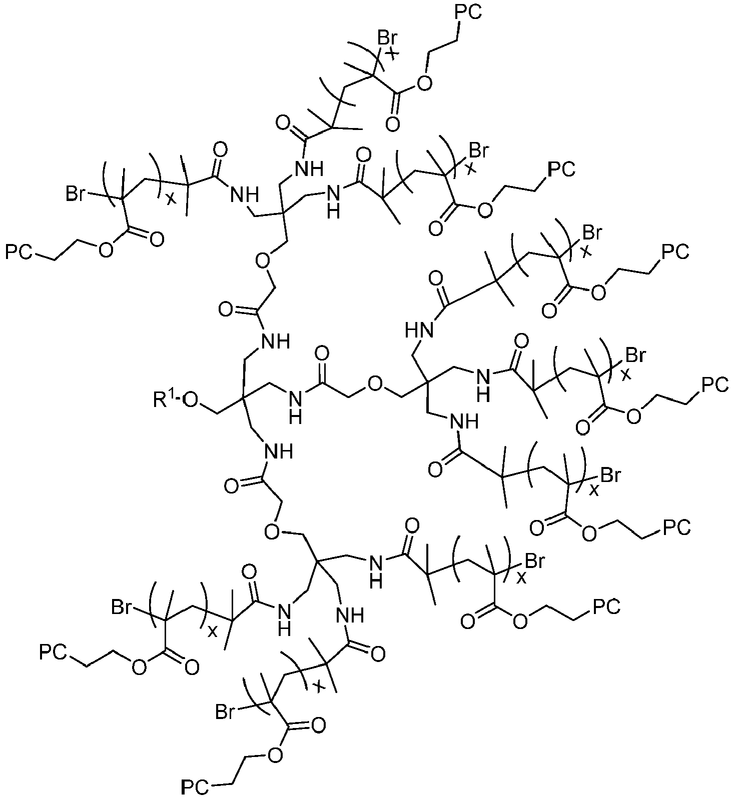 wherein PC is phosphorylcholine.

11. The polymer of claim 9, wherein:
R¹ is selected from the group consisting of LG¹ and L³-LG¹;
L³ is -(CH₂CH₂O)₁₋₁₀-; and
LG¹ is selected from the group consisting of maleimide, acetal, vinyl, allyl, aldehyde, -C(O)O-C₁₋₆ alkyl, hydroxy, diol, ketal, azide, alkyne, carboxylic acid, and succinimide, optionally wherein each LG¹ is independently selected from the group consisting of:
hydroxy, carboxy, vinyl, vinyloxy, allyl, allyloxy, aldehyde, azide, ethyne, propyne, propargyl, -C(O)O-C₁₋₆ alkyl,

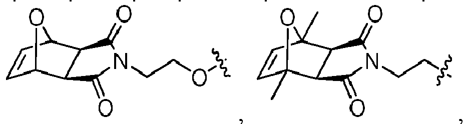

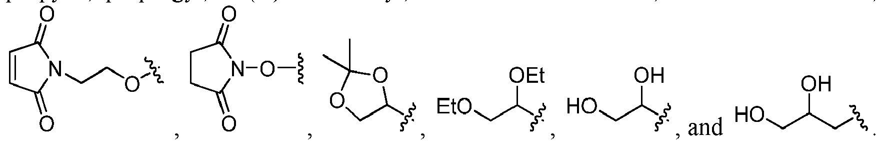

12. A process for preparing a high MW polymer in solution comprising:
providing a phosphorylcholine containing monomer and a solvent, wherein the monomer is selected from the group consisting of methacrylate, acrylate, acrylamide, and methacrylamide;
providing a mixture comprising an branched halogenated initiator, a catalyst, and a ligand; and
contacting the monomer and the solvent with the mixture under conditions such that an atom transfer radical polymerization occurs and proceeds as a homogenous solution with no precipitation, thereby forming a polymer having at least 5 polymer arms, a peak molecular weight of greater than 50 kDa as measured by multi-angle light scattering, and a polydispersity of less than 1.5 as determined by gel permeation chromatography.

13. The process according to claim 12 wherein the catalyst is copper bromide.

14. The process according to claim 12 wherein the ligand is 2,2'-bipyridyl.

15. The process according to any one of claims 12 to 14 wherein the solvent is an alcohol.

16. The process according to any one of claims 12 to 14 wherein the polymer has a polydispersity of less than 1.2.
